# EUROPEAN PATENT APPLICATION

(11) **EP 4 378 946 A1**
(43) Date of publication of application: **05.06.2024**
(21) Application number: 22849867.1
(22) Date of filing: 26.07.2022
(51) Int. Cl.: C07J 31/00, C07J 43/00, A61K 31/575, A61K 31/58, A61P 29/00

(54) **NOVEL COMPOUND THAT INHIBITS TNF-A GENERATION AND INFLAMMASOME ACTIVITY AND PREPARATION METHOD THEREFOR**

(30) Priority: 26.07.2021 KR 20210098148
(71) Applicant: Shaperon Inc., Seoul 06373 (KR); Seoul National University R & DB Foundation, Seoul 08826 (KR)
(72) Inventor: SEONG, Seung Yong, Seoul 05507 (KR)
(74) Representative: Vossius & Partner Patentanwälte Rechtsanwälte mbB
(86) International application number: PCT/KR2022/010998
(87) International publication number: WO 2023/008891

(57) **Abstract**

The present invention relates to a novel compound and a preparation method therefor. The compound according to the present invention exhibits inhibitory activity against the generation of TNF-α that is an inflammatory cytokine produced by inflammation initiation reaction, and against the generation of IL-1β by inflammasome activation, and thus, can be developed as a therapeutic agent for related inflammatory diseases.

## Description

### Technical Field

The present invention relates to a novel compound for inhibiting TNF-α production and activation of inflammatory complexes that can be used for the treatment of various inflammatory diseases and a producing method therefor, and relates to a novel compound prepared by reacting bile acid or a derivative thereof with a taurine derivative.

### Background of the invention

The causes of inflammation are known to be PAMPs (Pathogen-associated molecular patterns) derived from pathogens and DAMPs (Damage-associated molecular patterns) released from tissues owing to the tissue damages. Immune cells activated due to these molecular patterns and other inflammatory mediators produced by cells such as blood vessels respond to a biological protection, but if this response is not properly balanced, it causes inflammatory diseases.

Intractable inflammatory diseases have a high recurrence rate and require a long-term administration of their treatment measures. However, all the treatment measures developed to date does not meet the market demand, and therefore, the development of new treatment measures is necessary. So far, steroids, NSAIDs (non-steroidal anti-inflammatory drugs), antibody treatments, and JAK inhibitors are being prescribed for the treatment of inflammatory diseases. However, steroid drugs are only prescribed for a short period of time due to its systemic side effects, and NSAIDs are not showing sufficient efficacy in clinical trials. In addition, antibody treatments have relatively fast responses and can be administered for a long period of time, but this measures has the disadvantage of having side effects at the injection site, a gradual decrease in efficacy due to the production of neutralizing antibodies in the body, and high-cost burden for the patient. JAK inhibitors have excellent anti-inflammatory effects, but they have a risk of increasing severe infections by excessively suppressing T lymphocytes, and cardiovascular side effects and carcinogenic risks have been confirmed.

To overcome the unmet market demand, agents regulating inflammatory complexes are recently drawing the attentions for the treatment measures. Inflammatory complexes, which are the core of the body's immune response, play an important function in innate defense immunity as part of the immune response to microbial infections and cell damages. Inflammatory complexes are cytosolic multiprotein oligomers of the innate immune system. Among many inflammatory complexes, abnormal activation of the NLRP3 inflammasome is associated with the onset and worsening of various inflammatory diseases such as ulcerative colitis, gout, multiple sclerosis, arthritis, sepsis, and inflammatory neurological disease. The NLRP3 inflammasome is also activated by PAMPs from pathogens such as viruses and bacteria, and DAMP stimuli such as calcium influx, mitochondrial reactive oxygen species, and extracellular APT. Activation of the inflammatory complex not only activates gasdermin-D through caspase-1, but also promotes protein maturation and secretion of pro-IL-1β and pro-IL-18 into IL-1β and IL-18. Disorders in the regulation of inflammatory complex activation cause various diseases such as cancer, metabolic diseases, degenerative diseases, and inflammatory diseases.

Attempts to develop new drugs targeting the NLRP3 inflammatory complex, a major regulator of inflammatory response, used to be taken several times in the past. As it became to be known that the inflammatory complex is activated through canonical and non-canonical pathways consisting of pro-caspase, gasdermin D, ASC, NLR protein, AIM2, IF116, and pyrin, and various diseases occur when dysregulation of the inflammatory complex occurs, the development of drugs targeting proteins involved in inflammatory complex-related signaling has become active since 2010. P2X7 receptor antagonist, NLRP3 inhibitor, and caspase-1 inhibitor are the representatives of developed inflammatory complex regulators.

Among these, one of the important receptors for activation of the NLRP3 inflammatory complex is P2X7R. The P2X7 receptor is an ion channel receptor composed of a complex belonging to the P2Xn purine receptor group. When P2X7 is stimulated, it promotes the extracellular release of potassium ions and the rapid movement of calcium and sodium ions into the cells. High concentrations of ATP released from necrotic cells act as pro-inflammatory DAMPs, binding to the P2X7 receptor and activating the NLRP3 inflammatory complex. Ultimately, it causes the release of potassium ions through the P2X7 receptor and the imbalance of intracellular ions in cells, which activaties the NLRP3 inflammatory complex. As such, the P2X7 receptor, which plays an important role in activating inflammatory complexes, is widely expressed on various immune cells, epidermal cells, or nerve cells, and since the P2X7 receptor signaling activates both canonical and non-canonical pathways of NLRP3 activation, the interaction of ATP-P2X7 is very important in NLRP3 inflammasome activation.

P2X7 antagonists were developed by global pharmaceutical companies such as Pfizer, AstraZeneca, and Janssen for indications such as rheumatoid arthritis, Crohn's disease, and depression, but most of them were discontinued due to the lack of efficacy in clinical trials. Afterwards, venture companies continued the development, but as of 2020, there are only less than 10 therapeutic development pipelines targeting P2X7, Janssen 18F-JNJ-64413739 being developed for depression treatment is in the completion stage of the 1st clinical trials, and other pipelines remain in the preclinical or development stage. Caspase-1 inhibitor has no global development pipeline as it was discontinued in 2020 due to the lack of efficacy targets and safety issues in clinical trials. NLRP3 inhibitor is being developed by venture companies such as OLATEC, IMFLAZOME, IFM THERAPETICS, NODTHERA, and AC Immune for indications such as osteoarthritis, systolic heart failure, Parkinson's disease, inflammatory bowel disease, and Alzheimer's disease.

As mentioned above, most of these inflammatory complex modulator new drugs have not achieved success in clinical trials, and the reason for this is known to be that the new drug targets alone do not effectively and simultaneously suppress the various pro-inflammatory pathways that activate the NLRP3 inflammatory complex. there is. In other words, the currently known new drugs that regulate the inflammatory complex, such as P2X7 receptor antagonist, NLRP3 inhibitor, and caspase-1 inhibitor, selectively inhibit inflammatory cytokines triggered by inflammatory activity such as IL-1β and IL-18, but are triggered at the initiation stage of inflammation. There is a limitation in suppressing the inflammatory cytokine TNF-α. This is a basic limitation of new drugs that selectively target only NLRP3 or P2X7, and it can be said that the pharmacological mechanism that selectively blocks only the inflammatory activation stage cannot suppress the inflammatory response through various inflammation bypass pathways. In other words, there are many clinical reports of cases where patient drug responsiveness and anti-inflammatory effects for current inflammatory complex regulator new drugs are not met due to alternative activation of the compensatory bypass pathway.

In addition, it has been revealed that due to the genetic redundancy and polymorphisms inherent in NLRP3 and P2X7, current inflammatory complex modulator new drugs do not produce the targeted drug response rates in actual clinical trials. Since it exists in the similar NLRP inflammasome group, which has the same protein domain as the NLPR3 inflammasome, this is the reason that new drugs for controlling the inflammasome do not show sufficient effectiveness.

Hundreds to thousands of SNPs (single nucleotide polymorphisms) have been identified in the P2X7 protein and NLRP3 protein, and individual genetic diversity is very high. As a result, there are often large individual differences in drug response rate and effectiveness, and new drugs targeting these proteins are often individualized. It is unlikely that it will become a treatment with broad efficacy without deviation. As described above, the new drugs targeting inflammatory complexes developed to date, such as P2X7 receptor antagonist, NLRP3 inhibitor, and caspase-1 inhibitor, can be said to have many limitations in achieving the desired clinical efficacy.

In the present invention, in order to overcome the shortcomings of existing inflammatory complex-targeting new drugs, we developed a GPCR19-targeted therapeutic agent that regulates the P2X7R-inflammatory complex at the upstream level. GPCR19 is mainly expressed at a high level in innate immune cells, and because it selectively induces an immune cell-mediated anti-inflammatory effect, it has the advantage of having low non-specific side effects affecting normal cells. This is considering the fact that the target receptors of steroid drugs, which are existing anti-inflammatory drugs, are distributed throughout the body, so that in addition to the pharmacological action on the target, a wide range of systemic reactions are induced, and as a result, systemic metabolic side effects, increased risk of infection, and risk of highly carcinogenic side effects have been reported. In this case, it is believed that GPCR19-targeted new drugs will have great advantages in terms of safety.

To date, drugs targeting GPCR19 include TUDCA, INT-747 (Obeticholic acid, OCA), and INT-777 (S-EMCA). However, how efficiently they suppress inflammatory complexes has not been reported in detail. All of these are bile acid derivatives and have been reported as agonists of GPCR19, but so far, they have mainly been studied on the mechanism of suppressing inflammation by increasing cAMP production and inhibiting NF-kB. Clinical trials are currently underway for TUDCA for diseases such as type 2 diabetes, ulcerative colitis, and Lou Gehrig's disease, and for INT-747 for diseases such as biliary cirrhosis, non-alcoholic steatohepatitis, and liver cirrhosis. In the case of GPCR19-targeted treatments developed to date, there are no new drugs yet that have proven efficacy in clinical trials. In the case of the most recently developed new drugs, there are cases where phase 3 clinical trials targeting inflammatory diseases failed because the anti-inflammatory effect was not high.

The reason for the failure of these GPCR19-targeted treatments is that they overlook the fact that the complex of P2X7R and GPCR19 regulates the inflammatory complex. In other words, without an accurate review of the anti-inflammatory mechanism of GPCR19, new drugs were screened using cell lines that artificially highly expressed GPCR19. This was done through an artificial system that does not correctly reflect the initiation and activation stage of inflammation through GPCR19-P2X7R in the body. This is because it is a screened low-molecular-weight compound. Therefore, the present invention, which improves the drug screening system, overcomes existing failure cases. In the present invention, based on the understanding of the signaling system via the GPCR19-P2X7R-NLRP3 axis, we invented a small molecule compound with a wide range of anti-inflammatory effects that can block both the initiation and activation stages of inflammation and can simultaneously inhibit the cAMP pathway and NRLP pathway.

Bile acid is known as a GPCR19 modulator. Bile acids are amphipathic molecules that have both hydrophobic and hydrophilic regions. Looking at the production process of bile acids, cholesterol is used as a precursor and primary bile acids are produced by cytochrome P450 enzyme in the liver in the form of cholic acid and chenodeoxyholic acid, and later, by combining with taurine and glycine by hepatocytes, it becomes more hydrophilic and moves to the intestines, where it is changed into secondary bile acid by intestinal microorganisms. Secondary bile acids are reabsorbed into the liver through liver-intestinal circulation and move to various tissues through blood vessels to perform various physiological functions. Specifically, it plays a role in digesting fat through its amphipathic properties, inhibiting the overgrowth of harmful intestinal microorganisms through acidity, and excreting cholesterol during the intestinal circulation process. In addition, it is known to transmit signals through receptors such as farnesoid X receptor (FXR), a cell nuclear receptor, and cell membrane GPCR19. Specifically, bile acids bound to the FXR plays a role in suppressing gluconeogenesis and lipogenesis in the liver, and it bound to GPCR19 plays a role in promoting insulin secretion and suppressing cytokine secretion. In particular, because of that the bile acid bound to GPCR19 suppresses the inflammatory response, researches have been conducted on the treatment of diseases such as skin, nerves, stomach, intestines, liver, joints, etc. related to inflammation and autoimmune diseases using bile acids as a treatment.

Looking at the chemical structure of bile acid, it is a steroid structure with four rings and has the following chemical formula. Types of bile acids vary greatly depending on the differences of hydroxyl group position, steric position, number of hydroxyl groups, and etc. The main bile acids in the human body include cholic acid, glycocholic acid, deoxycholic acid, Lithocholic acid, etc. In addition, bile acids can be modified by the reactivity of the hydroxy group or carboxyl group, and due to amphiphilicity, chirality, etc., they are being used not only in the pharmaceutical field but also in various fields such as solution processing materials, thin films, and surfactants.

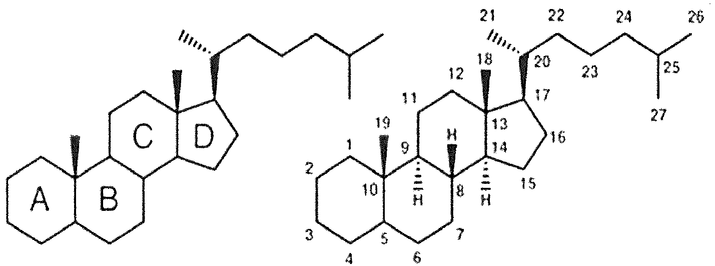

### <Structure of bile acid>

Taurine (or 2-aminoethanesulfonic acid) is an organic compound and a type of amino acid present in the cells and tissues of mammals, including humans. Taurine is rarely used in protein synthesis and mostly exists in the form of free amino acids, and is present in high concentrations in organs such as the brain, heart, liver, and kidneys, as well as in the skeleton and muscles.

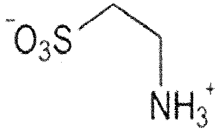

### <Structure of taurine>

Taurine has an inhibitory effect on the sympathetic nerves of the brain due to its structural characteristics of the β-amino acid of the sulfonic acid group. It is known that taurine is effective in stabilizing blood pressure and other vascular diseases, preventing strokes, and suppressing the production of low-density lipoprotein cholesterol. However, since taurine is difficult to manufacture its derivatives or compositions, taurine is generally used as a raw material. However, taurine can combine with cholic acid, a type of bile acid, to form taurocholic acid, which produces crystalline bile acid that is involved in emulsification of fat.

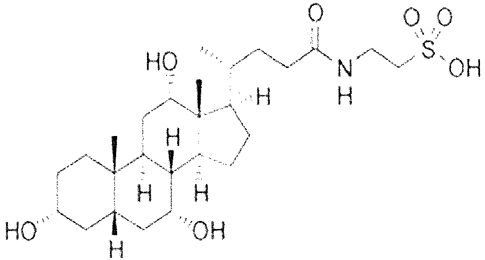

### <Structure of taurocholic acid>

In this regard, Korean Patent No. 10-1778687 discloses a deoxycholate-based amphipathic compound, and Korean Patent Publication No. 10-1994-0021065 discloses a taurine-bile acid derivative having therapeutic effects.

However, in the case of taurine, it is difficult to form a derivative of taurocholic acid, so there are almost no derivatives of taurocholic acid, and little is known about the therapeutic effect of using it. Accordingly, the present inventors made efforts to produce an inflammatory complex and TNF-α production inhibitor showing excellent effects. As a result, they disclosed that it is possible to produce the compounds of which a novel bile acid derivative and a taurine derivative are covalently bonded, and so a novel taurine-bile acid derivative compound is developed. The present invention is completed by synthesizing it and discovering its therapeutic use.

### Detailed Description of the Invention

### Technical Challenges

The present invention relates to a novel compound which is capable of simultaneously controlling the GPCR19-P2X7R-NLRP3 signaling system and the cAMP signaling system and a method for producing the same. Technical challenge of the present invention is to solve the problems of the difficult manufacturing process of the compounds containing conventional taurine derivatives, and a low yield rate and purity.

In addition, the aim of the present invention is to provide new compounds that can be developed as a therapeutic agent by disclosing that the novel compounds synthesized by the method according to the present invention inhibit the production of TNF-α and IL-1β caused by the activation of inflammatory complexes, which plays in an important role in the pathology of various inflammatory diseases, and by showing that the compounds have a useful therapeutic effect.

### Technical Solution

In order to achieve the above object, the present invention provides a compound represented by Formula 1 or a pharmaceutically acceptable salt thereof. 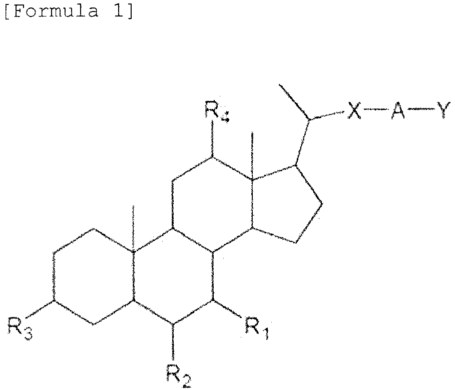 wherein,
R₁, R₂, R₃ and R₄ are each independently H, halo, C₁₋₅ alkyl, OH, or =O,
X is substituted or unsubstituted C₁₋₁₀ alkylene,
wherein when X is substituted, one or more hydrogen(s) is(are) substituted with linear or branched C₁₋₁₀ alkyl, halo, OH, SH, NH₂, CONH₂, or COOH,
A is C₁₋₁₀ alkylene, C₁₋₁₀ heteroalkylene, C₁₋₁₀ alkenylene, C₁₋₅ alkynylene, C₃₋₈ cycloalkylene, C₃₋₈ heterocycloalkylene, C₆₋₂₀ arylene, C₆₋₂₀ heteroarylene, -NR₅R₆C(O)-, -OR₆, -SR₆, -NR₅R₆, -C(O)R₆, -C(O)OR₆, -C(O)NR₅R₆, -CH(R₅)(R₆), -CH(R₅)(OR₆), -CH(OR₅)(OR₆), -CH(R₅)(SR₆), or -CH(SR₅)(SR₆);
R₅ is H, substituted or unsubstituted C₁₋₁₀ alkyl, substituted or unsubstituted C₁₋₁₀ heteroalkyl, substituted or unsubstituted C₃₋₂₀ cycloalkyl, substituted or unsubstituted C₃₋₂₀ heterocycloalkyl, substituted or unsubstituted C₆₋₂₀ aryl, substituted or unsubstituted C₆₋₂₀ heteroaryl, substituted or unsubstituted C₁₋₁₀ alkyl-C₆₋₂₀ cycloalkyl, substituted or unsubstituted C₁₋₁₀ alkyl-C₆₋₂₀ heterocycloalkyl, substituted or unsubstituted C₁₋₁₀ alkyl-C₆₋₂₀ aryl or substituted or unsubstituted C₁₋₁₀ alkyl-C₆₋₂₀ heteroaryl,
wherein, when R₅ is substituted, one or more hydrogen(s) is(are) linear
one or more hydrogen(s) is(are) substituted with linear or branched C₁₋₅ alkyl, =O, ORₐ, SRₐ, N(Rₐ)₂, or CON(Rₐ)₂, Rₐ is H or C1-5 alkyl,
R₆ is bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, substituted or unsubstituted C₆₋₂₀ arylene, or substituted or unsubstituted C₆₋₂₀ heteroarylene,
wherein, if R₆ is substitutde, one or more hydrogen(s) is (are) substituted with =O, linear or branched C₁₋₁₀ alkyl, C₀₋₁₀alkyl-C₆₋₂₀aryl, C₀₋₁₀alkyl-OH, C₀₋₁₀alkyl-NH₂, C₀₋₁₀alkyl-SH, or C₀₋₁₀alkyl-CONH₂; or R₅ and R₆ together form C₃₋₂₀ cycloalkylene, C₃₋₂₀ heterocycloalkylene, C₆₋₂₀ arylene or C₆₋₂₀ heteroarylene;
Y combines directly with A or with R₆, and is H, -(CH₂)ₙCH₃, -(CH₂)ₙCOOH, -(CH₂)ₙOH -(CH₂)ₙSO₃H, -(CH₂)ₙOSO₃H, -(CH₂)ₙPO₃H₂, -(CH₂)ₙOPO₃H₂, -(CH₂)ₙSO₃NH₂, -(CH₂)ₙCONH₂, or -(CH₂)ₙC(O)SO₃H,
wherein n is an integer from 0 to 10.

In one aspect of the invention, R₁, R₂, R₃ and R₄ are each independently H, OH, =O or C₁₋₅ alkyl.

In one aspect of the invention, R₁, R₂, R₃ and R₄ are each independently H, or OH, and wherein contains at least one OH.

In one aspect of the invention, R₁, R₂, R₃ and R₄ are each independently H, OH, =0 or C₁₋₅ alkyl, and wherein contains at least one or more =O or C₁₋₅ alkyl.

In one aspect of the invention, R₁, R₂, R₃ and R₄ are each independently H, or OH.

In one aspect of the invention, R₁, R₃ and R₄ are OH, and R₂ is H.

In one aspect of the invention, R₁ and R₂ are H, and R₃ and R₄ are OH.

In one aspect of the invention, R₁, R₂ and R₄ are H, and R₃ is OH.

In one aspect of the invention, R₁, R₂ and R₄ are H, and R₃ is =O.

In one aspect of the invention, R₁ and R₃ are OH, and R₂ is C₂ alkyl.

In one aspect of the invention, R₁, R₃ and R₄ are OH, R₂ is C₂ alkyl.

In one aspect of the invention, X is substituted or unsubstituted C₁₋₄ alkylene, and if substituted, one or more hydrogen(s) is(are) linear or branched C₁₋₄ alkyl. In one specific aspect of the invention, X is unsubstituted C₁₋₄ alkylene.

In one aspect of the invention, A is -NR₅R₆C(O)-, -OR₆, -SR₆, -NR₅R₆, -C(O)R₆, -C(O)OR₆, -C(O)NR₅R₆, -CH(R₅)(R₆), -CH(R₅)(OR₆), -CH(OR₅)(OR₆), - CH(R₅)(SR₆), or -CH(SR₅)(SR₆). In one specific aspect of the invention, A is -NR₅R₆C(O)-, -C(O)NR₅R₆, or -CH(R₅)(R₆).

In one aspect of the invention, R₅ is H, substituted or unsubstituted C₁₋₁₀ alkyl, substituted or unsubstituted C₁₋₁₀ heteroalkyl, substituted or unsubstituted C₃₋₂₀ cycloalkyl, substituted or unsubstituted C₃₋₂₀ heterocycloalkyl, substituted or unsubstituted C₆₋₂₀ aryl, substituted or unsubstituted C₁₋₁₀alkyl-C₆₋₂₀ aryl or substituted or unsubstituted C₁₋₁₀alkyl-C₆₋₂₀ heteroaryl,
wherein if R₅ is substituted, one or more hydrogen(s) is(are) substituted with linear or branched C₁₋₅ alkyl, =O, ORₐ, SRₐ, N(Rₐ)₂, or CON(Rₐ)₂, Rₐ is H or C₁₋₅ alkyl.

In one aspect of the invention, R₅ is H, or substituted or unsubstituted C₁₋₁₀ alkyl,
wherein if R₅ is substituted, one or more hydrogen(s) is(are) substituted with =O, OH, SH, NH₂, or CONH₂.

In one aspect of the invention, R₅ is substituted or unsubstituted C₃₋₂₀ cycloalkyl, substituted or unsubstituted C₃₋₂₀ heterocycloalkyl or substituted or unsubstituted C₆₋₂₀ aryl,
wherein if R₅ is substituted, one or more hydrogen(s) is(are) substituted with =0, OH, SH, NH₂, or CONH₂.

In one aspect of the invention, R₅ is unsubstituted C₃₋₁₀ cycloalkyl, unsubstituted C₃₋₁₀ heterocycloalkyl or unsubstituted C₆₋₁₀ aryl.

In one aspect of the invention, R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, or substituted or unsubstituted C₆₋₂₀ arylene,
wherein if R₆ is substituted, one or more hydrogen(s) is(are) substituted with =0, linear or branched C₁₋₁₀ alkyl, C₀₋₁₀alkyl-C₆₋₂₀aryl or C₀₋₁₀alkyl-OH.

In one aspect of the invention, R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, or substituted or unsubstituted C₆₋₂₀ arylene,
wherein if R₆ is substituted, one or more hydrogen(s) is(are) substituted with linear or branched C₁₋₅ alkyl, C₀₋₅ alkyl-OH, C₆₋₂₀aryl or CH₂-C₆₋₂₀aryl.

In one aspect of the invention, R₆ is a bond.

In one aspect of the invention, R₆ is substituted or unsubstituted C₁₋₅ alkylene, wherein if R₆ is substituted, one or more hydrogen(s) is(are) substituted with linear or branched C₁₋₅ alkyl, C₀₋₃ alkyl-OH, C₆ aryl or CH₂-C₆ aryl.

In one aspect of the invention, R₅ and R₆ together form C₃₋₁₀ cycloalkylene, C₃₋₁₀ heterocycloalkylene, C₆₋₁₀ arylene or C₆₋₁₀ heteroarylene.

In one aspect of the invention, R₅ and R₆ together form

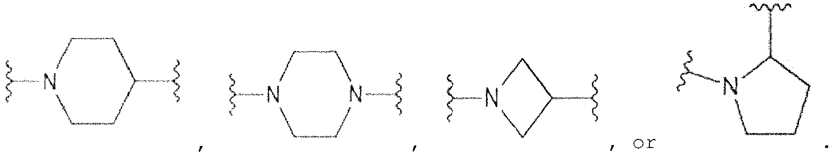

In one aspect of the invention, Y is H, -(CH₂)ₙCH₃, -(CH₂)ₙCOOH, - (CH₂)ₙOH, -(CH₂)ₙSO₃H or -(CH₂)ₙOSO₃H, n is an integer from 0 to 10.

In one aspect of the invention, Y is -(CH₂)ₙSO₃H, n is an integer from 0 to 5.

Further, the present invention provides a pharmaceutical composition for treating inflammatory diseases containing the above compound or a pharmaceutically acceptable salt thereof.

### Effects of the invention

The present invention relates to a novel compound for inhibiting TNF-α production and inflammatory complex activity and a method for producing the same, and the compound according to the present invention exhibits a useful anti-inflammatory activity and it can be developed as a treatment measures for the related inflammatory diseases.

### Detailed description

The present invention relates to a compound represented by Formula 1 or a pharmaceutically acceptable salt thereof.

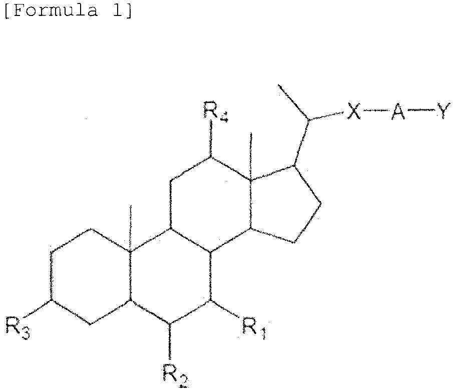

Hereinafter, the present invention is described in detail.

In the present invention, the compound represented by Formula 1 corresponds to a conjugate obtained by reacting a bile acid derivative with a taurine derivative. Bile acid derivatives refer to derivatives of the following structural formula, and the position of each carbon atom is indicated by a commonly used method.

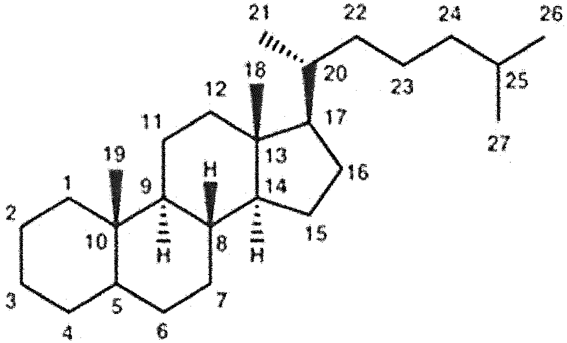

In the present invention, in the bile acid derivative, some of the H atoms substituted on the carbon ring may be substituted with other substituents. When substituted, for example, it may be substituted with C₁₋₅ alkyl, hydroxy, oxo (=O), etc. Examples of substituted structures include Deoxycholic acid, Lithocholic acid, Dehydrolithocholic acid, Obeticholic acid, INT-777, Cholic acid, and the like. However, this is an illustrative example and is not limited to this.

As used herein, "substituted or unsubstituted" refers to an atom that may be substituted or unsubstituted, "substituted" refers to a group having one or more substituents, and a substituent refers to a chemical moiety covalently bonded to the group. Alternatively, "unsubstituted" means that the chemical part is changed directly at the corresponding substituted part without having a substituent.

In this specification, "halo" refers to elements belonging to group 17, such as fluorine, chlorine, bromine, iodine, and the like.

In this specification, "alkyl" refers to a chemical moiety obtained by removing the H atom from the carbon atom of an aliphatic or alicyclic, saturated or unsaturated (unsaturated, fully unsaturated) carbonized H compound, for example, methyl, ethyl, propyl, butyl, pentyl, hexyl, isopropyl, isobutyl, sec-butyl, tert-butyl, isopentyl, neopentyl, and the like.

In this specification, "alkylene" is a divalent chemical moiety obtained by removing one more H atom from the alkyl, and does not include the removal of two carbon atoms from one carbon.

In this specification, "heteroalkyl" refers to alkyl containing one or more hetero atoms, and "heteroalkylene" refers to alkylene containing one or more hetero atoms.

In the present invention, "cycloalkyl" refers to an alkyl group, which is a cyclyl group, and refers to a chemical moiety obtained by removing the H atom from an alicyclic ring atom. Examples include cyclopropane, cyclobutane, cyclopentane, cyclohexane, cyclohexene, and the like.

As used herein, "heterocycloalkyl" means cycloalkyl containing one or more hetero atoms.

As used herein, "aryl" refers to a chemical moiety obtained by removing the H atom from the aromatic ring atom of an aromatic compound having a ring atom.

In the present invention, "heteroaryl" means aryl containing one or more hetero atoms. Examples include pyridine, pyrrolyl, quinoline, pyridyl, pyrimidinyl, and the like.

As used herein, "branched" means having one or more branched forms, and branching is not limited to carbon and means that it can bind to carbon.

As used herein, "linear" means not having a branched shape.

In this specification, a prefix (e.g., C₁₋₁₂, C₃₋₈, etc.) refers to the number of ring atoms or a range of the number of ring atoms, regardless of whether they are carbon atoms or hetero atoms. For example, the term "C₃₋₆ heterocycloalkyl" used herein refers to a heterocycloalkyl group having 3 to 6 ring atoms. Additionally, for example, the term "C₁₋₁₀ alkyl-C₆₋₂₀ aryl" used herein refers to a form in which C₁₋₁₀ alkyl and C₆₋₂₀ aryl are sequentially bonded.

In this specification, isomers are not excluded unless otherwise specified.

The present invention relates to a compound represented by Formula 1 or a pharmaceutically acceptable salt thereof. 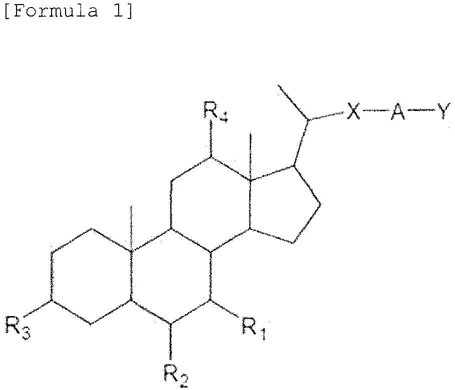 wherein,
R₁, R₂, R₃ and R₄ are each independently H, halo, C₁₋₅ alkyl, OH, or =0,
X is substituted or unsubstituted C₁₋₁₀ alkylene,
wherein when X is substituted, one or more hydrogens are substituted with linear or branched C₁₋₁₀ alkyl, halo, OH, SH, NH₂, CONH₂, or COOH,
A is C₁₋₁₀ alkylene, C₁₋₁₀ heteroalkylene, C₁₋₁₀ alkenylene, C₁₋₅ alkynylene, C₃₋₈ cycloalkylene, C₃₋₈ heterocycloalkylene, C₆₋₂₀ arylene, C₆₋₂₀ heteroarylene, -NR₅R₆C(O)-, -OR₆, -SR₆, -NR₅R₆, -C(O)R₆, -C(O)OR₆, -C(O)NR₅R₆, -CH(R₅)(R₆), -CH(R₅)(OR₆), -CH(OR₅)(OR₆), -CH(R₅)(SR₆), or -CH(SR₅)(SR₆);
R₅ is H, substituted or unsubstituted C₁₋₁₀ alkyl, substituted or unsubstituted C₁₋₁₀ heteroalkyl, substituted or unsubstituted C₃₋₂₀ cycloalkyl, substituted or unsubstituted C₃₋₂₀ heterocycloalkyl, substituted or unsubstituted C₆₋₂₀ aryl, substituted or unsubstituted C₆₋₂₀ heteroaryl, substituted or unsubstituted C₁₋₁₀ alkyl-C₆₋₂₀ cycloalkyl, substituted or unsubstituted C₁₋₁₀ alkyl-C₆₋₂₀ heterocycloalkyl, substituted or unsubstituted C₁₋₁₀alkyl-C₆₋₂₀ aryl or substituted or unsubstituted C₁₋₁₀alkyl-C₆₋₂₀ heteroaryl,
wherein if R₅ is substituted, one or more hydrogen(s) is(are) substituted with linear or branched C₁₋₅ alkyl, =O, ORₐ, SRₐ, N(Rₐ)₂, or CON(Rₐ)₂, and Rₐ is H or C₁₋₅ alkyl,
R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, substituted or unsubstituted C₆₋₂₀ arylene, or substituted or unsubstituted C₆₋₂₀ heteroarylene,
wherein if R₆ is substituted, one or more hydrogen(s) is(are) substituted with =O, linear or branched C₁₋₁₀ alkyl, C₀₋₁₀alkyl-C₆₋₂₀aryl, C₀₋₁₀alkyl-OH, C₀₋₁₀alkyl-NH₂, C₀₋₁₀alkyl-SH, or C₀₋₁₀alkyl-CONH₂; or R₅ and R₆ together form C₃₋₂₀ cycloalkylene, C₃₋₂₀ heterocycloalkylene, C₆₋₂₀ arylene or C₆₋₂₀ heteroarylene;
Y combines directly with A or with R₆, and is H, -(CH₂)ₙCH₃, -(CH₂)ₙCOOH, -(CH₂)ₙOH -(CH₂)ₙSO₃H, -(CH₂)ₙOSO₃H, -(CH₂)ₙPO₃H₂, -(CH₂)ₙOPO₃H₂, -(CH₂)ₙSO₃NH₂, -(CH₂)ₙCONH₂, or -(CH₂)ₙC(O)SO₃H,
wherein n is an integer from 0 to 10.

In one aspect of the invention, Formula 1 can be specifically expressed as the following Formula 2. 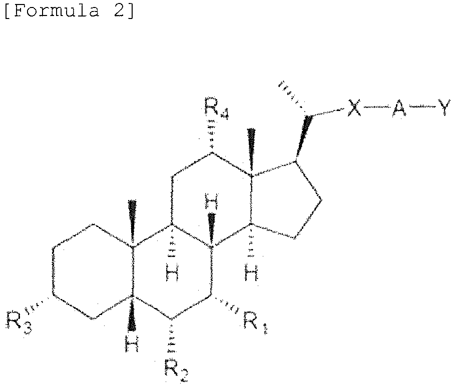 wherein, R₁, R₂, R₃, R₄, X, A and Y are as the same as defined above.

In one aspect of the invention, the compound or pharmaceutically acceptable salt thereof includes a compound expressed more specifically as Formula 3 below.

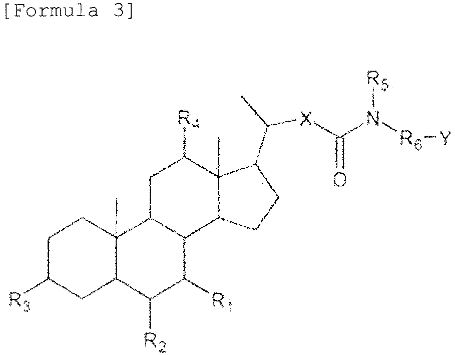

In addition, more specifically, the compound or a pharmaceutically acceptable salt thereof includes a compound represented by Formula 4 below.

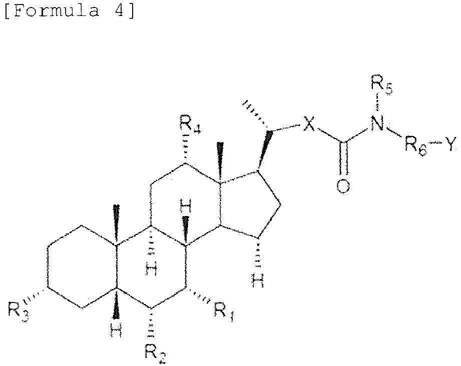

In Formula 3 and 4, R₁, R₂, R₃, R₄, R₅, R₆, X, and Y are as the same defined above.

In one specific aspect of the invention, R₁, R₂, R₃ and R₄ are each independently H, OH, =O or C₁₋₅ alkyl.

In one aspect of the invention, R₁, R₂, R₃ and R₄ are each independently H, or OH, wherein contains at least one or more OH.

In one aspect of the invention, R₁, R₂, R₃ and R₄ are each independently H, OH, =O or C1-5 alkyl, wherein contains at least one or more =0 or C1-5 alkyl.

In one aspect of the invention, R₁, R₂, R₃ and R₄ are each independently H, or OH.

In one aspect of the invention, R₃ is OH.

In one aspect of the invention, R₁ and R₃ are OH.

In one aspect of the invention, R₃ is =O.

In one aspect of the invention, R₁ and R₃ are OH, and R₂ is C₂ alkyl.

In one aspect of the invention, R₁, R₃ and R₄ are OH, and R₂ is C₂ alkyl.

In one aspect of the invention, R₁, R₃ and R₄ are OH, and R₂ is H.

In one aspect of the invention, R₁ and R₂ are H, R₃ and R₄ are OH.

In one aspect of the invention, R₁, R₂ and R₄ are H, and R₃ is OH.

In one aspect of the invention, R₁, R₂ and R₄ are H, R₃ is =O.

In one aspect of the invention, R₁ and R₃ are OH, R₂ is C₂ alkyl, and R₄ is H.

In one aspect of the invention, R₁, R₃ and R₄ are OH, and R₂ is C₂ alkyl.

In one aspect of the invention, the compounds or pharmaceutically acceptable salts thereof include compounds represented by the following Formulas 5 to 10, and more specifically, Formulas 5-2 to 10-2. 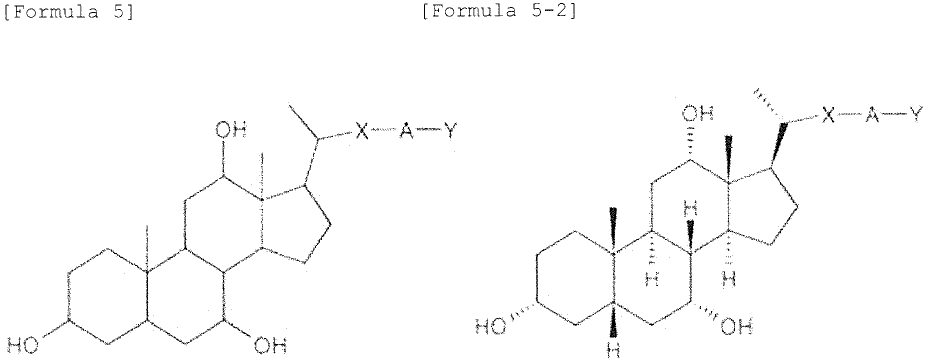 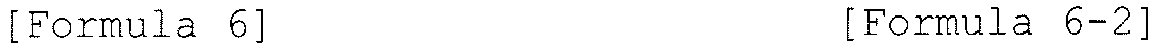 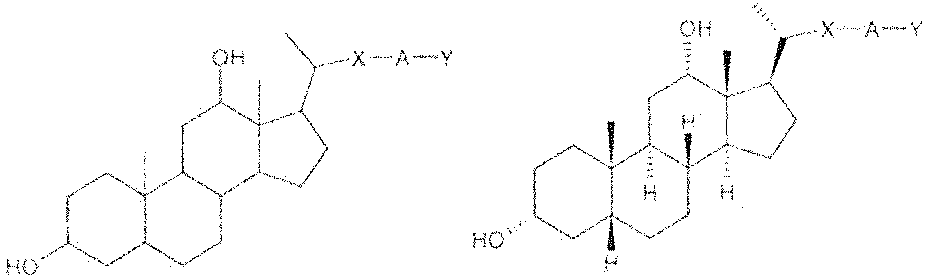 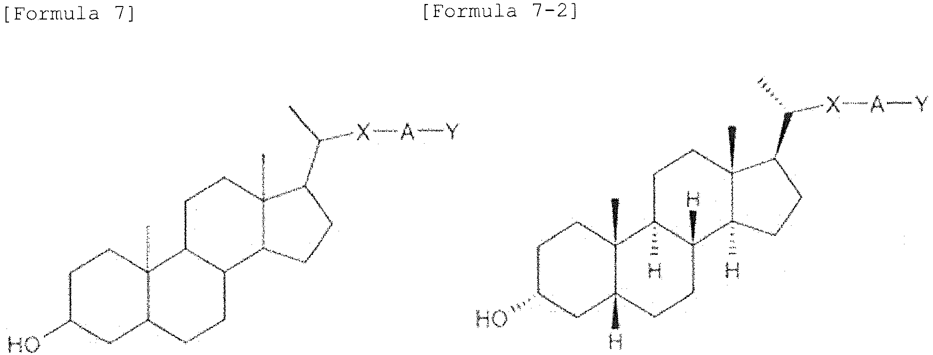 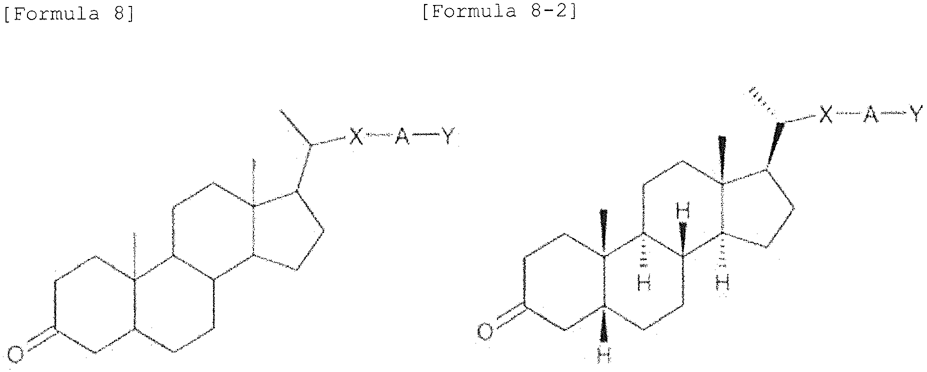 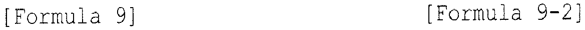 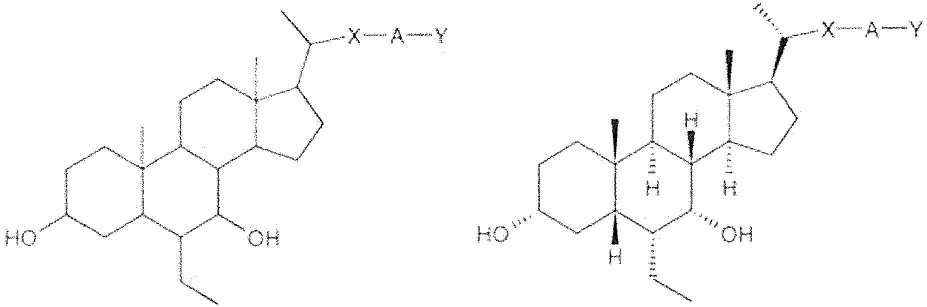 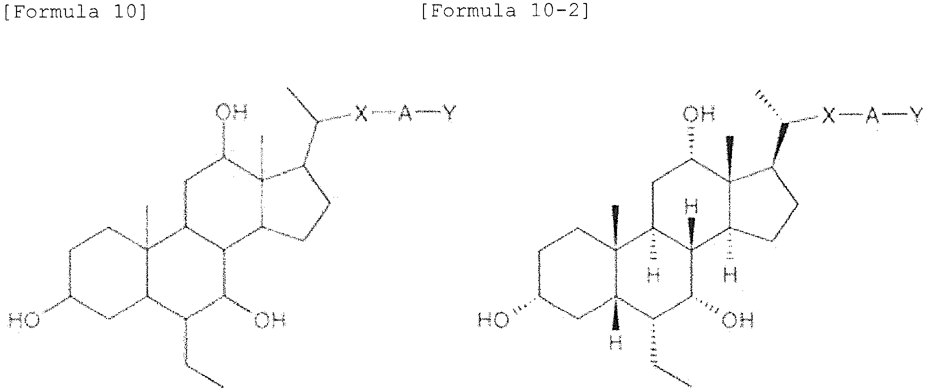 wherein, X, A and Y are as the same defined above.

In one aspect of the invention, X is substituted or unsubstituted C₁₋₄ alkylene, if substituted, one or more hydrogen(s) is(are) linear or branched C₁₋₄ alkyl.

In one aspect of the invention, X is unsubstituted C₁₋₄ alkylene.

In one aspect of the invention, A is -NR₅R₆C(O)-, -OR₆, -SR₆, -NR₅R₆, -C(O)R₆, -C(O)OR₆, -C(O)NR₅R₆, -CH(R₅)(R₆), -CH(R₅)(OR₆), -CH(OR₅)(OR₆), - CH(R₅)(SR₆), or -CH(SR₅)(SR₆).

In one aspect of the invention, A is -NR₅R₆C(O)-, -C(O)NR₅R₆, or - CH(R₅)(R₆).

In one aspect of the invention, R₅ is H, substituted or unsubstituted C₁₋₁₀ alkyl, substituted or unsubstituted C₁₋₁₀ heteroalkyl, substituted or unsubstituted C₃₋₂₀ cycloalkyl, substituted or unsubstituted C₃₋₂₀ heterocycloalkyl, substituted or unsubstituted C₆₋₂₀ aryl, substituted or unsubstituted C₁₋₁₀alkyl-C₆₋₂₀ aryl or substituted or unsubstituted C₁₋₁₀alkyl-C₆₋₂₀ heteroaryl,
wherein if R₅ is substituted, one or more hydrogen(s) is(are) subssituted with linear or branched C₁₋₅ alkyl, =0, ORₐ, SRₐ, N(Rₐ)₂, or CON(Rₐ)₂, Rₐ is H or C₁₋₅ alkyl.

In one aspect of the invention, R₅ is H, or substituted or unsubstituted C₁₋₁₀ alkyl,
wherein if R₅ is substituted, one or more hydrogen(s) is(are) substituted with =0, OH, SH, NH₂, or CONH₂.

In one aspect of the invention, R₅ is substituted or unsubstituted C₃₋₂₀ cycloalkyl, substituted or unsubstituted C₃₋₂₀ heterocycloalkyl or substituted or unsubstituted C₆₋₂₀ aryl,
wherein R₅ is substituted, one or more hydrogen(s) is (are) substituted with =O, OH, SH, NH₂, or CONH₂.

In one aspect of the invention, R₅ is unsubstituted C₃₋₁₀ cycloalkyl, unsubstituted C₃₋₁₀ heterocycloalkyl or unsubstituted C₆₋₁₀ aryl.

In one aspect of the invention, R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, or substituted or unsubstituted C₆₋₂₀ arylene,
wherein if R₆ is substituted, one or more hydrogen(s) is(are) substituted with =O, linear or branched C₁₋₁₀ alkyl, C₀₋₁₀alkyl-C₆₋₂₀aryl or C₀₋₁₀alkyl-OH.

In one aspect of the invention, R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, or substituted or unsubstituted C₆₋₂₀ arylene,
wherein if R₆ is substituted, one or more hydrogen(s) is(are) substituted with linear or branched C₁₋₅ alkyl, C₀₋₅ alkyl-OH, C₆₋₂₀aryl or CH₂-C₆₋₂₀aryl.

In one aspect of the invention, R₆ is a bond.

In one aspect of the invention, R₆ is substituted or unsubstituted C₁₋₅ alkylene,
wherein if R₆ is substituted, one or more hydrogen(s) is(are) substituted with linear or branched C₁₋₅ alkyl, C₀₋₃ alkyl-OH, C₆ aryl or CH₂-C₆ aryl.

In one aspect of the invention, R₅ and R₆ together form C₃₋₁₀ cycloalkylene, C₃₋₁₀ heterocycloalkylene, C₆₋₁₀ arylene or C₆₋₁₀ heteroarylene.

In one aspect of the invention, R₅ and R₆ together form

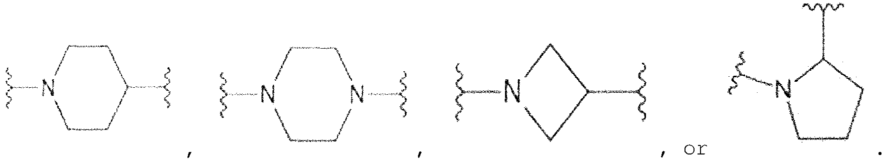

In one aspect of the invention, Y is H, -(CH₂)ₙCH₃, -(CH₂)ₙCOOH, - (CH₂)ₙOH, -(CH₂)ₙSO₃H or -(CH₂)ₙOSO₃H, and n is an integer from 0 to 10.

In one aspect of the invention, Y is -(CH₂)ₙSO₃H, and n is an integer from 0 to 5.

In one aspect of the invention, the compound represented by Formula 1 or a pharmaceutically acceptable salt thereof is any one selected from the group consisting of the following compounds or a pharmaceutically acceptable salt thereof.

[The following compounds and their pharmaceutically acceptable salts] (NC10001 ~ NC10019) 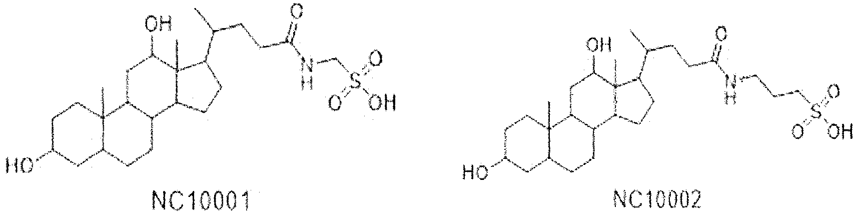 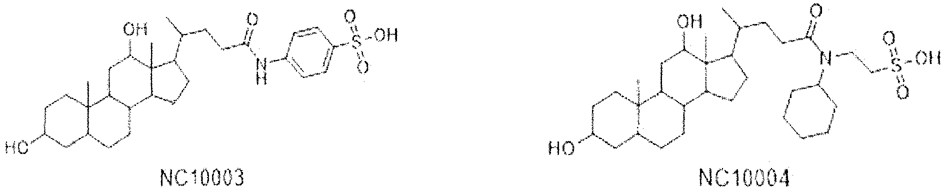 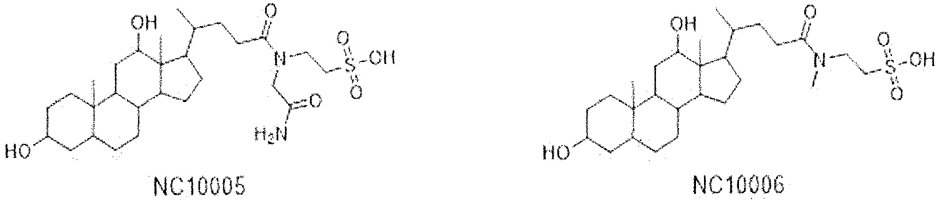 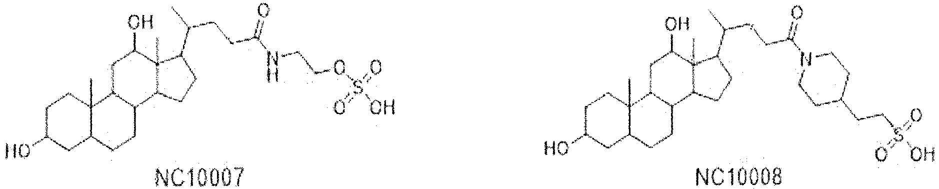 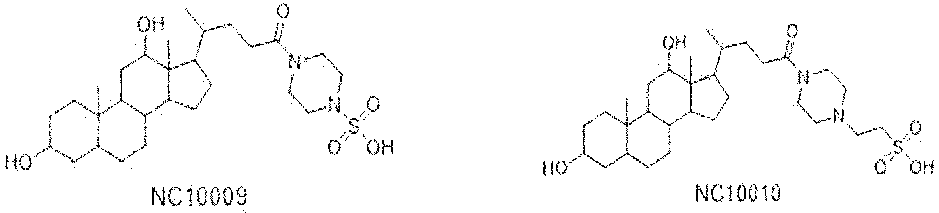 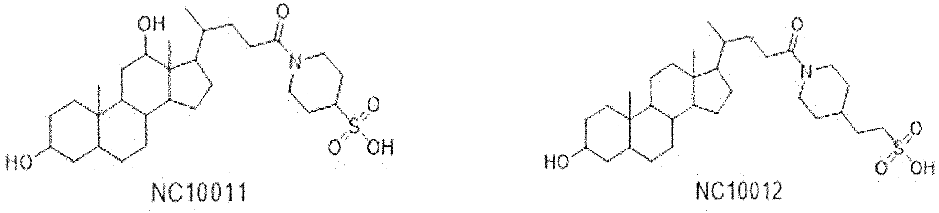 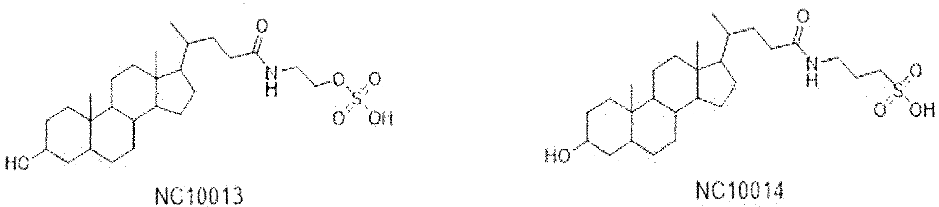 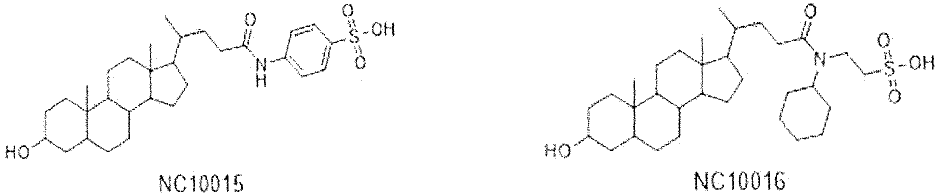 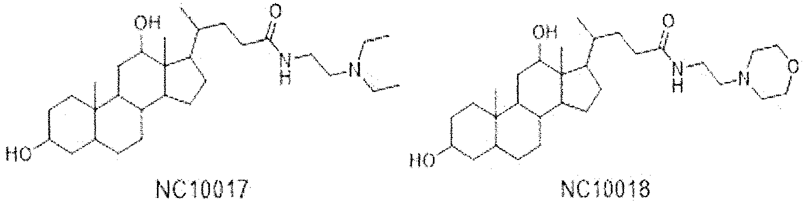 and

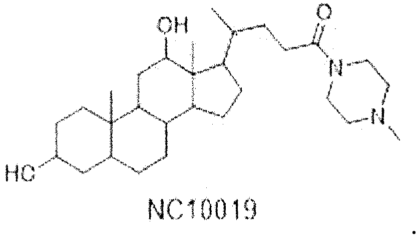

In one aspect of the invention, the compound represented by Formula 1 or a pharmaceutically acceptable salt thereof is any one selected from the group consisting of the following compounds or a pharmaceutically acceptable salt thereof.

[The following compounds and their pharmaceutically acceptable salts] (NC10021 ~ NC10056) 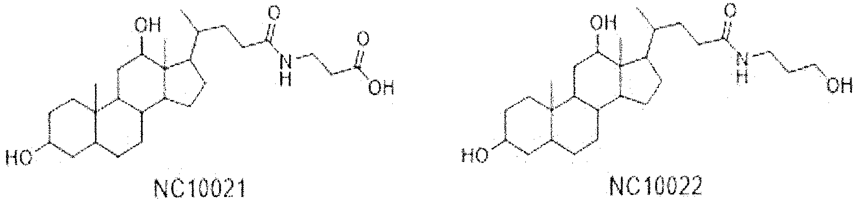 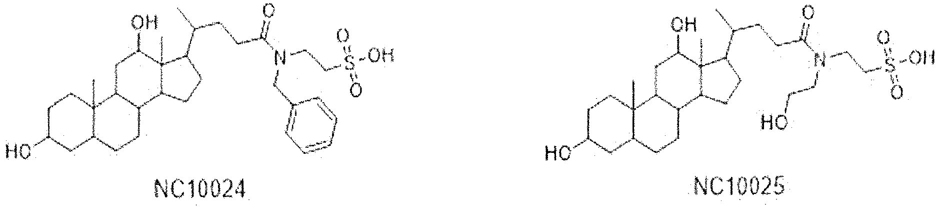 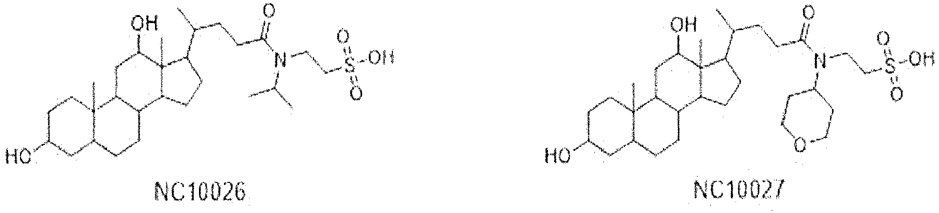 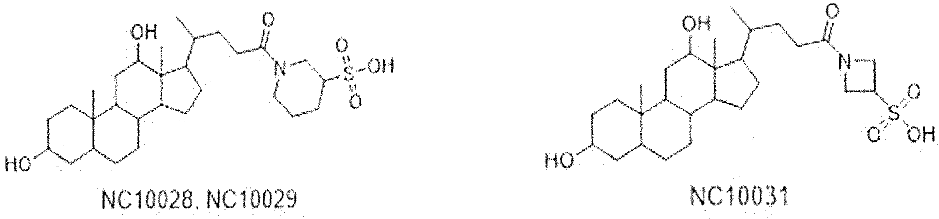 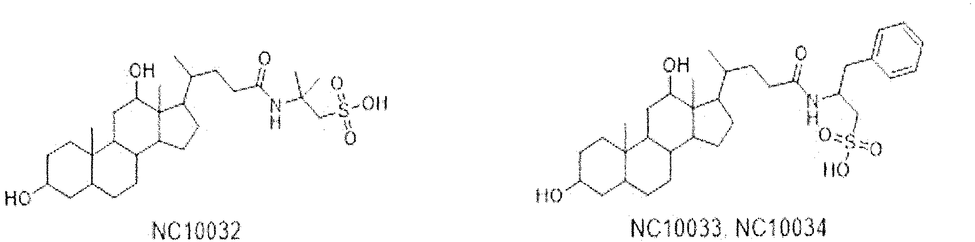 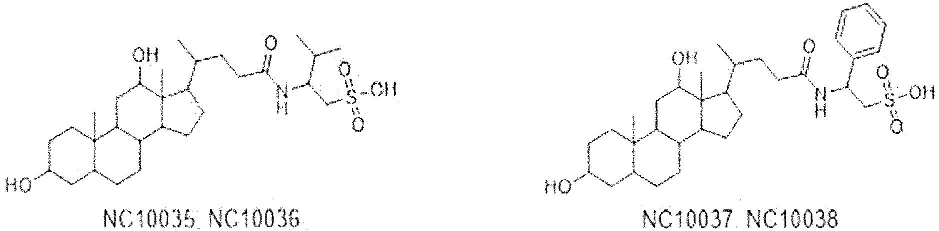 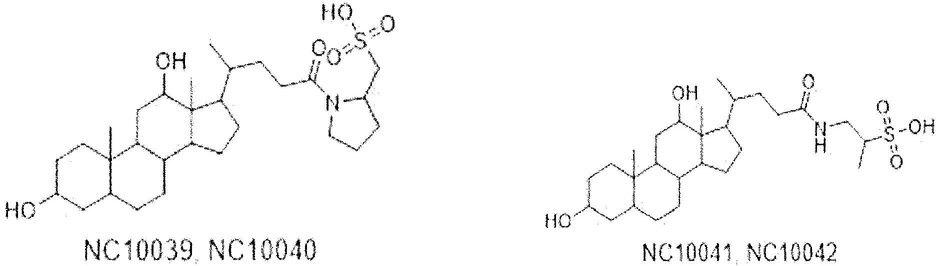 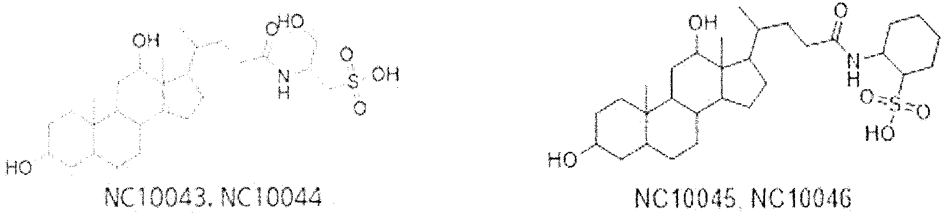 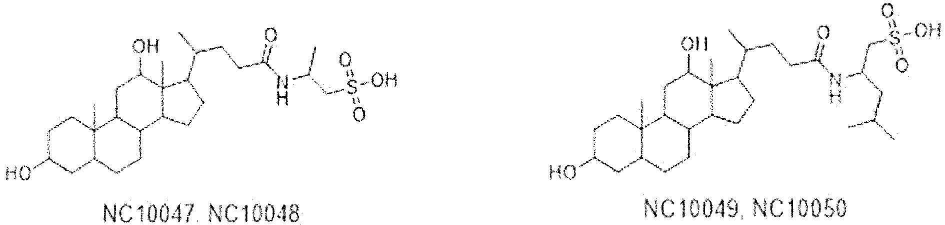 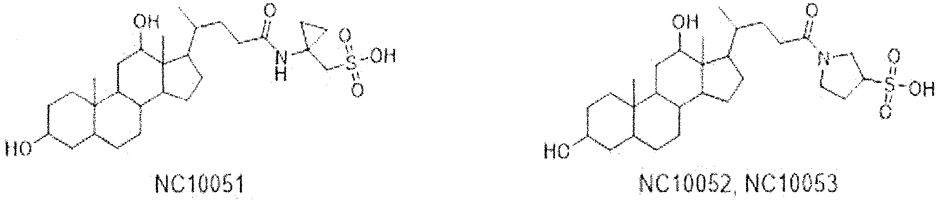 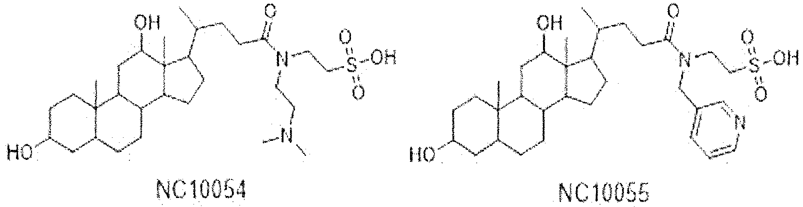 and

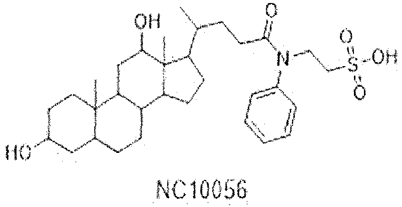

In one aspect of the invention, the compound represented by Formula 1 or a pharmaceutically acceptable salt thereof is any one selected from the group consisting of the following compounds or a pharmaceutically acceptable salt thereof.

[The following compounds and their pharmaceutically acceptable salts] (NC10058 ~ NC10093) 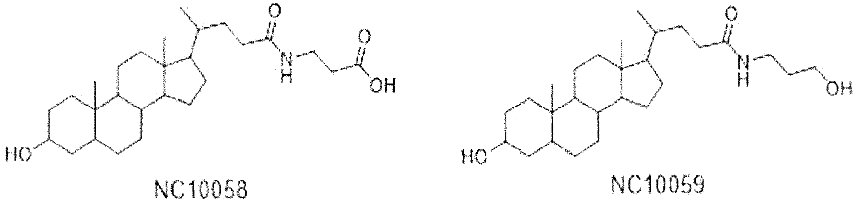 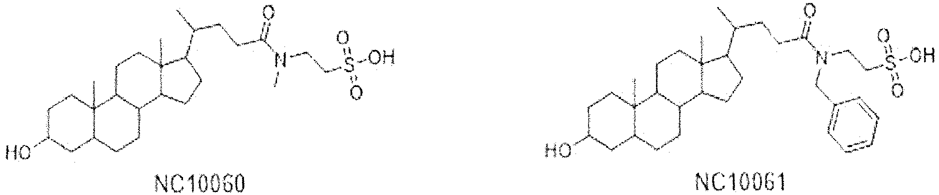 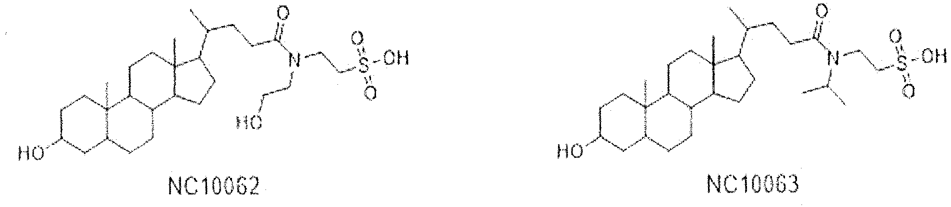 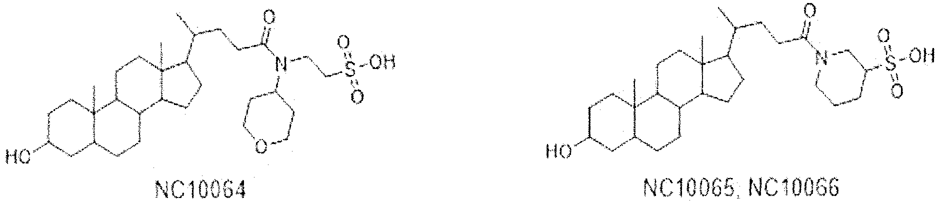 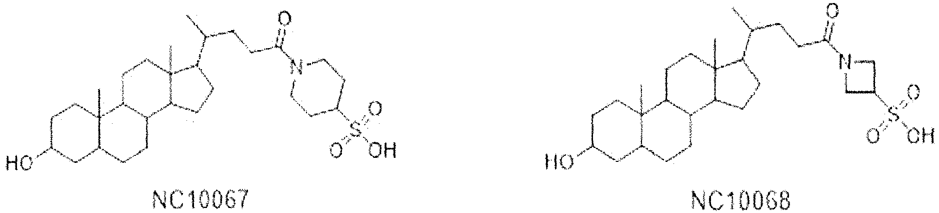 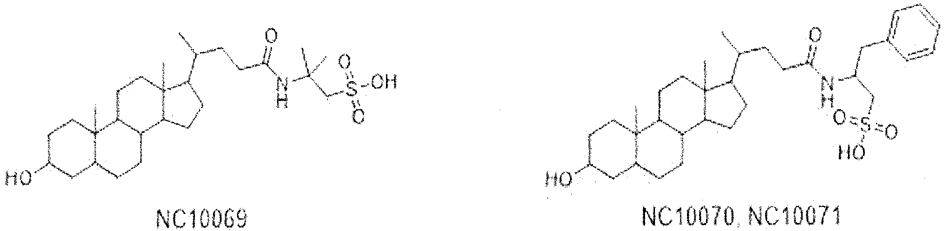 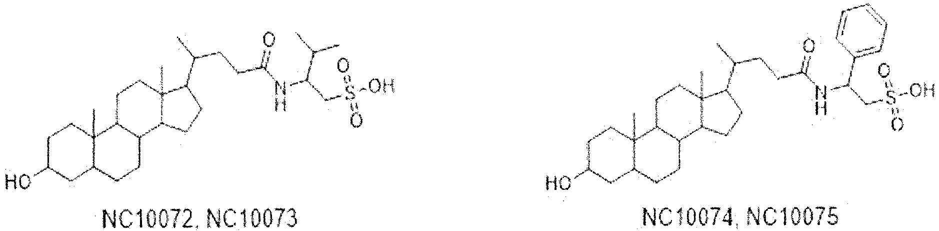 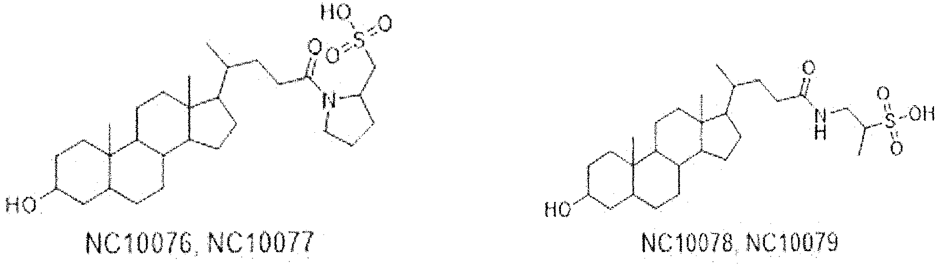 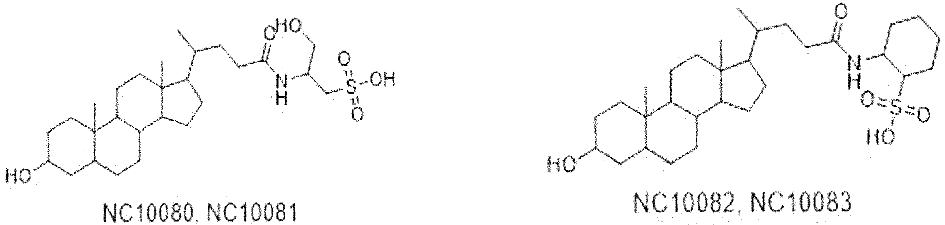 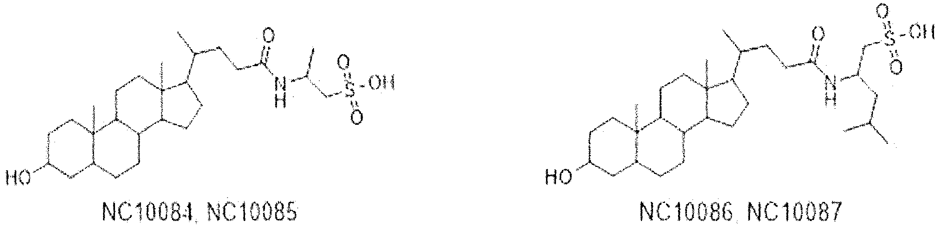 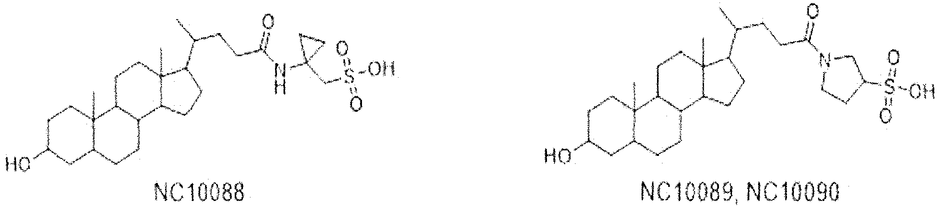 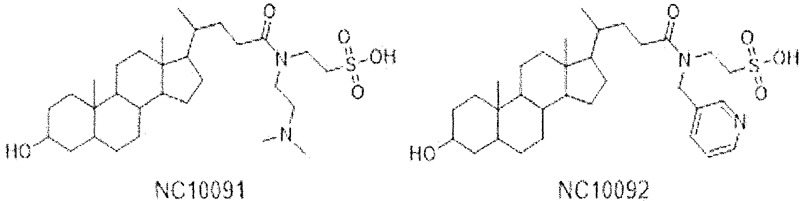 and

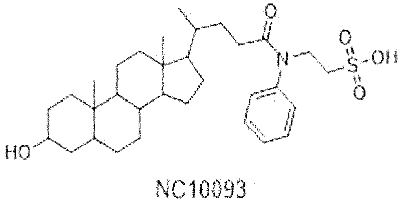

In one aspect of the invention, the compound represented by Formula 1 or a pharmaceutically acceptable salt thereof is any one selected from the group consisting of the following compounds or a pharmaceutically acceptable salt thereof.

[The following compounds and their pharmaceutically acceptable salts] (NC20002 ~ NC20037) 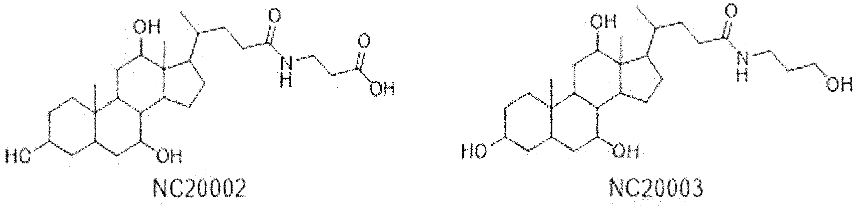 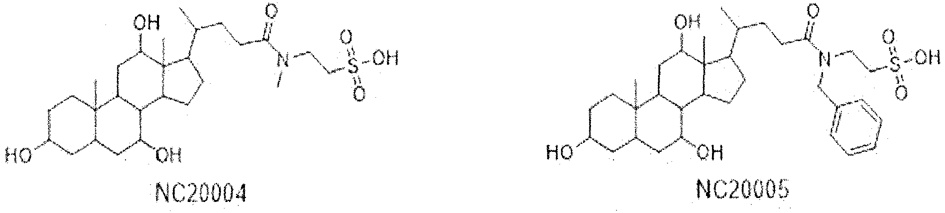 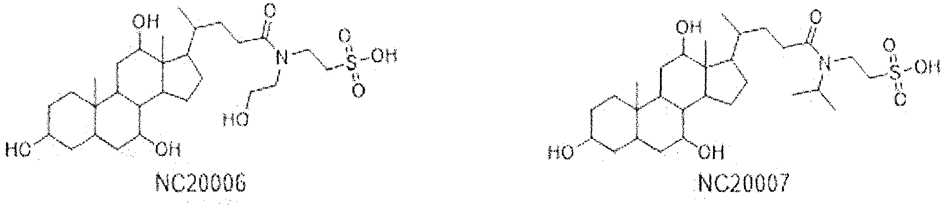 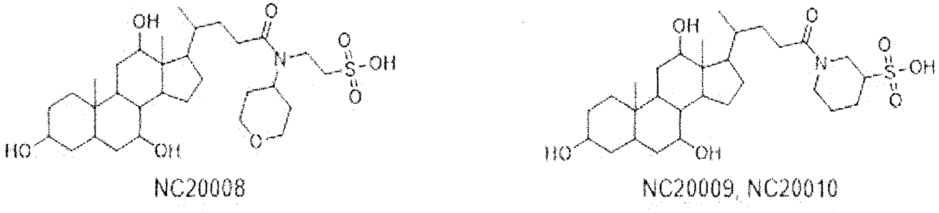 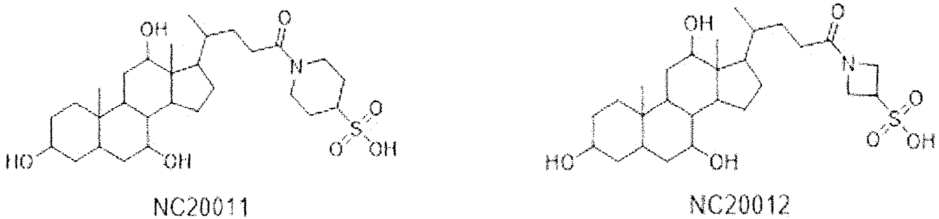 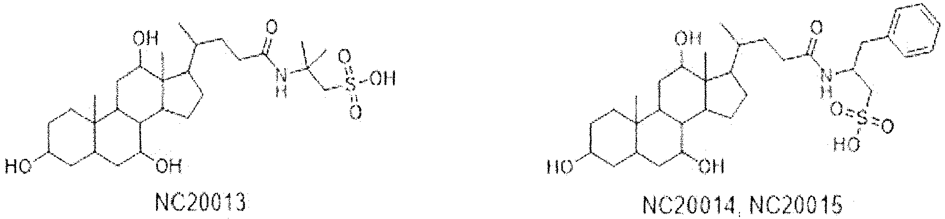 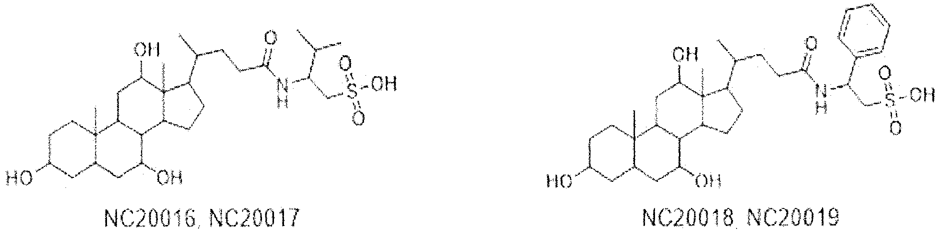 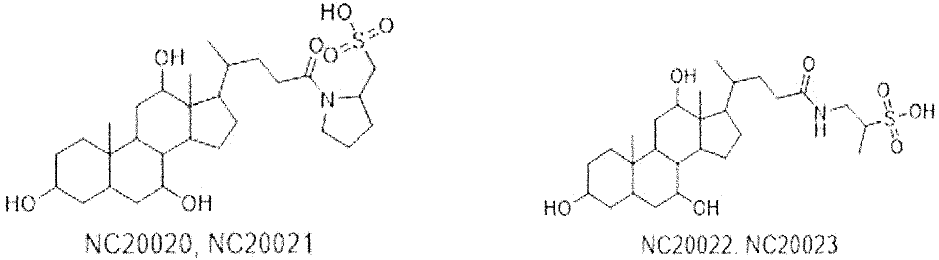 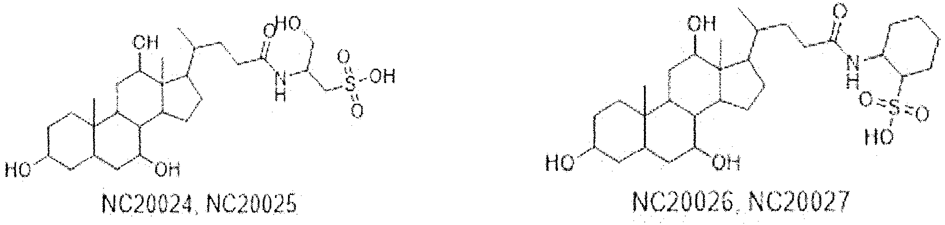 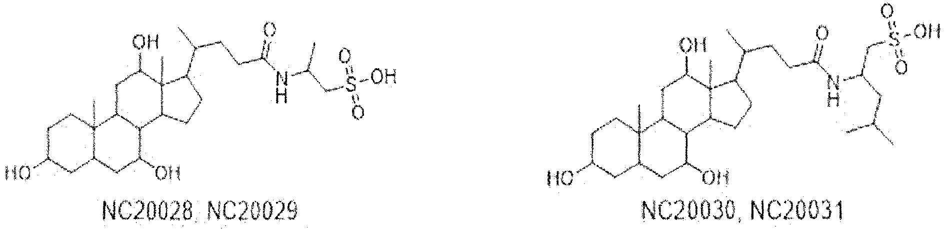 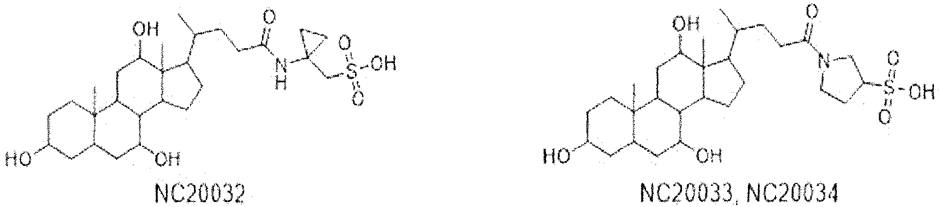 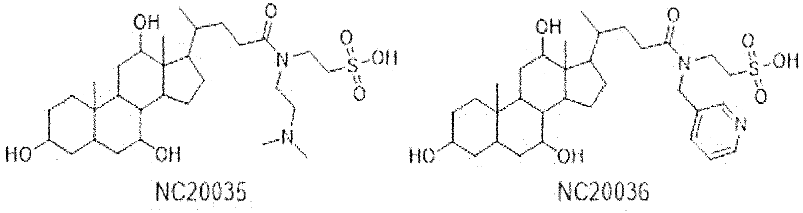 and

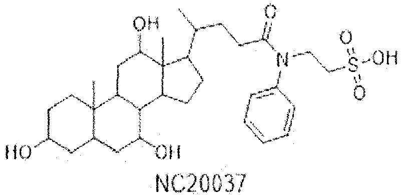

In one aspect of the invention, the compound represented by Formula 1 or a pharmaceutically acceptable salt thereof is any one selected from the group consisting of the following compounds or a pharmaceutically acceptable salt thereof.

[The following compounds and their pharmaceutically acceptable salts] (NC30002 ~ NC30037)

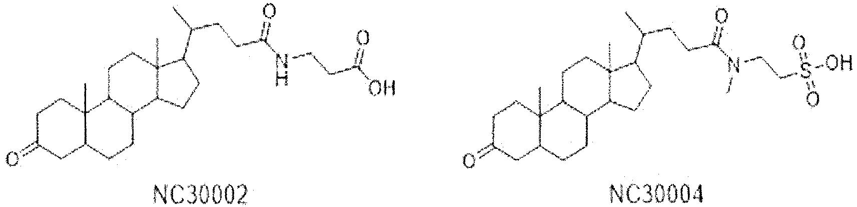

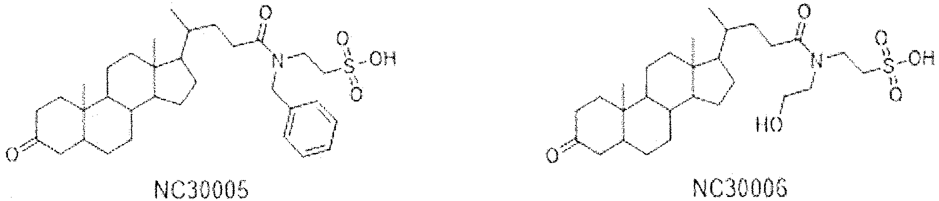

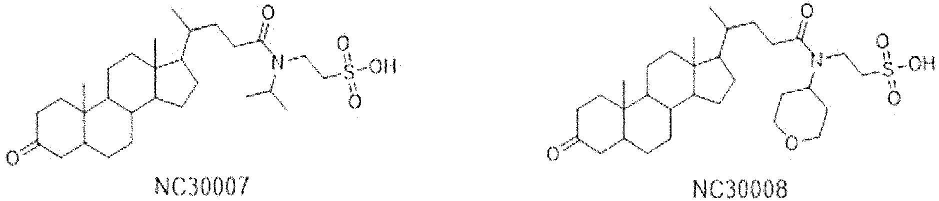

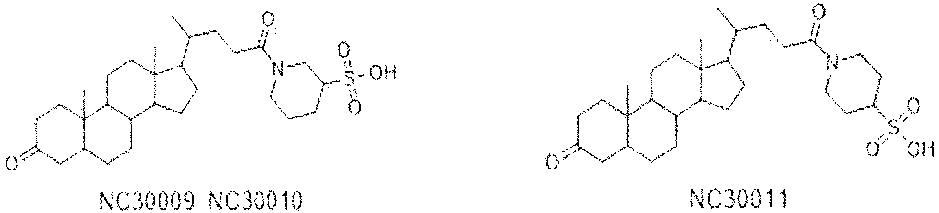

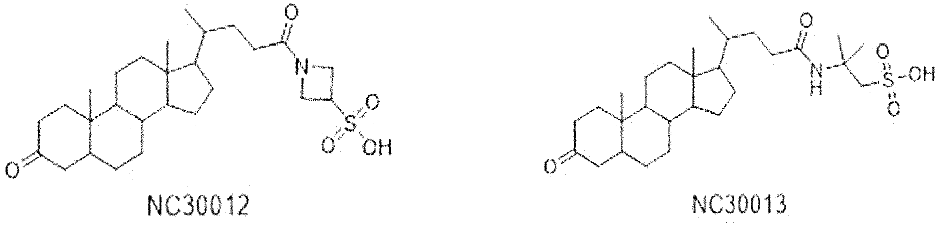

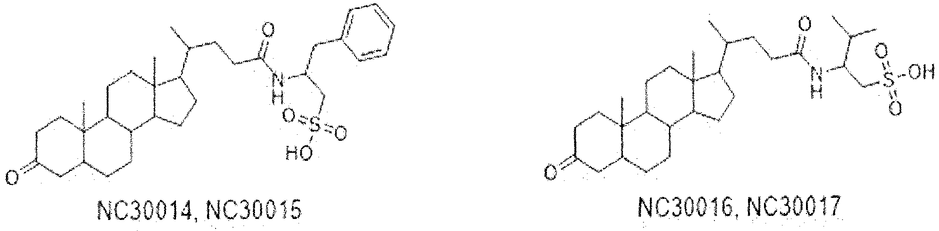

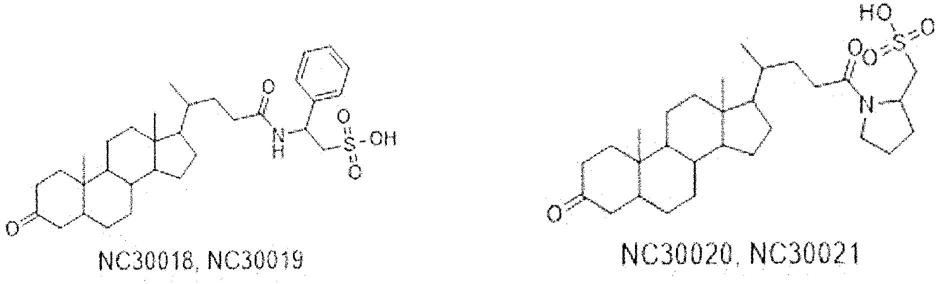

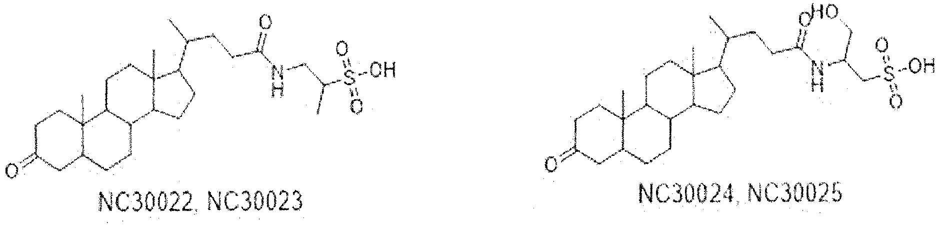

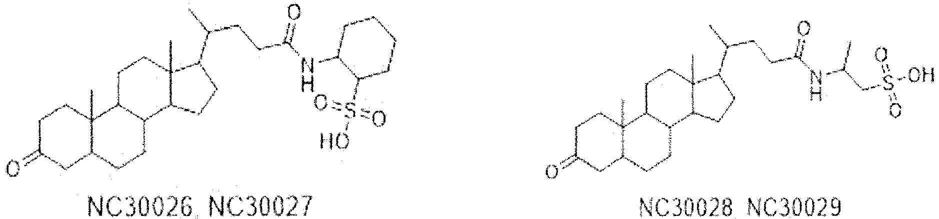

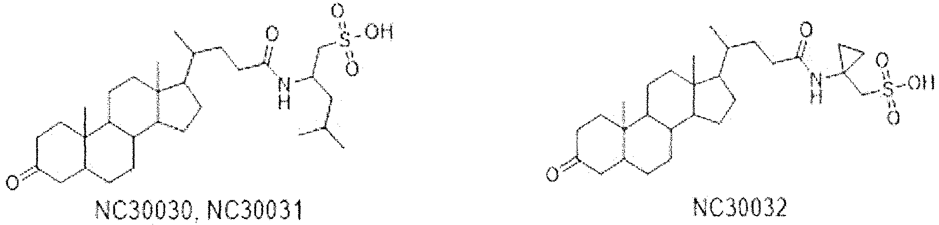

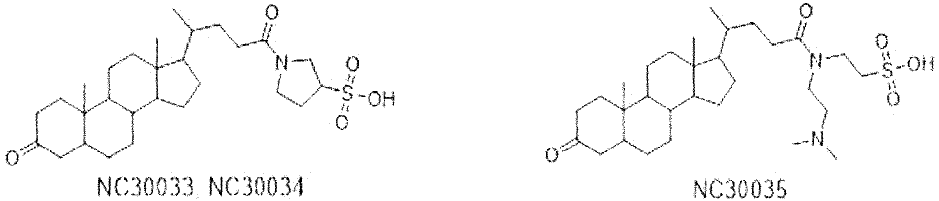

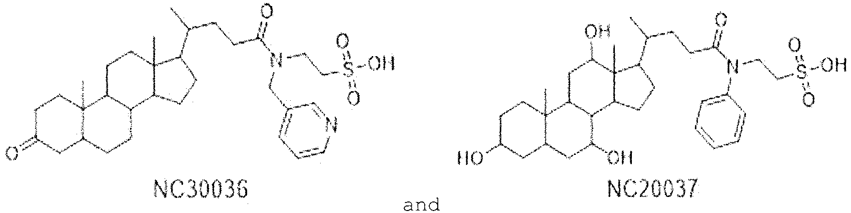

In one aspect of the invention, the compound represented by Formula 1 or a pharmaceutically acceptable salt thereof is any one selected from the group consisting of the following compounds or a pharmaceutically acceptable salt thereof.

[The following compounds and their pharmaceutically acceptable salts] (NC40002 ~ NC40037) 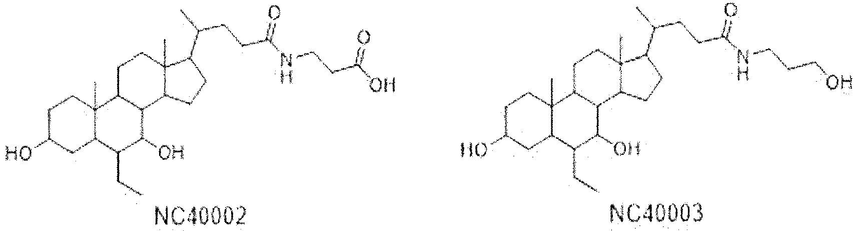 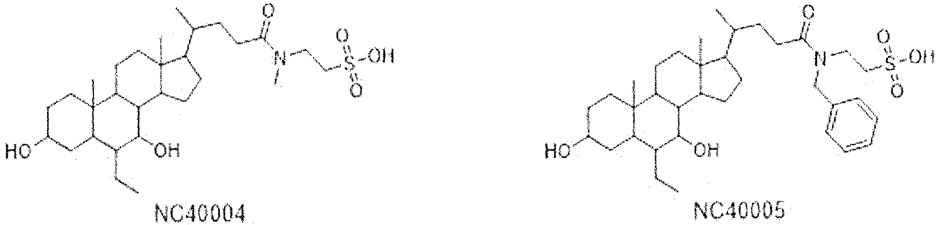 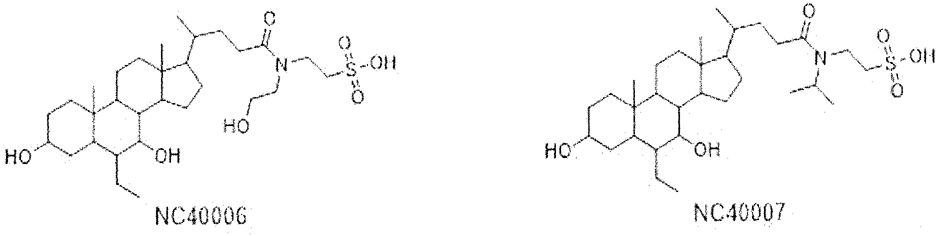 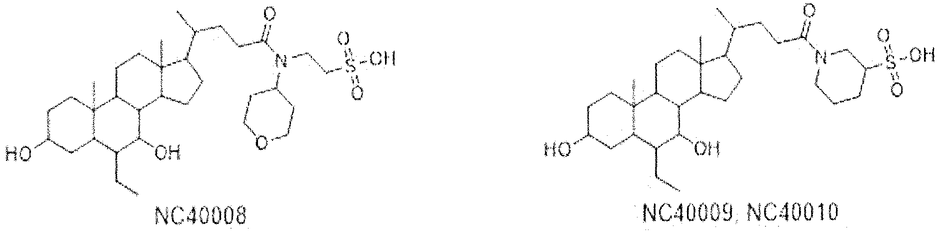 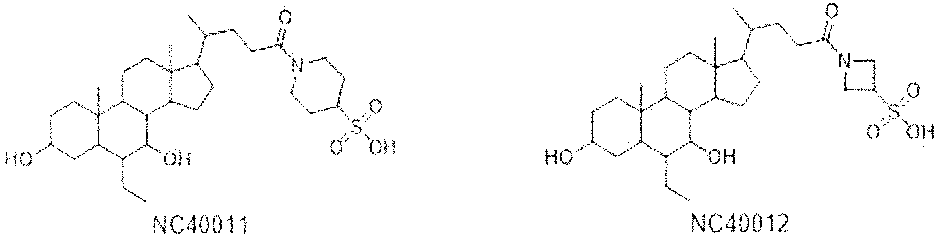 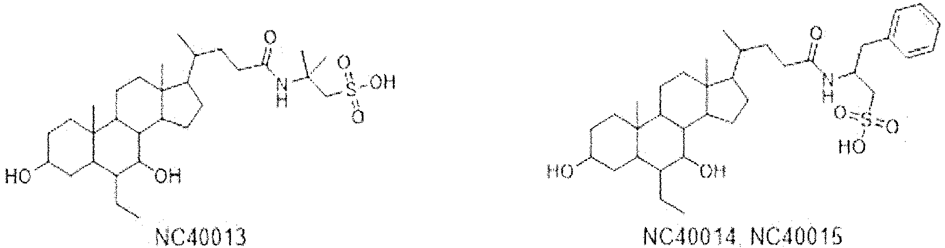 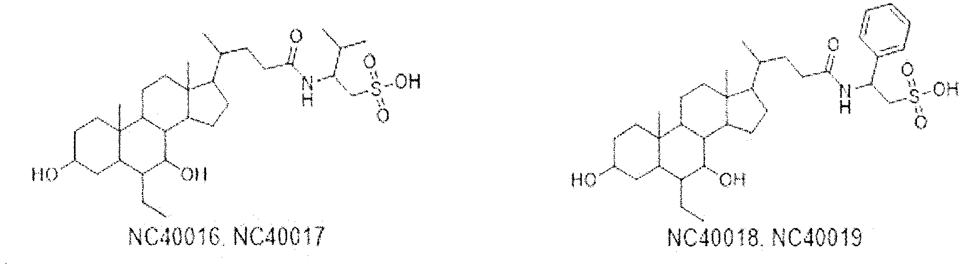 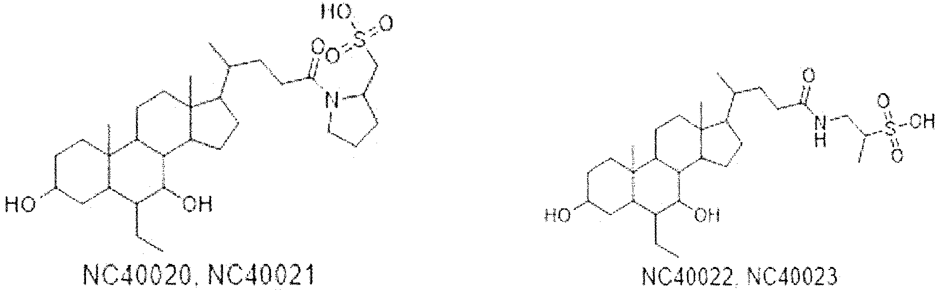 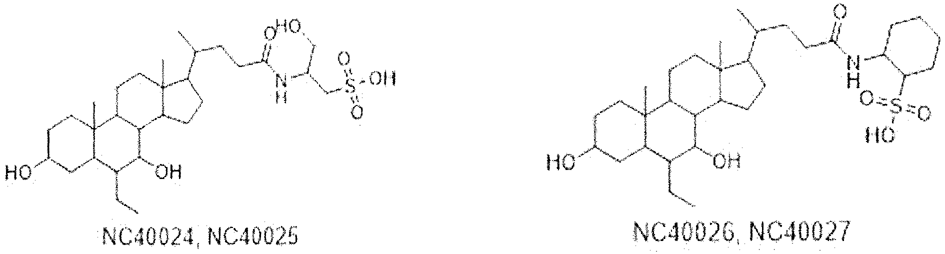 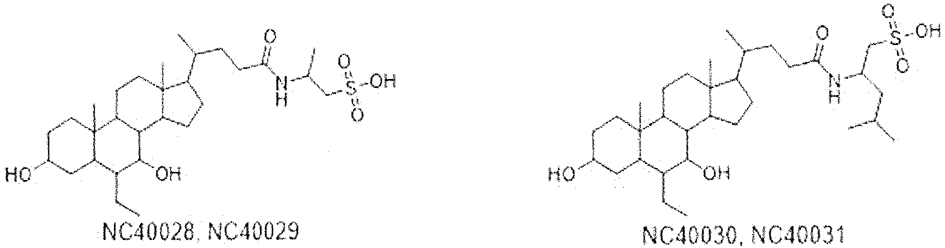 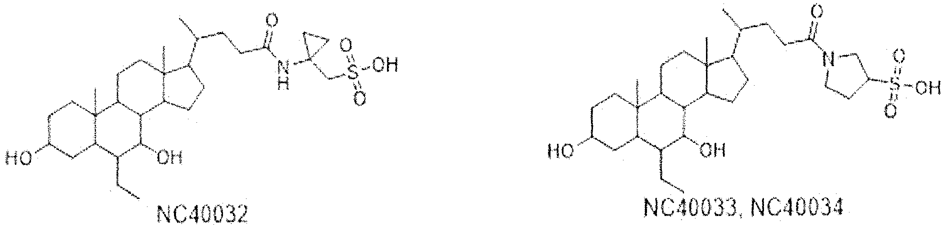 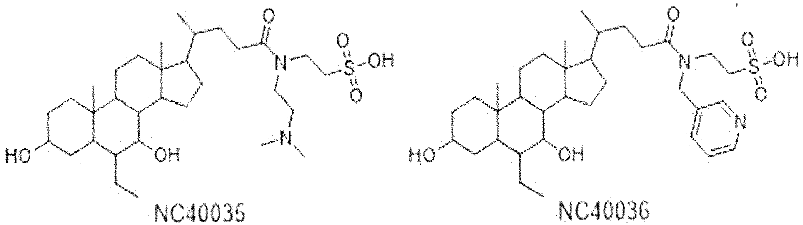 and

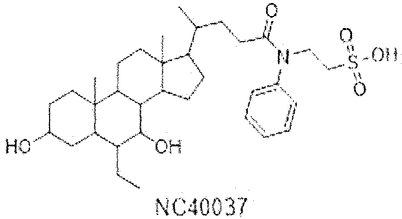

In one aspect of the invention, the compound represented by Formula 1 or a pharmaceutically acceptable salt thereof is any one selected from the group consisting of the following compounds or a pharmaceutically acceptable salt thereof.

[The following compounds and their pharmaceutically acceptable salts] (NC50002 ~NC50037)

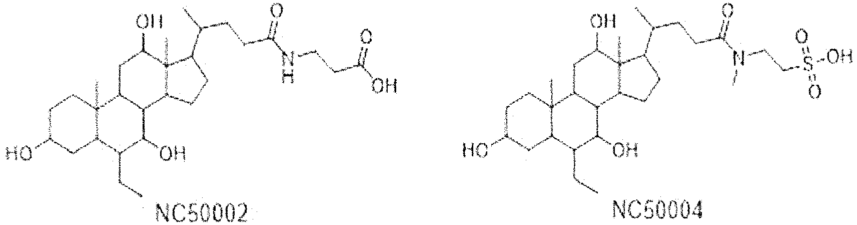

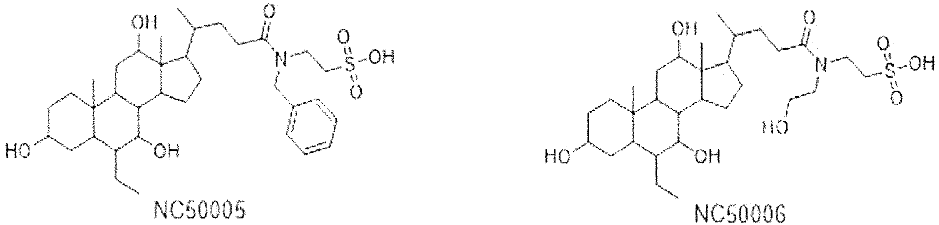

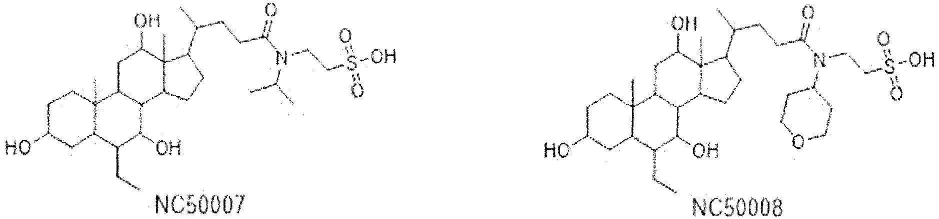

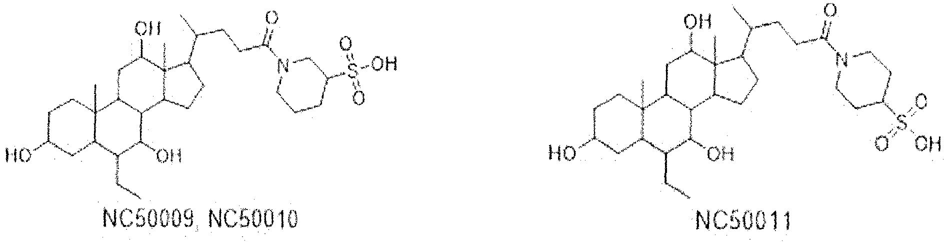

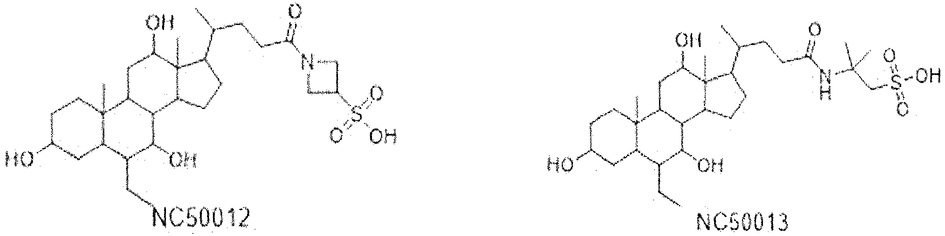

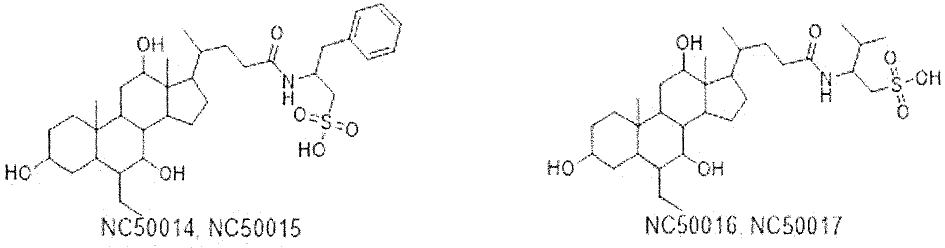

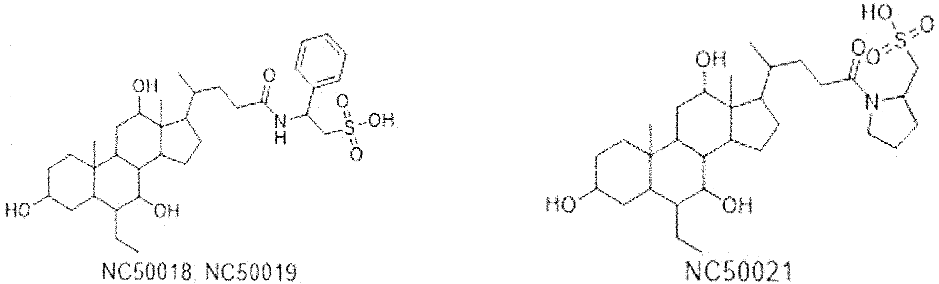

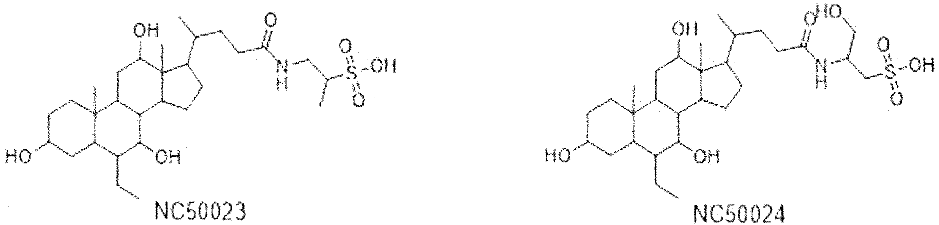

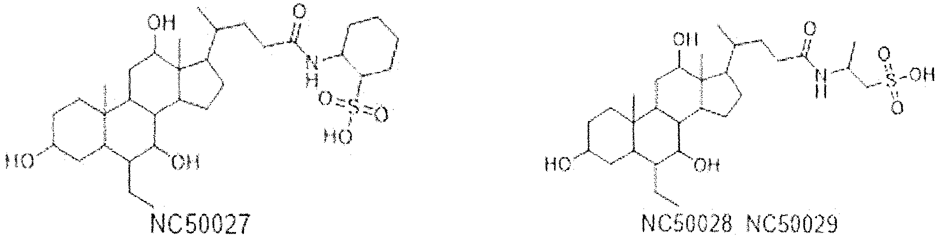

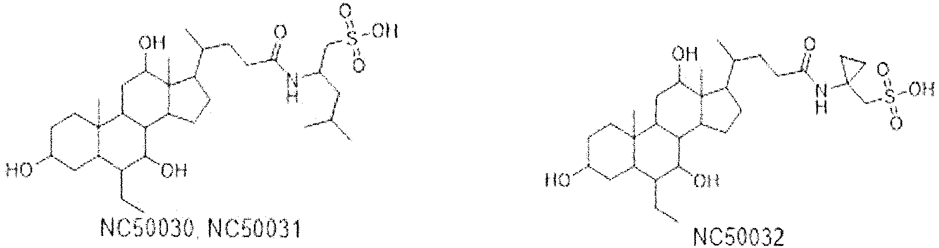

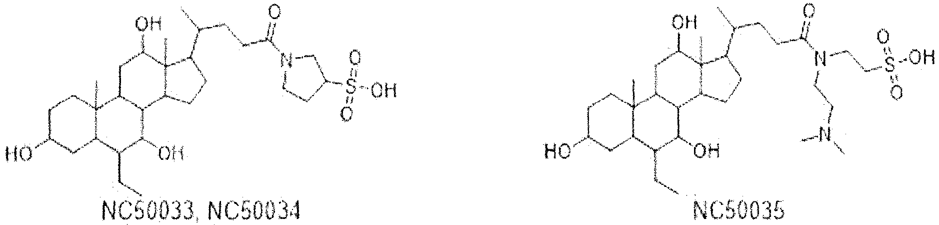

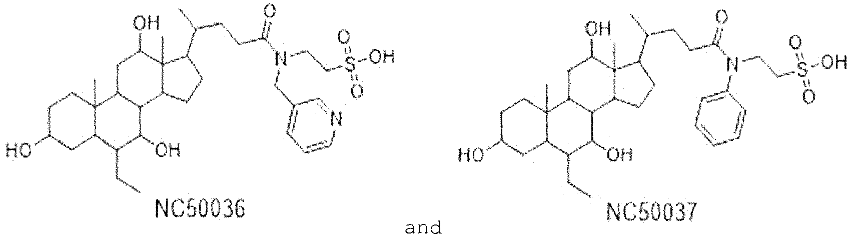

The present invention includes not only the compound represented by Formula 1, but also pharmaceutically acceptable salts thereof, and possible solvates, hydrates, racemates, or stereoisomers that can be prepared therefrom.

In one aspect of the invention, the compound represented by Formula 1 or a pharmaceutically acceptable salt thereof is any one selected from the group consisting of the following compounds or a pharmaceutically acceptable salt thereof. 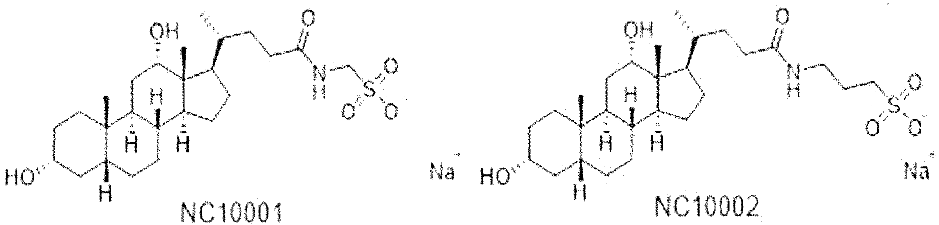 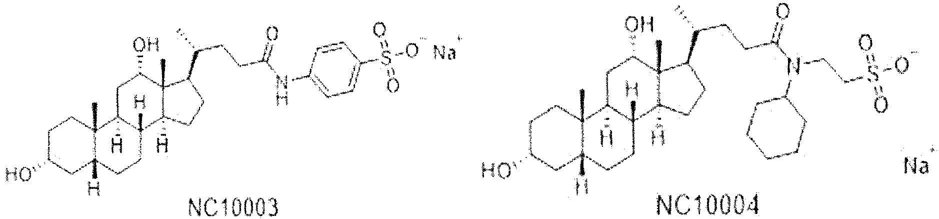 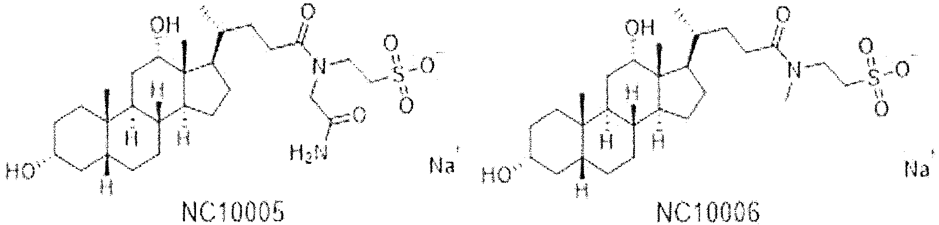 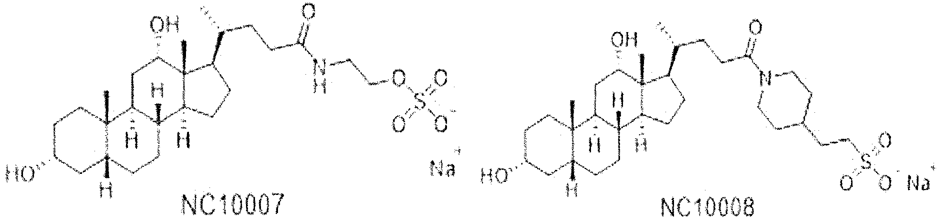 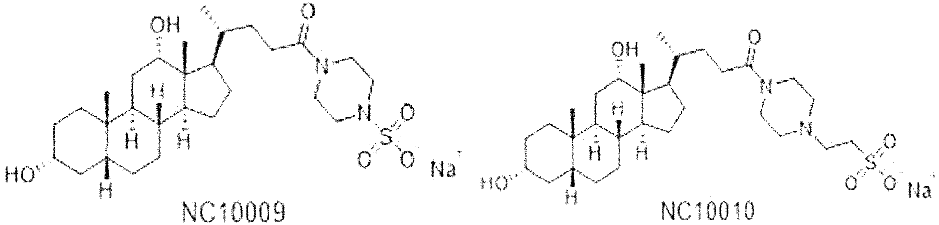 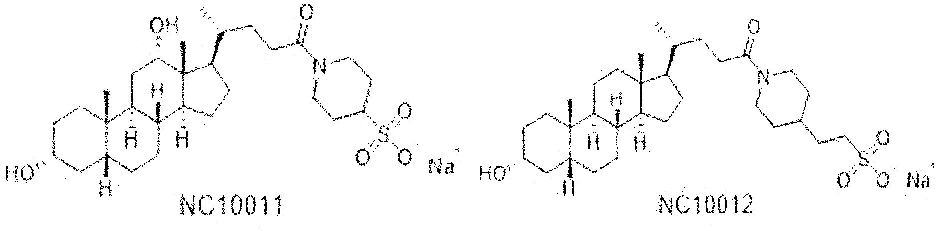 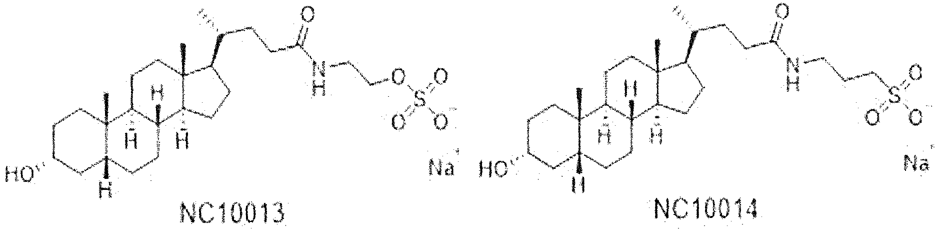 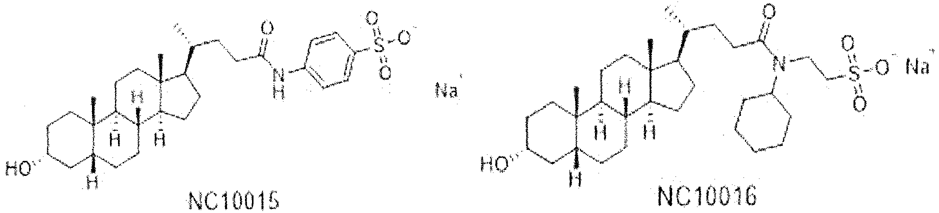 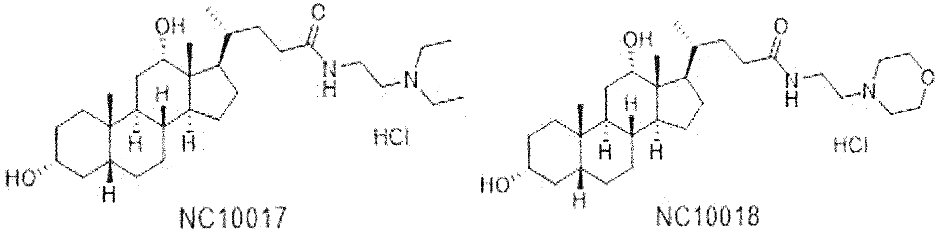 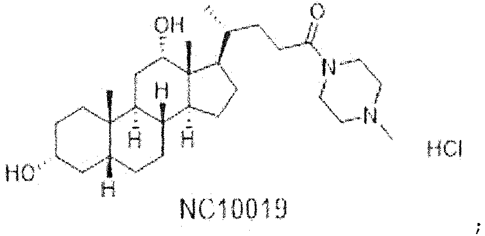 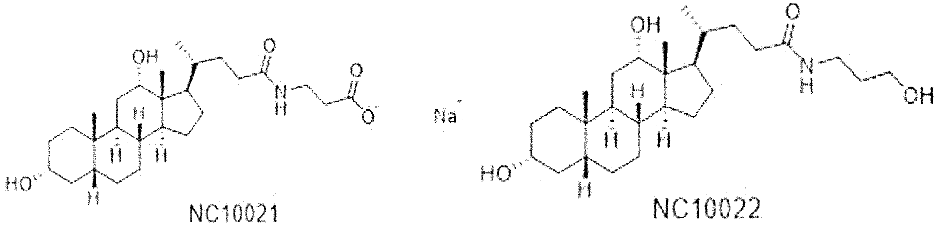 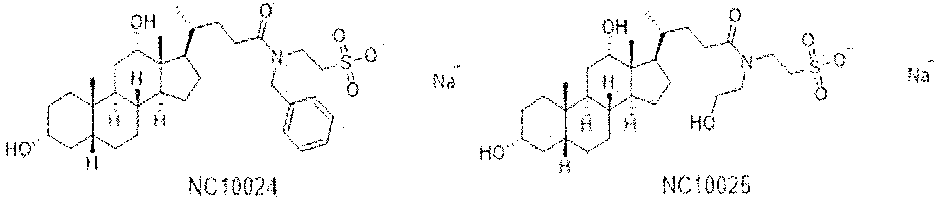 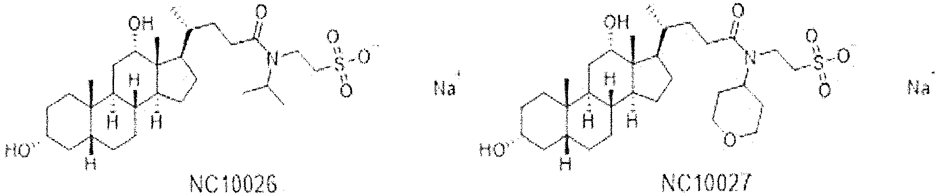 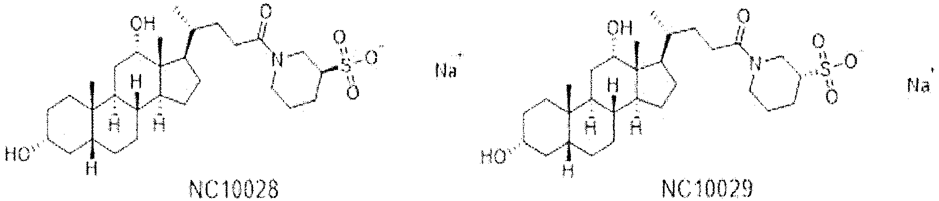 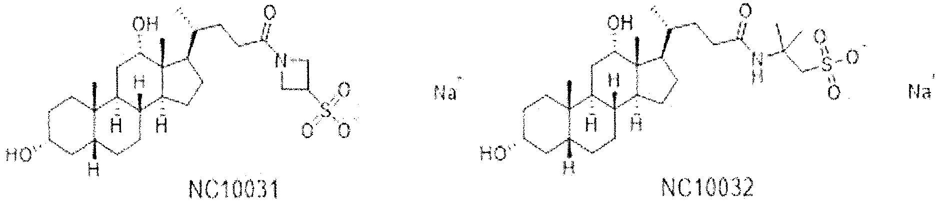 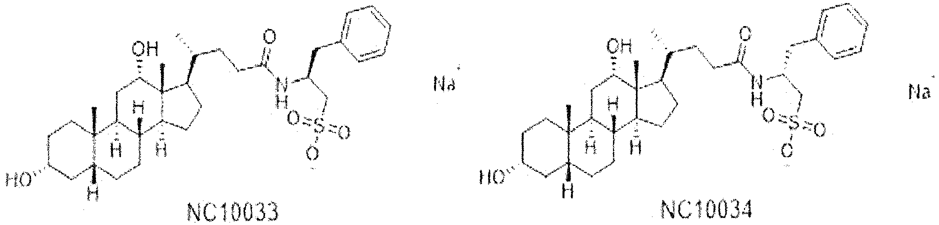 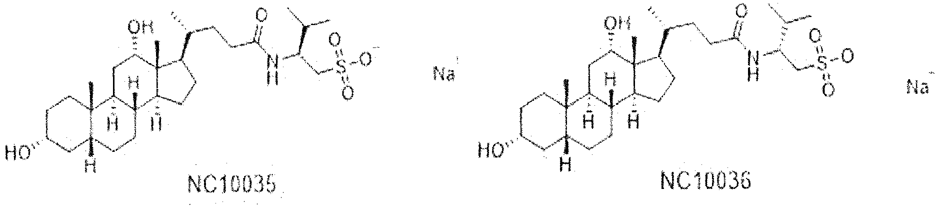 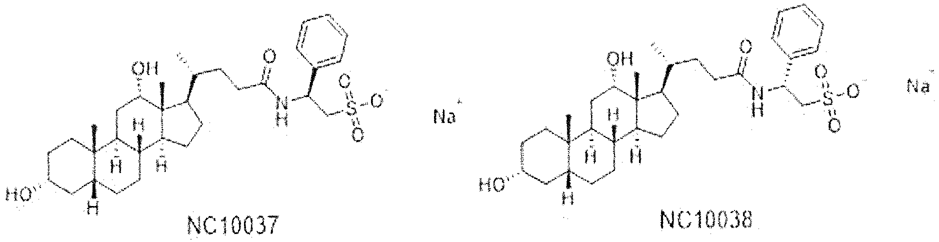 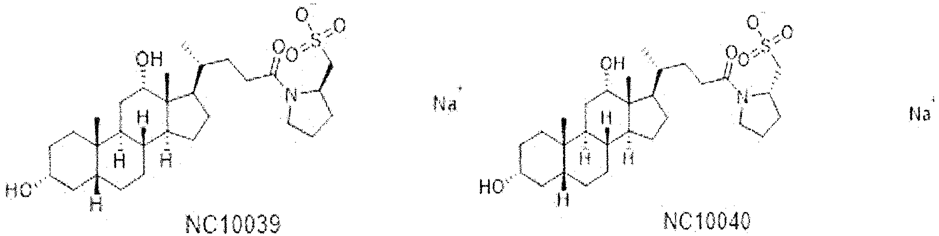 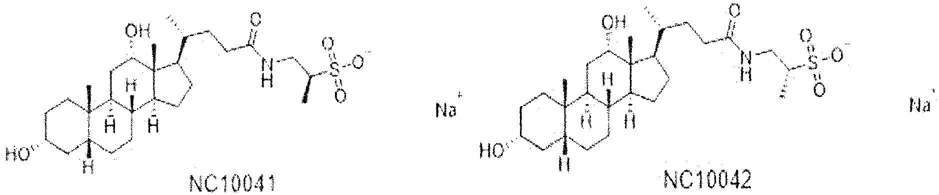 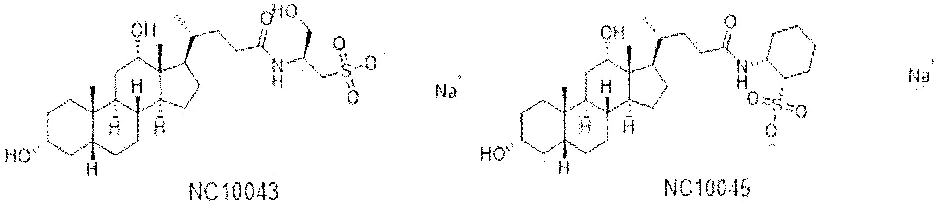 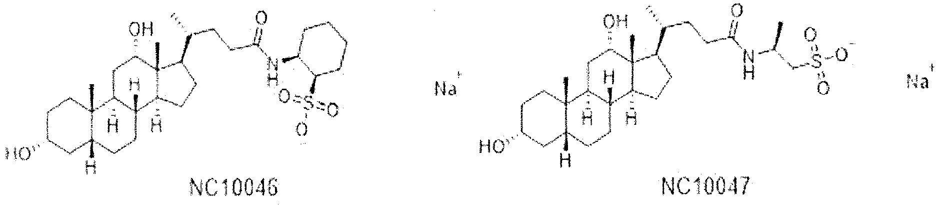 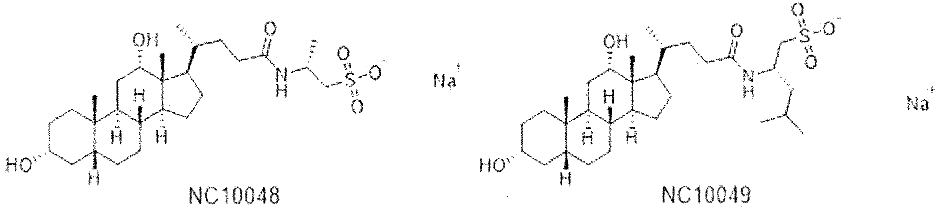 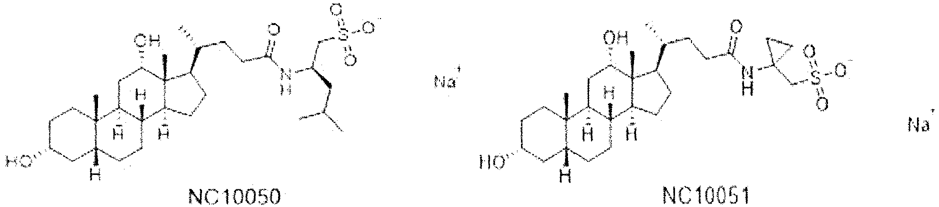 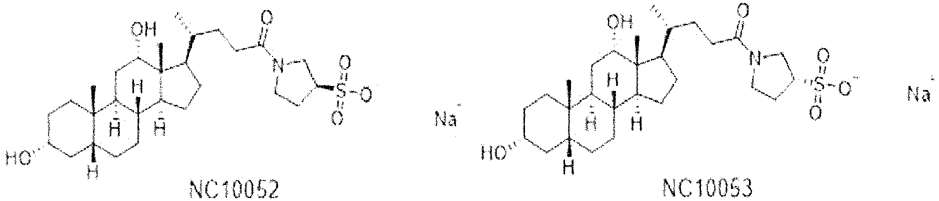 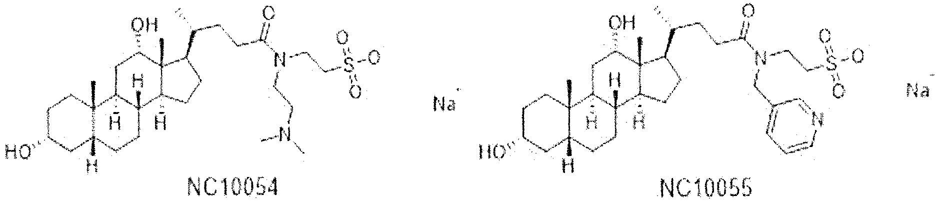 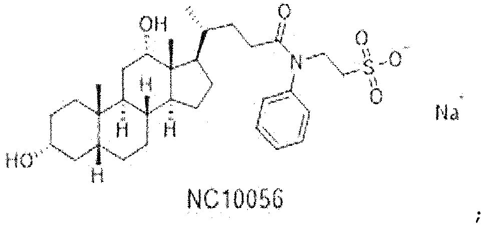 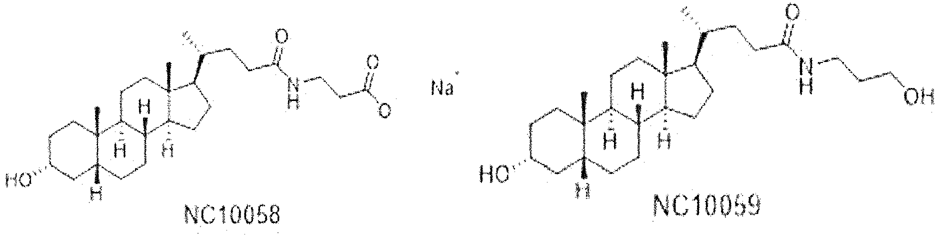 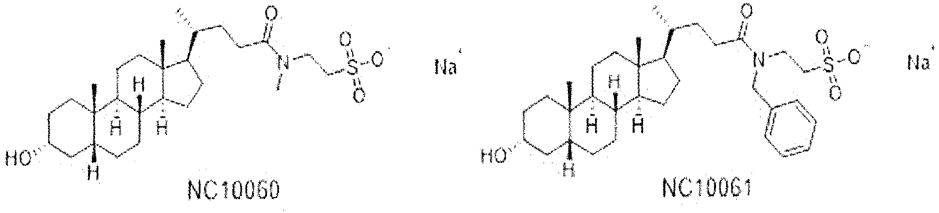 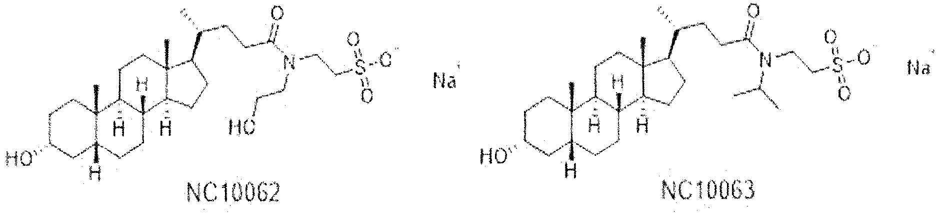 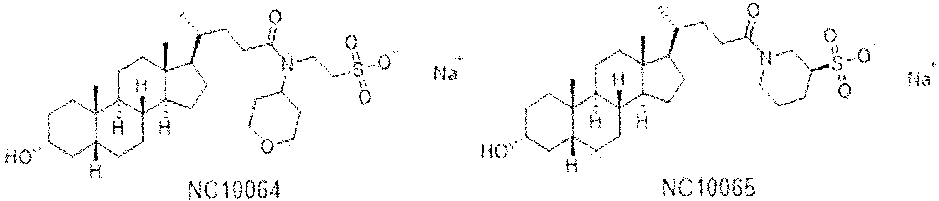 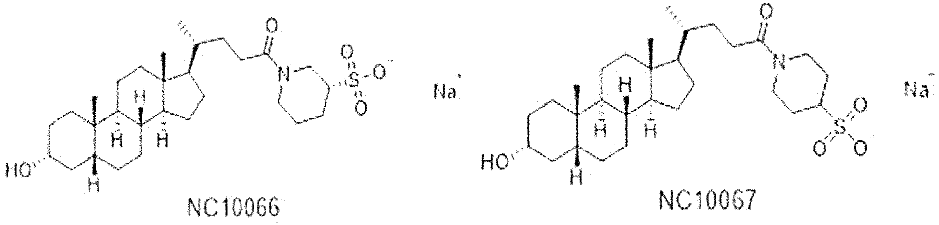 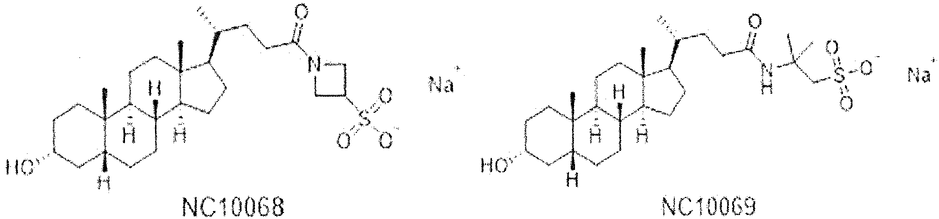 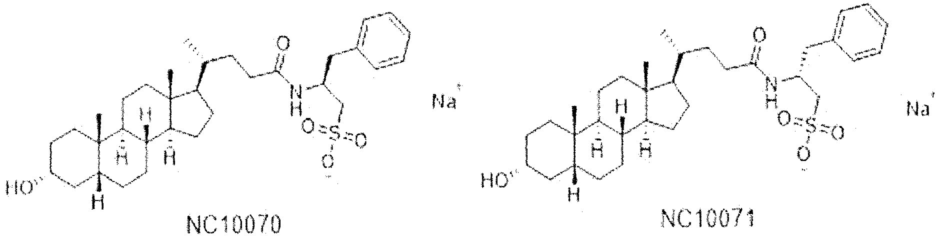 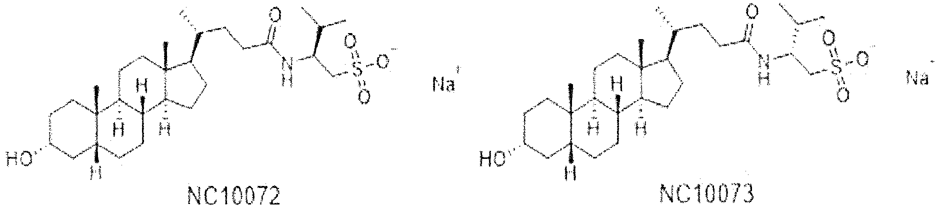 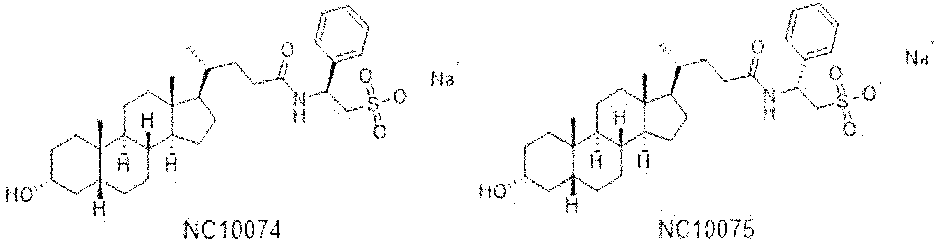 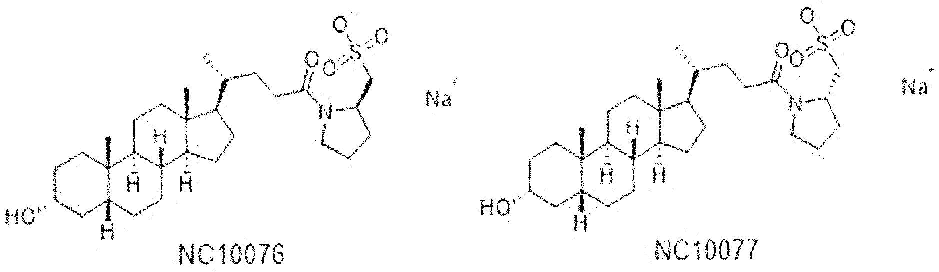 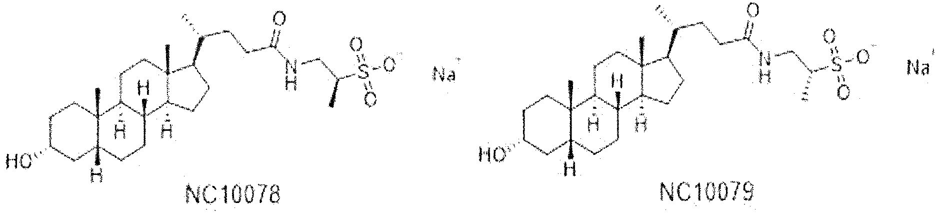 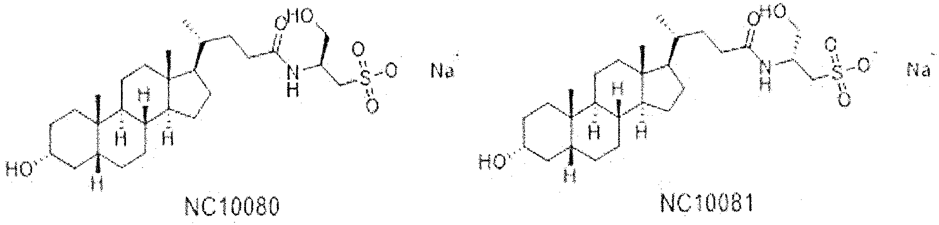 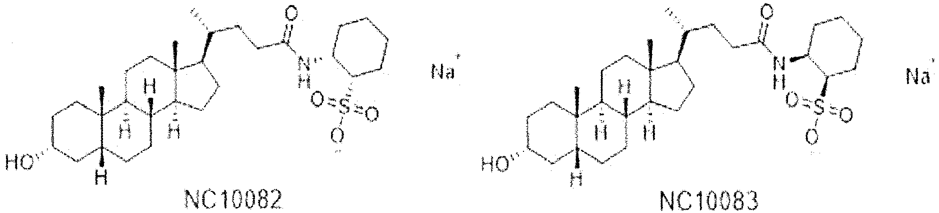 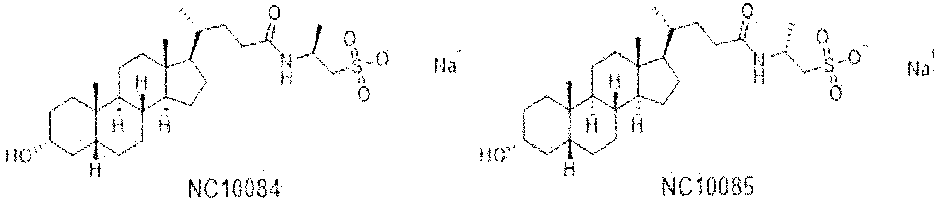 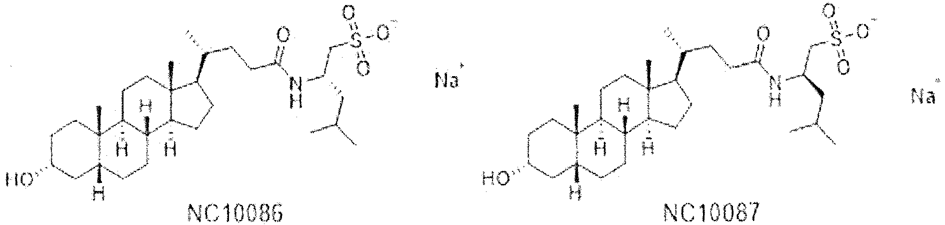 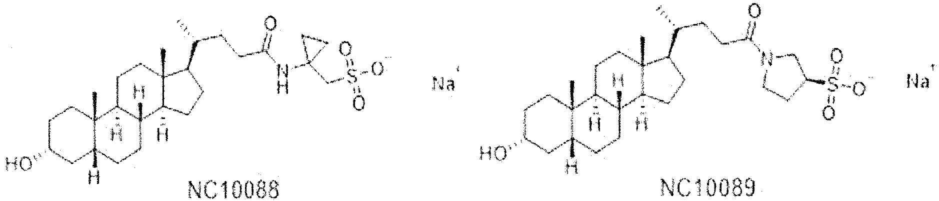 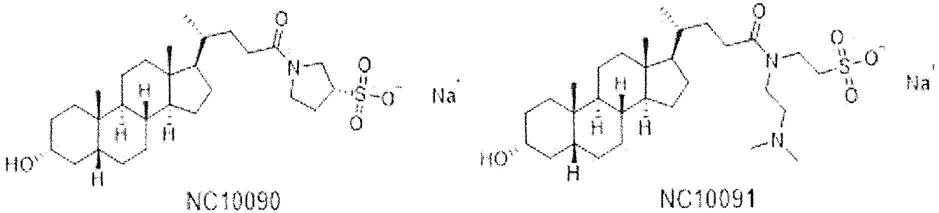 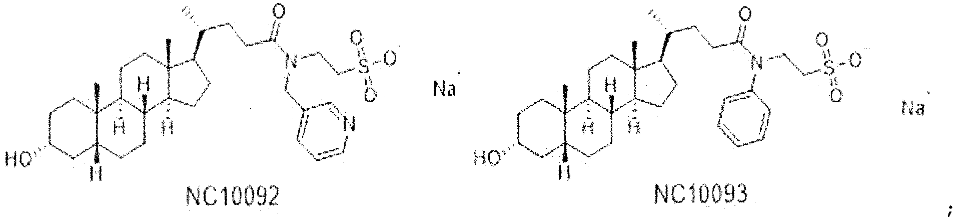 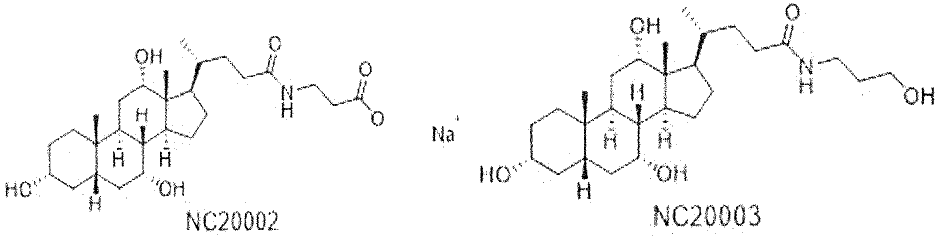 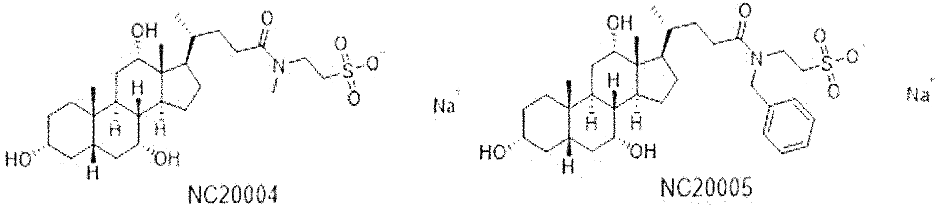 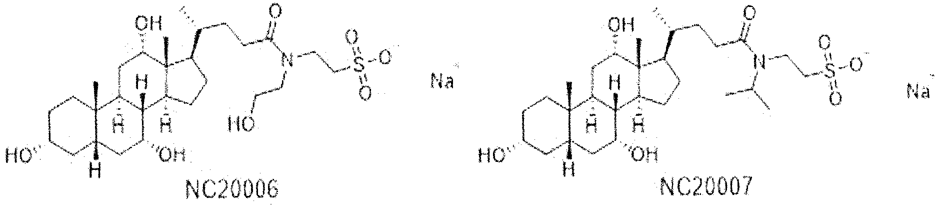 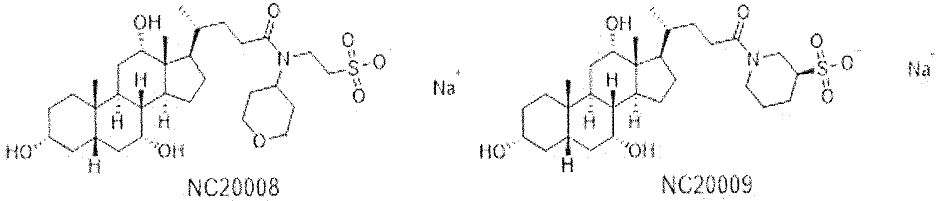 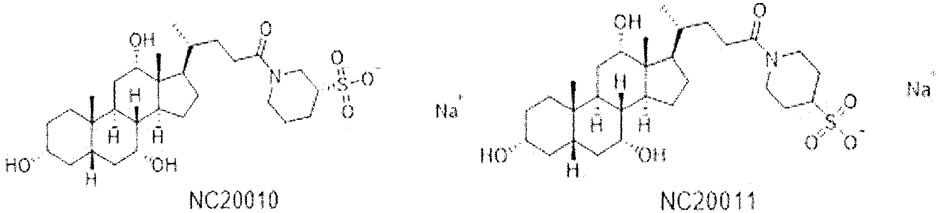 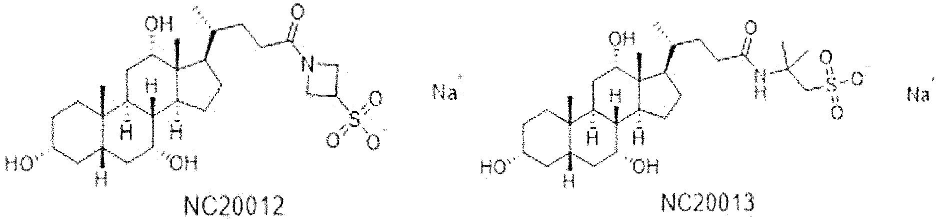 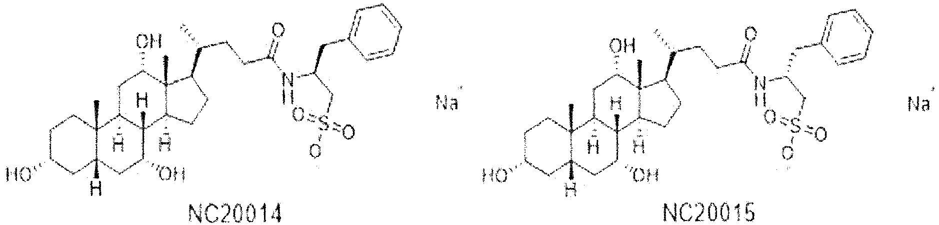 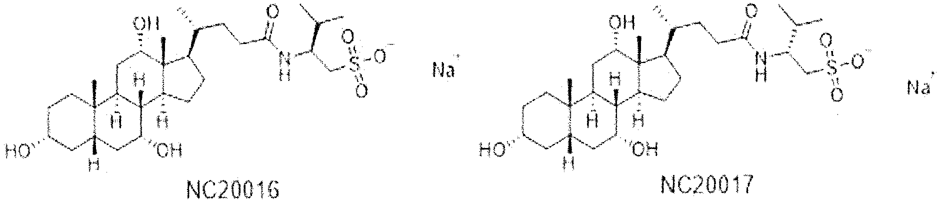 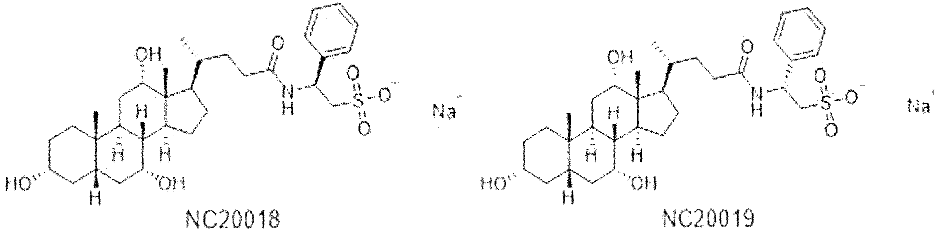 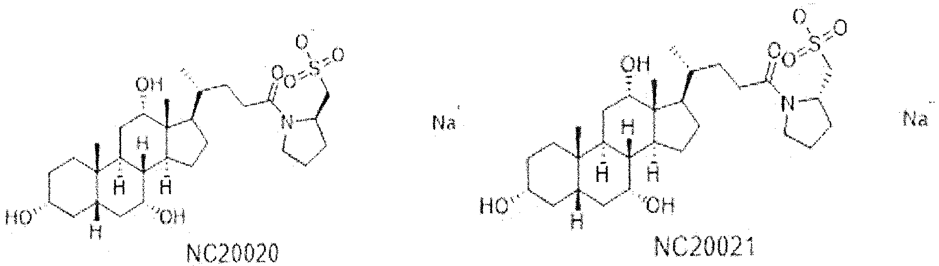 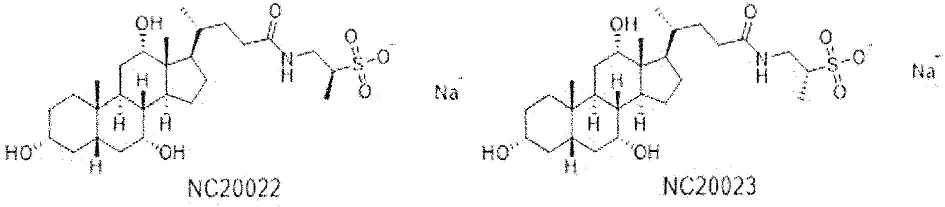 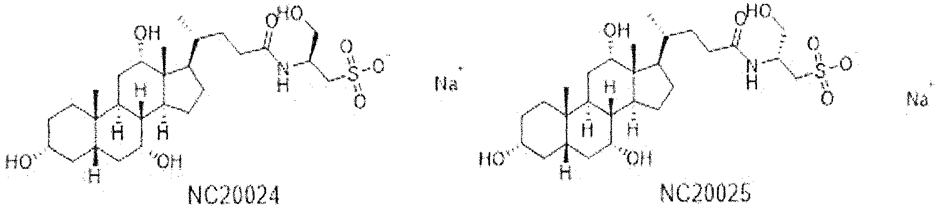 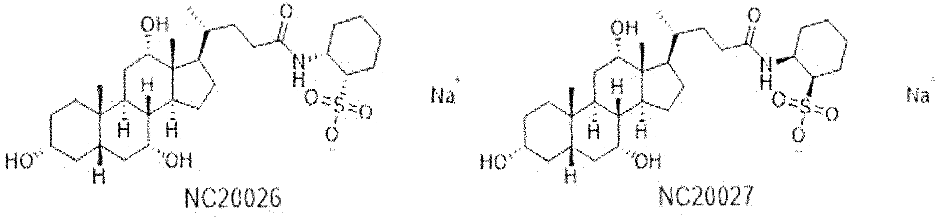 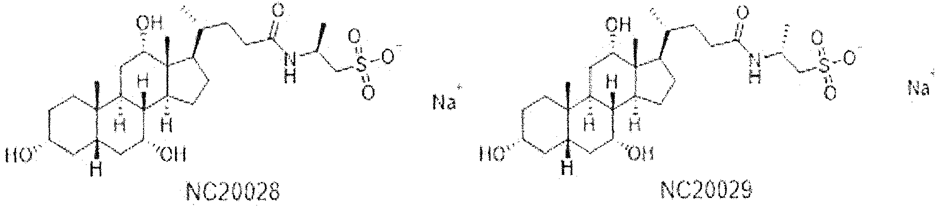 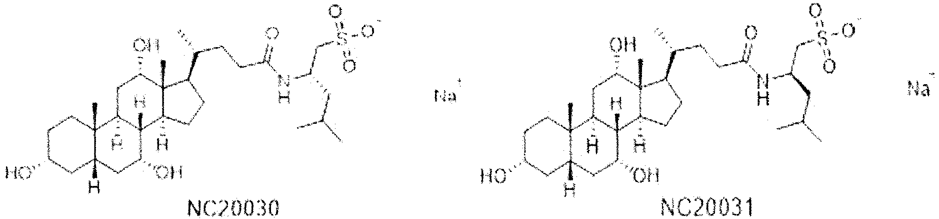 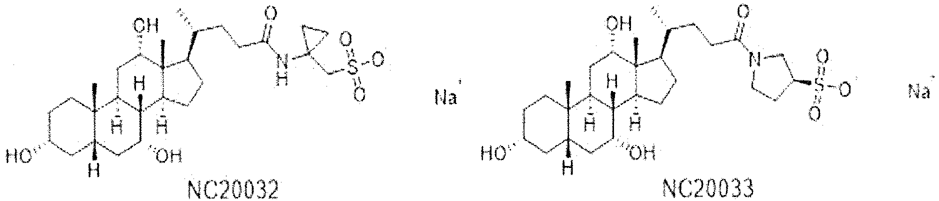 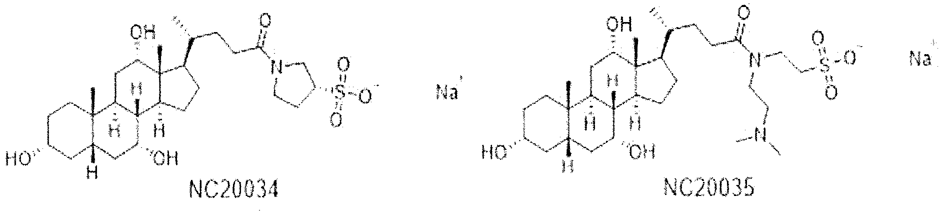 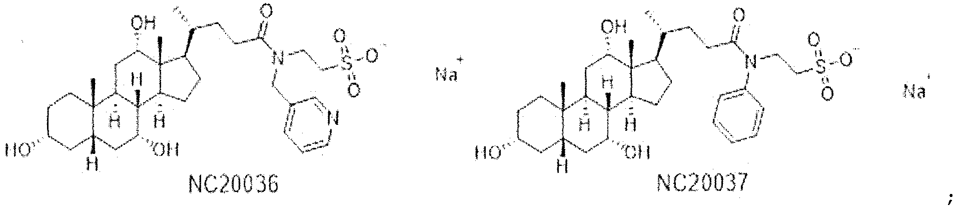 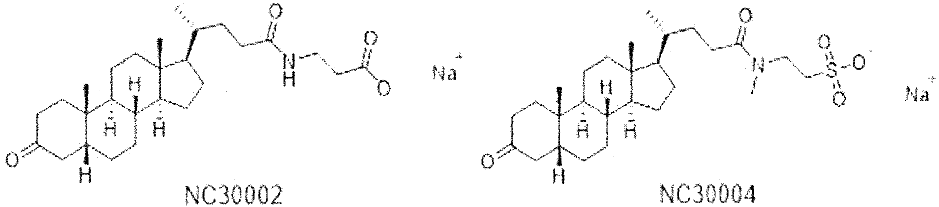 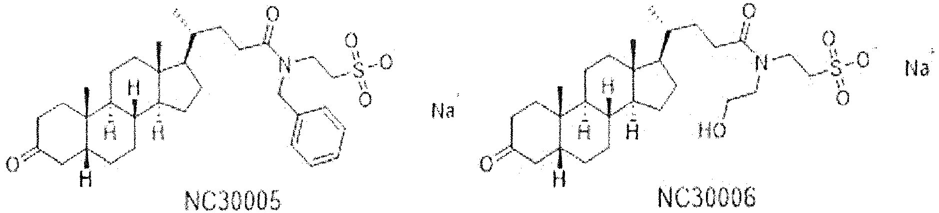 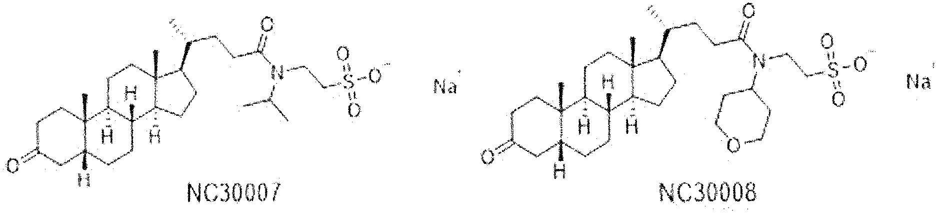 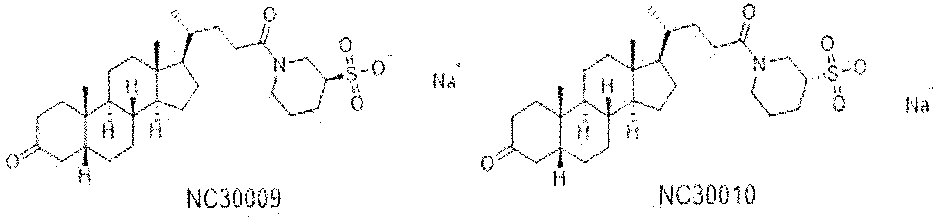 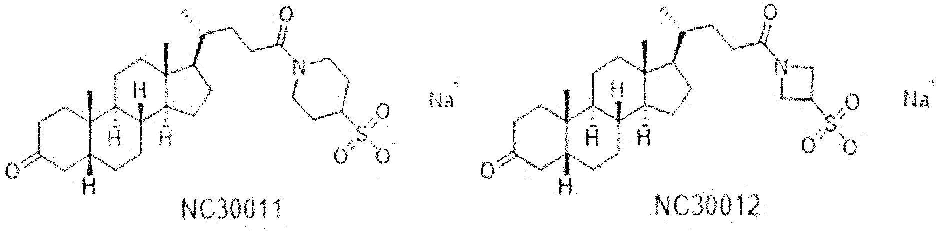 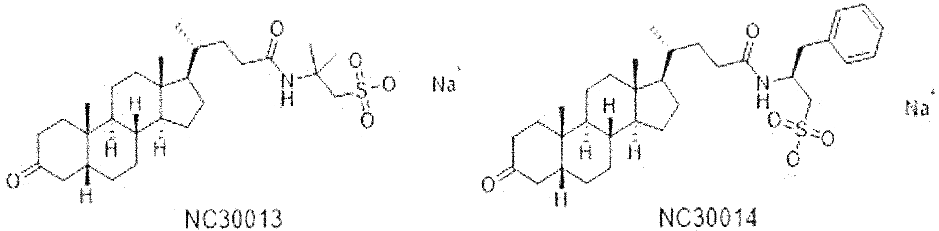 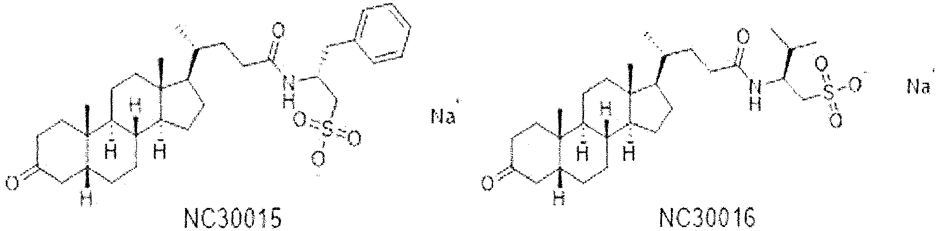 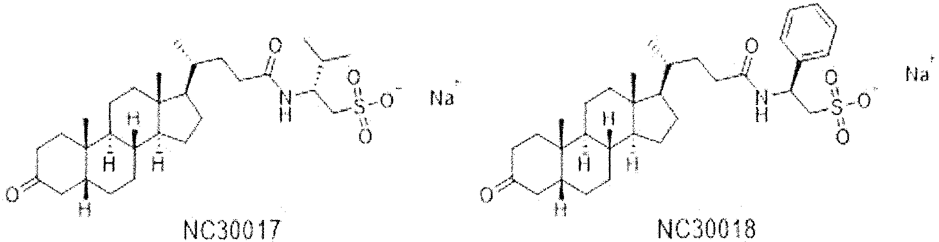 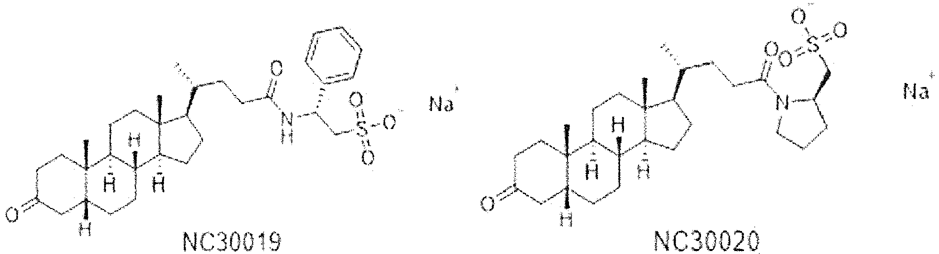 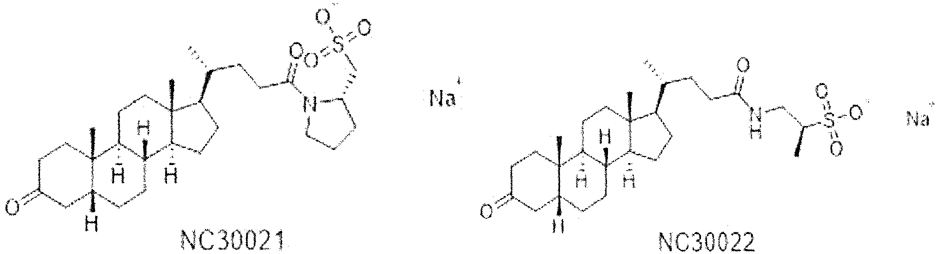 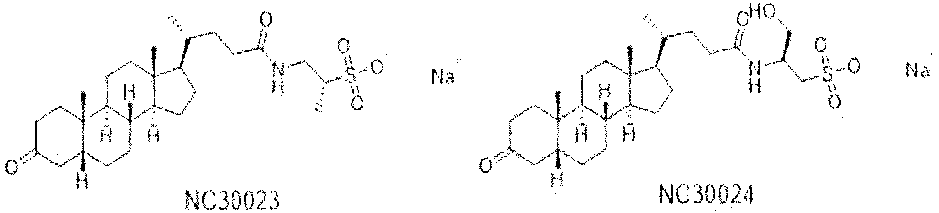 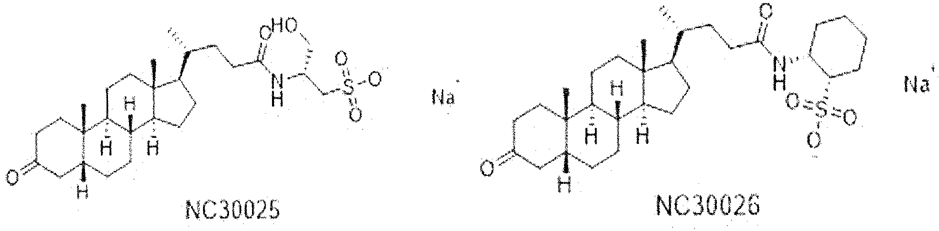 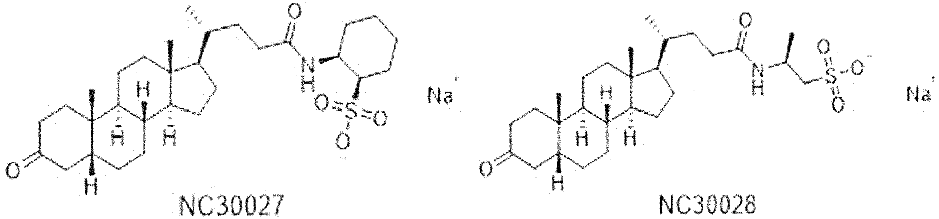 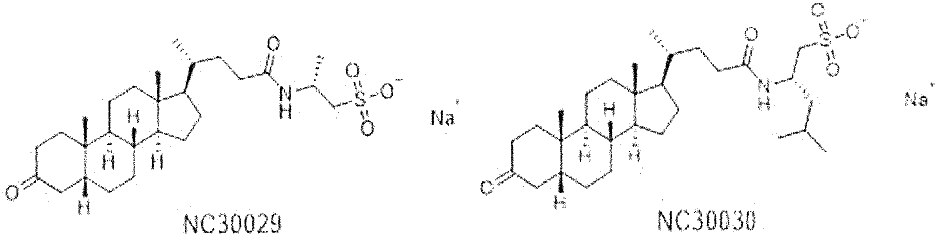 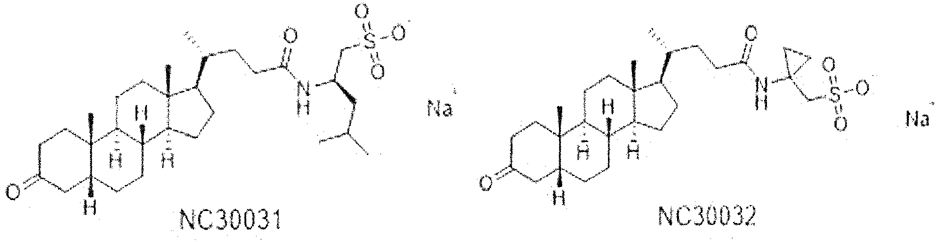 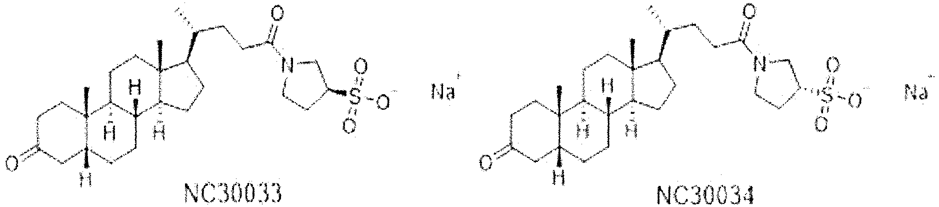 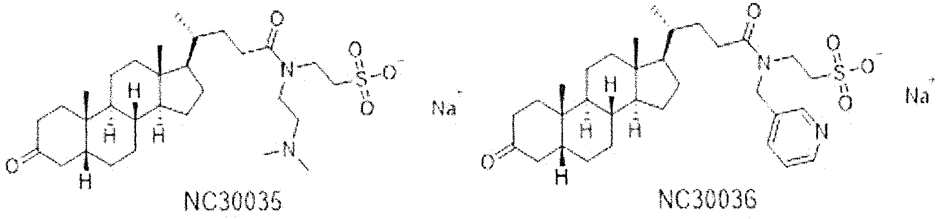 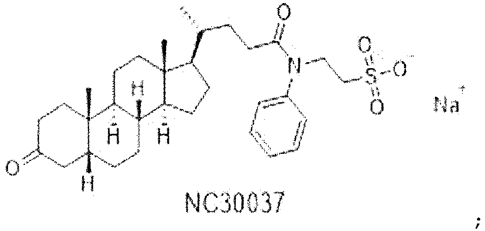 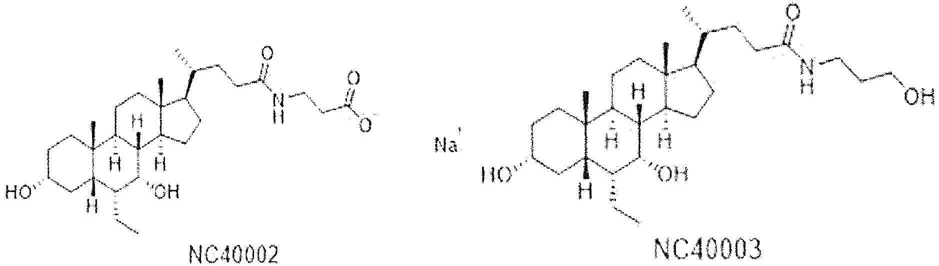 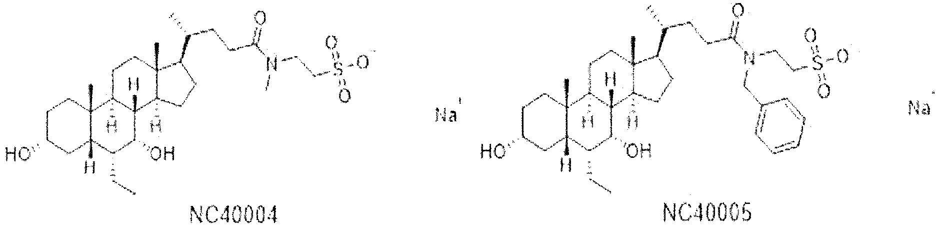 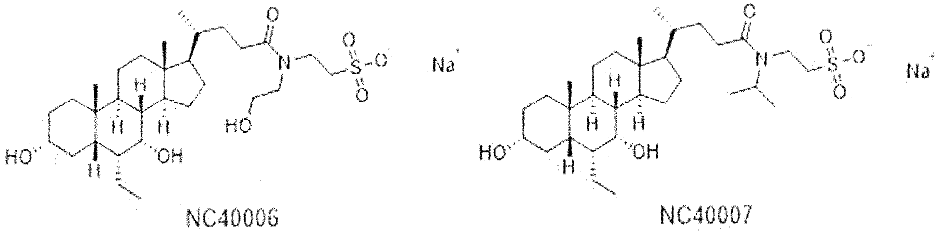 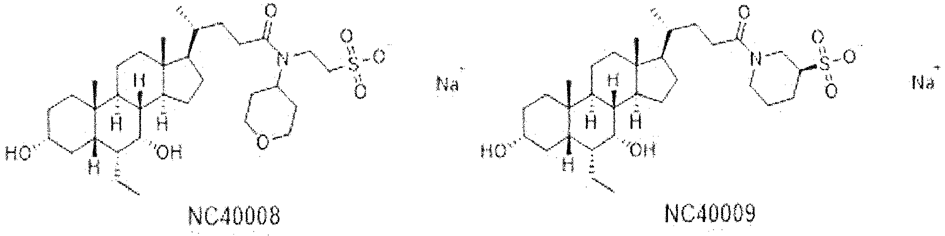 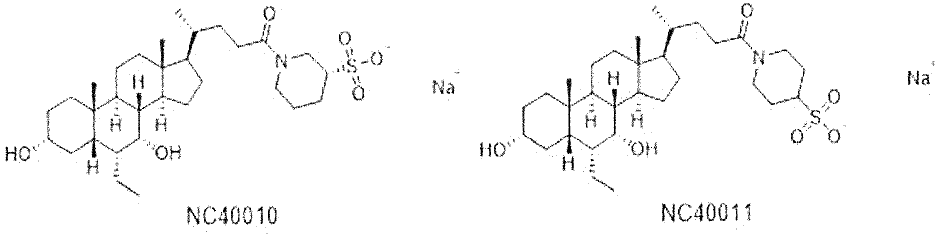 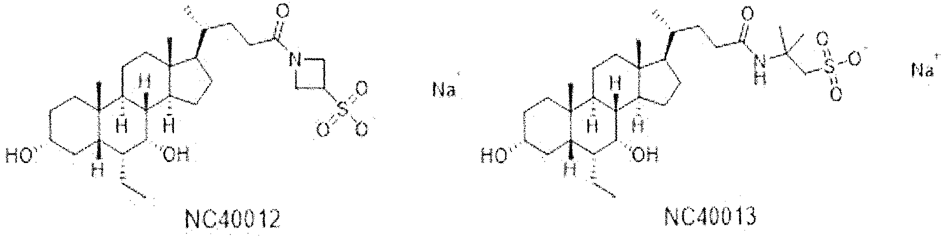 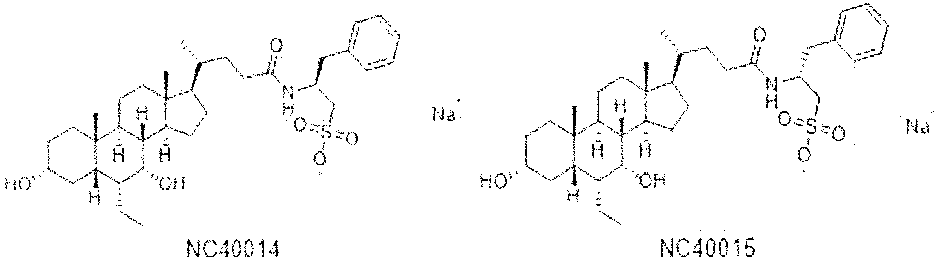 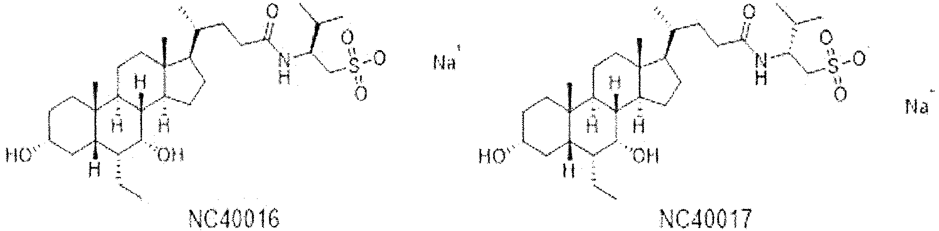 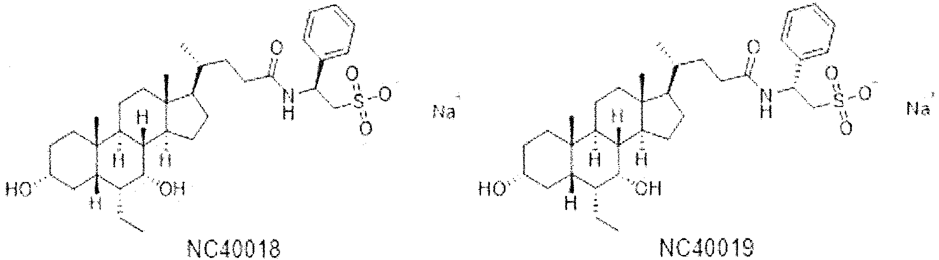 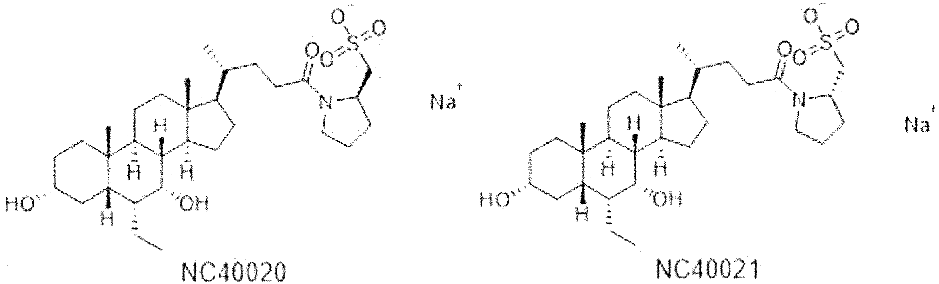 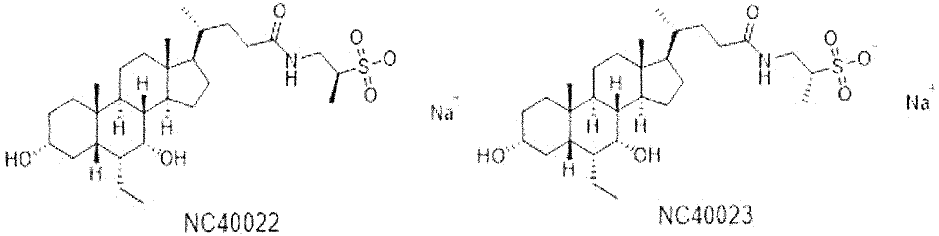 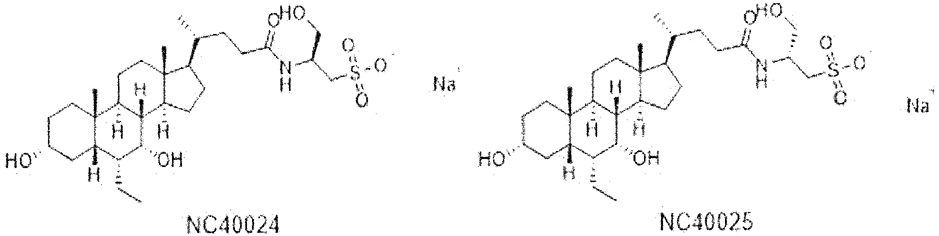 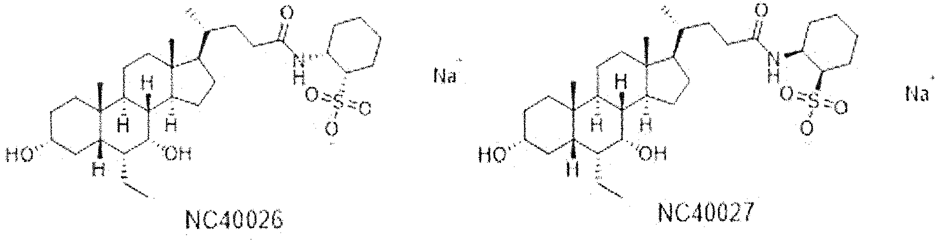 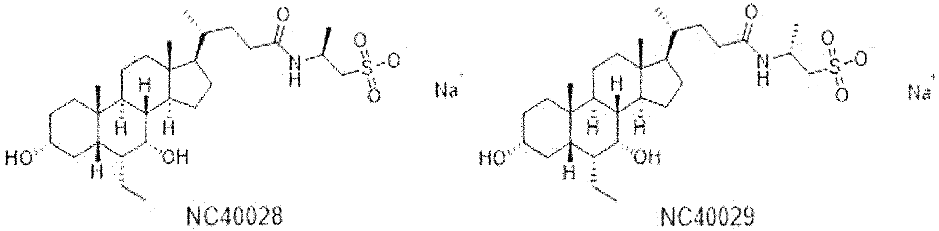 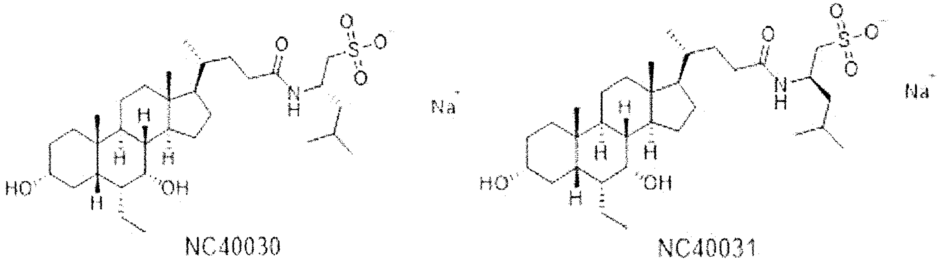 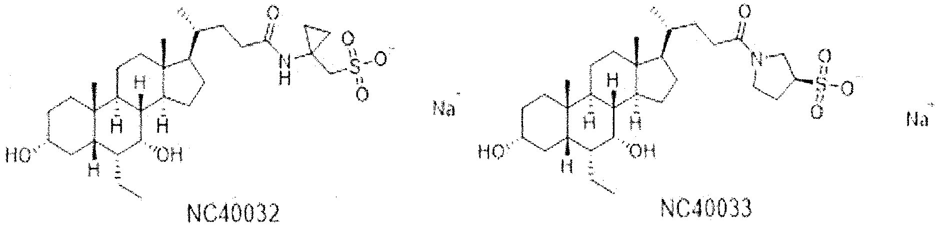 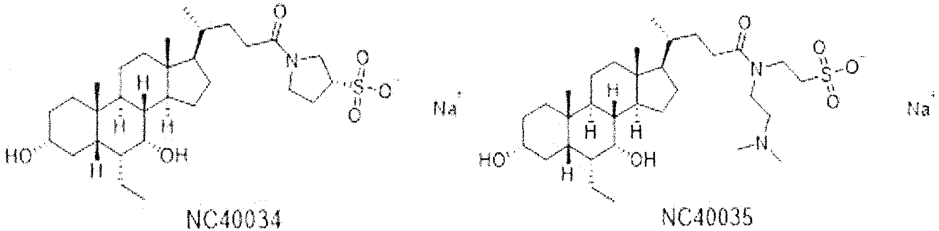 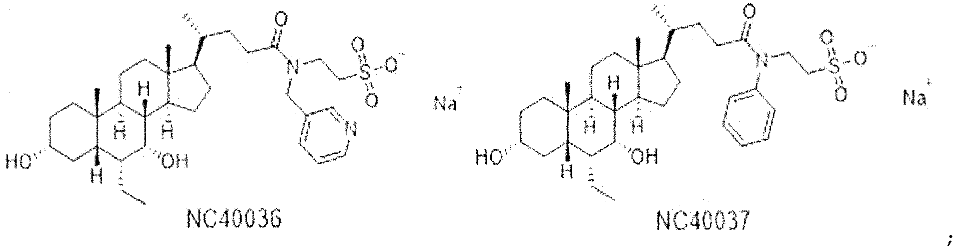 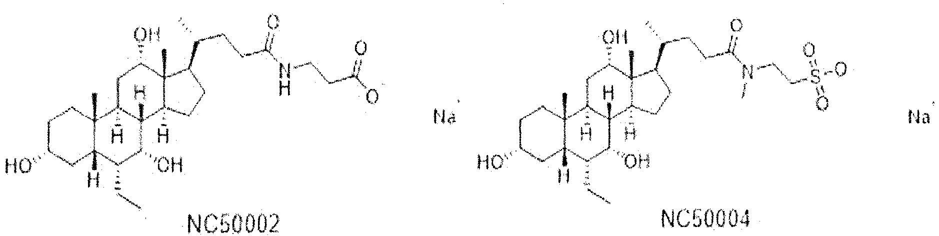 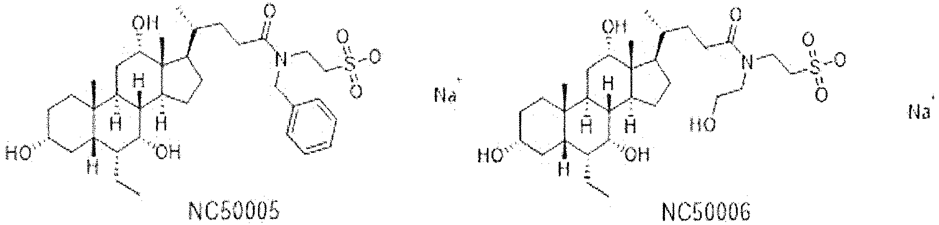 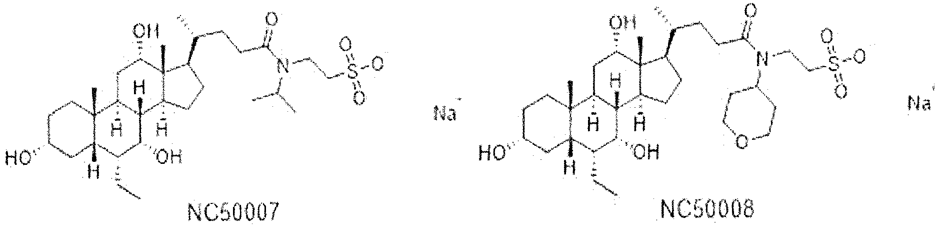 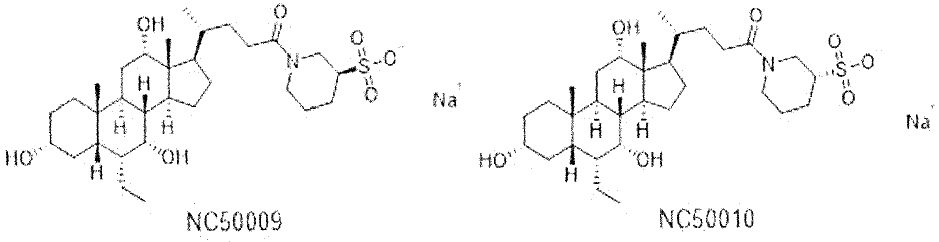 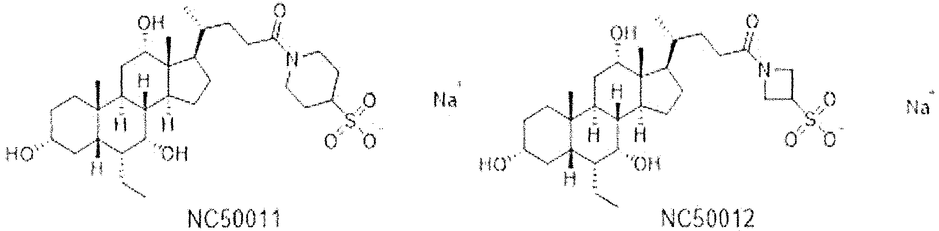 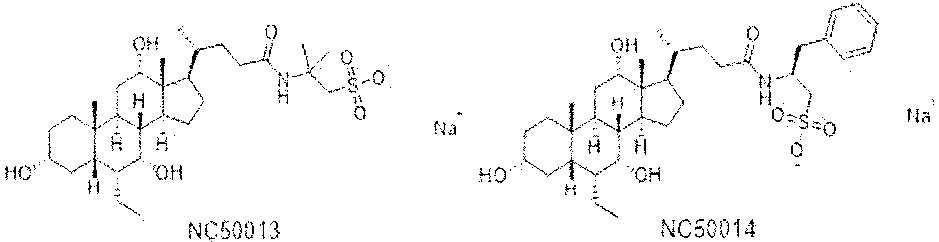 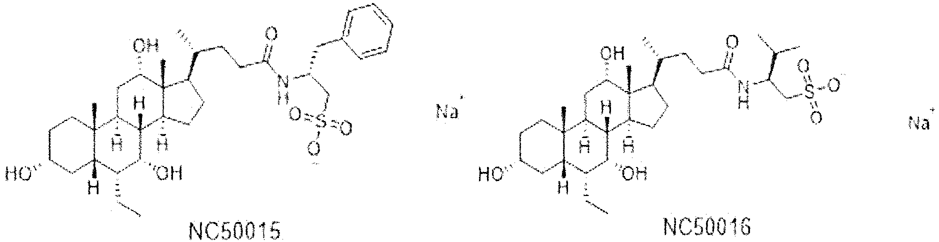 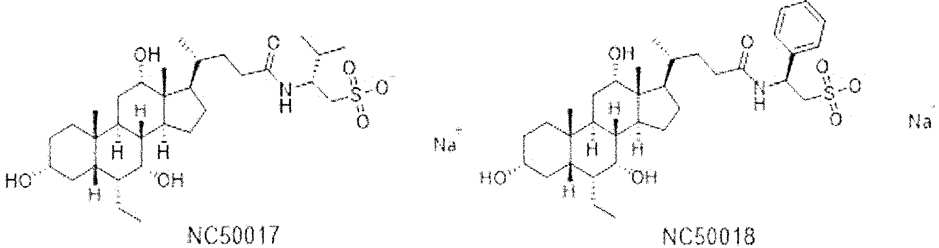 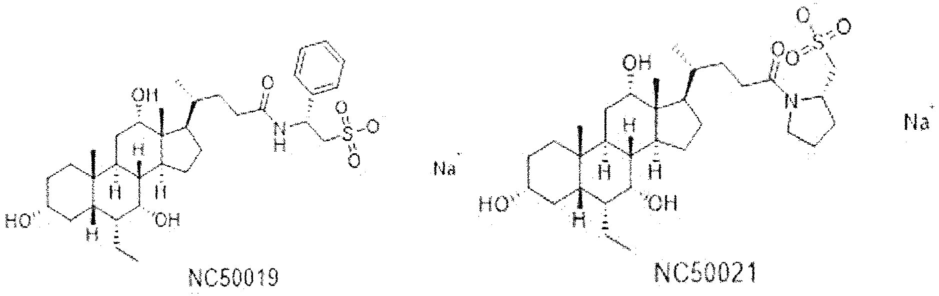 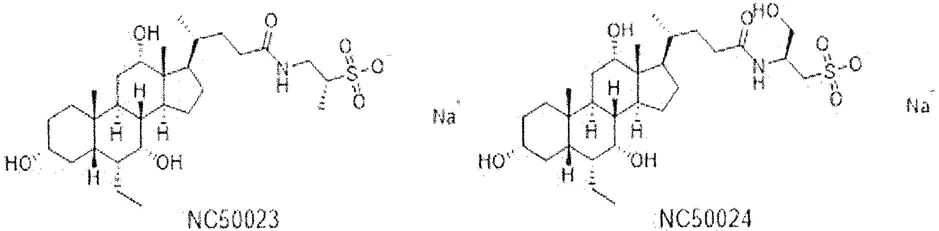 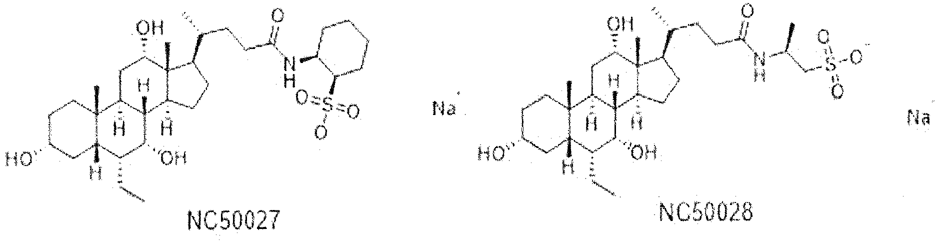 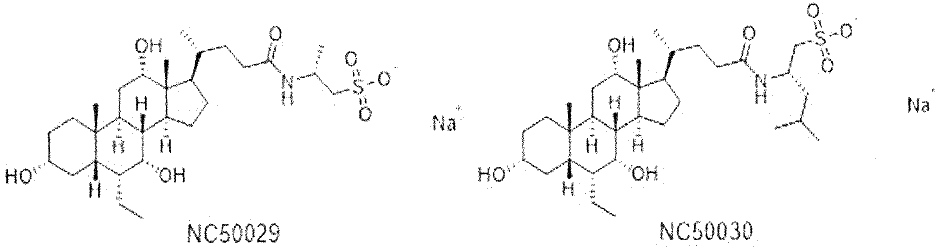 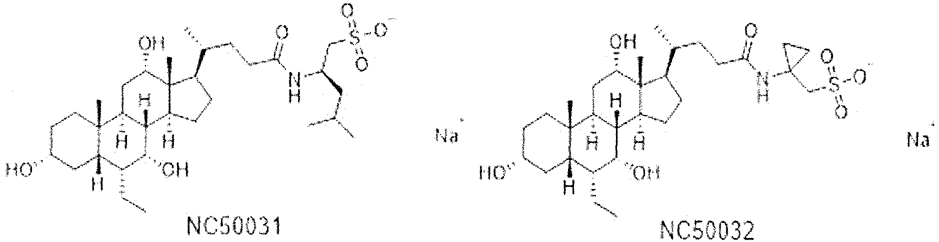 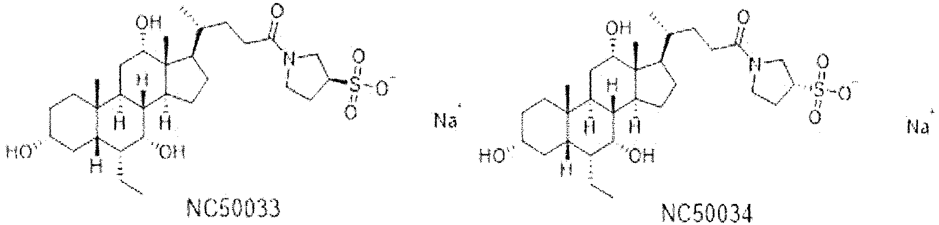 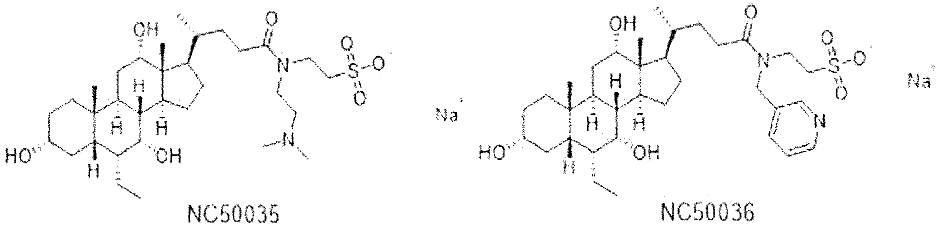 and 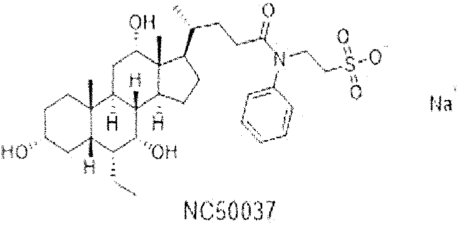 (wherein, Na⁺ can be replaced by other pharmaceutically acceptable salts)

The compound represented by Formula 1 of the present invention can be used in the form of a pharmaceutically acceptable salt, and an acid addition salt formed by a pharmaceutically acceptable free acid is useful as the salt. Acid addition salts are obtained from an inorganic acids such as hydrochloric acid, nitric acid, phosphoric acid, sulfuric acid, hydrobromic acid, hydroiodic acid, nitrous acid or phosphorous acid, aliphatic mono- and dicarboxylates, phenyl-substituted alkanoates, and hydroxy alkanoates and non-toxic organic acids such as alkanedioates, aromatic acids, aliphatic and aromatic sulfonic acids. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, and iodine, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitro benzoate, hydroxybenzoate, methylbenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate, mandelate, and the like.

The acid addition salt according to the present invention can be prepared by a conventional method, for example, by dissolving taurodeoxycholic acid represented by Formula 1 in an excessive amount of aqueous acid, and dissolving this salt cab be produced by precipitation using a water-miscible organic solvent, such as methanol, ethanol, acetone or acetonitril. Additionally, this mixture can be prepared by evaporating the solvent or excess acid and drying it, or by suction filtering the precipitated salt.

Additionally, a pharmaceutically acceptable metal salt can be prepared using a base. The alkali metal or alkaline earth metal salt is obtained, for example, by dissolving the compound in an excess of alkali metal hydroxide or alkaline earth metal hydroxide solution, filtering the insoluble compound salt, and evaporating and drying the filtrate. At this time, it is pharmaceutically appropriate to manufacture sodium, potassium or calcium salts as metal salts, and more specifically, it is suitable to manufacture sodium salts. Additionally, the corresponding silver salt is obtained by reacting an alkali metal or alkaline earth metal salt with an appropriate silver salt (e.g., silver nitrate).

In one specific aspect of the invention, salt may be selected from the group consisting of Sodium ,Hydrogen, Potassium, Magnesium, Calcium, Aluminum, Chloride, Hydrochloride, Sulfate, Carbonate, Nitrate, Phosphate, Ferrous salt, tartrate, Arginine, Benzathine, Chloroprocaine, Choline, Diethanolamine, thanolamine, Ethylenediamine, Histidine, Lithium, Lysine, Meglumine, Procaine, Triethylamine, Zinc, Acetate, Aspartate, Benzensulfonate, Benzoate, Besylate, Bicarbonate, Bitartrate, Bromide, Camsylate, Citrate, Decanoate, Edetate, Estolate, Esylate, Fumarate, Gluceptate, Gluconate, Glutamate, Glycolate, Glycollylarsanilate, Caproate, Hexanoate, Hexylresorcinate, Hydrabamine, Hydroxynaphthoate, Iodide, Isethionate, Lactate, Lactobionate, Malate, Maleate, Mandelate, Mesylate, Methylbromide, Methylnitrate, Methylsulfate, Mucate, Napsylate, Octanoate, Oleate, Pamoate, Pantothenate, Polygalacturonate, Propionate, Salicylate, Stearate, Subacetate, Succinate, Teoclate, Tosylate and Triethiodide. In one specific aspect of the invention, the pharmaceutically acceptable salt may be Sodium salt.

In one aspect of the invention, the compound or a pharmaceutically acceptable salt thereof may additionally include amino acids, peptides, saccharides, polysaccharides, or one or more conjugates thereof.

Additionally, the present invention provides a pharmaceutical composition containing the above compound or a pharmaceutically acceptable salt thereof.

Additionally, the present invention provides a pharmaceutical composition for treating inflammatory diseases containing the above compound or a pharmaceutically acceptable salt thereof.

The present invention also relates to a treatment method of administering the composition to a subject.

Additionally, the present invention relates to a pharmaceutical composition for treating inflammatory diseases by administering the composition to a subject.

In one aspect of the invention, the pharmaceutical composition may further include pharmaceutically acceptable ingredients. Examples of pharmaceutically acceptable ingredients may further include ARB(Angiotensin Receptor Blocker)-a compositions for the treatment of essential hypertension, ACEI(Angiotensin Converting Enzyme Inhibitor), CCB(Calcium Channel Blocker), BB(Beta Blocker), Diuretics, Biguanides-a composition for treating type 2 diabetes, SU(Sulfonyl Urea), Meglitinides, TZD(Thiazolidinedione), DPP-4i(Dipeptidyl Peptidase-4 inhibitor), SGLT-2i(Sodium Glucose Co-Transporter 2 inhibitor, GLP-1RA(Glucagon Like Peptide 1 Receptor Agonist), Acetaminophens-a composition for treating acute bronchitis, NSAIDs(Non-Steroidal Anti-Inflammatory Drugs), Penicilins, Cephalosporins, Monobactams, Carbapenems, Polymyxins, Aminoglycosides, Macrolides, Fluoroquinolones, Glycopeptides, HMG-CoA reductase inhibitors(β-hydroxy β-methylglutaryl-CoA reductase inhibitor)-a composition for treating dyslipidemia, PCSK9 inhibitors(Proprotein Convertase Subtilisin/Kexin type9 inhibitor), Fibrates, Resins, Omega-3, PPI(Proton Pump Inhibitor)-a composition for treating GERD, H₁-antagonist, H₂-antagonist-a composition for treating allergic rhinitis, Corticosteroids, LTRA(Leukotriene receptor antagonist), α₂-agonist, mast cell stabilizer, Anticholinergic agents, Cholinesterase inhibitor-a composition for treating dementia, NMDA receptor antagonist, antipsychotic agents, antidepressant, Acetaminophen-a commposition for treating osteoarthritis, NSAIDs, Tramadol, 5-HT₃ agonist-a composition for treating migraine, Ergot alkaloids.

The route of administration of the compound of the present invention may be, for example, oral or parenteral route. Here, parenteral refers to a broad route of administration, for example, intravenous, intraarterial, intraperitoneal, intramuscular, subcutaneous, intranasal, sublingual, intrathecal, inhalation, ocular, rectal, vaginal, intraventricular administration, and the like.

When formulating the composition, it is prepared using diluents or excipients such as commonly used fillers, extenders, binders, wetting agents, disintegrants, and surfactants.

Solid preparations for oral administration include tablets, pills, powders, granules, capsules, troches, and the like, and these solid preparations is prepared by mixing one or more compounds according to the present invention with at least one or more excipients, such as starch, calcium carbonate, sucrose, lactose, or gelatin. Additionally, in addition to simple excipients, lubricants such as magnesium styrate, talc are also used. Liquid preparations for oral administration include suspensions, oral solutions, emulsions, or syrups, and in addition to the commonly used simple diluents such as water and liquid paraffin, they can contain various excipients such as wetting agents, sweeteners, fragrances, preservatives, and the like.

Preparations for parenteral administration include sterilized aqueous solutions, non-aqueous solutions, suspensions, emulsions, freeze-dried preparations, suppositories, and the like.

Non-aqueous solvents and suspensions may include propylene glycol, polyethylene glycol, vegetable oil such as olive oil, and injectable ester such as ethyl oleate, and the like. As a base for suppositories, witepsol, macrogol, tween 61, cacao, laurel, glycerol, gelatin, and the like, can be used.

The composition according to the present invention is administered in a pharmaceutically effective amount. In the present invention, "pharmaceutically effective amount" means an amount sufficient to treat the disease with a reasonable benefit/risk ratio applicable to medical treatment, and the effective dose level can be determined by the type, severity, and activity of the patient's disease, sensitivity to the drug, time of administration, route of administration and excretion rate, duration of treatment, drugs used simultaneously, and other factors well known in the field of medicine. The composition of the present invention can be administered as an individual treatment or in combination with other treatments, and can be administered sequentially or simultaneously with conventional treatments, and can be administered singly or multiple times. Considering all of the above factors, it is important to administer an amount that can achieve maximum effect with the minimum amount without side effects, and this can be easily determined by a person skilled in the art.

Specifically, the effective amount of the compound according to the present invention may vary depending on the patient's age, gender, and weight, and is generally 0.01 mg to 100 mg per kg of body weight, more specifically 0.1 mg to 15 mg per kg of body weight daily or every other day, or divided into 1 to 3 doses per day. However, since it may increase or decrease depending on the route of administration, severity, gender, weight, age, and the like, the above dosage does not limit the scope of the present invention in any way.

In the present invention, for preparing the compound of Formula 1, bile acids or derivatives thereof can be used freely, whether isolated from animal carcasses, such as sheep, dogs, goats or rabbits, commercially available, or synthesized.

Hereinafter, the present invention is described in detail through examples and experimental examples.

However, the following examples and experimental examples are merely illustrative of the present invention, and the content of the present invention is not limited to the following examples and experimental examples.

### <Example 1> Method for producing taurine derivatives

### <Example 1-1> Preparation of taurine derivative (1)

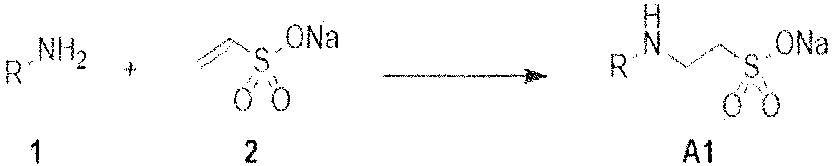

Amine (1) was added to 2.25 mL (5.0 mmol) of vinylsulfonic acid sodium salt (2) solution (25 wt%, in H₂O), and the resulting mixture was stirred at 100°C for 48 hours. After cooling, the reaction mixture was concentrated under reduced pressure and coevaporated with acetonitrile to obtain sodium sulfonate compound (**A1**).

### <Example 1-2> Preparation of taurine derivative (2)

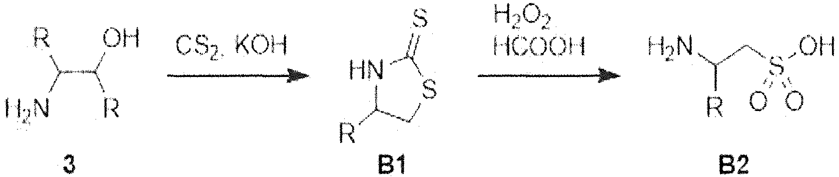

2.1 mL (35.0 mmol) of carbon disulfide in a mixture of 7.0 mmol of 2-aminoethan-1-ol (3) and 35.0 mL (35.0 mmol) of potassium hydroxide (1M in H₂O) was added and the resulting mixture was heated at 100°C for 16 hours. After cooling to room temperature, the mixture was extracted with dichloromethane (3 × 20 mL). The combined organic layer was dried with sodium sulfate, and the solvent was completely removed under reduced pressure to obtain thiazolidine-2-thione compound **(B1).**

7.0 mL (68.5 mmol) of hydrogen peroxide solution (30%) was dissolved in 35.0 mL (928 mmol) of formic acid at 0°C, and stirred at room temperature for 1 hour to obtain peroxyformic acid. The peroxyformic acid solution was cooled back to 0°C, 7.0 mmol of the thiazolidine-2-thione compound was added little by little, the resulting solution was stirred, and heated to room temperature. After 18 hours, the mixture was concentrated under reduced pressure and co-evaporated with acetonitrile. The obtained raw material was crystallized with ethanol and diethyl ether at 4°C to obtain pure sulfonic acid **(B2).**

### <Example 1-3> Preparation of taurine derivatives (3)

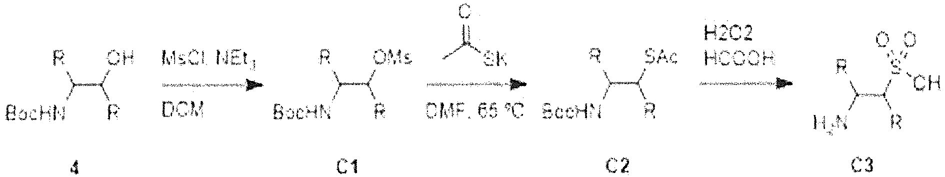

2.08 mL (15.0 mmol) of triethylamine and 35 mL of dichloromethane containing 10.0 mmol of tert-butyl(2-hydroxyethyl)carbamate (4) was added to the solution. The mixed solution was cooled to 0°C, and 0.93 ml (12.0 mmol) of mesyl chloride was added dropwise. The resulting mixture was stirred for 2 hours while slowly heating to room temperature. Saturated sodium bicarbonate was added to separate the layers, and the aqueous layer was extracted with dichloromethane (2 × 30 mL). The combined organic layer was washed with brine, dried over sodium sulfate, filtered, and concentrated under reduced pressure to obtain 2-((tert-butoxycarbonyl)amino)ethyl methane sulfonate**(C1)** was obtained.

Prepared by dissolving 2.28 g (20.0 mmol) of potassium thioacetate and 10.0 mmol of 2-(tert-butoxycarbonyl)amino)ethyl methane sulfonate **(C1)** in 50 mL of dry anhydrous *N*,*N*-DMF, and the resulting mixture was stirred at 65°C for 16 hours. Afterwards, 50 mL of water and 50 mL of ethyl acetate were added to separate the layers. The organic layer was washed with 80% saturated brine (4 × 30 mL). Afterwards, the organic layer was dried with sodium sulfate, filtered, and concentrated under reduced pressure. The residue was purified by flash column chromatography to obtain S-(2-((tert-butoxycarbonyl)amino)ethyl) ethane thioate (S-(2-((tert-butoxycarbonyl) amino) ethyl) ethane thioate(C2) was obtained.

4.25 mL (41.6 mmol) of hydrogen peroxide solution (30%) was dissolved in 20.8 mL (928 mmol) of formic acid at 0°C, and stirred at room temperature for 1 hour to obtain peroxyformic acid. The peroxyformic acid solution was cooled back to 0°C, 4.2 mmol of S-(2-((tert-butoxycarbonyl)amino)ethyl)ethane thioate (C2) was added dropwise, the resulting solution was stirred, and the resulting solution was stirred and cooled to room temperature. heated. After 18 hours, the mixture was concentrated under reduced pressure and co-evaporated with ethanol. The residue was washed once with DCM/EtOH and once with DCM to obtain pure sulfonic acid (C3).

### <Example 2> Preparation of bile acid derivatives

### <Example 2-1> Coupling of cholic acid derivatives and taurine derivatives

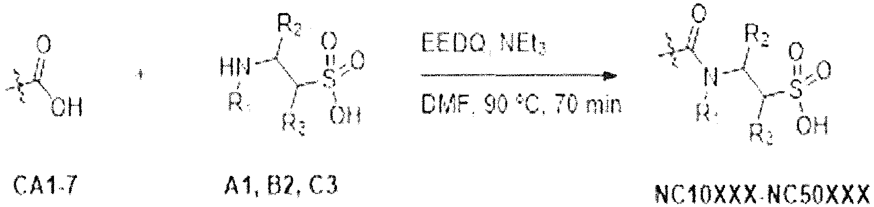

In 1 mL of dry anhydrous N,N-DMF containing 0.134 mmol of cholic acid and its derivatives (**CA1-7**), 0.147 mmol of taurine derivatives **(A1, B2, C3)** prepared in <Example 1-1> to <Example 1-3>, triethylamine 0.024 mL (0.174 mmol) and N-Ethoxycarbonyl-2-ethoxy-1,2,dihydroquinoline (EEDQ) 0.043 g (0.0174 mmol) was added, and the mixed solution was stirred at 90°C for 70 minutes. After cooling, the mixture was flushed with Dowex and rinsed with 4-6 mL of methanol. Afterwards, the mixture was concentrated and purified by basic preparative liquid chromatography-mass spectrometry (LCMS) to obtain compounds (NC10XXX to NC50XXX) containing 2-aminoethanesulfonic acid.

### <Example 2-2> Conversion to Na salt

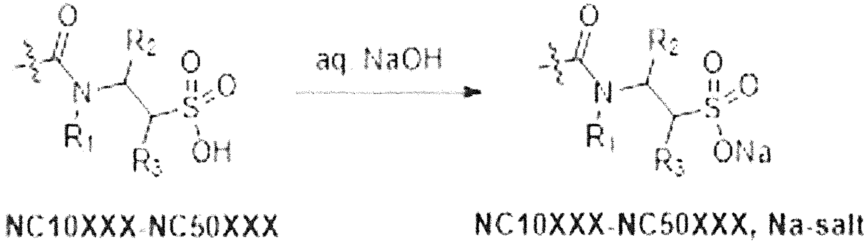

The compounds (NC10XXX-NC50XXX) obtained in <Example 2-1> were dissolved in 1 mL of MeCN/H₂O (9:1), and an equimolar amount of NaOH aqueous solution (0.1 M) was added. The resulting solution was concentrated in vacuum to obtain Na-salt compounds (NC10XXX-NC50XXX, **Na-salt**).

### <Example 3> Preparation of Compound 1

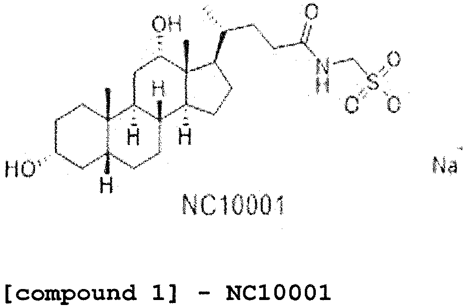

Compound 1 was prepared by the preparation method of <Example 1> and <Example 2> above. Specifically, 400 mg (10 mmol) of NaOH was dissolved in 40 mL of 95% ethanol, 1.11 g (10 mmol) of aminomethanesulfonic acid was added, and the mixture was heated to 50° C and stirred for 30 minutes. This was cooled to room temperature, 4.12 g (10.4 mmol) of deoxycholic acid (DCA) and 5.0 g (20.2 mmol) of EEDQ were added, washed with 5 mL of ethanol, heated to 40°C, and stirred for 15 hours. Afterwards, it was cooled to room temperature and stirred for 1 hour. The solvent was evaporated, 50 mL of acetone was added, and ultrasound was applied to obtain a solid. Afterwards, it was filtered, washed with acetone, and dried under vacuum for 5 hours to obtain 0.99 g of Compound 1. (yield 19%)

### <Example 4> Preparation of compound 2

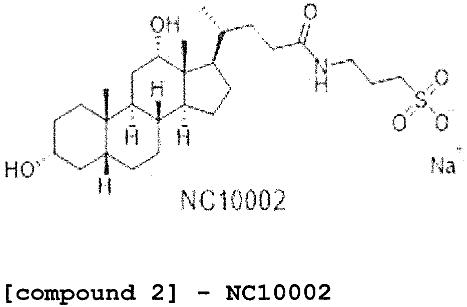

Compound 2 was prepared in the same manner as in the above example using 4.12 g (10.4 mmol) of deoxycholic acid (DCA), 1.39 g (10 mmol) of 3-Amino-1-propanesulfonic acid , 400 mg (10 mmol) of NaOH and 5.0 g (20.2 mmol) of EEDQ.

### <Example 5> Preparation of compound 3

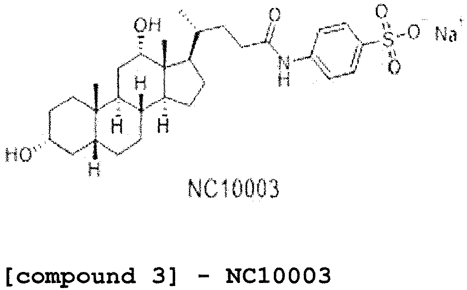

Compound 3 was prepared in the same manner as in the above example using 4.12 g (10.4 mmol) of deoxycholic acid (DCA), 1.73 g (10 mmol) of 4-aminobenzenesulfonic acid, 400 mg (10 mmol) of NaOH, and 5.0 g (20.2 mmol) of EEDQ.

### <Example 6> Preparation of compound 4

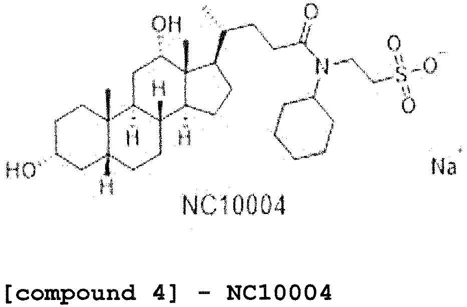

Compound 4 was prepared in the same manner as in the above example using 4.12 g (10.4 mmol) of deoxycholic acid (DCA), 2.07 g (10 mmol) of 2-(Cyclohexylamino)ethanesulfonic acid, 400 mg (10 mmol) of NaOH and 5.0 g using (20.2 mmol) of EEDQ. (yield 51%)

### <Example 7> Preparation of compound 5

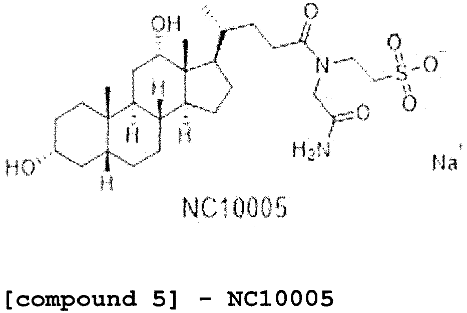

Compound 5 was prepared in the same manner as in the above example using 4.12 g (10.4 mmol) of deoxycholic acid (DCA), 1.82 g(10 mmol) of N-(2-Acetamido)-2-aminoethanesulfonic acid, 400 mg (10 mmol) of NaOH and 5.0 g (20.2 mmol) of EEDQ. (yield 79.0%)

### <Example 8> Preparation of compound 6

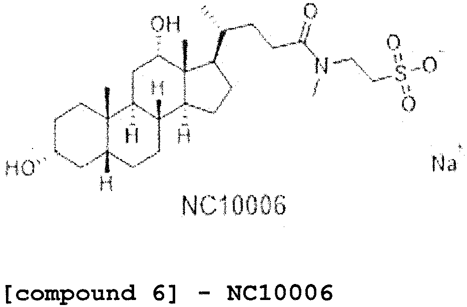

Compound 6 was prepared in the same manner as in the above example using 4.12 g (10.4 mmol) of deoxycholic acid (DCA), 2.5 g (10 mmol) of N-Methyltaurine sodium salt, 400 mg (10 mmol) of NaOH, and 5.0 g (20.2 mmol) of EEDQ.

### <Example 9> Preparation of compound 7

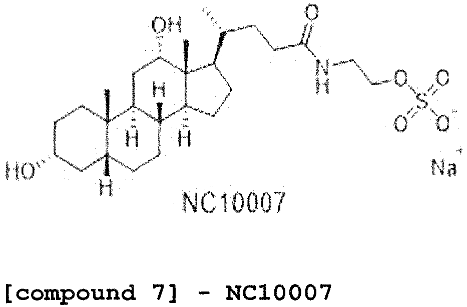

Compound 7 was prepared in the same manner as in the above example using 4.12 g (10.4 mmol) of deoxycholic acid (DCA), 1.41 g (10 mmol) of 2-Aminoethyl hydrogen sulfate, and 5.0 g (20.2 mmol) of EEDQ.

### <Example 10> Preparation of compound 8

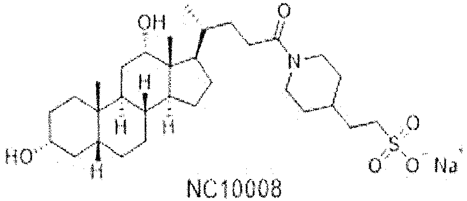

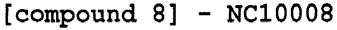

Compound 8 was prepared in the same manner as in the above example using 0.210 g (0.54 mmol) of deoxycholic acid (DCA), 96.6 mg ( 0.5 mmol) of 2-(Piperidin-4-yl)ethane-1-sulfonic acid, 20 mg (0.5 mmol) of NaOH, and 0.250 g (1.0 mmol) of EEDQ. (yield 74.6%)

### <Example 11> Preparation of compound 9

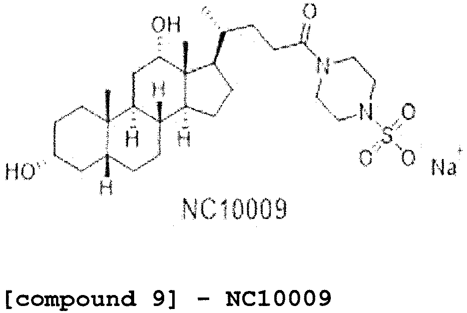

Compound 9 was prepared in the same manner as in the above example using 0.205 g (0.52 mmol) of Deoxycholic acid (DCA), 83.1 mg (0.5 mmol) of Piperazine-1-sulfonic acid, 20 mg (0.5 mmol) of NaOH and 0.250 g (1.0 mmol) of EEDQ. (yield 56.5%)

### <Example 12> Preparation of compound 10

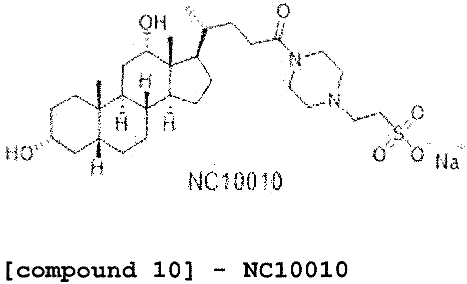

Compound 10 was prepared in the same manner as in the above example using 0.205 g (0.52 mmol) of deoxycholic acid (DCA), 97.1 mg (0.5 mmol) of 2-(piperazin-1-yl)ethane-1-sulfonic acid, 20 mg (0.5 mmol) of NaOH and 0.250 g (1.0 mmol) of EEDQ. (yield 71.1%)

### <Example 13> Preparation of compound 11

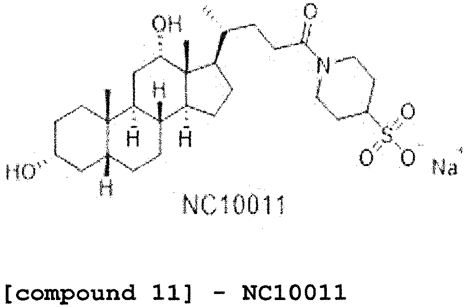

Compound 11 was prepared in the same manner as in the above example using 0.410 g (1.04 mmol) of deoxycholic acid (DCA), 165 mg (1.0 mmol) of piperidine-4-sulfonic acid, 40 mg (1.0 mmol) of NaOH and 0.500 g (2.0 mmol) of EEDQ. (yield 60.5%)

### <Example 14> Preparation of compound 12

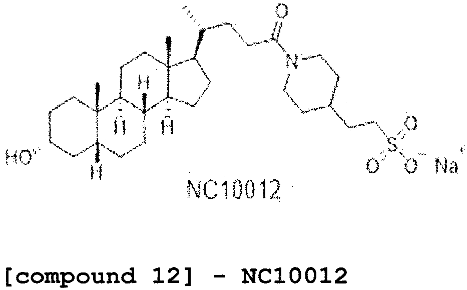

Compound 12 was prepared in the same manner as in the above example using 0.200 g (0.53 mmol) of Lithocholic acid (LCA), 96.2 mg (0.5 mmol) of 2-(Piperidin-4-yl)ethane-1-sulfonic acid, 20 mg (0.5 mmol) of NaOH, and 0.250 g (1.01 mmol) of EEDQ. (yield 73.2%)

### <Example 15> Preparation of compound 13

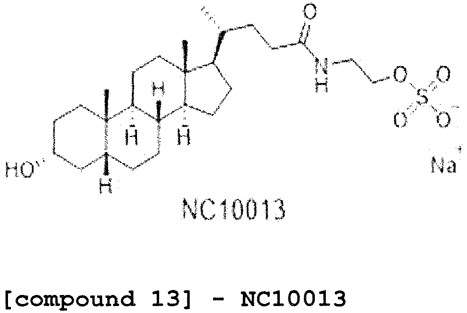

Compound 13 was prepared in the same manner as in the above example using 0.395 g (1.05 mmol) of Lithocholic acid (LCA), 141 mg (1.0 mmol) of 2-Aminoethyl hydrogen sulfate, 40 mg (1.0 mmol) of NaOH and 0.507 g (2.05 mmol) of EEDQ. (yield 61.3%)

### <Example 16> Preparation of compound 14

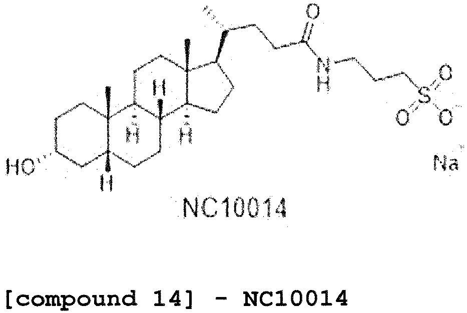

Compound 14 was prepared in the same manner as in the above example using 0.395 g (1.05 mmol) of Lithocholic acid (LCA), 139 mg (1.0 mmol) of 3-Amino-1-propanesulfonic acid, 40 mg (1.0 mmol) of NaOH and 0.507 g (2.05 mmol) of EEDQ. (yield 86.6%)

### <Example 17> Preparation of Compound 15

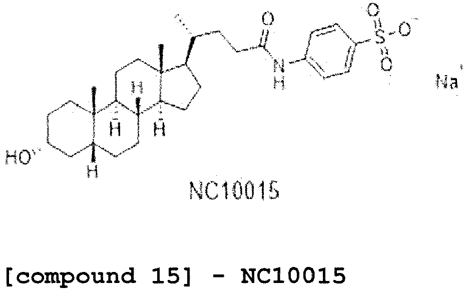

Compound 15 was prepared in the same manner as in the above example using 0.395 g (1.05 mmol) of lithocholic acid (LCA), 173 mg (1.0 mmol) of 4-aminobenzenesulfonic acid, 40 mg (1.0 mmol) of NaOH, and 0.505 g (2.05 mmol) of EEDQ. (yield 70.4%)

### <Example 18> Preparation of compound 16

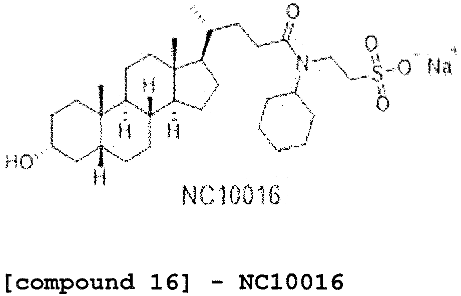

Compound 16 was prepared in the same manner as in the above example using 0.395 g (1.05 mmol) of Lithocholic acid (LCA), 207 mg (1.0 mmol) of 2-(Cyclohexylamino)ethanesulfonic acid, 40 mg (1.0 mmol) of NaOH and 0.507 g (2.05 mmol) of EEDQ . (yield 66.3%)

### <Example 19> Preparation of compound 17

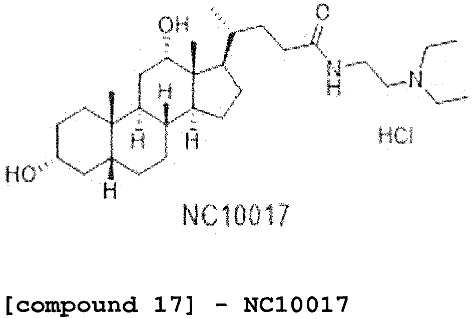

Compound 9 was prepared by the preparation method of <Example 1> and <Example 2> above. Specifically, deoxycholic acid (DCA) 3.92 g (10 mmol), hydroxybenzotriazole (HOBt) 1.42 g (10.5 mmol), N,N'-dicyclohexylcarbodiimide (DCC) 2.17 g (10.5 mmol) were mixed with 30 mL of DMF. To the mixture, 1.41 mL (10 mmol) of N,N-Diethylethylenediamine and 1.15 mL (10.4 mmol) of N-methylmorpholine (N-MMP) were added. This was heated to 40-45°C and stirred for 1 day. Afterwards, it was cooled to room temperature, filtered and washed with 10 mL of DMF. The solvent was evaporated under reduced pressure, dissolved in 20 mL of 2N HCl and 80 mL of H₂O, and washed with 100 mL of ethyl acetate X 2. Afterwards, it was neutralized with NaOH until the pH reached 8-9, and NaCl was added to saturate it. It was extracted with 100 mL of dichloromethane X 2, mixed with the organic layer, and dissolved in 10 mL of methanol. Afterwards, it was dried with Na₂SO₄, filtered and washed with dichloromethane, and the solvent was evaporated. Afterwards, compound 17 was obtained by sequentially filtering and washing using ethanol, 1N HCl ether, and acetone.

### <Example 20> Preparation of Compound 18

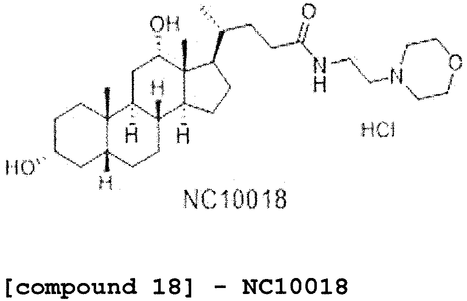

Compound 18 was prepared in the same manner as in the above example using 3.92 g (10 mmol) of Deoxycholic acid (DCA), 1.42 g (10.5 mmol) of HOBt, 2.17 g (10.5 mmol) of DCC, 1.30 g (10 mmol) of 4-(2-Aminoethyl)morpholine and 1.06 g (10.4 mmol) of N-MMP.

### <Example 21> Preparation of compound 19

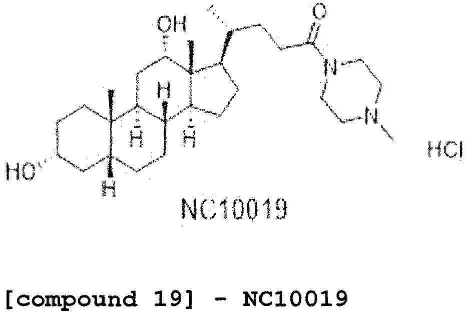

Compound 19 was prepared in the same manner as in the above example using 3.92 g (10 mmol) of deoxycholic acid (DCA), 1.42 g (10.5 mmol) of HOBt, 2.17 g (10.5 mmol) of DCC, 1.00 g (10 mmol) of 1-Methylpiperazine, and 1.06 g (10.4 mmol) of N-MMP.

### <Example 22> Preparation of bile acid derivatives

The following bile acid derivatives were prepared in the same manner as in the above examples, and the specific chemical structures, NMR data, and MS data of the bile acid derivatives are shown below.

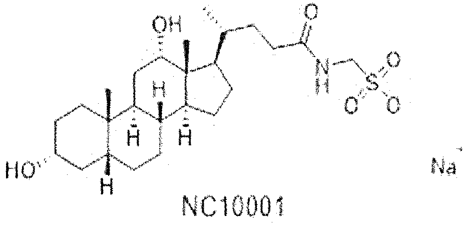

### NC10001

¹H NMR (400 MHz, DMSO-d₆) δ 8.04 (t, *J* = 6.2 Hz, 1H), 4.45 (s, 1H), 4.22-4.15 (m, 1H), 3.92-3.82 (m, 2H), 3.81-3.76 (m, 1H), 3.41-3.31 (m, 1H), 2.15 (ddd, *J* = 14.1, 10.6, 5.0 Hz, 1H), 2.00 (ddd, *J* = 14.1, 10.2, 6.0 Hz, 1H), 1.86-1.41 (m, 10H), 1.40-0.94 (m, 13H), 0.94-0.85 (m, 4H), 0.84 (s, 3H), 0.59 (s, 3H); LCMS (ESI) *m*/*z* calcd for C₂₅H₄₂NO₆S [M-Na]⁻ 484.3, found 484.6.

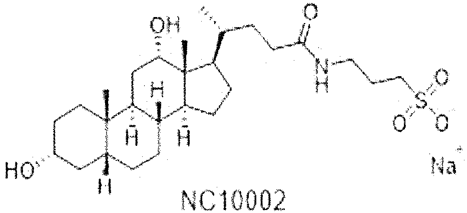

### NC10002

¹H NMR (400 MHz, DMSO-d₆) δ 7.78 (t, *J* = 5.6 Hz, 1H), 4.46 (d, *J* = 4.4 Hz, 1H), 4.19 (d, *J* = 4.1 Hz, 1H), 3.81-3.74 (m, 1H), 3.47-3.32 (m, 1H), 3.08-3.03 (m, 2H), 2.45-2.37 (m, 2H), 2.12-2.00 (m, 1H), 1.93 (ddd, *J* = 13.7, 9.6, 6.4 Hz, 1H), 1.86-0.95 (m, 25H), 0.94-0.85 (m, 4H), 0.84 (s, 3H), 0.58 (s, 3H); LCMS (ESI) *m*/*z* calcd for C₂₇H₄₆NO₆S [M-Na]⁻ 512.3, found 512.6.

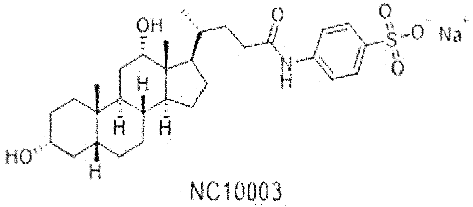

### NC10003

¹H NMR (400 MHz, DMSO-d₆) δ 9.92 (s, 1H), 7.56-7.47 (m, 4H), 4.47 (d, *J* = 4.2 Hz, 1H), 4.21 (d, *J* = 3.9 Hz, 1H), 3.83-3.76 (m, 1H), 3.49-3.38 (m, 1H), 2.34 (ddd, *J* = 14.1, 9.7, 4.6 Hz, 1H), 2.20 (ddd, *J* = 14.7, 9.0, 6.5 Hz, 1H), 1.87-1.67 (m, 5H), 1.67-1.41 (m, 5H), 1.41-1.13 (m, 11H), 1.12-0.86 (m, 6H), 0.84 (s, 3H), 0.60 (s, 3H); LCMS (ESI) *m*/*z* calcd for C₃₀H₄₄NO₆S [M-Na]⁻ 546.3, found 546.6.

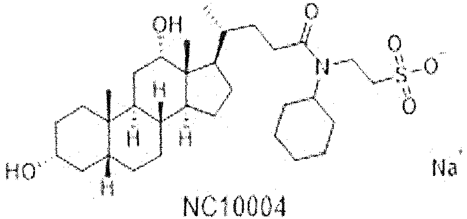

### NC10004

¹H NMR (400 MHz, DMSO-d₆) δ 9.45 (t, *J* = 4.9 Hz, 1H), 4.20 (d, *J* = 4.1 Hz, 1H), 4.15-4.07 (m, 0.5H), 3.83-3.76 (m, 1H), 3.57-3.47 (m, 0.5H), 3.46-3.35 (m, 3H), 2.66-2.54 (m, 2H), 2.33-2.11 (m, 2H), 1.86-0.86 (m, 37H), 0.84 (s, 3H), 0.59 (d, *J* = 1.9 Hz, 3H); LCMS (ESI) *m*/*z* calcd for C₃₂H₅₄NO₆S [M-Na]⁻ 580.4, found 580.8.

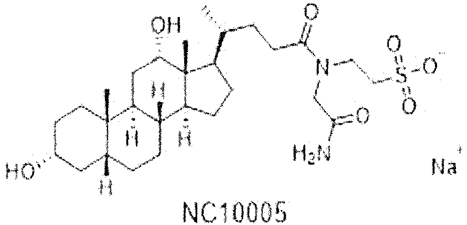

### NC10005

¹H NMR (400 MHz, DMSO-d₆) δ 7.52 (s, 0.4H) 7.42 (s, 0.6H), 7.11 (s, 0.4H), 6.91 (s, 0.6H), 4.49-4.40 (m, 1H), 4.20 (d, *J* = 4.1 Hz, 0.6H), 4.18 (d, *J* = 4.1 Hz, 0.4H), 3.91 (s, 1H), 3.83-3.71 (m, 2H), 3.61-3.51 (m, 1H), 3.49-3.41 (m, 1H), 3.41-3.31 (m, 1H), 2.69-2.65 (m, 1.2H), 2.59-2.56 (m, 0.8H), 2.49-2.38 (m, 0.6H), 2.33-2.21 (m, 0.6H), 2.21-2.10 (m, 0.4H) 2.07-1.97 (m, 0.4H), 1.87-1.41 (m, 10H), 1.40-0.79 (m, 20H), 0.60 (s, 1.8H), 0.59 (s, 1.2H); LCMS (ESI) m/z calcd for C₂₈H₄₇N₂O₇S [M-Na]⁻ 555.3, found 555.6.

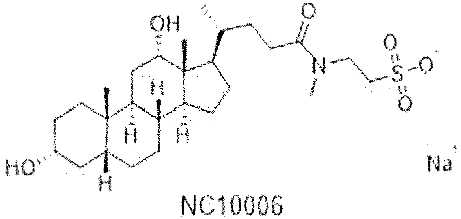

### NC10006

¹H NMR (400 MHz, DMSO-d₆) δ 4.45 (s, 1H), 4.25-4.16 (m, 1H), 3.79 (s, 1H), 3.55-3.41 (m, 2H), 3.40-3.30 (m, 1H), 2.93 (s, 1.5H), 2.75 (s, 1.5H), 2.69-2.62 (m, 1H), 2.60-2.53 (m, 1H), 2.36-2.10 (m, 2H), 1.86-1.70 (m, 4H), 1.68-1.41 (m, 6H), 1.40-1.11 (m, 11H), 1.09-0.85 (m, 6H), 0.84 (s, 3H), 0.59 (s, 3H); LCMS (ESI) *m*/*z* calcd for C₂₇H₄₆NO₆S [M-Na]⁻ 512.3, found 512.6.

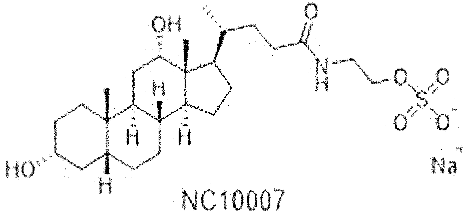

### NC10007

¹H NMR (400 MHz, DMSO-d₆) δ 7.85 (t, *J* = 5.6 Hz, 1H), 4.45 (d, *J* = 4.3 Hz, 1H), 4.18 (d, *J* = 4.1 Hz, 1H), 3.82-3.75 (m, 1H), 3.69 (t, *J =* 6.1 Hz, 2H), 3.42-3.31 (m, 1H), 3.19 (q, *J* = 5.9 Hz, 2H), 2.14-2.03 (m, 1H), 1.95 (ddd, *J* = 13.9, 9.7, 6.3 Hz, 1H), 1.86-1.69 (m, 4H), 1.68-1.40 (m, 6H), 1.40-1.09 (m, 11H), 1.08-0.85 (m, 6H), 0.84 (s, 3H), 0.59 (s, 3H); LCMS (ESI) *m*/*Z* calcd for C₂₆H₄₄NO₇S [M-Na]⁻ 514.3, found 514.5.

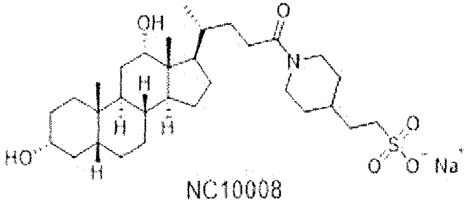

### NC10008

¹H NMR (400 MHz, DMSO-d₆) δ 4.46 (d, *J* = 4.2 Hz, 1H), 4.34 (d, *J* = 12.9 Hz, 1H), 4.19 (d, *J* = 4.0 Hz, 1H), 3.84-3.75 (m, 2H), 3.42-3.30 (m, 1H), 2.98-2.87 (m, 1H), 2.48-2.36 (m, 3H), 2.34-2.22 (m, 1H), 2.22-2.10 (m, 1H), 1.86-1.41 (m, 15H), 1.40-0.85 (m, 19H), 0.84 (s, 3H), 0.59 (s, 3H); LCMS (ESI) *m*/*z* calcd for C₃₁H₅₂NO₆S [M-Na]⁻ 566.4, found 566.7.

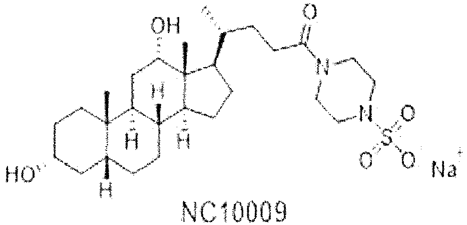

### NC10009

¹H NMR (400 MHz, DMSO-d₆) δ 4.95 (d, *J* = 4.2 Hz, 1H), 4.19 (d, *J* = 4.0 Hz, 1H), 3.82-3.75 (m, 1H), 3.48-3.35 (m, 5H), 2.83-2.65 (m, 4H), 2.30 (ddd, *J* = 15.4, 10.3, 5.2 Hz, 1H), 2.17 (ddd, *J* = 15.3, 9.9, 5.9 Hz, 1H), 1.86-1.69 (m, 4H), 1.67-1.41 (m, 6H), 1.39-1.12 (m, 11H), 1.10-0.86 (m, 6H), 0.84 (s, 3H), 0.59 (s, 3H); LCMS (ESI) *m*/*z* calcd for C₂₈H₄₇N₂O₆S [M-Na]⁻ 539.3, found 539.5.

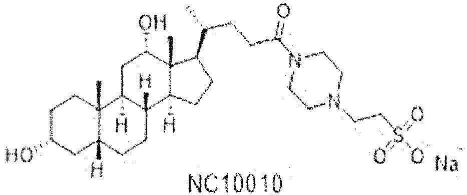

### NC10010

¹H NMR (500 MHz, CD₃OD) δ3.98-3.94 (m, 1H), 3.61-3.46 (m, 5H), 3.07-2.99 (m, 2H), 2.89-2.82 (m, 2H), 2.56-2.54 (m, 2H), 2.52-2.40 (m, 3H), 2.35-2.27 (m, 1H), 1.96-1.68 (m, 7H), 1.67-1.56 (m, 3H), 1.55-1.23 (m, 11H), 1.22-0.91 (m, 9H), 0.72 (s, 3H); LCMS (ESI) *m*/*Z* calcd for C₃₀H₅₁N₂O₆S [M-Na]⁻ 567.3, found 567.8.

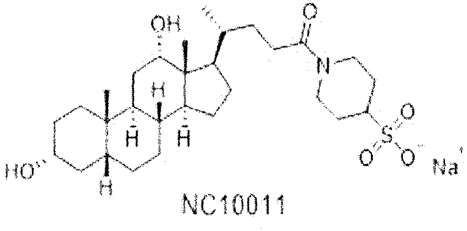

### NC10011

¹H NMR (500 MHz, CD₃OD) δ4.65-4.55 (m, 1H), 4.06-4.04 (m,1H), 3.97-3.95 (m, 1H), 3.57-3.47 (m, 1H), 3.13 (td, *J* = 13.2, 2.6 Hz, 1H), 2.90 (tt, *J* = 11.7, 3.8 Hz, 1H), 2.69-2.60 (m, 1H), 2.52-2.40 (m, 1H), 2.38-2.26 (m, 1H), 2.23-2.08 (m, 2H), 1.95-1.68 (m, 8H), 1.68-1.55 (m, 4H), 1.55-1.23 (m, 11H), 1.22-1.06 (m, 2H), 1.04 (d, *J* = 6.5 Hz, 3H), 0.98 (td, *J* = 14.1, 3.3 Hz, 1H), 0.93 (s, 3H), 0.72 (s, 3H); LCMS (ESI) *m*/*Z* calcd for C₂₉H₄₈NO₆S [M-Na]⁻ 538.3, found 538.7.

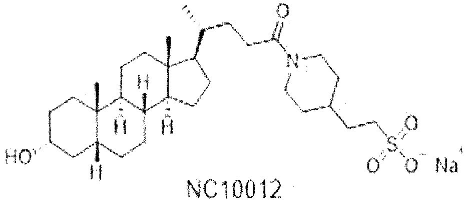

### NC10012

¹H NMR (400 MHz, DMSO-d₆) δ 4.50 (s, 1H), 4.33 (d, J = 13.1 Hz, 1H), 3.80 (d, *J* = 13.4 Hz, 1H), 3.53-3.35 (m, 1H), 2.95-2.89 (m, 1H), 2.48-2.36 (m, 3H), 2.35-2.22 (m, 1H), 2.22-2.11 (m, 1H), 1.96-1.87 (m, 1H), 1.85-1.72 (m, 2H), 1.71-1.43 (m, 10H), 1.42-0.77 (m, 26H), 0.60 (s, 3H); LCMS (ESI) *m*/*z* calcd for C₃₁H₅₂NO₅S [M-Na]⁻ 550.4, found 550.3.

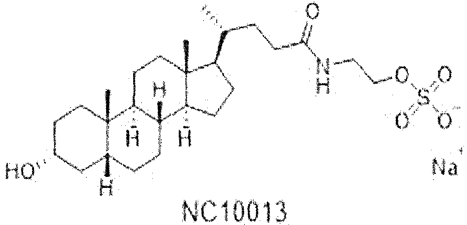

### NC10013

¹H NMR (500 MHz, CD₃OD) δ 4.31-4.26 (m, 1H), 4.05-4.01 (m, 1H), 3.60-3.49 (m, 1H), 3.46-3.42 (m, 1H), 3.23-3.19 (m, 1H), 2.49-2.08 (m, 2H), 2.03-2.00 (m,1H), 1.96-1.70 (m, 5H), 1.66-1.57 (m, 2H), 1.51-1.24 (m, 12H), 1.24-1.05 (m, 5H), 1.03-0.93 (m, 7H), 0.694 (s, 1.5H), 0.691 (s, 1.5H); LCMS (ESI) *m*/*Z* calcd for C₂₆H₄₄NO₆S [M-Na]⁻ 498.3, found 498.6.

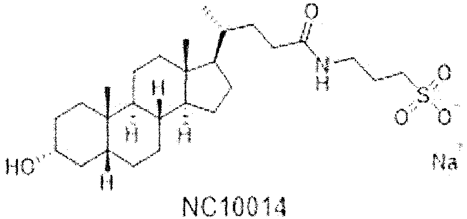

### NC10014

¹H NMR (400 MHz, DMSO-d₆) δ 7.80 (t, *J* = 5.6 Hz, 1H), 4.49 (d, *J* = 4.5 Hz, 1H), 3.59-3.24 (m, 1H), 3.05 (q, *J* = 6.6 Hz, 2H), 2.46-2.37 (m, 2H), 2.11-2.01 (m, 1H), 1.99-1.87 (m, 2H), 1.83-1.43 (m, 9H), 1.41-0.96 (m, 17H), 0.95-0.80 (m, 7H), 0.60 (s, 3H); LCMS (ESI) *m*/*Z* calcd for C₂₇H₄₆NO₅S [M-Na]⁻496.3, found 496.6.

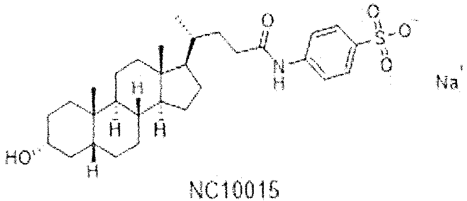

### NC10015

¹H NMR (400 MHz, DMSO-d₆) δ 9.92 (s, 1H), 7.57-7.46 (m, 4H), 4.45 (d, *J* = 4.5 Hz, 1H), 3.42-3.31 (m, 1H), 2.33 (ddd, *J* = 14.7, 9.7, 5.2 Hz, 1H), 2.20 (ddd, *J* = 14.7, 9.3, 6.6 Hz, 1H), 1.97-1.90 (m, 1H), 1.88-1.45 (m, 7H), 1.45-0.97 (m, 17H), 0.97-0.89 (m, 4H), 0.87 (s, 3H), 0.62 (s, 3H); LCMS (ESI) *m*/*z* calcd for C₃₀H₄₄NO₅S [M-Na]⁻ 530.3, found 530.7.

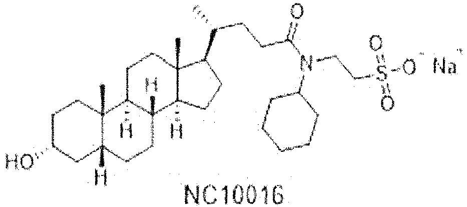

### NC10016

¹H NMR (500 MHz, CD₃OD) δ 3.73-3.61 (m, 2H), 3.58-3.47 (m, 1H), 3.08-3.00 (m, 2H), 2.49-2.38 (m, 1H), 2.37-2.25 (m, 1H), 2.05-1.99 (m, 1H), 1.95-1.52 (m, 14H), 1.52-1.05 (m, 21H), 1.04-0.92 (m, 7H), 0.70 (d, *J* = 4.9 Hz, 3H); LCMS (ESI) *m*/*z* calcd for C₃₂H₅₄NO₅S [M-Na]⁻ 564.4, found 564.6.

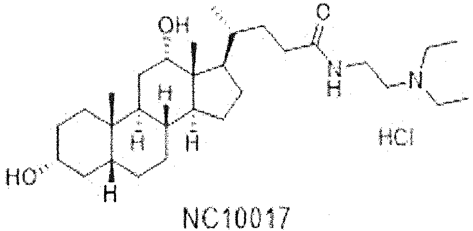

### NC10017

¹H NMR (500 MHz, CD₃OD) δ 3.96 (t, *J* = 3.0 Hz, 1H), 3.58-3.48 (m, 3H), 3.30-3.22 (m, 6H), 2.32 (ddd, *J* = 14.0, 10.6, 4.9 Hz, 1H), 2.16 (ddd, *J* = 14.1, 9.9, 6.2 Hz, 1H), 1.95-1.73 (m, 7H), 1.67-1.56 (m, 3H), 1.56-1.24 (m, 17H), 1.22-1.07 (m, 2H), 1.03 (d, *J* = 6.4 Hz, 3H), 1.01-0.95 (m, 1H), 0.93 (s, 3H), 0.71 (s, 3H); LCMS (ESI) *m*/*z* calcd for C₃₀H₅₅N₂O₃ [M-Cl]⁺ 491.4, found 491.3.

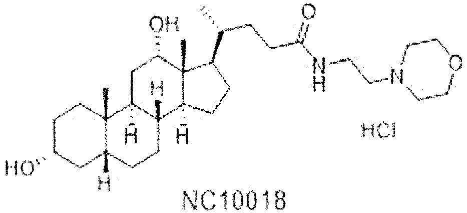

### NC10018

¹H NMR (500 MHz, CD₃OD) δ 4.12-4.02 (m, 2H), 3.97-3.94 (m, 1H), 3.84-3.73 (m, 2H), 3.65-3.48 (m, 5H), 3.29 (t, *J* = 6.0 Hz, 2H), 3.23-3.12 (m, 2H), 2.33 (ddd, *J* = 13.9, 10.6, 4.7 Hz, 1H), 2.22-2.13 (m, 1H), 1.95-1.73 (m, 7H), 1.67-1.55 (m, 3H), 1.55-1.33 (m, 9H), 1.33-1.23 (m, 2H), 1.23-1.07 (m, 2H), 1.05-0.95 (m, 4H), 0.93 (s, 3H), 0.71 (s, 3H); LCMS (ESI) *m*/*z* calcd for C₃₀H₅₃N₂O₄ [M-Cl]⁺ 505.4, found 505.3.

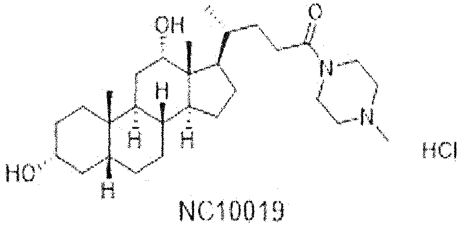

### NC10019ⁱ

¹H NMR (500 MHz, CD₃OD) δ 4.66 (s, 1H), 4.20 (s, 1H), 3.97-3.95 (m,1H), 3.73-3.36 (m, 4H), 3.28-2.96 (m, 3H), 2.93 (s, 3H), 2.51 (ddd, *J* = 15.7, 10.9, 5.1 Hz, 1H), 2.36 (ddd, *J* = 15.6, 10.5, 5.6 Hz, 1H), 1.95-1.70 (m, 7H), 1.68-1.56 (m, 3H), 1.56-1.23 (m, 11H), 1.23-1.06 (m, 2H), 1.04 (d, *J* = 6.5 Hz, 3H), 1.02-0.95 (m, 1H), 0.94 (s, 3H), 0.72 (s, 3H); LCMS (ESI) *m*/*z* calcd for C₂₉H₅₁N₂O₃ [M-Cl]⁺ 475.4, found 475.3.

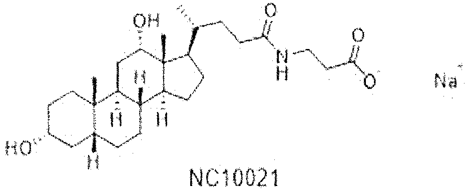

### NC10021

¹H NMR (400 MHz, DMSO-d₆) δ 7.79 (t, *J* = 5.4 Hz, 1H), 4.47 (s, 1H), 4.22 (s, 1H), 3.78 (s, 1H), 3.41-3.29 (m, 1H), 3.12-3.07 (m, 2H), 2.04 (ddd, *J* = 14.8, 10.1, 5.1 Hz, 1H), 1.97-1.85 (m, 3H), 1.85-1.41 (m, 10H), 1.40-0.93 (m, 13H), 0.93-0.85 (m, 4H), 0.84 (s, 3H), 0.59 (s, 3H); LCMS (ESI) *m*/*z* calcd for C₂₇H₄₄NO₅ [M-Na]⁻ 462.3, found 462.2.

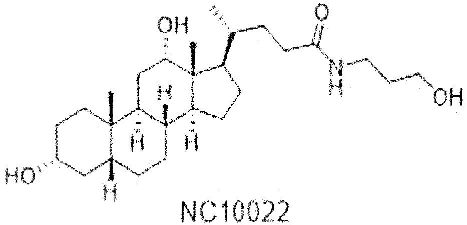

### NC10022

¹H NMR (500 MHz, CD₃OD) δ 3.96 (t, *J* = 3.0 Hz, 1H), 3.58 (t, *J* = 6.3 Hz, 2H), 3.56-3.49 (m, 1H), 3.25 (td, *J* = 6.9, 1.1 Hz, 2H), 2.29-2.20 (m, 1H), 2.15-2.05 (m, 1H), 1.95-1.74 (m, 7H), 1.74-1.66 (m, 2H), 1.65-1.56 (m, 3H), 1.56-1.23 (m, 11H), 1.23-1.06 (m, 2H), 1.05-0.94 (m, 4H), 0.93 (s, 3H), 0.71 (s, 3H); LCMS (ESI) *m*/*z* calcd for C₂₇H₄₆NO₄ [M-H]⁻ 448.3, found 448.1.

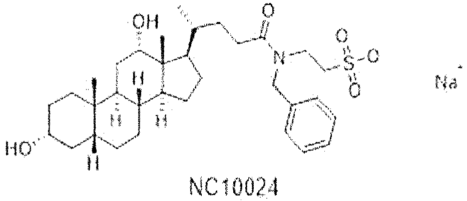

### NC10024

1H NMR (500 MHz, CD₃OD) δ 7.41-7.34 (m, 1H), 7.34-7.19 (m, 4H), 4.74 (s, 1H), 4.63 (s, 1H), 3.99-3.94 (m, 0.5H), 3.93-3.88 (m, 0.5H), 3.83-3.67 (m, 2H), 3.57-3.46 (m, 1H), 3.10-2.96 (m, 2H), 2.62-2.52 (m, 0.5H), 2.52-2.38 (m, 1H), 2.35-2.25 (m, 0.5H), 1.96-1.68 (m, 7H), 1.68-1.03 (m, 18H), 1.02-0.90 (m, 5H), 0.71 (s, 1.5H), 0.67 (s, 1.5H); LCMS (ESI) *m*/*z* calcd for C₃₃H₅₀NO₆S [M-Na]⁻ 588.3, found 588.2.

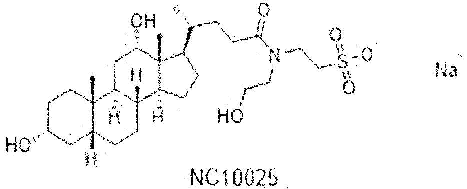

### NC10025

¹H NMR (500 MHz, CD₃OD) δ 3.99-3.93 (m, 1H), 3.89-3.77 (m, 1H), 3.77-3.64 (m, 3H), 3.60-3.44 (m, 3H), 3.15-3.01 (m, 2H), 2.57-2.45 (m, 1H), 2.43-2.30 (m, 1H), 1.96-1.70 (m, 7H), 1.66-1.55 (m, 3H), 1.55-1.24 (m, 11H), 1.23-0.94 (m, 6H), 0.93 (s, 3H), 0.73-0.69 (m, 3H); LCMS (ESI) *m*/*z* calcd for C₂₈H₄₈NO₇S [M-Na]⁻ 542.3, found 542.4.

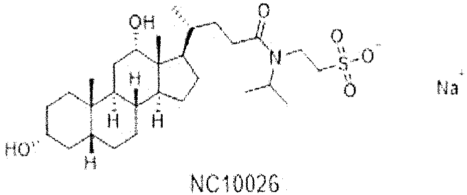

### NC10026

¹H NMR (500 MHz, CD₃OD) δ 4.66-4.56 (m, 0.5H), 4.23-4.13 (m, ,0.5H), 3.99-3.94 (m, 1H), 3.72-3.65 (m, 1H), 3.65-3.58 (m, 1H), 3.57-3.46 (m, 1H), 3.10-3.01 (m, 2H), 2.51-2.40 (m, 1H), 2.38-2.26 (m, 1H), 1.95-1.69 (m, 7H), 1.67-1.57 (m, 3H), 1.55-1.23 (m, 15H), 1.23-1.07 (m, 4H), 1.06-0.94 (m, 4H), 0.934 (s, 1.5H), 0.932 (s, 1.5H), 0.72 (s, 1.5H), 0.71 (s, 1.5H); LCMS (ESI) *m*/*z* calcd for C₂₉H₅₀NO₆S [M-Na]⁻ 540.3, found 540.2.

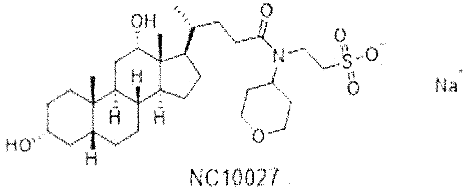

### NC10027

¹H NMR (500 MHz, CD₃OD) δ 4.44 (tt, *J* = 12.2, 4.0 Hz, 0.5H), 4.06-3.91 (m, 3.5H), 3.77-3.62 (m, 2H), 3.56-3.41 (m, 3H), 3.09-3.01 (m, 2H), 2.54-2.43 (m, 1H), 2.36 (ddd, *J* = 14.7, 10.2, 5.8 Hz, 1H), 2.05-1.23 (m, 25H), 1.23-1.02 (m, 5H), 1.01-0.94 (m, 1H), 0.935 (s, 1.5H), 0.931 (s, 1.5H), 0.72 (s, 1.5H), 0.71 (s, 1.5H); LCMS (ESI) *m*/*z* calcd for C₃₁H₅₂NO₇S [M-Na]⁻ 582.3, found 582.2.

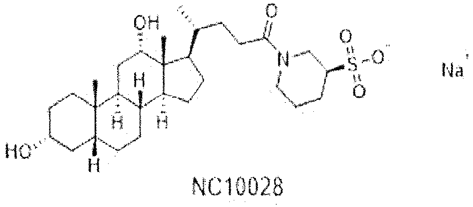

### NC10028

¹H NMR (500 MHz, CD₃OD) δ 5.03 (d, *J* = 10.6 Hz, 0.4H), 4.43 (d, *J* = 13.0 Hz, 0.6H), 4.33-4.26 (m, 0.6H), 4.00-3.89 (m, 1.4H), 3.57-3.46 (m, 1H), 3.24 (dd, *J* = 13.5, 11.1 Hz, 0.6H), 3.01 (td, *J* = 13.2, 2.7 Hz, 0.4H), 2.83-2.56 (m, 2H), 2.56-2.40 (m, 1H), 2.39-2.21 (m, 2H), 1.96-1.69 (m, 9H), 1.67-1.57 (m, 3H), 1.57-1.24 (m, 12H), 1.23-0.94 (m, 6H), 0.93 (s, 3H), 0.71 (s, 3H); LCMS (ESI) *m*/*z* calcd for C₂₉H₄₈NO₆S [M-Na]⁻ 538.3, found 538.2.

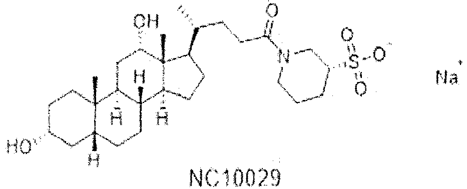

### NC10029

¹H NMR (500 MHz, CD₃OD) δ 5.02 (d, *J* = 10.4 Hz, 0.4H), 4.44 (d, *J* = 13.0 Hz, 0.6H), 4.34-4.27 (m, 0.6H), 3.99-3.89 (m, 1.4H), 3.56-3.46 (m, 1H), 3.23 (dd, *J* = 13.6, 11.2 Hz, 0.6H), 3.01 (td, *J* = 13.2, 2.8 Hz, 0.4H), 2.83-2.54 (m, 2H), 2.53-2.24 (m, 3H), 1.95-1.68 (m, 9H), 1.67-1.55 (m, 3H), 1.55-1.23 (m, 12H), 1.23-1.02 (m, 5H), 1.02-0.95 (m, 1H), 0.93 (s, 3H), 0.72 (s, 3H); LCMS (ESI) *m*/*z* calcd for C₂₉H₄₈NC₆S [M-Na]⁻ 538.3, found 538.2.

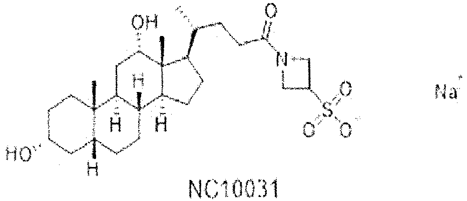

### NC10031

¹H NMR (400 MHz, DMSO-d₆) δ 4.45 (d, *J* = 4.0 Hz, 1H), 4.21 (d, *J* = 4.0 Hz, 1H), 4.16 (td, *J* = 8.4, 3.8 Hz, 1H), 4.05-3.96 (m, 1H), 3.85 (t, *J* = 9.0 Hz, 1H), 3.80-3.71 (m, 2H), 3.44-3.35 (m, 2H), 2.06-1.94 (m, 1H), 1.94-1.40 (m, 11H), 1.40-0.85 (m, 17H), 0.84 (s, 3H), 0.59 (s, 3H); LCMS (ESI) *m*/*z* calcd for C₂₇H₄₄NO₆S [M-Na]⁻ 510.3, found 510.1.

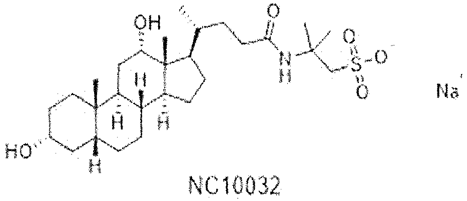

### NC10032

¹H NMR (500 MHz, CD₃OD) δ 3.95 (t, *J* = 3.0 Hz, 1H), 3.51 (tt, *J* = 11.1, 4.6 Hz, 1H), 3.20-3.11 (m, 2H), 2.25-2.16 (m, 1H), 2.10-2.00 (m, 1H), 1.95-1.71 (m, 7H), 1.66-1.23 (m, 20H), 1.23-1.05 (m, 2H), 1.03-0.94 (m, 4H), 0.93 (s, 3H), 0.71 (s, 3H); LCMS (ESI) *m*/*Z* calcd for C₂₈H₄₈NO₆S [M-Na]⁻ 526.3, found 526.2.

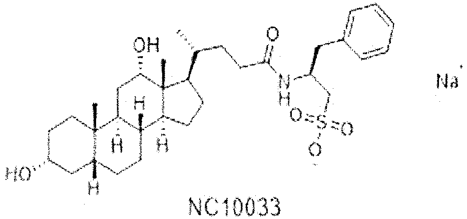

### NC10033

¹H NMR (500 MHz, CD₃OD) δ 7.29-7.22 (m, 4H), 7.22-7.14 (m, 1H), 4.51-4.43 (m, 1H), 3.94 (t, *J* = 3.0 Hz, 1H), 3.52 (tt, *J* = 11.1, 4.6 Hz, 1H), 3.17 (dd, *J* = 13.6, 6.1 Hz, 1H), 3.04-2.94 (m, 2H), 2.91 (dd, *J* = 13.7, 7.9 Hz, 1H), 2.18 (ddd, *J* = 13.8, 10.6, 5.1 Hz, 1H), 2.02 (ddd, *J* = 13.8, 10.3, 6.2 Hz, 1H), 1.95-1.73 (m, 6H), 1.73-1.32 (m, 12H), 1.31-1.03 (m, 5H), 1.03-0.94 (m, 4H), 0.93 (s, 3H), 0.69 (s, 3H); LCMS (ESI) *m*/*z* calcd for C₃₃H₅₀NO₆S [M-Na]⁻ 588.3, found 588.3.

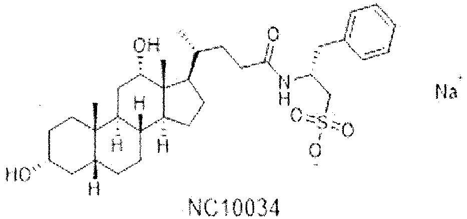

### NC10034

¹H NMR (400 MHz, DMSO-d₆) δ 7.69 (d, *J* = 7.5 Hz, 1H), 7.29-7.11 (m, 5H), 4.45 (d, *J* = 4.3 Hz, 1H), 4.22-4.06 (m, 2H), 3.78 (s, 1H), 3.41-3.31 (m, 1H), 3.13 (dd, *J* = 13.3, 6.0 Hz, 1H), 2.75 (dd, *J =* 13.3, 6.9 Hz, 1H), 2.54-2.51 (m, 2H), 2.07-1.94 (m, 1H), 1.93-1.41 (m, 11H), 1.38-0.93 (m, 13H), 0.89 (d, *J* = 6.5 Hz, 4H), 0.84 (s, 3H), 0.58 (s, 3H); LCMS (ESI) *m*/*z* calcd for C₃₃H₅₀NO₆S [M-Na]⁻ 588.3, found 588.3.

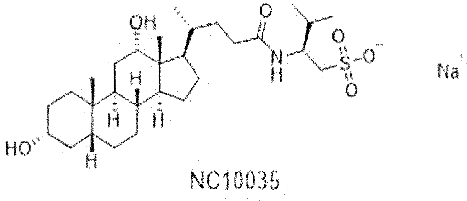

### NC10035

¹H NMR (500 MHz, CD₃OD) δ4.24-4.17 (m, 1H), 3.96 (t, *J* = 3.0 Hz, 1H), 3.52 (tt, *J* = 11.1, 4.5 Hz, 1H), 2.98-2.92 (m, 2H), 2.30 (ddd, *J* = 13.8, 10.5, 5.1 Hz, 1H), 2.16-2.03 (m, 2H), 1.96-1.73 (m, 7H), 1.67-1.23 (m, 14H), 1.23-1.05 (m, 2H), 1.02 (d, *J* = 6.5 Hz, 3H), 1.01-0.94 (m, 1H), 0.94-0.88 (m, 9H), 0.71 (s, 3H); LCMS (ESI) *m*/*z* calcd for C₂₉H₅₀NO₆S [M-Na]⁻ 540.3, found 540.2.

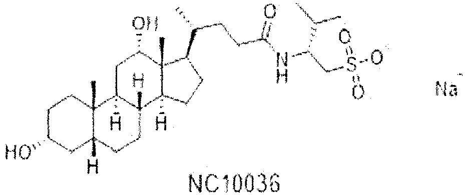

### NC10036

¹H NMR (400 MHz, DMSO-d₆) δ 7.46 (d, *J* = 8.5 Hz, 1H), 4.45 (d, *J* = 4.3 Hz, 1H), 4.19 (d, *J* = 4.0 Hz, 1H), 3.98-3.87 (m, 1H), 3.78 (s, 1H), 3.41-3.28 (m, 1H), 2.54-2.51 (m, 2H), 2.14-1.91 (m, 3H), 1.86-1.40 (m, 10H), 1.40-0.85 (m, 17H), 0.84 (s, 3H), 0.75 (d, *J* = 6.8 Hz, 6H), 0.58 (s, 3H); LCMS (ESI) *m*/*z* calcd for C₂₉H₅₀NO₆S [M-Na]⁻ 540.3, found 540.2.

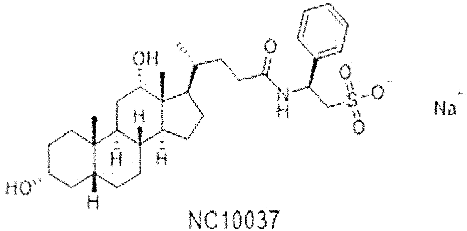

### NC10037

¹H NMR (500 MHz, CD₃OD) δ 7.38-7.33 (m, 2H), 7.33-7.26 (m, 2H), 7.24-7.19 (m, 1H), 5.35 (dd, *J* = 10.0, 3.7 Hz, 1H), 3.97-3.92 (m, 1H), 3.52 (tt, *J* = 11.1, 4.6 Hz, 1H), 3.26 (dd, *J* = 14.3, 10.0 Hz, 1H), 3.10 (dd, *J* = 14.3, 3.8 Hz, 1H), 2.32 (ddd, *J* = 13.7, 10.0, 5.0 Hz, 1H), 2.16 (ddd, *J* = 13.9, 9.6, 6.7 Hz, 1H), 1.95-1.73 (m, 7H), 1.65-1.54 (m, 3H), 1.54-1.03 (m, 13H), 1.02-0.94 (m, 4H), 0.93 (s, 3H), 0.67 (s, 3H); LCMS (ESI) *m*/*z* calcd for C₃₂H₄₈NO₆S [M-Na]⁻ 574.3, found 574.2.

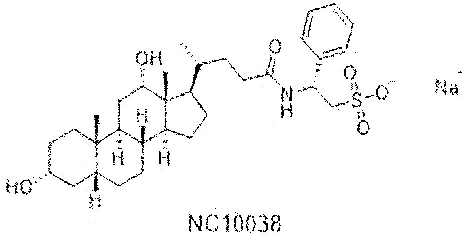

### NC10038

¹H NMR (500 MHz, CD₃OD) δ 7.37-7.27 (m, 4H), 7.24-7.18 (m, 1H), 5.35 (dd, *J* = 10.0, 3.7 Hz, 1H), 3.97-3.92 (m, 1H), 3.51 (tt, *J* = 11.1, 4.5 Hz, 1H), 3.26 (dd, *J* = 14.3, 10.0 Hz, 1H), 3.09 (dd, *J* = 14.3, 3.7 Hz, 1H), 2.30 (ddd, *J* = 14.0, 9.6, 4.6 Hz, 1H), 2.18 (ddd, *J* = 13.7, 8.7, 7.2 Hz, 1H), 1.95-1.73 (m, 7H), 1.64-1.09 (m, 15H), 1.09-0.94 (m, 5H), 0.93 (s, 3H), 0.65 (s, 3H); LCMS (ESI) *m*/*z* calcd for C₃₂H₄₈NO₆S [M-Na]⁻ 574.3, found 574.2.

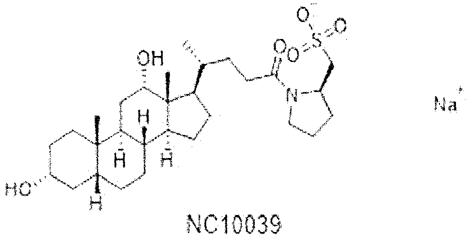

### NC10039

¹H NMR (500 MHz, CD₃OD) δ 4.52-4.37 (m, 1H), 3.99-3.93 (m, 1H), 3.59-3.32 (m, 3.5H), 3.02-2.90 (m, 1H), 2.73 (dd, *J* = 13.3, 10.6 Hz, 0.5H), 2.60-2.51 (m, 0.5H), 2.47-2.28 (m, 2H), 2.21 (ddd, *J =* 15.1, 10.1, 5.9 Hz, 0.5H), 2.11-1.69 (m, 10H), 1.67-1.23 (m, 14H), 1.23-1.07 (m, 2H), 1.05 (d, *J* = 6.5 Hz, 1.5H), 1.03 (d, *J* = 6.5 Hz, 1.5H), 1.01-0.94 (m, 1H), 0.93 (s, 3H), 0.71 (s, 3H); LCMS (ESI) *m*/*z* calcd for C₂₉H₄₈NO₆S [M-Na]⁻ 538.3, found 538.2.

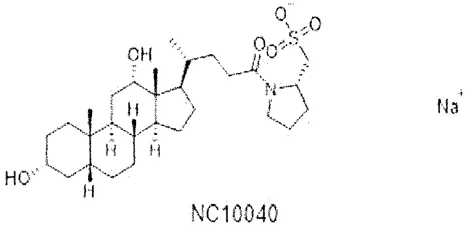

### NC10040

¹H NMR (500 MHz, CD₃OD) δ 4.52-4.38 (m, 1H), 3.99-3.93 (m, 1H), 3.60-3.33 (m, 3.5H), 3.02-2.90 (m, 1H), 2.74 (dd, *J* = 13.3, 10.6 Hz, 0.5H), 2.50-2.39 (m, 2H), 2.35 (ddd, *J* = 15.3, 10.6, 5.1 Hz, 0.5H), 2.23 (ddd, *J* = 14.6, 10.2, 5.9 Hz, 0.5H), 2.11-1.70 (m, 10H), 1.67-1.23 (m, 14H), 1.23-1.07 (m, 2H), 1.05 (d, *J* = 6.5 Hz, 1.5 H), 1.03 (d, *J* = 6.5 Hz, 1.5H), 0.933 (s, 1.5H), 0.931 (s, 1.5H), 1.01-0.95 (m, 1H), 0.933 (s, 1.5H), 0.931 (s, 1.5H), 0.713 (s, 1.5H), 0.711 (s, 1.5H); LCMS (ESI) *m*/*z* calcd for C₂₉H₄₈NO₆S [M-Na]⁻538.3, found 538.2.

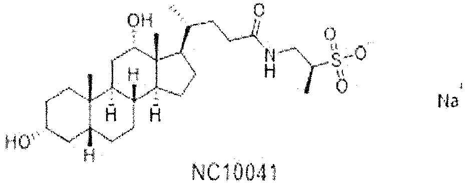

### NC10041

¹H NMR (400 MHz, DMSO-d₆) δ 7.62-7.55 (m, 1H), 4.45 (d, *J* = 4.3 Hz, 1H), 4.19 (d, *J* = 3.9 Hz, 1H), 3.78 (s, 1H), 3.41-3.22 (m, 2H), 3.17-3.06 (m, 1H), 2.48-2.41 (m, 1H), 2.06 (ddd, *J* = 14.5, 9.8, 5.0 Hz, 1H), 2.00-1.88 (m, 1H), 1.86-1.41 (m, 10H), 1.40-0.85 (m, 20H), 0.84 (s, 3H), 0.58 (s, 3H); LCMS (ESI) *m*/*z* calcd for C₂₇H₄₆NO₆S [M-Na]⁻ 512.3, found 512.2.

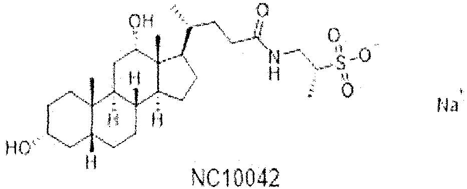

### NC10042

¹H NMR (500 MHz, CD₃OD) δ 3.96 (t, *J* = 3.0 Hz, 1H), 3.59-3.41 (m, 3H), 2.99-2.89 (m, 1H), 2.27 (ddd, *J* = 13.8, 10.1, 5.0 Hz, 1H), 2.12 (ddd, *J* = 13.9, 9.7, 6.6 Hz, 1H), 1.95-1.74 (m, 7H), 1.67-1.55 (m, 3H), 1.55-1.24 (m, 14H), 1.22-1.04 (m, 2H), 1.02 (d, *J* = 6.5 Hz, 3H), 1.01-0.94 (m, 1H), 0.93 (s, 3H), 0.71 (s, 3H); LCMS (ESI) *m*/*z* calcd for C₂₇H₄₆NO₆S [M-Na]⁻ 512.3, found 512.2.

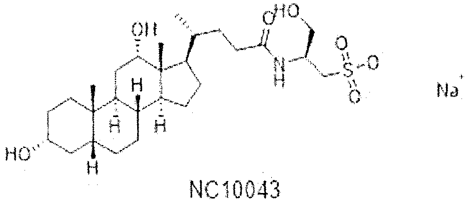

### NC10043

¹H NMR (500 MHz, CD₃OD) δ 4.34-4.26 (m, 1H), 3.96 (t, *J* = 3.2 Hz, 1H), 3.76-3.65 (m, 2H), 3.52 (tt, *J* = 11.1, 4.5 Hz, 1H), 3.14-3.07 (m, 1H), 3.06-2.99 (m, 1H), 2.32-2.25 (m, 1H), 2.17-2.09 (m, 1H), 1.95-1.73 (m, 7H), 1.66-1.55 (m, 3H), 1.55-1.23 (m, 11H), 1.23-1.06 (m, 2H), 1.04-0.94 (m, 4H), 0.93 (s, 3H), 0.71 (s, 3H); LCMS (ESI) *m*/*z* calcd for C₂₇H₄₆NO₇S [M-Na]⁻ 528.3, found 528.2.

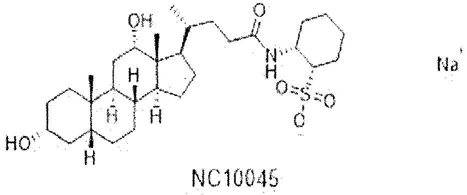

### NC10045

¹H NMR (400 MHz, DMSO-d₆) δ 7.61 (d, *J* = 4.5 Hz, 1H), 4.45 (d, *J* = 4.4 Hz, 1H), 4.20 (d, *J* = 4.0 Hz, 1H), 3.93-3.84 (m, 1H), 3.81-3.75 (m, 1H), 3.41-3.28 (m, 1H), 2.48-2.42 (m, 1H), 2.25-2.13 (m, 1H), 2.08-1.86 (m, 2H), 1.86-1.67 (m, 7H), 1.67-1.41 (m, 6H), 1.41-0.85 (m, 21H), 0.84 (s, 3H), 0.58 (s, 3H); LCMS (ESI) *m*/*z* calcd for C₃₀H₅₀NO₆S [M-Na]⁻ 552.3, found 552.3.

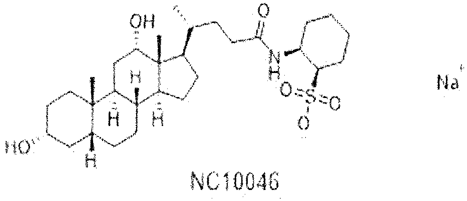

### NC10046

¹H NMR (500 MHz, CD₃OD) δ 4.33-4.27 (m, 1H), 3.96 (t, *J* = 3.0 Hz, 1H), 3.51 (tt, *J* = 11.1, 4.6 Hz, 1H), 2.91 (dt, *J* = 10.1, 4.0 Hz, 1H), 2.32 (ddd, *J* = 14.2, 10.2, 5.2 Hz, 1H), 2.25-2.18 (m, 1H), 2.13 (ddd, *J* = 14.3, 9.9, 6.5 Hz, 1H), 2.07-1.74 (m, 10H), 1.67-1.57 (m, 3H), 1.55-1.23 (m, 15H), 1.22-1.05 (m, 2H), 1.02 (d, *J* = 6.5 Hz, 3H), 1.00-0.94 (m, 1H), 0.93 (s, 3H), 0.71 (s, 3H); LCMS (ESI) *m*/*z* calcd for C₃₀H₅₀NO₆S [M-Na]⁻ 552.3, found 552.3.

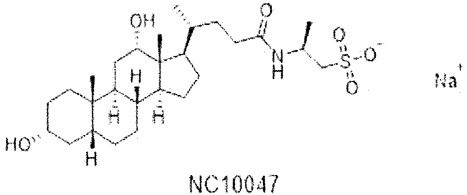

### NC10047

¹H NMR (500 MHz, CD₃OD) δ 4.37-4.27 (m, 1H), 3.98-3.93 (m, 1H), 3.52 (tt, *J* = 11.1, 4.6 Hz, 1H), 3.05 (dd, *J* = 13.9, 5.5 Hz, 1H), 2.87 (dd, *J* = 13.9, 7.0 Hz, 1H), 2.23 (ddd, *J* = 13.8, 10.2, 5.1 Hz, 1H), 2.08 (ddd, *J* = 13.8, 9.7, 6.5 Hz, 1H), 1.95-1.73 (m, 7H), 1.66-1.57 (m, 3H), 1.57-1.23 (m, 14H), 1.22-1.04 (m, 2H), 1.01 (d, *J* = 6.5 Hz, 3H), 1.00-0.94 (m, 1H), 0.93 (s, 3H), 0.71 (s, 3H); LCMS (ESI) *m*/*z* calcd for C₂₇H₄₆NO₆S [M-Na]⁻ 512.3, found 512.2.

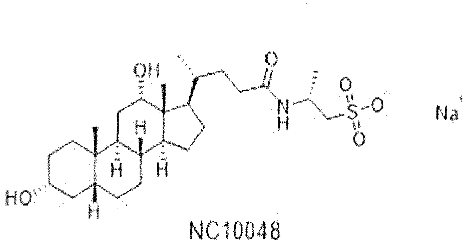

### NC10048

¹H NMR (400 MHz, DMSO-d₆) δ 7.67 (d, *J* = 7.4 Hz, 1H), 4.45 (d, J = 4.3 Hz, 1H), 4.19 (s, 1H), 4.05-3.91 (m, 1H), 3.78 (s, 1H), 3.41-3.28 (m, 1H), 2.60 (dd, *J* = 13.4, 4.6 Hz, 1H), 2.45 (dd, *J* = 13.4, 8.0 Hz, 1H), 2.02 (ddd, *J* = 14.6, 9.9, 5.1 Hz, 1H), 1.94-1.40 (m, 11H), 1.40-0.85 (m, 20H), 0.84 (s, 3H), 0.58 (s, 3H); LCMS (ESI) *m*/*Z* calcd for C₂₇H₄₆NO₆S [M-Na]⁻ 512.3, found 512.2.

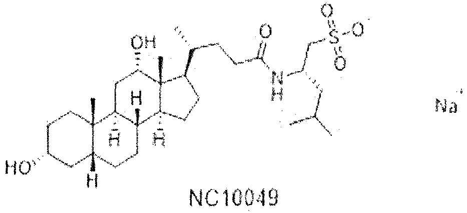

### NC10049

¹H NMR (500 MHz, CD₃OD) δ 4.39-4.31 (m, 1H), 3.96 (t, *J* = 3.0 Hz, 1H), 3.52 (tt, *J* = 11.1, 4.6 Hz, 1H), 3.02 (dd, *J* = 14.0, 5.6 Hz, 1H), 2.90 (dd, *J* = 14.0, 6.3 Hz, 1H), 2.25 (ddd, *J* = 13.8, 9.9, 5.1 Hz, 1H), 2.10 (ddd, *J* = 13.7, 9.5, 6.9 Hz, 1H), 1.95-1.74 (m, 7H), 1.73-1.23 (m, 17H), 1.23-1.04 (m, 2H), 1.04-0.95 (m, 4H), 0.95-0.89 (m, 9H), 0.71 (s, 3H); LCMS (ESI) *m*/*Z* calcd for C₃₀H₅₂NO₆S [M-Na]⁻ 554.4, found 554.3.

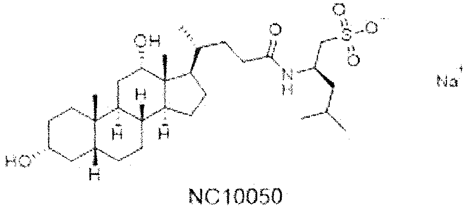

### NC10050

¹H NMR (400 MHz, DMSO-d₆) δ 7.54 (d, *J* = 8.6 Hz, 1H), 4.45 (d, *J* = 4.3 Hz, 1H), 4.18 (d, *J* = 3.7 Hz, 1H), 4.12-4.01 (m, 1H), 3.78 (s, 1H), 3.41-3.29 (m, 1H), 2.59 (dd, *J* = 13.5, 4.5 Hz, 1H), 2.46 (dd, *J* = 13.5, 7.5 Hz, 1H), 2.05-1.86 (m, 2H), 1.83-1.41 (m, 12H), 1.40-0.74 (m, 27H), 0.58 (s, 3H); LCMS (ESI) *m*/*z* calcd for C₃₀H₅₂NO₆S [M-Na]⁻ 554.4, found 554.3.

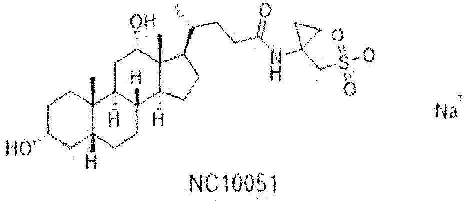

### NC10051

¹H NMR (500 MHz, CD₃OD) δ 3.97-3.92 (m, 1H), 3.56-3.46 (m, 1H), 3.03 (s, 2H), 2.21 (ddd, *J* = 13.9, 10.2, 5.0 Hz, 1H), 2.06 (ddd, *J* = 14.0, 9.7, 6.5 Hz, 1H), 1.94-1.70 (m, 7H), 1.66-1.56 (m, 3H), 1.55-1.22 (m, 11H), 1.22-1.04 (m, 2H), 1.02-0.94 (m, 6H), 0.93 (s, 3H), 0.81-0.76 (m, 2H), 0.70 (s, 3H); LCMS (ESI) *m*/*z* calcd for C₂₈H₄₆NO₆S [M-Na]⁻ 524.3, found 524.3.

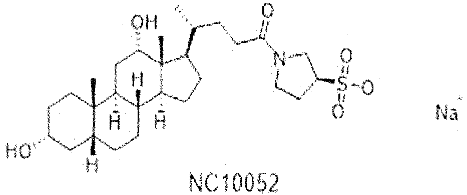

### NC10052

¹H NMR (400 MHz, 1DMSO-d₆) δ4.45 (d, *J* = 4.3 Hz, 1H), 4.22 (d, *J* = 4.0 Hz, 0.5H), 4.20 (d, *J* = 3.4 Hz, 0.5H), 3.79 (s, 1H), 3.63-3.30 (m, 4.5H), 3.25-3.14 (m, 1H), 3.13-3.04 (m, 0.5H), 2.26-1.87 (m, 4H), 1.86-1.67 (m, 4H), 1.67-1.41 (m, 6H), 1.40-1.12 (m, 11H), 1.12-0.80 (m, 9H), 0.60 (s, 1.5H), 0.59 (s, 1.5H); LCMS (ESI) *m*/*z* calcd for C₂₈H₄₆NO₆S [M-Na]⁻ 524.3, found 524.2.

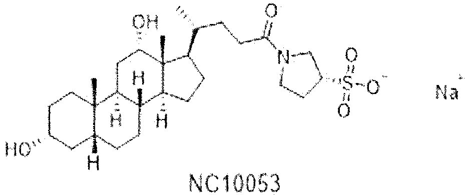

### NC10053

¹H NMR (500 MHz, CD₃OD) δ 3.98-3.93 (m, 1H), 3.90-3.65 (m, 3H), 3.65-3.40 (m, 3H), 2.47-2.18 (m, 4H), 1.95-1.71 (m, 7H), 1.66-1.56 (m, 3H), 1.56-1.24 (m, 11H), 1.23-1.07 (m, 2H), 1.04 (d, J = 6.4 Hz, 1.5H), 1.03 (d, J = 6.5 Hz, 1.5H), 1.01-0.94 (m, 1H), 0.93 (s, 3H), 0.72 (s, 1.5H), 0.71 (s, 1.5H); LCMS (ESI) *m*/*z* calcd for C₂₈H₄₆NO₆S [M-Na]⁻ 524.3, found 524.2.

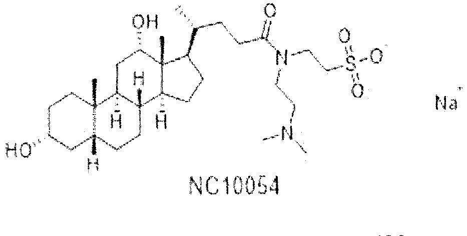

### NC10054

¹H NMR (500 MHz, CD₃OD) δ 3.99-3.93 (m, 1H), 3.81-3.75 (m, 1H), 3.74-3.68 (m, 1H), 3.60-3.46 (m, 3H), 3.11-3.02 (m, 2H), 2.59-2.33 (m, 4H), 2.31 (s, 3H), 2.29 (s, 3H), 1.95-1.72 (m, 7H), 1.68-1.56 (m, 3H), 1.55-1.23 (m, 11H), 1.23-1.06 (m, 2H), 1.06-1.02 (m, 3H), 0.98 (td, *J* = 14.2, 3.1 Hz, 1H), 0.93 (s, 3H), 0.72 (s, 3H); LCMS (ESI) *m*/*z* calcd for C₃₀H₅₃N₂O₆S [M-Na]⁻ 569.4, found 569.3.

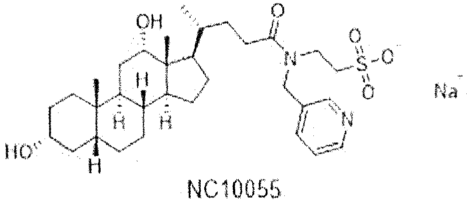

### NC10055

¹H NMR (500 MHz, CD₃OD) δ8.53-8.41 (m, 2H), 7.80-7.76 (m, 0.7H), 7.76-7.72 (m, 0.3H), 7.49-7.45 (m, 0.3H), 7.43-7.38 (m, 0.7H), 4.84 (s, 1H), 4.69-4.65 (m, 1H), 3.96 (t, *J* = 3.0 Hz, 0.7H), 3.91 (t, *J* = 3.0 Hz, 0.3H), 3.84-3.69 (m, 2H), 3.56-3.46 (m, 1H), 3.11-3.04 (m, 2H), 2.64-2.54 (m, 0.7H), 2.52-2.39 (m, 1H), 2.36-2.27 (m, 0.3H), 1.95-1.69 (m, 7H), 1.67-1.55 (m, 3H), 1.55-1.31 (m, 9H), 1.32-1.03 (m, 6H), 1.02-0.89 (m, 5H), 0.71 (s, 2H), 0.68 (s, 1H); LCMS (ESI) *m*/*z* calcd for C₃₂H₄₉N₂O₆S [M-Na]⁻ 589.3, found 589.3.

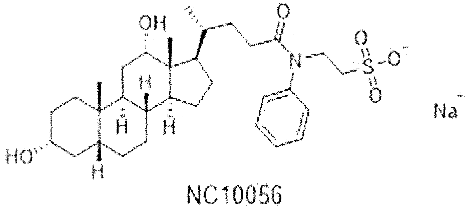

### NC10056

¹H NMR (500 MHz, CD₃OD) δ 7.52-7.46 (m, 2H), 7.45-7.38 (m, 1H), 7.35-7.29 (m, 2H), 4.13-4.06 (m, 2H), 3.85 (t, *J* = 3.0 Hz, 1H), 3.50 (tt, *J* = 11.1, 4.6 Hz, 1H), 3.09-3.02 (m, 2H), 2.19-2.09 (m, 1H), 2.03-1.94 (m, 1H), 1.93-1.62 (m, 7H), 1.62-1.34 (m, 10H), 1.32-0.87 (m, 10H), 0.76 (d, *J* = 6.1 Hz, 3H), 0.63 (s, 3H); LCMS (ESI) *m*/*z* calcd for C₃₂H₄₈NO₆S [M-Na]⁻ 574.3, found 574.3.

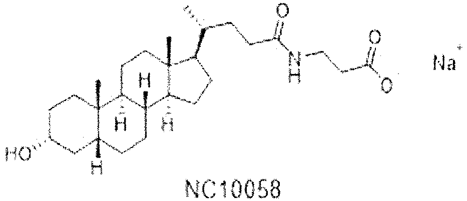

### NC10058

¹H NMR (500 MHz, CD₃OD) δ 3.53 (tt, *J* = 11.1, 4.6 Hz, 1H), 3.39 (t, *J* = 7.0 Hz, 2H), 2.34 (t, *J* = 7.0 Hz, 2H), 2.22 (ddd, *J* = 13.8, 10.4, 5.2 Hz, 1H), 2.07 (ddd, *J* = 13.8, 10.1, 6.4 Hz, 1H), 2.01 (dt, *J* = 12.5, 3.2 Hz, 1H), 1.95-1.85 (m, 2H), 1.84-1.71 (m, 3H), 1.66-1.57 (m, 2H), 1.51-1.23 (m, 12H), 1.23-1.03 (m, 5H), 1.03-0.93 (m, 7H), 0.69 (s, 3H); LCMS (ESI) m/Z calcd for C₂₇H₄₄NO₄ [M-Na]⁻ 446.3, found 446.3.

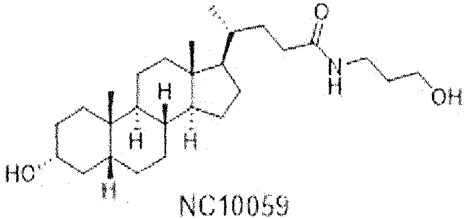

### NC10059

¹H NMR (500 MHz, CD₃OD) δ 3.58 (t, *J* = 6.3 Hz, 2H), 3.56-3.50 (m, 1H), 3.25 (td, *J* = 6.9, 1.3 Hz, 2H), 2.23 (ddd, *J* = 13.7, 10.0, 5.2 Hz, 1H), 2.09 (ddd, *J* = 13.8, 9.7, 6.7 Hz, 1H), 2.02 (dt, *J* = 12.3, 3.5 Hz, 1H), 1.96-1.85 (m, 1H), 1.85-1.74 (m, 3H), 1.73-1.67 (m, 2H), 1.66-1.56 (m, 2H), 1.51-1.24 (m, 13H), 1.23-1.05 (m, 5H), 1.03-0.92 (m, 7H), 0.69 (s, 3H); LCMS (ESI) *m*/*z* calcd for C₂₇H₄₆NO₃ [M-H]⁻ 432.3, found 432.3.

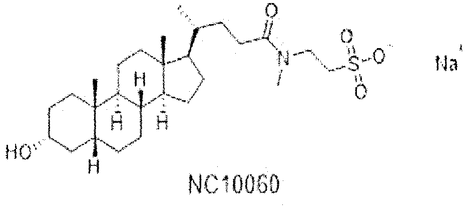

### NC10060

¹H NMR (400 MHz, DMSO-d₆) δ 4.42 (t, *J* = 5.6 Hz, 1H), 3.57-3.42 (m, 2H), 3.40-3.27 (m, 1H), 2.93 (s, 1.5H), 2.75 (s, 1.5H), 2.70-2.58 (m, 1H), 2.58-2.52 (m, 1H), 2.37-2.06 (m, 2H), 1.97-1.87 (m, 1H), 1.87-1.73 (m, 2H), 1.72-1.44 (m, 5H), 1.42-0.96 (m, 17H), 0.95-0.81 (m, 7H), 0.61 (s, 3H); LCMS (ESI) *m*/*z* calcd for C₂₇H₄₆NO₅S [M-Na]⁻ 496.3, found 496.2.

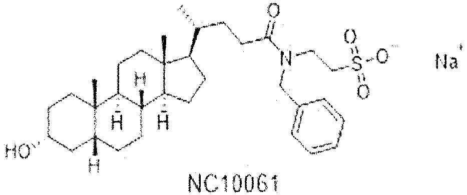

### NC10061

¹H NMR (500 MHz, CD₃OD) δ 7.41-7.34 (m, 1H), 7.34-7.20 (m, 4H), 4.79-4.69 (m, 1H), 4.68-4.58 (m, 1H), 3.84-3.68 (m, 2H), 3.57-3.48 (m, 1H), 3.07 (t, *J* = 7.1 Hz, 1H), 3.05-2.97 (m, 1H), 2.57 (ddd, *J* = 15.2, 10.3, 5.2 Hz, 0.5H), 2.50-2.33 (m, 1H), 2.27 (ddd, *J* = 15.4, 10.4, 5.7 Hz, 0.5H), 2.06-1.68 (m, 6H), 1.66-0.85 (m, 26H), 0.70 (s, 1.5H), 0.65 (s, 1.5H); LCMS (ESI) *m*/*z* calcd for C₃₃H₅₀NO₅S [M-Na]⁻ 572.3, found 572.3.

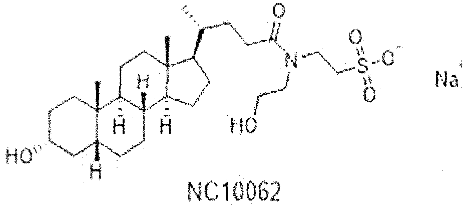

### NC10062

¹H NMR (500 MHz, CD₃OD) δ 3.89-3.78 (m, 1H), 3.77-3.69 (m, 2H), 3.67 (t, *J* = 5.7 Hz, 1H), 3.58-3.44 (m, 3H), 3.13-3.04 (m, 2H), 2.55-2.44 (m, 1H), 2.41-2.29 (m, 1H), 2.05-1.99 (m, 1H), 1.98-1.85 (m, 2H), 1.85-1.71 (m, 3H), 1.60 (s, 2H), 1.52-1.04 (m, 17H), 1.02-0.94 (m, 7H), 0.69 (s, 3H); LCMS (ESI) *m*/*z* calcd for C₂₈H₄₈NO₆S [M-Na]⁻ 526.3, found 526.3.

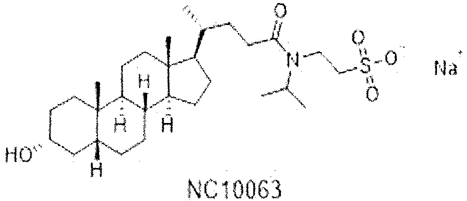

### NC10063

¹H NMR (500 MHz, CD₃OD) δ 4.66-4.55 (m, 0.5H), 4.21-4.12 (m, 0.5H), 3.72-3.65 (m, 1H), 3.65-3.58 (m, 1H), 3.58-3.47 (m, 1H), 3.10-3.01 (m, 2H), 2.44 (ddd, *J* = 15.3, 10.5, 5.1 Hz, 1H), 2.37-2.25 (m, 1H), 2.05-1.99 (m, 1H), 1.95-1.85 (m, 2H), 1.85-1.71 (m, 3H), 1.66-1.57 (m, 2H), 1.51-1.04 (m, 23H), 1.03-0.93 (m, 7H), 0.70 (s, 1.5H), 0.69 (s, 1.5H); LCMS (ESI) *m*/*z* calcd for C₂₉H₅₀NO₅S [M-Na]⁻ 524.3, found 524.3.

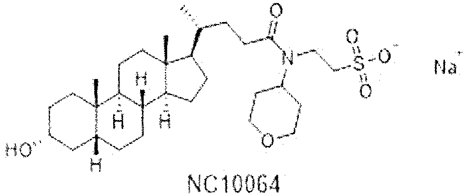

### NC10064

¹H NMR (500 MHz, CD₃OD) δ 4.43 (tt, *J* = 12.1, 3.9 Hz, 0.5H), 4.06-3.89 (m, 2.5H), 3.75-3.62 (m, 2H), 3.58-3.42 (m, 3H), 3.08-3.01 (m, 2H), 2.53-2.42 (m, 1H), 2.40-2.30 (m, 1H), 2.05-1.85 (m, 5H), 1.85-1.70 (m, 3H), 1.70-1.54 (m, 4H), 1.50-1.04 (m, 17H), 1.03-0.95 (m, 4H), 0.949 (s, 1.5H), 0.945 (s, 1.5H), 0.70 (s, 1.5H), 0.69 (s, 1.5H); LCMS (ESI) *m*/*z* calcd for C₃₁H₅₂NO₆S [M-Na]⁻ 566.4, found 566.3.

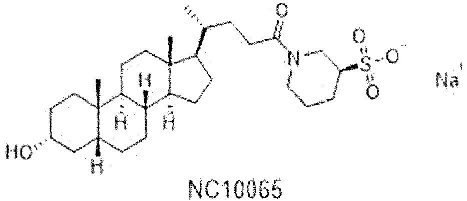

### NC10065

¹H NMR (500 MHz, CD₃OD) δ 5.05-4.98 (m, 0.4H), 4.42 (d, J = 12.9 Hz, 0.6H), 4.32-4.25 (m, 0.6H), 3.92 (d, *J* = 12.9 Hz, 0.4H), 3.52 (tt, *J* = 11.4, 4.3 Hz, 1H), 3.24 (dd, *J* = 13.5, 11.1 Hz, 0.6H), 3.01 (td, *J* = 13.2, 2.8 Hz, 0.4H), 2.84-2.56 (m, 2H), 2.56-2.39 (m, 1H), 2.38-2.24 (m, 2H), 2.02 (dt, *J* = 12.4, 3.1 Hz, 1H), 1.98-1.68 (m, 7H), 1.66-1.57 (m, 2H), 1.54-1.05 (m, 18H), 1.03-0.97 (m, 4H), 0.95 (s, 3H), 0.69 (s, 3H); LCMS (ESI) *m*/*z* calcd for C₂₉H₄₈NO₅S [M-Na]⁻ 522.3, found 522.3.

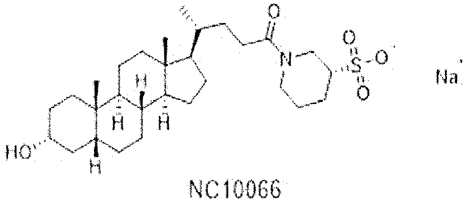

### NC10066

¹H NMR (400 MHz, DMSO-d₆) δ 4.71 (d, *J* = 13.6 Hz, 0.5H), 4.42 (s, 1H), 4.28 (d, *J* = 12.9 Hz, 0.5H), 4.03 (d, *J* = 13.2 Hz, 0.5H), 3.77 (d, *J* = 13.3 Hz, 0.5H), 3.41-3.27 (m, 1H), 2.99-2.88 (m, 0.5H), 2.85-2.75 (m, 0.5H), 2.42-2.24 (m, 2.5H), 2.22-2.08 (m, 1.5H), 2.06-1.98 (m, 1H), 1.96-1.89 (m, 1H), 1.83-1.73 (m, 2H), 1.72-1.43 (m, 7H), 1.43-0.96 (m, 18H), 0.94-0.84 (m, 7H), 0.61 (s, 3H); LCMS (ESI) *m*/*z* calcd for C₂₉H₄₈NO₅S [M-Na]⁻ 522.3, found 522.3.

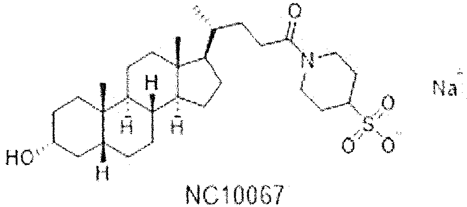

### NC10067

¹H NMR (400 MHz, DMSO-d₆) δ 4.45-4.32 (m, 2H), 3.85 (d, *J* = 13.5 Hz, 1H), 3.41-3.27 (m, 1H), 2.92 (t, *J* = 12.5 Hz, 1H), 2.47-2.37 (m, 2H), 2.36-2.10 (m, 2H), 1.96-1.72 (m, 5H), 1.72-1.44 (m, 5H), 1.44-0.96 (m, 19H), 0.95-0.82 (m, 7H), 0.61 (s, 3H). ; LCMS (ESI) *m*/*z* calcd for C₂₉H₄₈NO₅S [M-Na]⁻522.3, found 522.2.

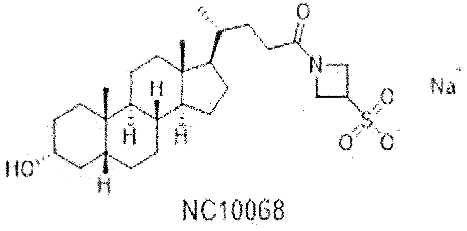

### NC10068

¹H NMR (500 MHz, CD₃OD) δ4.92 (t, *J* = 8.8 Hz, 1H), 4.36-4.30 (m, 1H), 4.21-4.11 (m, 2H), 3.85-3.76 (m, 1H), 3.58-3.48 (m, 1H), 2.25-2.12 (m, 1H), 2.11-1.98 (m, 2H), 1.95-1.85 (m, 2H), 1.85-1.69 (m, 3H), 1.65-1.57 (m, 2H), 1.51-1.05 (m, 17H), 1.03-0.98 (, 1H), 0.96 (d, *J* = 6.5 Hz, 3H), 0.95 (s, 3H), 0.69 (s, 3H); LCMS (ESI) *m*/*z* calcd for C₂₇H₄₄NO₅S [M-Na]⁻ 494.3, found 494.2.

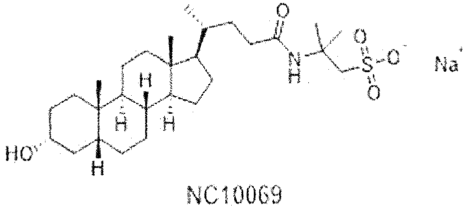

### NC10069

¹H NMR (500 MHz, CD₃OD) δ3.57-3.48 (m, 1H), 3.21-3.09 (m, 2H), 2.19 (ddd, *J* = 13.9, 10.3, 5.2 Hz, 1H), 2.08-1.98 (m, 2H), 1.96-1.85 (m, 2H), 1.84-1.71 (m, 3H), 1.67-1.56 (m, 2H), 1.53 (s, 6H), 1.50-1.04 (m, 17H), 1.03-0.92 (m, 7H), 0.69 (s, 3H); LCMS (ESI) m/z calcd for C₂₈H₄₈NO₅S [M-Na]⁻ 510.3, found 510.3.

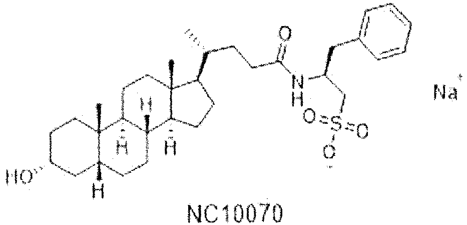

### NC10070

¹H NMR (500 MHz, CD₃OD) δ 7.29-7.22 (m, 4H), 7.22-7.14 (m, 1H), 4.50-4.42 (m, 1H), 3.58-3.48 (m, 1H), 3.20-3.13 (m, 1H), 3.05-2.95 (m, 2H), 2.92 (dd, *J* = 13.6, 7.8 Hz, 1H), 2.16 (ddd, *J* = 13.8, 10.8, 5.1 Hz, 1H), 2.00 (ddd, *J* = 13.8, 10.5, 6.2 Hz, 2H), 1.96-1.57 (m, 7H), 1.50-1.04 (m, 17H), 1.03-0.95 (m, 1H), 0.94 (s, 3H), 0.92 (d, *J* = 6.6 Hz, 3H), 0.67 (s, 3H); LCMS (ESI) *m*/*z* calcd for C₃₃H₅₀NO₅S [M-Na]⁻ 572.3, found 572.3.

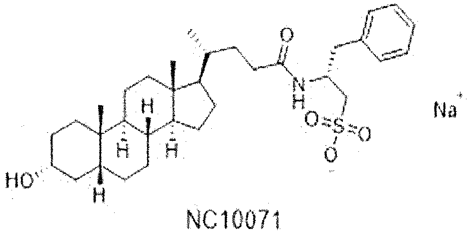

### NC10071

¹H NMR (500 MHz, CD₃OD) δ 7.30-7.22 (m, 4H), 7.22-7.14 (m, 1H), 4.52-4.43 (m, 1H), 3.53 (tt, *J* = 11.1, 4.6 Hz, 1H), 3.16 (dd, *J* = 13.7, 6.1 Hz, 1H), 3.04-2.93 (m, 2H), 2.91 (dd, *J* = 13.7, 7.8 Hz, 1H), 2.17 (ddd, *J* = 13.8, 10.4, 5.1 Hz, 1H), 2.06-1.96 (m, 2H), 1.96-1.55 (m, 7H), 1.51-1.04 (m, 17H), 0.99 (td, *J* = 14.1, 3.3 Hz, 1H), 0.94 (s, 3H), 0.91 (d, *J* = 6.5 Hz, 3H), 0.66 (s, 3H); LCMS (ESI) *m*/*z* calcd for C₃₃H₅₀NO₅S [M-Na]⁻ 572.3, found 572.3.

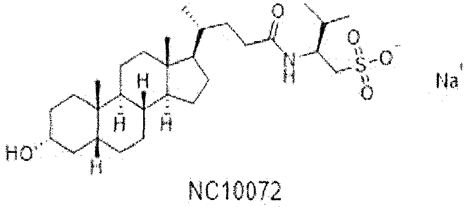

### NC10072

¹H NMR (500 MHz, CD₃OD) δ 4.23-4.16 (m, 1H), 3.53 (tt, *J* = 11.1, 4.6 Hz, 1H), 3.01-2.90 (m, 2H), 2.28 (ddd, *J* = 13.7, 10.6, 5.2 Hz, 1H), 2.15-1.98 (m, 3H), 1.97-1.86 (m, 2H), 1.85-1.71 (m, 3H), 1.65-1.56 (m, 2H), 1.51-1.23 (m, 12H), 1.23-1.03 (m, 5H), 1.03-0.88 (m, 13H), 0.69 (s, 3H); LCMS (ESI) *m*/*z* calcd for C₂₉H₅₀NO₅S [M-Na]⁻ 524.3, found 524.3.

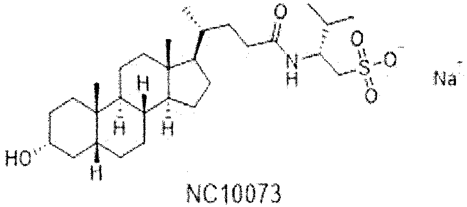

### NC10073

¹H NMR (400 MHz, DMSO-d₆) δ 7.46 (d, *J* = 8.4 Hz, 1H), 4.42 (d, *J* = 4.6 Hz, 1H), 3.98-3.87 (m, 1H), 3.41-3.27 (m, 1H), 2.55-2.51 (m, 2H), 2.15-1.88 (m, 4H), 1.84-1.72 (m, 2H), 1.72-1.43 (m, 5H), 1.41-0.95 (m, 17H), 0.94-0.82 (m, 7H), 0.76 (s, 3H), 0.74 (s, 3H), 0.60 (s, 3H); LCMS (ESI) *m*/*z* calcd for C₂₉H₅₀NO₅S [M-Na]⁻ 524.3, found 524.3.

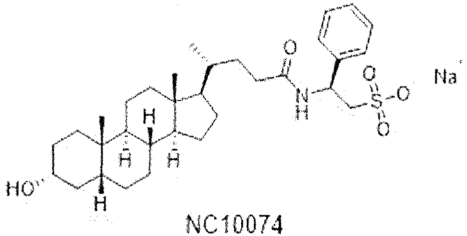

### NC10074

¹H NMR (500 MHz, CD₃OD) δ 7.37-7.27 (m, 4H), 7.24-7.19 (m, 1H), 5.35 (dd, *J* = 10.0, 3.7 Hz, 1H), 3.53 (tt, *J* = 11.1, 4.6 Hz, 1H), 3.26 (dd, *J* = 14.3, 10.0 Hz, 1H), 3.09 (dd, *J* = 14.3, 3.8 Hz, 1H), 2.31 (ddd, *J* = 13.9, 10.1, 5.4 Hz, 1H), 2.15 (ddd, *J* = 14.0, 9.8, 6.5 Hz, 1H), 1.99 (dt, *J* = 12.5, 3.3 Hz, 1H), 1.95-1.71 (m, 5H), 1.66-1.52 (m, 2H), 1.52-1.21 (m, 12H), 1.21-0.90 (m, 12H), 0.65 (s, 3H); LCMS (ESI) *m*/*z* calcd for C₃₂H₄₈NO₅S [M-Na]⁻558.3, found 558.3.

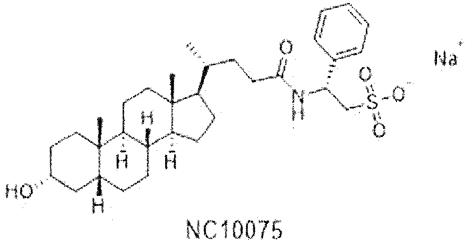

### NC10075

¹H NMR (500 MHz, CD₃OD) δ 7.37-7.26 (m, 4H), 7.24-7.18 (m, 1H), 5.35 (dd, *J* = 10.1, 3.7 Hz, 1H), 3.53 (tt, *J* = 11.1, 4.6 Hz, 1H), 3.26 (dd, *J* = 14.3, 10.1 Hz, 1H), 3.09 (dd, *J* = 14.3, 3.7 Hz, 1H), 2.29 (ddd, *J* = 14.4, 9.7, 5.0 Hz, 1H), 2.17 (ddd, *J* = 13.9, 9.2, 7.1 Hz, 1H), 2.00 (dt, *J* = 12.4, 3.2 Hz, 1H), 1.95-1.71 (m, 5H), 1.65-1.52 (m, 2H), 1.51-0.91 (m, 24H), 0.63 (s, 3H); LCMS (ESI) *m*/*z* calcd for C₃₂H₄₈NO₅S [M-Na]⁻ 558.3, found 558.3.

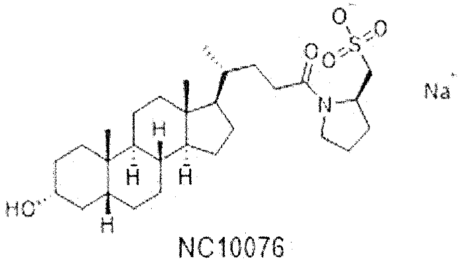

### NC10076

¹H NMR (500 MHz, CD₃OD) δ 4.52-4.44 (m, 0.5H), 4.44-4.37 (m, 0.5H), 3.59-3.32 (m, 3.5H), 3.02-2.90 (m, 1H), 2.73 (dd, *J* = 13.3, 10.6 Hz, 0.5H), 2.55 (ddd, *J* = 15.3, 10.6, 5.3 Hz, 0.5H), 2.47-2.27 (m, 2H), 2.19 (ddd, *J* = 14.7, 10.4, 5.8 Hz, 0.5H), 2.11-1.85 (m, 6H), 1.85-1.70 (m, 3H), 1.66-1.56 (m, 2H), 1.50-1.03 (m, 17H), 1.02-0.91 (m, 4H), 0.95 (s, 3H), 0.69 (s, 3H); LCMS (ESI) *m*/*z* calcd for C₂₉H₄₈NO₅S [M-Na]⁻ 522.3, found 522.3.

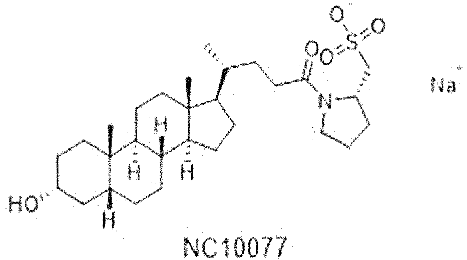

### NC10077

¹H NMR (500 MHz, CD₃OD) δ 4.51-4.44 (m, 0.5H), 4.44-4.37 (m, 0.5H), 3.59-3.32 (m, 3.5H), 3.01-2.90 (m, 1H), 2.74 (dd, *J* = 13.3, 10.6 Hz, 0.5H), 2.48-2.38 (m, 2H), 2.34 (ddd, *J* = 15.5, 10.8, 5.2 Hz, 0.5H), 2.21 (ddd, *J* = 14.7, 10.5, 5.8 Hz, 0.5H), 2.10-1.85 (m, 6H), 1.85-1.71 (m, 3H), 1.67-1.56 (m, 2H), 1.50-1.03 (m, 17H), 1.03-0.96 (m, 4H), 0.95 (s, 3H), 0.694 (s, 1.5H), 0.690 (s, 1.5H); LCMS (ESI) *m*/*z* calcd for C₂₉H₄₈NO₅S [M-Na]⁻ 522.3, found 522.3.

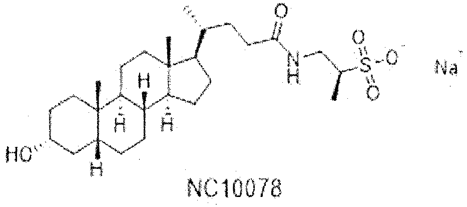

### NC10078

¹H NMR (500 MHz, CD₃OD) δ 3.61-3.48 (m, 2H), 3.44 (dd, *J* = 13.8, 6.7 Hz, 1H), 3.00-2.90 (m, 1H), 2.25 (ddd, *J* = 13.9, 10.2, 5.1 Hz, 1H), 2.11 (ddd, *J* = 13.9, 9.8, 6.7 Hz, 1H), 2.01 (dt, *J* = 12.4, 3.2 Hz, 1H), 1.96-1.85 (m, 2H), 1.85-1.71 (m, 3H), 1.66-1.57 (m, 2H), 1.51-1.24 (m, 15H), 1.23-1.03 (m, 5H), 1.03-0.98 (m, 1H), 0.96 (d, *J* = 6.6 Hz, 3H), 0.95 (s, 3H), 0.69 (s, 3H); LCMS (ESI) *m*/*z* calcd for C₂₇H₄₆NO₅S [M-Na]⁻ 496.3, found 496.2.

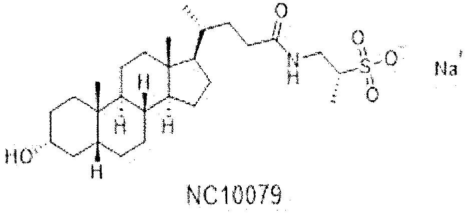

### NC10079

¹H NMR (500 MHz, CD₃OD) δ 3.59-3.49 (m, 2H), 3.46 (dd, *J* = 13.7, 6.8 Hz, 1H), 2.99-2.89 (m, 1H), 2.26 (ddd, *J* = 13.9, 10.3, 5.2 Hz, 1H), 2.11 (ddd, *J* = 14.0, 9.9, 6.6 Hz, 1H), 2.01 (dt, *J* = 12.4, 3.2 Hz, 1H), 1.96-1.85 (m, 2H), 1.84-1.71 (m, 3H), 1.66-1.56 (m, 2H), 1.51-1.23 (m, 15H), 1.23-1.04 (m, 5H), 1.03-0.98 (m, 1H), 0.96 (d, *J* = 0.65 Hz, 3H), 0.95 (s, 3H), 0.69 (s, 3H); LCMS (ESI) *m*/*z* calcd for C₂₇H₄₆NO₅S [M-Na]⁻ 496.3, found 496.2.

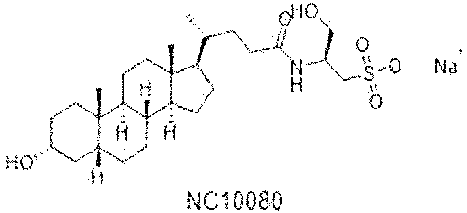

### NC10080

¹H NMR (500 MHz, CD₃OD) δ 4.34-4.25 (m, 1H), 3.75-3.65 (m, 2H), 3.53 (tt, *J* = 10.5, 4.6 Hz, 1H), 3.13-3.07 (m, 1H), 3.06-3.00 (m, 1H), 2.32-2.17 (m, 1H), 2.16-1.98 (m, 2H), 1.97-1.85 (m, 2H), 1.85-1.71 (m, 3H), 1.67-1.55 (m, 2H), 1.51-1.23 (m, 12H), 1.23-1.04 (m, 5H), 1.03-0.90 (m, 7H), 0.69 (s, 3H); LCMS (ESI) *m*/*z* calcd for C₂₇H₄₆NO₆S [M-Na]⁻ 512.3, found 512.2.

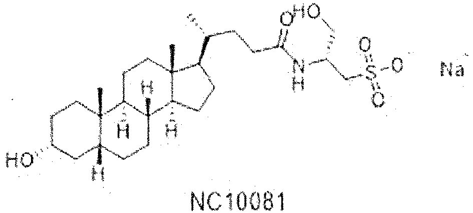

### NC10081

¹H NMR (500 MHz, CD₃OD) δ 4.33-4.26 (m, 1H), 3.76-3.64 (m, 2H), 3.57-3.48 (m, 1H), 3.14-3.07 (m, 1H), 3.07-2.99 (m, 1H), 2.32-2.17 (m, 1H), 2.16-1.98 (m, 2H), 1.96-1.85 (m, 2H), 1.85-1.72 (m, 3H), 1.66-1.55 (m, 2H), 1.51-1.23 (m, 12H), 1.23-1.04 (m, 5H), 1.03-0.91 (m, 7H), 0.69 (s, 3H); LCMS (ESI) *m*/*z* calcd for C₂₇H₄₆NO₆S [M-Na]⁻ 512.3, found 512.3.

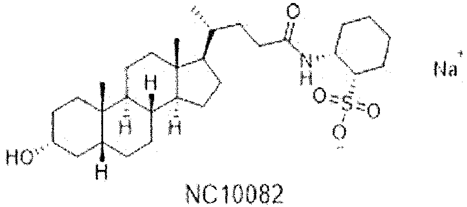

### NC10082

¹H NMR (500 MHz, CD₃OD) δ 4.33-4.27 (m, 1H), 3.52 (tt, *J* = 11.1, 4.6 Hz, 1H), 2.91 (dt, *J* = 10.2, 4.0 Hz, 1H), 2.31-2.19 (m, 2H), 2.15 (ddd, *J* = 14.1, 9.6, 6.9 Hz, 1H), 2.07-1.85 (m, 6H), 1.84-1.71 (m, 3H), 1.66-1.56 (m, 2H), 1.55-1.24 (m, 16H), 1.23-1.03 (m, 5H), 1.02-0.98 (m, 1H), 0.97 (d, *J* = 6.6 Hz, 3H), 0.95 (s, 3H), 0.68 (s, 3H); LCMS (ESI) *m*/*z* calcd for C₃₀H₅₀NO₅S [M-Na]⁻ 536.3, found 536.3.

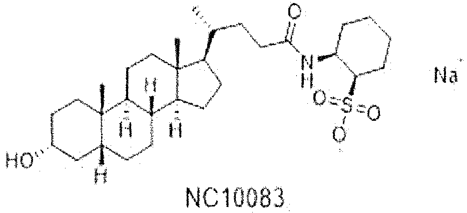

### NC10083

¹H NMR (500 MHz, CD₃OD) δ 4.33-4.27 (m, 1H), 3.53 (tt, *J* = 11.1, 4.6 Hz, 1H), 2.91 (dt, *J* = 10.1, 4. 0 Hz, 1H), 2. 31 (ddd, *J* = 14.2, 10.3, 5.3 Hz, 1H), 2.25-2.18 (m, 1H), 2.12 (ddd, *J* = 14.3, 10.1, 6.4 Hz, 1H), 2.06-1.85 (m, 6H), 1.84-1.72 (m, 3H), 1.66-1.56 (m, 2H), 1.55-1.24 (m, 16H), 1.24-1.03 (m, 5H), 1.03-0.98 (m, 1H), 0.96 (d, *J* = 6.5 Hz, 3H), 0.95 (s, 3H), 0.69 (s, 3H); LCMS (ESI) *m*/*z* calcd for C₃₀H₅₀NO₅S [M-Na]⁻ 536.3, found 536.3.

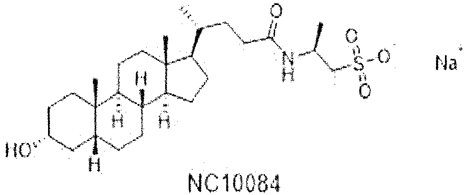

### NC10084

¹H NMR (500 MHz, CD₃OD) δ 4.37-4.27 (m, 1H), 3.58-3.48 (m, 1H), 3.05 (dd, *J* = 13.9, 5.5 Hz, 1H), 2.88 (dd, *J* = 13.9, 7.0 Hz, 1H), 2.22 (ddd, *J* = 13.8, 10.3, 5.2 Hz, 1H), 2.12-1.98 (m, 2H), 1.96-1.85 (m, 2H), 1.85-1.71 (m, 3H), 1.66-1.56 (m, 2H), 1.51-1.24 (m, 15H), 1.23-1.03 (m, 5H), 1.03-0.97 (m, 1H), 0.96 (d, *J* = 6.5 Hz, 3H), 0.94 (s, 3H), 0.69 (s, 3H); LCMS (ESI) m/z calcd for C₂₇H₄₆NO₅S [M-Na]⁻ 496.3, found 496.2.

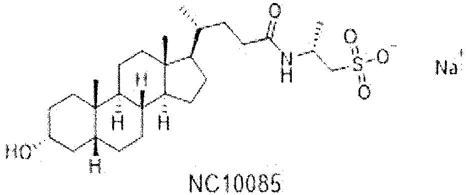

### NC10085

¹H NMR (500 MHz, CD₃OD) δ 4.37-4.27 (m, 1H), 3.53 (tt, *J* = 11.1, 4.6 Hz, 1H), 3.05 (dd, *J* = 13.9, 5.6 Hz, 1H), 2.88 (dd, *J* = 13.9, 6.9 Hz, 1H), 2.22 (ddd, *J* = 13.8, 10.2, 5.1 Hz, 1H), 2.11-1.98 (m, 2H), 1.96-1.85 (m, 2H), 1.85-1.71 (m, 3H), 1.66-1.56 (m, 2H), 1.50-1.24 (m, 15H), 1.23-1.04 (m, 5H), 1.03-0.97 (m, 1H), 0.96 (d, *J* = 6.5 Hz, 3H), 0.94 (s, 3H), 0.69 (s, 3H); LCMS (ESI) *m*/*z* calcd for C₂₇H₄₆NO₅S [M-Na]⁻ 496.3, found 496.2.

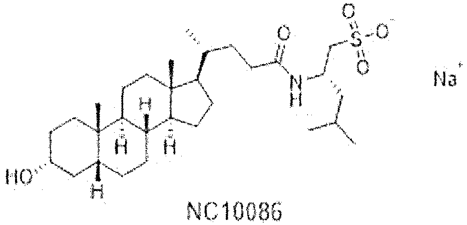

### NC10086

¹H NMR (500 MHz, CD₃OD) δ 4.39-4.30 (m, 1H), 3.53 (tt, *J* = 11.1, 4.6 Hz, 1H), 3.03 (dd, *J* = 14.0, 5.6 Hz, 1H), 2.90 (dd, *J* = 14.0, 6.2 Hz, 1H), 2.23 (ddd, *J* = 13.8, 9.9, 5.3 Hz, 1H), 2.08 (ddd, *J* = 13.8, 9.6, 6.8 Hz, 1H), 2.02 (dt, *J* = 12.4, 3.2 Hz, 1H), 1.96-1.85 (m, 2H), 1.85-1.72 (m, 3H), 1.70-1.52 (m, 5H), 1.51-1.24 (m, 12H), 1.23-1.04 (m, 5H), 1.04-0.89 (m, 13H), 0.69 (s, 3H); LCMS (ESI) *m*/*z* calcd for C₃₀H₅₂NO₅S [M-Na]⁻ 538.4, found 538.3.

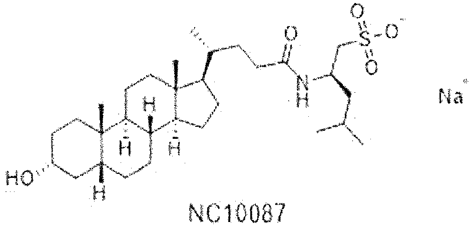

### NC10087

¹H NMR (500 MHz, CD₃OD) δ 4.42-4.33 (m, 1H), 3.53 (tt, *J* = 11.1, 4.6 Hz, 1H), 3.01 (dd, *J* = 14.0, 5.6 Hz, 1H), 2.90 (dd, *J* = 14.0, 6.3 Hz, 1H), 2.22 (ddd, *J* = 14.2, 9.4, 5.1 Hz, 1H), 2.10 (ddd, *J* = 13.8, 9.0, 7.4 Hz, 1H), 2.01 (dt, *J* = 12.5, 3.2 Hz, 1H), 1.96-1.85 (m, 2H), 1.85-1.71 (m, 3H), 1.71-1.51 (m, 5H), 1.50-1.04 (m, 17H), 1.04-0.89 (m, 13H), 0.68 (s, 3H); LCMS (ESI) *m*/*z* calcd for C₃₀H₅₂NO₅S [M-Na]⁻ 538.4, found 538.3.

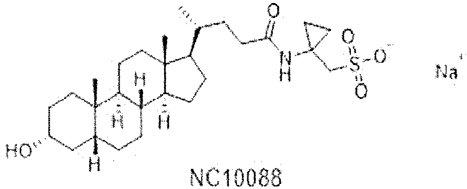

### NC10088

¹H NMR (500 MHz, CD₃OD) δ 3.53 (tt, *J* = 11.1, 4.6 Hz, 1H), 3.08-2.97 (m, 2H), 2.20 (ddd, *J* = 14.0, 10.4, 5.2 Hz, 1H), 2.09-1.97 (m, 2H), 1.96-1.85 (m, 2H), 1.85-1.70 (m, 3H), 1.66-1.56 (m, 2H), 1.51-1.23 (m, 12H), 1.22-1.04 (m, 5H), 1.03-0.90 (m, 9H), 0.82-0.76 (m, 2H), 0.68 (s, 3H); LCMS (ESI) *m*/*z* calcd for C₂₈H₄₆NO₅S [M-Na]⁻ 508.3, found 508.3.

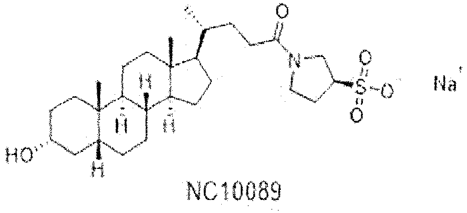

### NC10089

¹H NMR (500 MHz, CD₃OD) δ 3.88-3.65 (m, 3H), 3.65-3.39 (m, 3H), 2.47-2.16 (m, 4H), 2.09-1.99 (m, 1H), 1.95-1.85 (m, 2H), 1.85-1.71 (m, 3H), 1.67-1.56 (m, 2H), 1.53-1.04 (m, 17H), 1.04-0.92 (m, 7H), 0.73-0.65 (m, 3H); LCMS (ESI) *m*/*z* calcd for C₂₈H₄₆NO₅S [M-Na]⁻ 508.3, found 508.3.

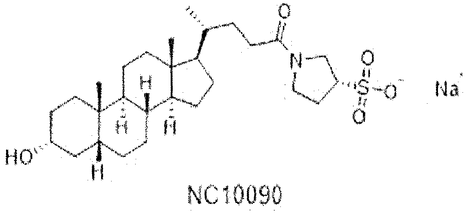

### NC10090

¹H NMR (400 MHz, DMSO-d₆) δ 4.43 (s, 1H), 3.65-3.29 (m, 4.5H), 3.27-3.14 (m, 1H), 3.13-3.04 (m, 0.5H), 2.26-2.14 (m, 1H), 2.14-1.87 (m, 4H), 1.85-1.73 (m, 2H), 1.72-1.44 (m, 5H), 1.43-0.96 (m, 17H), 0.94-0.81 (m, 7H), 0.61 (s, 3H); LCMS (ESI) *m*/*z* calcd for C₂₈H₄₆NO₅S [M-Na]⁻ 508.3, found 508.3.

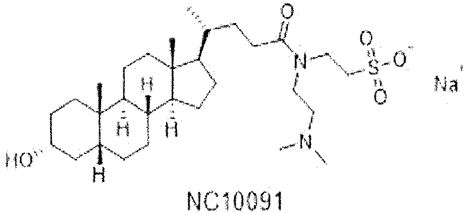

### NC10091

¹H NMR (500 MHz, CD₃OD) δ 3.80-3.74 (m, 1H), 3.73-3.68 (m, 1H), 3.59-3.44 (m, 3H), 3.12-3.02 (m, 2H), 2.58-2.22 (m, 10H), 2.02 (dt, *J* = 12.5, 3.1 Hz, 1H), 1.96-1.85 (m, 2H), 1.84-1.71 (m, 3H), 1.66-1.57 (m, 2H), 1.52-1.05 (m, 17H), 1.03-0.96 (m, 4H), 0.95 (s, 3H), 0.70 (s, 1.5H), 0.69 (s, 1.5H); LCMS (ESI) MIZ calcd for C₃₀H₅₃N₂O₅S [M-Na]⁻ 553.4, found 553.3.

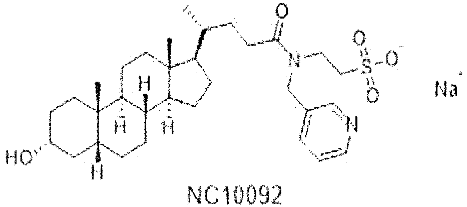

### NC10092

¹H NMR (500 MHz, CD₃OD) δ 8.52-8.41 (m, 2H), 7.81-7.72 (m, 1H), 7.51-7.37 (m, 1H), 4.86-4.79 (m, 0.6H), 4.72-4.65 (m, 1.4H), 3.84-3.68 (m, 2H), 3.57-3.48 (m, 1H), 3.11-3.02 (m, 2H), 2.63-2.53 (m, 0.3H), 2.34-2.25 (m, 1H), 2.51-2.37 (m, 0.7H), 2.05-1.69 (m, 6H), 1.66-1.54 (m, 2H), 1.52-1.03 (m, 17H), 1.02-0.96 (m, 3H), 0.96-0.92 (m, 3H), 0.86 (d, *J* = 6.5 Hz, 1H), 0.69 (s, 2H), 0.65 (s, 1H); LCMS (ESI) *m*/*z* calcd for C₃₂H₄₉N₂O₅S [M-Na]⁻ 573.3, found 573.3.

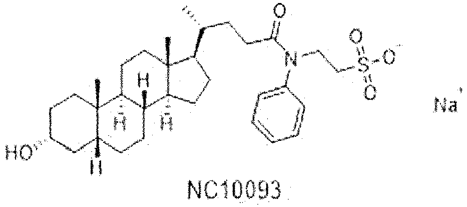

### NC10093

¹H NMR (500 MHz, CD₃OD) δ 7.53-7.47 (m, 2H), 7.46-7.39 (m, 1H), 7.35-7.29 (m, 2H), 4.13-4.06 (m, 2H), 3.52 (tt, *J* = 11.1, 4.6 Hz, 1H), 3.09-3.02 (m, 2H), 2.17-2.08 (m, 1H), 2.01-1.83 (m, 3H), 1.83-1.51 (m, 6H), 1.51-0.94 (m, 18H), 0.92 (s, 3H), 0.69 (d, *J* = 6.0 Hz, 3H), 0.61 (s, 3H); LCMS (ESI) *m*/*z* calcd for C₃₂H₄₈NO₅S [M-Na]⁻ 558.3, found 558.3.

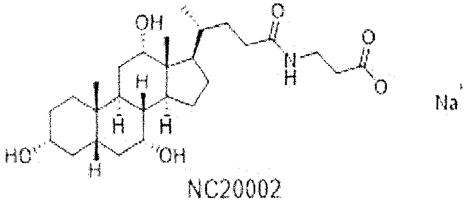

### NC20002

¹H NMR (400 MHz, DMSO-d₆) δ 7.83 (t, *J* = 5.6 Hz, 1H), 4.31 (s, 1H), 4.09 (s, 1H), 4.01 (s, 1H), 3.78 (s, 1H), 3.61 (s, 1H), 3.24-3.13 (m, 3H), 2.32 (t, *J* = 6.9 Hz, 2H), 2.28-1.87 (m, 5H), 1.83-1.55 (m, 6H), 1.51-1.06 (m, 11H), 1.02-0.82 (m, 5H), 0.81 (s, 3H), 0.58 (s, 3H); LCMS (ESI) *m*/*z* calcd for C₂₇H₄₄NO₆ [M-Na]⁻ 478.3, found 478.2.

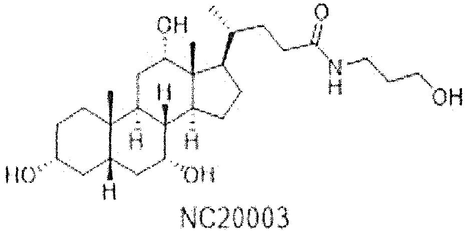

### NC20003

¹H NMR (500 MHz, CD₃OD) δ 3.98-3.93 (m, 1H), 3.82-3.77 (m, 1H), 3.58 (t, *J* = 6.4 Hz, 2H), 3.41-3.33 (m, 1H), 3.25 (t, *J* = 7.3 Hz, 2H), 2.33-2.20 (m, 3H), 2.11 (ddd, *J* = 13.7, 9.5, 6.7 Hz, 1H), 2.04-1.49 (m, 15H), 1.48-1.24 (m, 5H), 1.12 (qd, *J* = 12.0, 5.8 Hz, 1H), 1.03 (d, *J* = 6.4 Hz, 3H), 1.02-0.94 (m, 1H), 0.92 (s, 3H), 0.71 (s, 3H); LCMS (ESI) m/Z calcd for C₂₇H₄₆NO₅ [M-H]⁻ 464.3, found 464.1.

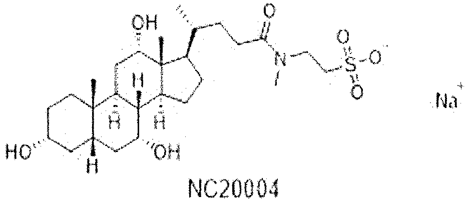

### NC20004

¹H NMR (400 MHz, DMSO-d₆) δ 4.30 (s, 1H), 4.12 (s, 1H), 4.01 (s, 1H), 3.79 (s, 1H), 3.61 (s, 1H), 3.56-3.42 (m, 2H), 3.17 (s, 1H), 2.93 (s, 1.5H) 2.75 (s, 1.5H), 2.69-2.60 (m, 1H), 2.58-2.52 (m, 1H), 2.36-2.07 (m, 4H), 2.05-1.94 (m, 1H), 1.85-1.55 (m, 6H), 1.51-1.07 (m, 11H), 1.03-0.82 (m, 5H), 0.81 (s, 3H), 0.59 (s, 3H); LCMS (ESI) *m*/*z* calcd for C₂₇H₄₆NO₇S [M-Na]⁻ 528.3, found 528.1.

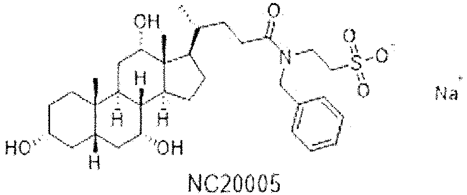

### NC20005

¹H NMR (500 MHz, CD₃OD) δ 7.41-7.34 (m, 1H), 7.34-7.20 (m, 4H), 4.74 (s, 1H), 4.63 (s, 1H), 3.99-3.94 (m, 0.5H) 3.92-3.88 (m, 0.5H), 3.82-3.68 (m, 3H), 3.37 (m, 1H), 3.10-2.96 (m, 2H), 2.63-2.54 (m, 0.5H) 2.52-2.39 (m, 1H), 2.36-2.19 (m, 2.5H), 2.06-1.18 (m, 18H), 1.17-0.88 (m, 8H), 0.72 (s, 1.5H), 0.68 (s, 1.5H); LCMS (ESI) *m*/*z* calcd for C₃₃H₅₀NO₇S [M-Na]⁻ 604.3, found 604.2.

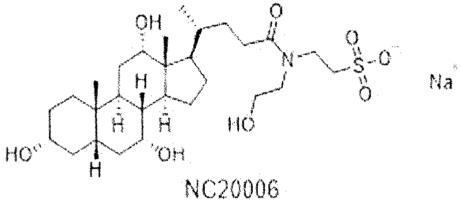

### NC20006

¹H NMR (500 MHz, CD₃OD) δ 3.99-3.93 (m, 1H), 3.89-3.82 (m, 1H), 3.82-3.77 (m, 1H), 3.77-3.64 (m, 3H), 3.60-3.52 (m, 1H), 3.51-3.44 (m, 1H), 3.41-3.32 (m, 1H), 3.15-3.04 (m, 2H), 2.55-2.47 (m, 1H), 2.41-2.33 (m, 1H), 2.32-2.23 (m, 2H), 2.04-1.70 (m, 7H), 1.69-1.49 (m, 6H), 1.49-1.27 (m, 5H), 1.17-1.02 (m, 4H), 0.98 (td, *J* = 14.2, 3.5 Hz, 1H), 0.92 (s, 3H), 0.72 (s, 3H); LCMS (ESI) *m*/*z* calcd for C₂₈H₄₈NO₈S [M-Na]⁻ 558.3, found 558.2.

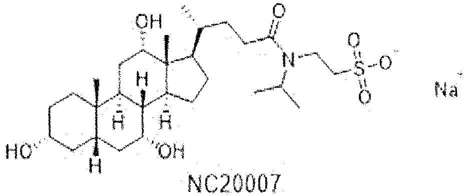

### NC20007

¹H NMR (400 MHz, DMSO-d₆) δ 4.55-4.42 (m, 0.6H), 4.31 (s, 1H), 4.11 (s, 1H), 4.15-3.96 (m, 1.4H), 3.79 (s, 1H), 3.61 (s, 1H), 3.45-3.37 (m, 1H), 3.36-3.27 (m, 1H), 3.17 (s, 1H), 2.67-2.54 (m, 2H), 2.34-2.09 (m, 4H), 2.02-1.94 (m,1H), 1.85-1.55 (m, 6H), 1.51-1.07 (m, 14H), 1.03 (d, *J* = 6.8, 3H), 0.97-0.94 (m, 1H), 0.93 (d, *J* = 6.4, 3H), 0.87-0.83 (m, 1H), 0.81 (s, 3H), 0.59 (s, 3H); LCMS (ESI) *m*/*z* calcd for C₂₉H₅₀NO₇S [M-Na]⁻ 556.3, found 556.2.

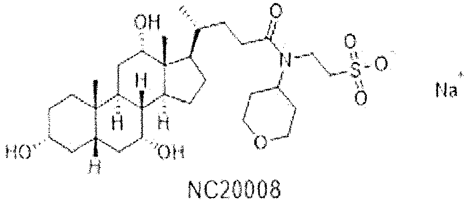

### NC20008

¹H NMR (500 MHz, CD₃OD) δ 4.44 (tt, *J* = 12.1, 3.9 Hz, 0.5H) 4.06-3.91 (m, 3.5H), 3.83-3.77 (m, 1H), 3.75-3.63 (m, 2H), 3.53-3.41 (m, 2H), 3.41-3.32 (m, 1H), 3.08-3.01 (m, 2H), 2.55-2.44 (m, 1H), 2.41-2.21 (m, 3H), 2.06-1.24 (m, 22H), 1.18-1.08 (m, 1H), 1.07-1.05 (m, 3H), 0.98 (tt, *J* = 14.2, 3.5 Hz, 1H), 0.922 (s, 1.5H), 0.919 (s, 1.5H), 0.73 (s, 1.5H), 0.72 (s, 1.5H); LCMS (ESI) *m*/*z* calcd for C₃₁H₅₂NO₈S [M-Na]⁻ 598.3, found 598.2.

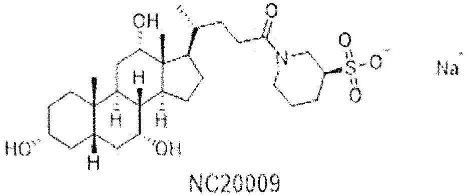

### NC20009

¹H NMR (400 MHz, DMSO-d₆) δ 4.72 (d, *J* = 12.7 Hz, 0.5H), 4.37-4.22 (m, 1.5H), 4.16-4.07 (m, 1H), 4.07-3.95 (m, 1.5H), 3.84-3.70 (m, 1.5H), 3.61 (s, 1H), 3.24-3.11 (m, 1H), 2.99-2.89 (m, 0.5H), 2.86-2.76 (m, 0.5H), 2.42-2.09 (m, 6H), 2.08-1.91 (m, 2H), 1.86-1.07 (m, 20H), 1.01-0.82 (m, 5H), 0.81 (s, 3H), 0.59 (s, 3H); LCMS (ESI) *m*/*z* calcd for C₂₉H₄₈NO₇S [M-Na]⁻ 554.3, found 554.2.

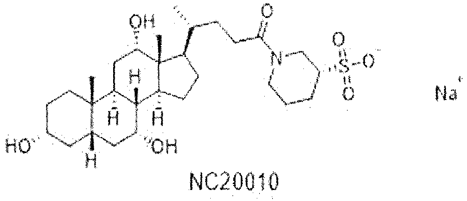

### NC20010

¹H NMR (500 MHz, CD₃OD) δ 5.02 (d, *J* = 9.7 Hz, 0.5H), 4.44 (d, *J* = 13.3 Hz, 0.5H), 4.34-4.27 (m, 0.5H), 3.98-3.94 (m, 1.5H), 3.82-3.77 (m, 1H), 3.41-3.33 (m, 1H), 3.23 (dd, *J* = 13.6, 11.2 Hz, 0.5H), 3.01 (td, *J* = 13.2, 2.8 Hz, 0.5H), 2.84-2.55 (m, 2H), 2.54-2.42 (m, 1H), 2.42-2.21 (m, 4H), 2.07-1.69 (m, 9H), 1.69-1.25 (m, 12H), 1.17-1.08 (m, 1H), 1.06 (d, *J* = 6.5, 1.5H), 1.04 (d, *J* = 6.4, 1.5H), 1.02-0.94 (m, 1H), 0.92 (s, 3H), 0.72 (s, 3H); LCMS (ESI) *m*/*z* calcd for C₂₉H₄₈NO₇S [M-Na]⁻ 554.3, found 554.2.

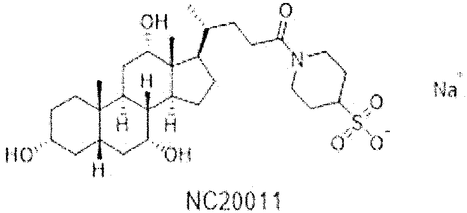

### NC20011

¹H NMR (400 MHz, D₂O) δ 4.50 (d, *J* = 13.5 Hz, 1H), 4.15-4.02 (m, 2H), 3.92-3.85 (m, 1H), 3.55-3.44 (m, 1H), 3.20 (t, *J* = 13.1 Hz, 1H), 3.14-3.03 (m, 1H), 2.78-2.67 (m, 1H), 2.59-2.29 (m, 2H), 2.23-1.22 (m, 24H), 1.22-1.09 (m, 1H), 1.08-0.95 (m, 4H), 0.91 (s, 3H), 0.72 (s, 3H); LCMS (ESI) *m*/*z* calcd for C₂₉H₄₈NO₇S [M-Na]⁻ 554.3, found 554.2.

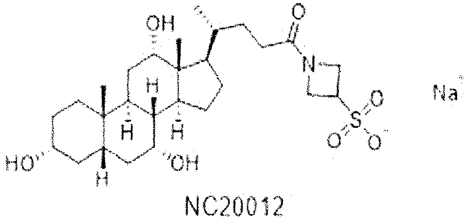

### NC20012

¹H NMR (500 MHz, CD₃OD) δ 4.47-4.38 (m, 1H), 4.38-4.31 (m, 1H), 4.21-4.11 (m, 2H), 3.98-3.93 (m, 1H), 3.85-3.76 (m, 2H), 3.41-3.32 (m, 1H), 2.35-2.14 (m, 3H), 2.13-1.69 (m, 8H), 1.69-1.49 (m, 6H), 1.49-1.27 (m, 5H), 1.12 (qd, *J* = 11.8, 5.3 Hz, 1H), 1.05-0.94 (m, 4H), 0.92 (s, 3H), 0.72 (s, 3H); LCMS (ESI) *m*/*z* calcd for C₂₇H₄₄NO₇S [M-Na]⁻ 526.3, found 526.1.

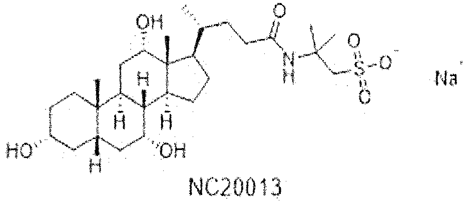

### NC20013

¹H NMR (400 MHz, DMSO-d₆) δ 8.15 (s, 1H), 4.31 (d, *J* = 4.5 Hz, 1H), 4.11 (d, *J* = 3.4 Hz, 1H), 4.01 (d, *J* = 3.4 Hz, 1H), 3.78 (s, 1H), 3.61 (s, 1H), 3.23-3.11 (m, 1H), 2.61 (s, 2H), 2.29-2.08 (m, 2H), 2.04-1.91 (m, 2H), 1.90-1.52 (m, 7H), 1.50-1.07 (m, 17H), 1.02-0.82 (m, 5H), 0.81 (s, 3H), 0.58 (s, 3H); LCMS (ESI) *m*/*z* calcd for C₂₈H₄₈NO₇S [M-Na]⁻ 542.3, found 542.2.

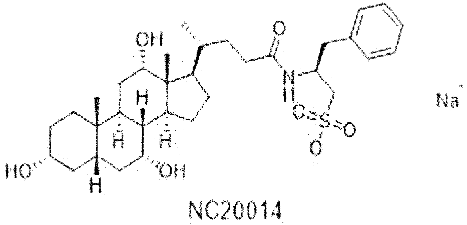

### NC20014

¹H NMR (500 MHz, CD₃OD) δ 7.30-7.21 (m, 4H), 7.20-7.14 (m, 1H), 4.51-4.42 (m, 1H), 3.95-3.94 (m, 1H), 3.82-3.78 (m, 1H), 3.42-3.34 (m, 1H), 3.21-3.13 (m, 1H), 3.03-2.96 (m, 2H), 2.91 (dd, *J* = 13.6, 7.8 Hz, 1H), 2.34-2.13 (m, 3H), 2.07-1.92 (m, 3H), 1.92-1.49 (m, 11H), 1.48-1.33 (m, 3H), 1.32-1.18 (m, 2H), 1.16-0.93 (m, 5H), 0.92 (s, 3H), 0.70 (s, 3H); LCMS (ESI) *m*/*z* calcd for C₃₃H₅₀NO₇S [M-Na]⁻ 604.3, found 604.2.

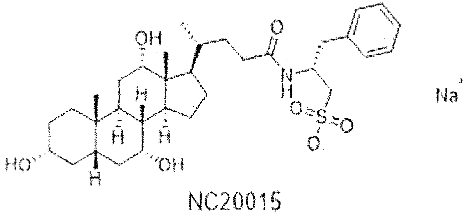

### NC20015

¹H NMR (500 MHz, CD₃OD) δ 7.30-7.22 (m, 4H), 7.22-7.14 (m, 1H), 4.52-4.44 (m, 1H), 3.96-3.91 (m, 1H), 3.82-3.77 (m, 1H), 3.41-3.32 (m, 1H), 3.17 (dd, *J* = 13.6, 6.1 Hz, 1H), 3.03-2.95 (m, 2H), 2.91 (dd, *J* = 13.7, 7.8 Hz, 1H), 2.34-2.15 (m, 3H), 2.08-1.49 (m, 14H), 1.48-1.19 (m, 5H), 1.16-1.04 (m, 1H), 1.03-0.93 (m, 4H), 0.92 (s, 3H), 0.69 (s, 3H); LCMS (ESI) *m*/*z* calcd for C₃₃H₅₀NO₇S [M-Na]⁻ 604.3, found 604.2.

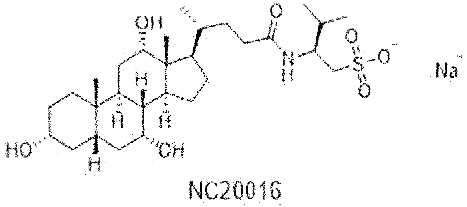

### NC20016

¹H NMR (500 MHz, CD₃OD) δ 4.24-4.17 (m, 1H), 3.98-3.93 (m, 1H), 3.82-3.77 (m, 1H), 3.41-3.33 (m, 1H), 2.99-2.92 (m, 2H), 2.35-2.21 (m, 3H), 2.17-2.04 (m, 2H), 2.03-1.69 (m, 7H), 1.69-1.49 (m, 6H), 1.49-1.27 (m, 5H), 1.17-1.06 (m, 1H), 1.03 (d, *J* = 6.5 Hz, 3H), 1.02-0.94 (m, 1H), 0.94-0.89 (m, 9H), 0.72 (s, 3H); LCMS (ESI) *m*/*z* calcd for C₂₉H₅₀NO₇S [M-Na]⁻ 556.3, found 556.2.

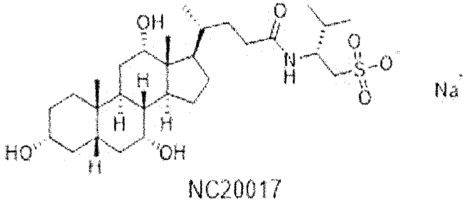

### NC20017

¹H NMR (500 MHz, CD₃OD) δ 4.23-4.21 (m, 1H), 3.98-3.92 (m, 1H), 3.83-3.75 (m, 1H), 3.41-3.32 (m, 1H), 2.95 (d, *J* = 6.1 Hz, 2H), 2.34-2.21 (m, 3H), 2.21-2.12 (m, 1H), 2.12-2.03 (m, 1H), 2.03-1.69 (m, 7H), 1.69-1.49 (m, 6H), 1.49-1.25 (m, 5H), 1.16-1.07 (m, 1H), 1.04 (d, *J* = 6.4 Hz, 3H), 1.02-0.94 (m, 1H), 0.94-0.89 (m, 9H), 0.71 (s, 3H); LCMS (ESI) *m*/*z* calcd for C₂₉H₅₀NO₇S [M-Na]⁻ 556.3, found 556.2.

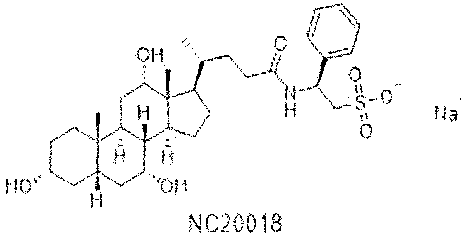

### NC20018

¹H NMR (400 MHz, DMSO-d₆) δ 8.14 (d, *J* = 6.2 Hz, 1H), 7.29-7.19 (m, 4H), 7.18-7.11 (m, 1H), 5.01-4.92 (m, 1H), 4.31 (d, *J* = 4.5 Hz, 1H), 4.11 (s, 1H), 4.01 (s, 1H), 3.78 (s, 1H), 3.61 (s, 1H), 3.23-3.11 (m, 1H), 2.85 (dd, *J* = 13.8, 9.2 Hz, 1H), 2.66 (dd, *J* = 13.8, 4.2 Hz, 1H), 2.29-2.06 (m, 3H), 2.04-1.89 (m, 2H), 1.85-1.55 (m, 6H), 1.50-1.08 (m, 11H), 0.99-0.82 (m, 5H), 0.81 (s, 3H), 0.57 (s, 3H); LCMS (ESI) *m*/*z* calcd for C₃₂H₄₈NO₇S [M-Na]⁻590.3, found 590.3.

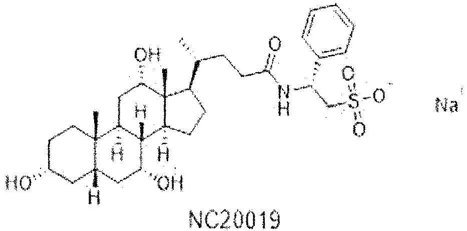

### NC20019

¹H NMR (500 MHz, CD₃OD) δ 7.37-7.27 (m, 4H), 7.24-7.18 (m, 1H), 5.35 (dd, *J* = 10.0, 3.7 Hz, 1H), 3.96-3.91 (m, 1H), 3.81-3.75 (m, 1H), 3.41-3.32 (m, 1H), 3.26 (dd, *J* = 14.3, 10.0 Hz, 1H), 3.10 (dd, *J* = 14.3, 3.7 Hz, 1H), 2.36-2.14 (m, 4H), 2.02-1.48 (m, 13H), 1.48-1.27 (m, 4H), 1.26-1.17 (m, 1H), 1.11-0.94 (m, 5H), 0.92 (s, 3H), 0.65 (s, 3H); LCMS (ESI) *m*/*z* calcd for C₃₂H₄₈NO₇S [M-Na]⁻ 590.3, found 590.3.

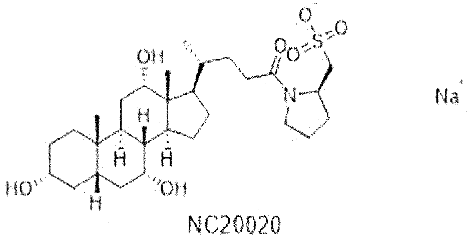

### NC20020

¹H NMR (400 MHz, DMSO-d₆) δ 4.31 (s, 1H), 4.23-4.07 (m, 2H), 4.01 (s, 1H), 3.79 (s, 1H), 3.61 (s, 1H), 3.44-3.27 (m, 1H), 3.26-3.11 (m, 2H), 2.92 (dd, *J* = 12.7, 2.9 Hz, 0.5H), 2.61-2.52 (m, 0.5H), 2.44-1.91 (m, 7H), 1.91-1.54 (m, 9H), 1.51-1.06 (m, 11H), 1.03-0.82 (m, 5H), 0.81 (s, 3H), 0.59 (s, 1.5H), 0.58 (s, 1.5H); LCMS (ESI) *m*/*z* calcd for C₂₉H₄₈NO₇S [M-Na]⁻ 554.3, found 554.4.

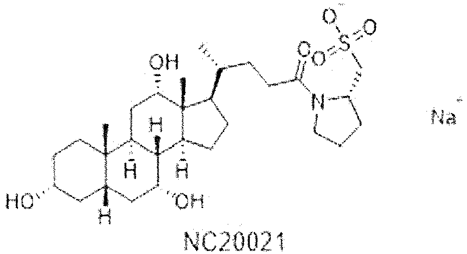

### NC20021

¹H NMR (500 MHz, CD₃OD) δ 4.52-4.38 (m, 1H), 3.99-3.93 (m, 1H), 3.82-3.77 (m, 1H), 3.60-3.52 (m, 0.5H), 3.52-3.26 (m, 3H), 3.02-2.92 (m, 1H), 2.74 (dd, *J* = 13.3, 10.6 Hz, 0.5H), 2.50-2.40 (m, 2H), 2.39-2.19 (m, 3H), 2.11-1.70 (m, 10H), 1.68-1.49 (m, 6H), 1.49-1.25 (m, 5H), 1.17-1.08 (m, 1H), 1.06 (d, *J* = 6.5 Hz, 1.5H), 1.04 (d, *J* = 6.5 Hz, 1.5H), 1.02-0.94 (m, 1H), 0.92 (s, 3H), 0.72 (s, 3H); LCMS (ESI) *m*/*z* calcd for C₂₉H₄₈NO₇S [M-Na]⁻ 554.3, found 554.3.

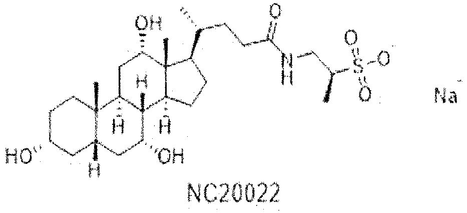

### NC20022

¹H NMR (500 MHz, CD₃OD) δ 3.99-3.93 (m, 1H), 3.82-3.77 (m, 1H), 3.58 (dd, *J* = 13.8, 6.0 Hz, 1H), 3.43 (dd, *J* = 13.8, 6.8 Hz, 1H), 3.40-3.32 (m, 1H), 3.00-2.90 (m, 1H), 2.34-2.21 (m, 3H), 2.13 (ddd, *J* = 13.9, 9.5, 6.8 Hz, 1H), 2.04-1.70 (m, 7H), 1.69-1.49 (m, 6H), 1.47-1.31 (m, 5H), 1.29 (d, *J* = 6.9 Hz, 3H), 1.17-1.06 (m, 1H), 1.03 (d, *J* = 6.4 Hz, 3H), 1.01-0.94 (m, 1H), 0.92 (s, 3H), 0.71 (s, 3H); LCMS (ESI) *m*/*z* calcd for C₂₇H₄₆NO₇S [M-Na]⁻ 528.3, found 528.2.

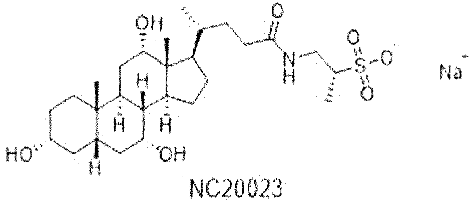

### NC20023

¹H NMR (400 MHz, DMSO-d₆) δ 7.63-7.55 (m, 1H), 4.32 (d, *J* = 4.4 Hz, 1H), 4.10 (d, *J* = 3.3 Hz, 1H), 4.01 (d, *J* = 3.3 Hz, 1H), 3.78 (s, 1H), 3.61 (s, 1H), 3.30-3.09 (m, 3H), 2.49-2.42 (m, 1H), 2.28-1.87 (m, 5H), 1.84-1.52 (m, 6H), 1.50-1.08 (m, 11H), 1.02 (d, *J* = 6.8 Hz, 3H), 0.99-0.82 (m, 5H), 0.80 (s, 3H), 0.58 (s, 3H); LCMS (ESI) *m*/*z* calcd for C₂₇H₄₆NO₇S [M-Na]⁻ 528.3, found 528.2.

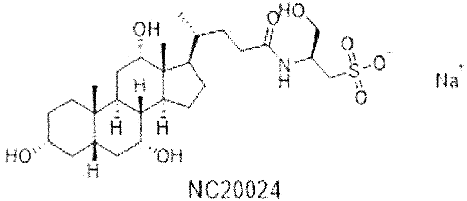

### NC20024

¹H NMR (500 MHz, CD₃OD) δ 4.34-4.27 (m, 1H), 3.99-3.92 (m, 1H), 3.84-3.77 (m, 1H), 3.75-3.64 (m, 2H), 3.42-3.32 (m, 1H), 3.14-3.06 (m, 1H), 3.05-2.97 (m, 1H), 2.34-2.20 (m, 3H), 2.18-2.03 (m, 1H), 2.03-1.69 (m, 7H), 1.69-1.49 (m, 6H), 1.49-1.25 (m, 5H), 1.17-1.06 (m, 1H), 1.03-1.01 (m, 3H), 1.01-0.93 (m, 1H), 0.92 (s, 3H), 0.71 (s, 3H); LCMS (ESI) *m*/*z* calcd for C₂₇H₄₆NO₈S [M-Na]⁻ 544.3, found 544.2.

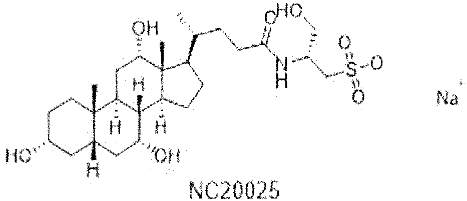

### NC20025

¹H NMR (500 MHz, CD₃OD) δ 4.35-4.27 (m, 1H), 4.00-3.92 (m, 1H), 3.83-3.77 (m, 1H), 3.76-3.64 (m, 2H), 3.41-3.32 (m, 1H), 3.14-3.06 (m, 1H), 3.05-2.98 (m, 1H), 2.34-2.20 (m, 3H), 2.18-2.03 (m, 1H), 2.03-1.69 (m, 7H), 1.69-1.49 (m, 6H), 1.49-1.24 (m, 5H), 1.17-1.06 (m, 1H), 1.03-0.94 (m, 4H), 0.92 (s, 3H), 0.71 (s, 3H); LCMS (ESI) *m*/*z* calcd for C₂₇H₄₆NO₈S [M-Na]⁻ 544.3, found 544.2.

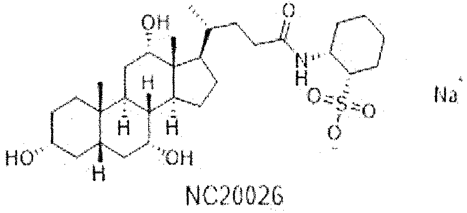

### NC20026

¹H NMR (500 MHz, CD₃OD) δ 4.34-4.28 (m, 1H), 4.00-3.93 (m, 1H), 3.82-3.77 (m, 1H), 3.41-3.32 (m, 1H), 2.91 (dt, *J* = 10.3, 4.0 Hz, 1H), 2.34-2.12 (m, 5H), 2.07-1.69 (m, 10H), 1.68-1.25 (m, 15H), 1.16-1.06 (m, 1H), 1.04 (d, *J* = 6.4 Hz, 3H), 0.98 (td, *J* = 14.2, 3.4 Hz, 1H), 0.92 (s, 3H), 0.71 (s, 3H); LCMS (ESI) *m*/*z* calcd for C₃₀H₅₀NO₇S [M-Na]⁻ 568.3, found 568.3.

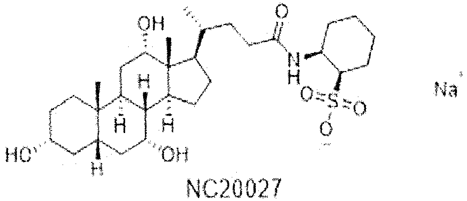

### NC20027

¹H NMR (500 MHz, CD₃OD) δ 4.33-4.27 (m, 1H), 3.98-3.93 (m, 1H), 3.82-3.77 (m, 1H), 3.41-3.32 (m, 1H), 2.91 (dt, *J* = 10.1, 4.1 Hz, 1H), 2.37-2.18 (m, 4H), 2.14 (ddd, *J* = 14.2, 9.9, 6.5 Hz, 1H), 2.07-1.70 (m, 10H), 1.69-1.27 (m, 15H), 1.17-1.06 (m, 1H), 1.03 (d, *J* = 6.5 Hz, 3H), 0.98 (td, *J* = 14.1, 3.5 Hz, 1H), 0.92 (s, 3H), 0.72 (s, 3H); LCMS (ESI) *m*/*z* calcd for C₃₀H₅₀NO₇S [M-Na]⁻ 568.3, found 568.3.

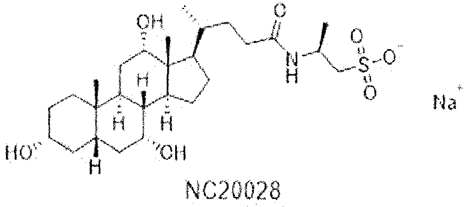

### NC20028

¹H NMR (400 MHz, DMSO-d₆) δ 7.67 (d, *J* = 7.4 Hz, 1H), 4.31 (d, *J* = 4.3 Hz, 1H), 4.10 (s, 1H), 4.04-3.91 (m, 2H), 3.78 (s, 1H), 3.61 (s, 1H), 3.24-3.11 (m, 1H), 2.60 (dd, *J* = 13.4, 4.6 Hz, 1H), 2.48-2.41 (m, 1H), 2.29-2.08 (m, 2H), 2.07-1.52 (m, 9H), 1.52-1.06 (m, 14H), 1.02-0.73 (m, 8H), 0.58 (s, 3H); LCMS (ESI) *m*/*z* calcd for C₂₇H₄₆NO₇S [M-Na]⁻ 528.3, found 528.2.

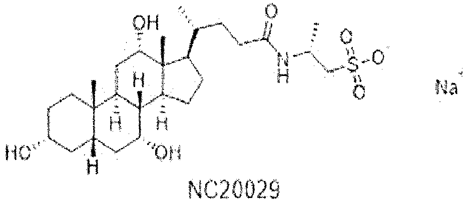

### NC20029

¹H NMR (500 MHz, CD₃OD) δ 4.37-4.27 (m, 1H), 3.99-3.93 (m, 1H), 3.82-3.77 (m, 1H), 3.41-3.33 (m, 1H), 3.08-3.02 (m, 1H), 2.91-2.84 (m, 1H), 2.34-2.19 (m, 3H), 2.13-2.04 (m, 1H), 2.04-1.69 (m, 7H), 1.69-1.48 (m, 6H), 1.48-1.25 (m, 8H), 1.11 (qd, *J* = 11.9, 5.8 Hz, 1H), 1.02 (d, *J* = 6.4 Hz, 3H), 1.01-0.93 (m, 1H), 0.92 (s, 3H), 0.71 (s, 3H); LCMS (ESI) *m*/*z* calcd for C₂₇H₄₆NO₇S [M-Na]⁻ 528.3, found 528.2.

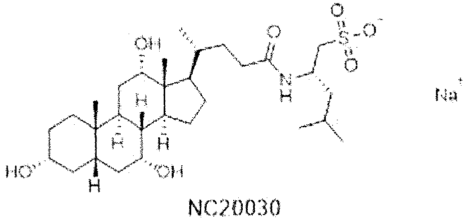

### NC20030

¹H NMR (500 MHz, CD₃OD) δ 4.39-4.31 (m, 1H), 3.98-3.93 (m, 1H), 3.82-3.77 (m, 1H), 3.41-3.32 (m, 1H), 3.03 (dd, *J* = 14.0, 5.5 Hz, 1H), 2.90 (dd, *J* = 14.0, 6.3 Hz, 1H), 2.34-2.19 (m, 3H), 2.15-2.05 (m, 1H), 2.04-1.49 (m, 16H), 1.49-1.25 (m, 5H), 1.11 (qd, *J* = 12.0, 5.7 Hz, 1H), 1.03 (d, *J* = 6.5 Hz, 3H), 1.01-0.90 (m, 10H), 0.71 (s, 3H); LCMS (ESI) *m*/*z* calcd for C₃₀H₅₂NO₇S [M-Na]⁻ 570.3, found 570.3.

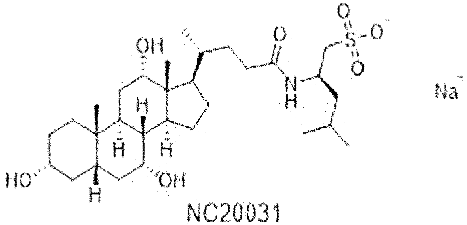

### NC20031

¹H NMR (500 MHz, CD₃OD) δ 4.42-4.33 (m, 1H), 3.99-3.93 (m, 1H), 3.82-3.77 (m, 1H), 3.41-3.32 (m, 1H), 3.01 (dd, *J* = 14.0, 5.5 Hz, 1H), 2.90 (dd, *J* = 14.0, 6.4 Hz, 1H), 2.34-2.19 (m, 3H), 2.17-2.06 (m, 1H), 2.04-1.49 (m, 16H), 1.49-1.23 (m, 5H), 1.10 (qd, *J* = 12.0, 5.9 Hz, 1H), 1.03 (d, *J* = 6.5 Hz, 3H), 1.01-0.89 (m, 10H), 0.71 (s, 3H); LCMS (ESI) *m*/*z* calcd for C₃₀H₅₂NO₇S [M-Na]⁻ 570.3, found 570.3.

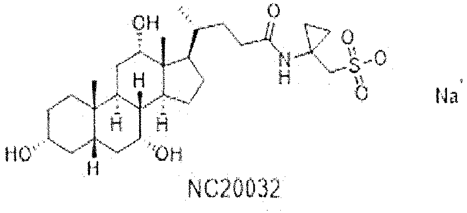

### NC20032

¹H NMR (400 MHz, DMSO-d₆) δ 7.78 (s, 1H), 4.31 (d, *J* = 4.4 Hz, 1H), 4.09 (d, *J* = 3.4 Hz, 1H), 4.01 (d, *J* = 3.3 Hz, 1H), 3.77 (s, 1H), 3.61 (s, 1H), 3.22-3.11 (m, 1H), 2.70-2.59 (m, 2H), 2.28-2.08 (m, 2H), 2.04-1.91 (m, 2H), 1.90-1.52 (m, 7H), 1.49-1.06 (m, 11H), 1.01-0.72 (m, 10H), 0.61-0.50 (m, 5H); LCMS (ESI) *m*/*z* calcd for C₂₈H₄₆NO₇S [M-Na]⁻ 540.3, found 540.2.

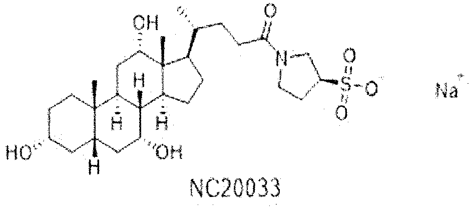

### NC20033

¹H NMR (500 MHz, CD₃OD) δ 3.99-3.93 (m, 1H), 3.89-3.32 (m, 7H), 2.47-2.18 (m, 6H), 2.05-1.70 (m, 7H), 1.69-1.26 (m, 11H), 1.18-0.94 (m, 5H), 0.92 (s, 3H), 0.724 (s, 1.5H), 0.719 (s, 1.5H); LCMS (ESI) *m*/*z* calcd for C₂₈H₄₆NO₇S [M-Na]⁻ 540.3, found 540.2.

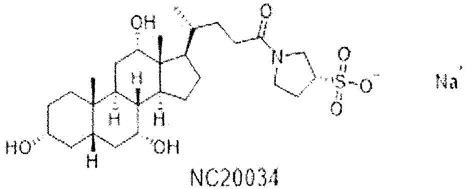

### NC20034

¹H NMR (500 MHz, CD₃OD) δ 3.98-3.93 (m, 1H), 3.89-3.32 (m, 7H), 2.47-2.18 (m, 6H), 2.06-1.70 (m, 7H), 1.70-1.50 (m, 6H), 1.49-1.26 (m, 5H), 1.18-0.95 (m, 5H), 0.92 (s, 3H), 0.723 (s, 1.5H), 0.720 (s, 1.5H); LCMS (ESI) *m*/*z* calcd for C₂₈H₄₆NO₇S [M-Na]⁻ 540.3, found 540.2.

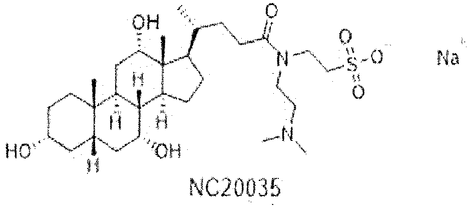

### NC20035

¹H NMR (400 MHz, DMSO-d₆) δ 4.30 (s, 1H), 4.12 (s, 1H), 4.01 (s, 1H), 3.79 (s, 1H), 3.61 (s, 1H), 3.54-3.39 (m, 2H), 3.31-3.23 (m, 2H), 3.21-3.12 (m, 1H), 2.70-2.61 (m, 1H), 2.60-2.53 (m, 1H), 2.39-2.06 (m, 12H), 2.03-1.92 (m, 1H), 1.85-1.56 (m, 6H), 1.50-1.07 (m, 11H), 1.01-0.82 (m, 5H), 0.81 (s, 3H), 0.59 (s, 3H); LCMS (ESI) *m*/*z* calcd for C₃₀H₅₃N₂O₇S [M-Na]⁻ 585.4, found 585.3.

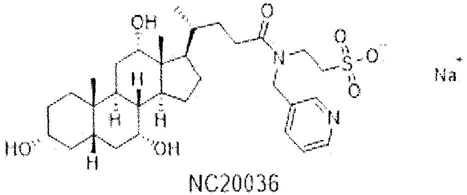

### NC20036

¹H NMR (400 MHz, DMSO-d₆) δ 8.51-8.38 (m, 2H), 7.62-7.54 (m, 1H), 7.43-7.29 (m, 1H), 4.65 (s, 0.5H), 4.59-4.45 (m, 1.5H), 4.30 (s, 1H), 4.16-3.97 (m, 2H), 3.82-3.72 (m, 1H), 3.61 (s, 1H), 3.54-3.41 (m, 2H), 3.22-3.11 (m, 1H), 2.69-2.54 (m, 2H), 2.48-2.37 (m, 1H), 2.37-2.07 (m, 3H), 2.04-1.90 (m, 1H), 1.85-1.55 (m, 6H), 1.50-1.07 (m, 11H), 0.96 (d, *J* = 6.3 Hz, 3H), 0.89-0.73 (m, 5H), 0.62-0.52 (m, 3H); LCMS (ESI) *m*/*z* calcd for C₃₂H₄₉N₂O₇S [M-Na]⁻605.3, found 605.3.

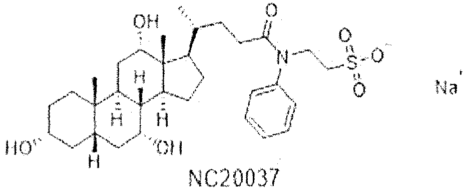

### NC20037

¹H NMR (500 MHz, CD₃OD) δ 7.52-7.46 (m, 2H), 7.44-7.38 (m, 1H), 7.34-7.29 (m, 2H), 4.13-4.06 (m, 2H), 3.87-3.83 (m, 1H), 3.79-3.75 (m, 1H), 3.39-3.33 (m, 1H), 3.09-3.03 (m, 2H), 2.32-2.09 (m, 3H), 2.05-1.87 (m, 3H), 1.82-1.47 (m, 11H), 1.46-1.32 (m, 2H), 1.32-1.14 (m, 3H), 1.13-1.01 (m, 1H), 1.01-0.92 (m, 1H), 0.90 (s, 3H), 0.77 (d, *J* = 6.1 Hz, 3H), 0.64 (s, 3H); LCMS (ESI) *m*/*z* calcd for C₃₂H₄₈NO₇S [M-Na]⁻ 590.3, found 590.3.

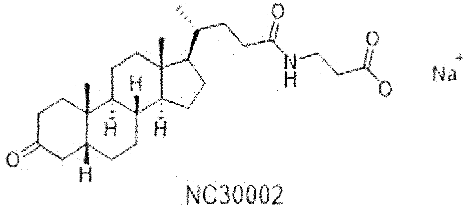

### NC30002

¹H NMR (500 MHz, CD₃OD) δ 3.39 (t, *J* = 7.0 Hz, 2H), 2.82 (d, *J* = 13.5 Hz, 1H), 2.51-2.40 (m, 1H), 2.34 (t, *J* = 7.0 Hz, 2H), 2.22 (ddd, *J* = 13.8, 10.4, 5.2 Hz, 1H), 2.12-2.01 (m, 4H), 1.99-1.86 (m, 2H), 1.82-1.72 (m, 2H), 1.68-1.57 (m, 2H), 1.54-1.07 (m, 15H), 1.04 (s, 3H), 0.96 (d, *J* = 6.5 Hz, 3H), 0.73 (s, 3H); LCMS (ESI) *m*/*z* calcd for C₂₇H₄₂NO₄ [M-Na]⁻ 444.3, found 444.2.

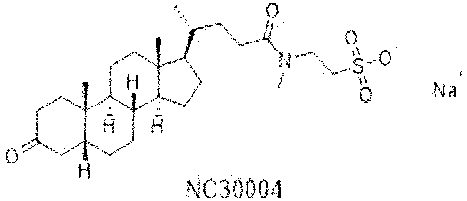

### NC30004

¹H NMR (400 MHz, DMSO-d₆) δ 3.57-3.41 (m, 2H), 2.93 (s, 1.5H), 2.82-2.69 (m, 2.5H), 2.69-2.61 (m, 1H), 2.59-2.52 (m, 1H), 2.42-2.06 (m, 3H), 2.01-1.88 (m, 3H), 1.86-1.48 (m, 7H), 1.46-0.99 (m, 14H), 0.95 (s, 3H), 0.89 (d, 3H), 0.64 (s, 3H); LCMS (ESI) *m*/*z* calcd for C₂₇H₄₄NO₅S [M-Na]⁻ 494.3, found 494.2.

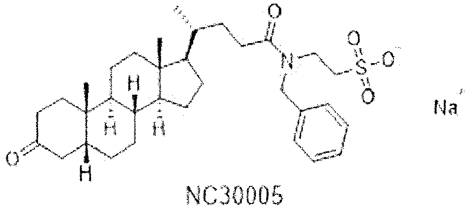

### NC30005

¹H NMR (500 MHz, CD₃OD) δ 7.41-7.34 (m, 1H), 7.34-7.19 (m, 4H), 4.74 (s, 1H), 4.63 (s, 1H), 3.84-3.69 (m, 2H), 3.07 (t, *J* = 7.1 Hz, 1H), 3.03-2.98 (m, 1H), 2.90-2.79 (m, 1H), 2.62-2.52 (m, 0.5H), 2.52-2.35 (m, 2H), 2.32-2.20 (m, 0.5H), 2.14-1.70 (m, 8H), 1.69-0.93 (m, 20.5H), 0.86 (d, *J* = 6.5 Hz, 1.5H), 0.74 (s, 1.5H), 0.69 (s, 1.5H); LCMS (ESI) *m*/*z* calcd for C₃₃H₄₈NO₅S [M-Na]⁻ 570.3, found 570.3.

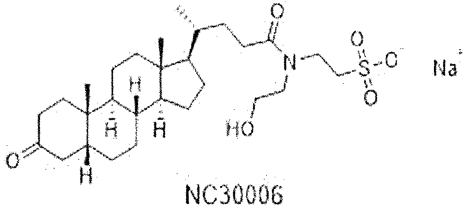

### NC30006

¹H NMR (500 MHz, CD₃OD) δ 3.89-3.79 (m, 1H), 3.76-3.64 (m, 3H), 3.58-3.52 (m, 1H), 3.50-3.45 (m, 1H), 3.13-3.04 (m, 2H), 2.89-2.79 (m, 1H), 2.57-2.41 (m, 2H), 2.41-2.29 (m, 1H), 2.12-2.02 (m, 3H), 1.99-1.88 (m, 3H), 1.83-1.72 (m, 2H), 1.69-1.56 (m, 2H), 1.56-1.08 (m, 14H), 1.05 (s, 3H), 1.02-0.93 (m, 3H), 0.73 (s, 3H); LCMS (ESI) *m*/*z* calcd for C₂₈H₄₆NO₆S [M-Na]⁻ 524.3, found 524.3.

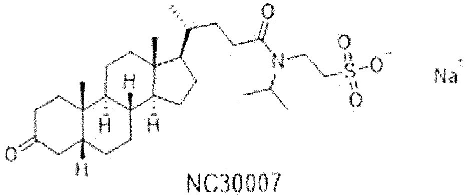

### NC30007

¹H NMR (500 MHz, CD₃OD) δ 4.66-4.56 (m, 0.5H), 4.23-4.12 (m, 0.5H), 3.71-3.64 (m, 1H), 3.64-3.57 (m, 1H), 3.09-3.02 (m, 2H), 2.84 (td, J = 14.3, 3.7 Hz, 1H), 2.52-2.40 (m, 2H), 2.37-2.26 (m, 1H), 2.13-2.01 (m, 3H), 2.00-1.86 (m, 3H), 1.84-1.71 (m, 2H), 1.69-1.57 (m, 2H), 1.56-1.07 (m, 20H), 1.048 (s, 1.5H), 1.045 (s, 1.5H), 1.02-0.93 (m, 3H), 0.74 (s, 1.5H), 0.73 (s, 1.5H); LCMS (ESI) m/z calcd for C₂₉H₄₈NO₅S [M-Na]⁻ 522.3, found 522.3.

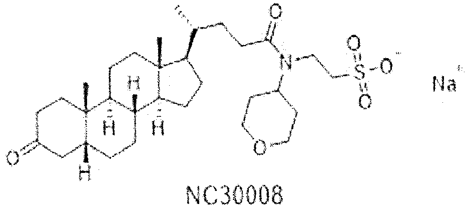

### NC30008

¹H NMR (500 MHz, CD₃OD) δ 4.44 (tt, *J* = 12.1, 3.9 Hz, 0.5H), 4.06-3.90 (m, 2.5H), 3.75-3.61 (m, 2H), 3.53-3.42 (m, 2H), 3.08-3.00 (m, 2H), 2.83 (td, J = 14.2, 3.5 Hz, 1H), 2.54-2.30 (m, 3H), 2.12-2.02 (m, 3H), 2.02-1.84 (m, 5H), 1.83-1.71 (m, 1H), 1.71-1.24 (m, 15H), 1.24-1.08 (m, 4H), 1.05 (s, 1.5H), 1.04 (s, 1.5H), 1.02-0.93 (m, 3H), 0.74 (s, 1.5H), 0.73 (s, 1.5H); LCMS (ESI) *m*/*Z* calcd for C₃₁H₅₀NO₆S [M-Na]⁻ 564.3, found 564.3.

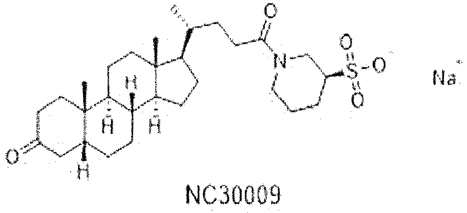

### NC30009

¹H NMR (500 MHz, CD₃OD) δ 5.02 (d, *J* = 10.2 Hz, 0.4H), 4.42 (d, *J* = 13.3 Hz, 0.6H), 4.32-4.25 (m, 0.6H), 3.92 (d, *J* = 13.0 Hz, 0.4H), 3.24 (dd, *J* = 13.5, 11.1 Hz, 0.6H), 3.01 (td, *J* = 13.2, 2.8 Hz, 0.4H), 2.90-2.57 (m, 3H), 2.56-2.39 (m, 2H), 2.38-2.25 (m, 2H), 2.13-1.99 (m, 3H), 1.99-1.69 (m, 7H), 1.69-1.57 (m, 2H), 1.57-1.24 (m, 11H), 1.24-1.07 (m, 4H), 1.05 (s, 3H), 1.01-0.98 (m, 3H), 0.73 (s, 3H); LCMS (ESI) m/z calcd for C₂₉H₄₆NO₅S [M-Na]⁻520.3, found 520.3.

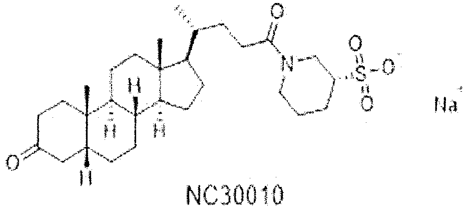

### NC30010

¹H NMR (500 MHz, CD₃OD) δ 5.02 (d, *J* = 9.9 Hz, 0.4H), 4.44 (d, *J* = 13.0 Hz, 0.6H), 4.33-4.25 (m, 0.6H), 3.92 (d, *J* = 13.5 Hz, 0.4H), 3.23 (dd, *J* = 13.5, 11.2 Hz, 0.6H), 3.01 (td, *J* = 13.2, 2.8 Hz, 0.4H), 2.89-2.55 (m, 3H), 2.52-2.40 (m, 2H), 2.40-2.25 (m, 2H), 2.12-1.99 (m, 3H), 1.99-1.70 (m, 7H), 1.69-1.58 (m, 2H), 1.56-1.24 (m, 11H), 1.24-1.07 (m, 4H), 1.05 (s, 3H), 1.01-0.98 (m, 3H), 0.74 (s, 3H); LCMS (ESI) *m*/*z* calcd for C₂₉H₄₆NO₅S [M-Na]⁻520.3, found 520.3.

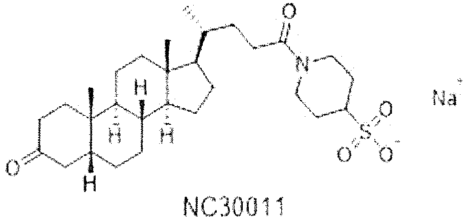

### NC30011

¹H NMR (400 MHz, DMSO-d₆) δ 4.37 (d, *J* = 12.9 Hz, 1H), 3.85 (d, *J* = 13.4 Hz, 1H), 2.98-2.87 (m, 1H), 2.81-2.69 (m, 1H), 2.48-2.11 (m, 5H), 2.01-1.67 (m, 9H), 1.67-1.49 (m, 3H), 1.46-1.00 (m, 16H), 0.96 (s, 3H), 0.90 (d, *J* = 6.5 Hz, 3H), 0.65 (s, 3H); LCMS (ESI) m/Z calcd for C₂₉H₄₆NO₅S [M-Na]⁻520.3, found 520.2.

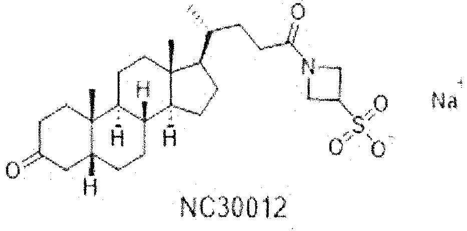

### NC30012

¹H NMR (400 MHz, DMSO-d₆) δ 4.16 (td, *J* = 8.4, 2.3 Hz, 1H), 4.05-3.97 (m, 1H), 3.85 (t, *J* = 9.0 Hz, 1H), 3.80-3.71 (m, 1H), 3.46-3.38 (m, 1H), 2.75 (t, *J* = 14.1 Hz, 1H), 2.36 (td, *J* = 14.7, 5.4 Hz, 1H), 2.05-1.67 (m, 9H), 1.66-1.49 (m, 3H), 1.47-0.99 (m, 14H), 0.96 (s, 3H), 0.88 (d, *J* = 6.7 Hz, 3H), 0.64 (d, *J* = 2.0 Hz, 3H); LCMS (ESI) *m*/*z* calcd for C₂₇H₄₂NO₅S [M-Na]⁻ 492.3, found 492.2.

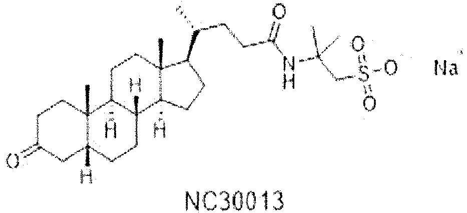

### NC30013

¹H NMR (500 MHz, CD₃OD) δ 3.15-3.10 (m, 2H), 2.89-2.80 (m, 1H), 2.52-2.42 (m, 1H), 2.20 (ddd, *J* = 14.0, 10.3, 5.2 Hz, 1H), 2.13-1.99 (m, 4H), 1.99-1.85 (m, 3H), 1.83-1.71 (m, 2H), 1.68-1.57 (m, 2H), 1.56-1.24 (m, 10H), 1.53 (s, 6H), 1.24-1.07 (m, 4H), 1.04 (s, 3H), 0.96 (d, *J* = 6.6 Hz, 3H), 0.73 (s, 3H); LCMS (ESI) *m*/*z* calcd for C₂₈H₄₆NO₅S [M-Na]⁻ 508.3, found 508.3.

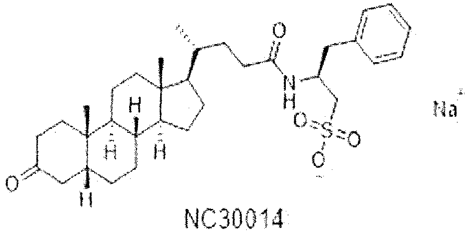

### NC30014

¹H NMR (500 MHz, CD₃OD) δ 7.30-7.22 (m, 4H), 7.21-7.14 (m, 1H), 4.50-4.43 (m, 1H), 3.16 (dd, *J* = 13.6, 6.3 Hz, 1H), 3.04-2.94 (m, 2H), 2.91 (dd, *J* = 13.6, 7.8 Hz, 1H), 2.88-2.80 (m, 1H), 2.52-2.41 (m, 1H), 2.17 (ddd, J = 13.8, 10.7, 5.2 Hz, 1H), 2.12-1.83 (m, 7H), 1.82-1.75 (m, 1H), 1.73-1.57 (m, 3H), 1.56-1.06 (m, 14H), 1.04 (s, 3H), 0.93 (d, *J* = 6.6 Hz, 3H), 0.71 (s, 3H); LCMS (ESI) *m*/*z* calcd for C₃₃H₄₈NO₅S [M-Na]⁻ 570.3, found 570.3.

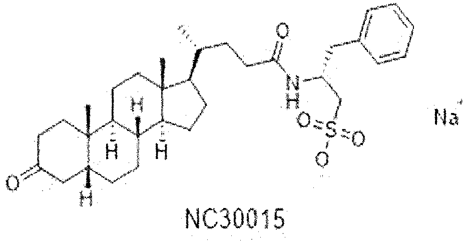

### NC30015

¹H NMR (500 MHz, CD₃OD) δ 7.30-7.22 (m, 4H), 7.22-7.14 (m, 1H), 4.52-4.44 (m, 1H), 3.16 (dd, J = 13.6, 6.1 Hz, 1H), 3.03-2.94 (m, 2H), 2.91 (dd, *J* = 13.6, 7.8 Hz, 1H), 2.88-2.80 (m, 1H), 2.52-2.41 (m, 1H), 2.18 (ddd, *J* = 13.8, 10.4, 5.1 Hz, 1H), 2.13-1.83 (m, 7H), 1.83-1.75 (m, 1H), 1.74-1.56 (m, 3H), 1.56-1.07 (m, 14H), 1.04 (s, 3H), 0.92 (d, *J* = 6.6 Hz, 3H), 0.70 (s, 3H); LCMS (ESI) *m*/*z* calcd for C₃₃H₄₈NO₅S [M-Na]⁻ 570.3, found 570.4.

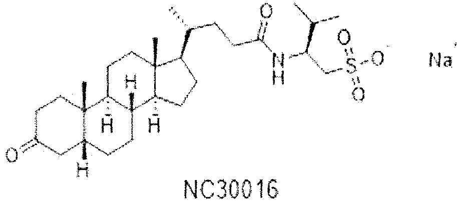

### NC30016

¹H NMR (500 MHz, CD₃OD) δ 4.23-4.16 (m, 1H), 2.96 (dd, *J* = 6.1, 1.9 Hz, 2H), 2.88-2.80 (m, 1H), 2.52-2.41 (m, 1H), 2.28 (ddd, *J* = 13.8, 10.6, 5.2 Hz, 1H), 2.15-2.02 (m, 5H), 1.99-1.87 (m, 3H), 1.87-1.75 (m, 2H), 1.69-1.56 (m, 2H), 1.56-1.07 (m, 14H), 1.05 (s, 3H), 0.98 (d, *J* = 6.6 Hz, 3H), 0.92 (t, *J* = 6.5 Hz, 6H), 0.73 (s, 3H); LCMS (ESI) *m*/*z* calcd for C₂₉H₄₈NO₅S [M-Na]⁻ 522.3, found 522.3.

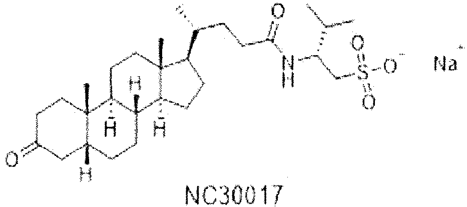

### NC30017

¹H NMR (400 MHz, DMSO-d₆) δ 7.97 (d, *J* = 8.5 Hz, 1H), 3.99-3.88 (m, 1H), 2.75 (t, *J* = 14.1 Hz, 1H), 2.56-2.51 (m, 2H), 2.36 (td, *J* = 14.5, 5.3 Hz, 1H), 2.14-1.89 (m, 6H), 1.87-1.62 (m, 5H), 1.62-1.49 (m, 2H), 1.46-0.98 (m, 14H), 0.95 (s, 3H), 0.89 (d, *J* = 6.5 Hz, 3H), 0.76 (d, *J* = 6.8 Hz, 6H), 0.63 (s, 3H); LCMS (ESI) *m*/*z* calcd for C₂₉H₄₈NO₅S [M-Na]⁻ 522.3, found 522.3.

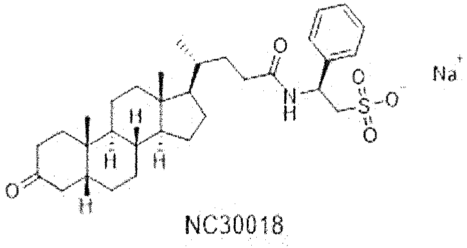

### NC30018

¹H NMR (500 MHz, CD₃OD) δ 7.37-7.27 (m, 4H), 7.24-7.18 (m, 1H), 5.34 (dd, *J* = 10.0, 3.7 Hz, 1H), 3.26 (dd, *J* = 14.3, 10.0 Hz, 1H), 3.09 (dd, *J* = 14.3, 3.8 Hz, 1H), 2.89-2.79 (m, 1H), 2.51-2.41 (m, 1H), 2.31 (ddd, *J* = 14.0, 10.2, 5.3 Hz, 1H), 2.15 (ddd, *J* = 14.0, 10.0, 6.5 Hz, 1H), 2.11-2.00 (m, 3H), 2.00-1.84 (m, 3H), 1.83-1.74 (m, 2H), 1.65-1.56 (m, 2H), 1.55-1.06 (m, 14H), 1.04 (s, 3H), 0.95 (d, *J* = 6.5 Hz, 3H), 0.70 (s, 3H); LCMS (ESI) *m*/*z* calcd for C₃₂H₄₆NO₅S [M-Na]⁻ 556.3, found 556.3.

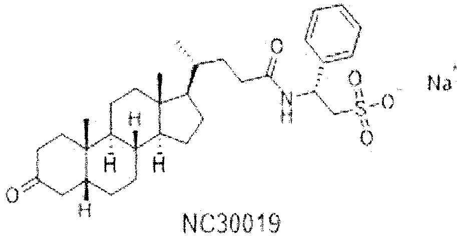

### NC30019

¹H NMR (500 MHz, CD₃OD) δ 7.36-7.27 (m, 4H), 7.24-7.19 (m, 1H), 5.35 (dd, *J* = 10.1, 3.6 Hz, 1H), 3.26 (dd, *J* = 14.3, 10.1 Hz, 1H), 3.09 (dd, *J* = 14.3, 3.7 Hz, 1H), 2.88-2.80 (m, 1H), 2.51-2.41 (m, 1H), 2.30 (ddd, *J* = 13.8, 9.7, 5.0 Hz, 1H), 2.17 (ddd, *J* = 13.9, 9.3, 7.1 Hz, 1H), 2.12-2.00 (m, 3H), 1.99-1.83 (m, 3H), 1.83-1.73 (m, 2H), 1.71-1.55 (m, 2H), 1.55-1.06 (m, 14H), 1.05 (s, 3H), 0.97 (d, *J* = 6.5 Hz, 3H), 0.67 (s, 3H); LCMS (ESI) *m*/*z* calcd for C₃₂H₄₆NO₅S [M-Na]⁻ 556.3, found 556.3.

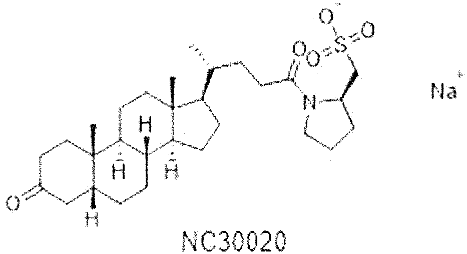

### NC30020

¹H NMR (500 MHz, CD₃OD) δ 4.52-4.45 (m, 0.5H), 4.43-4.36 (m, 0.5H), 3.58-3.51 (m, 0.5H), 3.50-3.28 (m, 2H), 3.02-2.90 (m, 1H), 2.85 (td, *J* = 14.2, 4.1 Hz, 1H), 2.74 (dd, *J* = 13.3, 10.6 Hz, 0.5H), 2.56 (ddd, *J* = 15.3, 10.6, 5.3 Hz, 0.5H), 2.51-2.27 (m, 3H), 2.20 (ddd, *J* = 14.7, 10.4, 5.9 Hz, 0.5H), 2.12-1.87 (m, 9H), 1.85-1.71 (m, 2H), 1.69-1.57 (m, 2H), 1.56-1.07 (m, 14H), 1.05 (s, 1.5H), 1.04 (s, 1.5H), 1.00 (d, *J* = 6.6 Hz, 1.5H), 0.98 (d, *J* = 6.6 Hz, 1.5H), 0.73 (s, 3H); LCMS (ESI) m/z calcd for C₂₉H₄₆NO₅S [M-Na]⁻ 520.3, found 520.3.

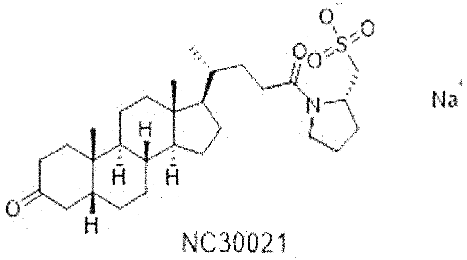

### NC30021

¹H NMR (400 MHz, DMSO-d₆) δ4.24-4.10 (m, 1H), 3.42-3.27 (m, 1H), 3.27-3.11 (m, 1H), 2.91 (dd, *J* = 12.7, 2.9 Hz, 0.5H), 2.76 (td, *J* = 14.1, 6.4 Hz, 1H), 2.60-2.52 (m, 0.5H), 2.43-2.03 (m, 5H), 2.00-1.89 (m, 3H), 1.88-1.48 (m, 10H), 1.47-0.99 (m, 14H), 0.96 (s, 3H), 0.93-0.86 (m, 3H), 0.65 (s, 3H); LCMS (ESI) *m*/*z* calcd for C₂₉H₄₆NO₅S [M-Na]⁻ 520.3, found 520.4.

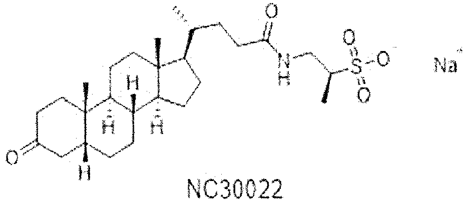

### NC30022

¹H NMR (500 MHz, CD₃OD) δ 3.57 (dd, *J* = 13.8, 6.1 Hz, 1H), 3.44 (dd, *J* = 13.8, 6.7 Hz, 1H), 3.00-2.90 (m, 1H), 2.89-2.80 (m, 1H), 2.52-2.41 (m, 1H), 2.26 (ddd, *J* = 13.9, 10.2, 5.2 Hz, 1H), 2.17-2.02 (m, 4H), 1.99-1.85 (m, 3H), 1.85-1.75 (m, 2H), 1.68-1.57 (m, 2H), 1.57-1.24 (m, 13H), 1.24-1.07 (m, 4H), 1.05 (s, 3H), 0.97 (d, *J* = 6.5 Hz, 3H), 0.73 (s, 3H); LCMS (ESI) *m*/*z* calcd for C₂₇H₄₄NO₅S [M-Na]⁻ 494.3, found 494.2.

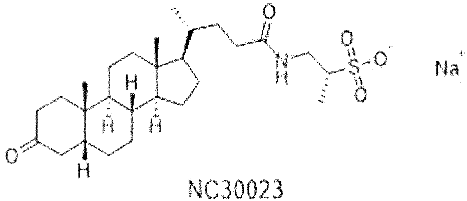

### NC30023

¹H NMR (400 MHz, DMSO-d₆) δ 7.59 (t, *J* = 5.6 Hz, 1H), 3.28-3.20 (m, 1H), 3.19-3.09 (m, 1H), 2.75 (t, *J* = 14.1 Hz, 1H), 2.48-2.30 (m, 2H), 2.07 (ddd, *J* = 14.6, 9.9, 5.2 Hz, 1H), 2.00-1.88 (m, 4H), 1.87-1.49 (m, 7H), 1.47-1.04 (m, 14H), 1.02 (d, *J* = 6.8 Hz, 3H), 0.96 (s, 3H), 0.88 (d, *J* = 6.5 Hz, 3H), 0.64 (s, 3H); LCMS (ESI) m/z calcd for C₂₇H₄₄NO₅S [M-Na]⁻ 494.3, found 494.2.

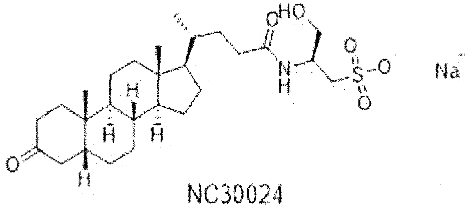

### NC30024

¹H NMR (500 MHz, CD₃OD) δ 4.34-4.26 (m, 1H), 3.75-3.64 (m, 2H), 3.14-3.06 (m, 1H), 3.06-2.98 (m, 1H), 2.89-2.79 (m, 1H), 2.51-2.40 (m, 1H), 2.33-2.23 (m, 1H), 2.16-1.99 (m, 4H), 1.99-1.87 (m, 3H), 1.85-1.74 (m, 2H), 1.68-1.56 (m, 2H), 1.56-1.24 (m, 10H), 1.24-1.06 (m, 4H), 1.05 (s, 3H), 0.97 (d, *J* = 6.5 Hz, 3H), 0.73 (s, 3H); LCMS (ESI) *m*/*z* calcd for C₂₇H₄₄NO₆S [M-Na]⁻510.3, found 510.2.

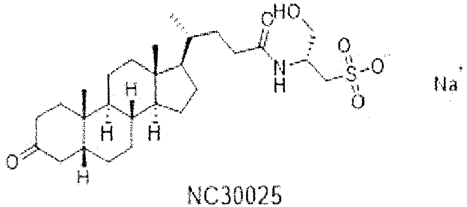

### NC30025

¹H NMR (500 MHz, CD₃OD) δ 4.33-4.26 (m, 1H), 3.76-3.65 (m, 2H), 3.13-3.00 (m, 2H), 2.89-2.80 (m, 1H), 2.51-2.40 (m, 1H), 2.32-2.24 (m, 1H), 2.16-2.00 (m, 4H), 1.99-1.86 (m, 3H), 1.85-1.74 (m, 2H), 1.68-1.57 (m, 2H), 1.56-1.24 (m, 10H), 1.24-1.06 (m, 6H), 1.05 (s, 3H), 0.97 (d, *J* = 6.5 Hz, 3H), 0.73 (s, 3H) ; LCMS (ESI) *m*/*z* calcd for C₂₇H₄₄NO₆S [M-Na]⁻ 510.3, found 510.3.

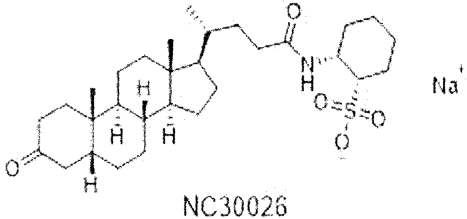

### NC30026

¹H NMR (500 MHz, CD₃OD) δ 4.34-4.28 (m, 1H), 2.91 (dt, *J* = 10.2, 4.0 Hz, 1H), 2.88-2.80 (m, 1H), 2.52-2.42 (m, 1H), 2.32-1.84 (m, 12H), 1.84-1.74 (m, 2H), 1.69-1.57 (m, 2H), 1.57-1.24 (m, 14H), 1.24-1.06 (m, 4H), 1.04 (s, 3H), 0.98 (d, *J* = 6.5 Hz, 3H), 0.72 (s, 3H); LCMS (ESI) m/Z calcd for C₃₀H₄₈NO₅S [M-Na]⁻ 534.3, found 534.4.

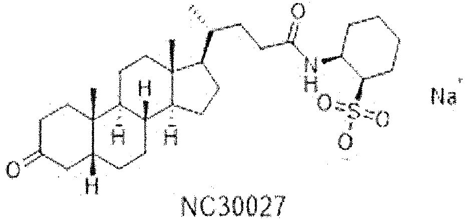

### NC30027

¹H NMR (500 MHz, CD₃OD) δ 4.33-4.26 (m, 1H), 2.91 (dt, *J* = 10.1, 4.1 Hz, 1H), 2.88-2.80 (m, 1H), 2.51-2.41 (m, 1H), 2.31 (ddd, *J* = 14.2, 10.4, 5.2 Hz, 1H), 2.26-2.18 (m, 1H), 2.16-1.74 (m, 12H), 1.69-1.57 (m, 2H), 1.57-1.24 (m, 14H), 1.24-1.06 (m, 4H), 1.05 (s, 3H), 0.97 (d, *J* = 6.5 Hz, 3H), 0.73 (s, 3H); LCMS (ESI) *m*/*z* calcd for C₃₀H₄₈NO₅S [M-Na]⁻ 534.3, found 534.3.

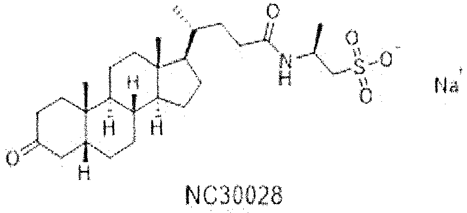

### NC30028

¹H NMR (500 MHz, CD₃OD) δ 4.37-4.26 (m, 1H), 3.05 (dd, *J* = 13.9, 5.6 Hz, 1H), 2.92-2.80 (m, 2H), 2.52-2.41 (m, 1H), 2.22 (ddd, *J* = 13.8, 10.3, 5.2 Hz, 1H), 2.13-2.01 (m, 4H), 1.99-1.85 (m, 3H), 1.84-1.74 (m, 2H), 1.68-1.57 (m, 2H), 1.56-1.24 (m, 13H), 1.23-1.07 (m, 4H), 1.05 (s, 3H), 0.97 (d, J = 6.5 Hz, 3H), 0.73 (s, 3H); LCMS (ESI) *m*/*z* calcd for C₂₇H₄₄NO₅S [M-Na]⁻494.3, found 494.2.

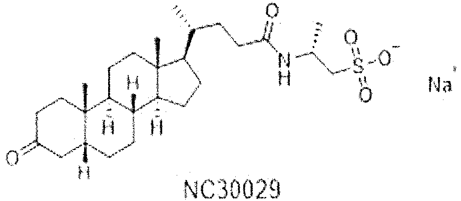

### NC30029

¹H NMR (400 MHz, DMSO-d₆) δ 7.68 (d, *J* = 7.5 Hz, 1H), 4.04-3.92 (m, 1H), 2.81-2.69 (m, 1H), 2.60 (dd, *J* = 13.4, 4.6 Hz, 1H), 2.49-2.30 (m, 2H), 2.09-1.48 (m, 12H), 1.47-0.99 (m, 17H), 0.96 (s, 3H), 0.88 (d, J = 6.5 Hz, 3H), 0.64 (s, 3H); LCMS (ESI) *m*/*z* calcd for C₂₇H₄₄NO₅S [M-Na]⁻ 494.3, found 494.2.

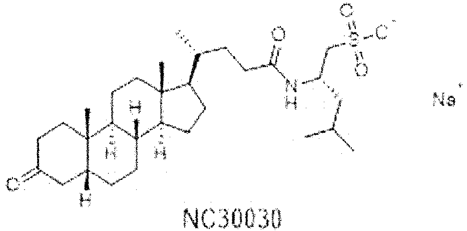

### NC30030

¹H NMR (500 MHz, CD₃OD) δ 4.39-4.30 (m, 1H), 3.03 (dd, *J* = 14.0, 5.6 Hz, 1H), 2.90 (dd, *J* = 14.0, 6.2 Hz, 1H), 2.87-2.79 (m, 1H), 2.51-2.40 (m, 1H), 2.24 (ddd, *J* = 13.8, 9.9, 5.3 Hz, 1H), 2.14-2.02 (m, 4H), 1.99-1.87 (m, 3H), 1.84-1.75 (m, 2H), 1.71-1.24 (m, 15H), 1.23-1.07 (m, 4H), 1.05 (s, 3H), 1.00-0.91 (m, 9H), 0.73 (s, 3H); LCMS (ESI) m/Z calcd for C₃₀H₅₀NO₅S [M-Na]⁻536.3, found 536.3.

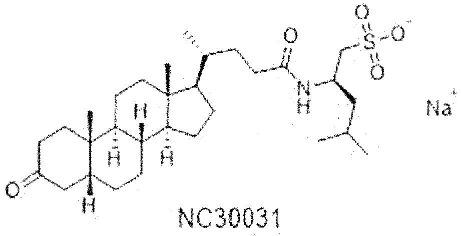

### NC30031

¹H NMR (500 MHz, CD₃OD) δ 4.42-9.33 (m, 1H), 3.01 (dd, *J* = 14.1, 5.6 Hz, 1H), 2.94-2.80 (m, 2H), 2.52-2.41 (m, 1H), 2.22 (ddd, *J* = 14.2, 9.4, 5.2 Hz, 1H), 2.15-2.02 (m, 4H), 1.99-1.85 (m, 3H), 1.85-1.75 (m, 2H), 1.71-1.06 (m, 19H), 1.05 (s, 3H), 1.00-0.91 (m, 9H), 0.72 (s, 3H); LCMS (ESI) *m*/*z* calcd for C₃₀H₅₀NO₅S [M-Na]⁻ 536.3, found 536.3.

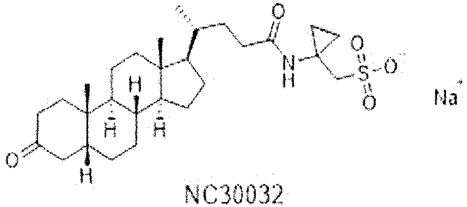

### NC30032

¹H NMR (500 MHz, CD₃OD) δ 3.08-2.97 (m, 2H), 2.88-2.80 (m, 1H), 2.52-2.41 (m, 1H), 2.20 (ddd, *J* = 14.0, 10.4, 5.2 Hz, 1H), 2.12-2.00 (m, 4H), 1.99-1.86 (m, 3H), 1.83-1.71 (m, 2H), 1.68-1.57 (m, 2H), 1.57-1.24 (m, 10H), 1.23-1.07 (m, 4H), 1.04 (s, 3H), 1.01-0.90 (m, 5H), 0.80-0.76 (m, 2H), 0.72 (s, 3H); LCMS (ESI) *m*/*z* calcd for C₂₈H₄₄NO₅S [M-Na]⁻ 506.3, found 506.2.

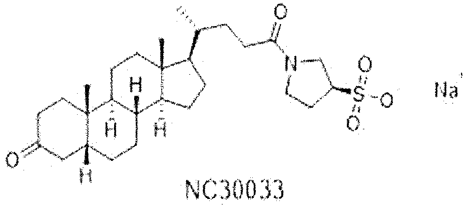

### NC30033

¹H NMR (400 MHz, DMSO-d₆) δ 3.65-3.26 (m, 3.5H), 3.25-3.13 (m, 1H), 3.13-3.04 (m, 0.5H), 2.81-2.69 (m, 1H), 2.43-2.30 (m, 1H), 2.27-2.14 (m, 1H), 2.14-1.86 (m, 6H), 1.86-1.50 (m, 7H), 1.46-1.00 (m, 14H), 0.96 (s, 3H), 0.93-0.82 (m, 3H), 0.68-0.60 (m, 3H); LCMS (ESI) *m*/*z* calcd for C₂₈H₄₄NO₅S [M-Na]⁻506.3, found 506.3.

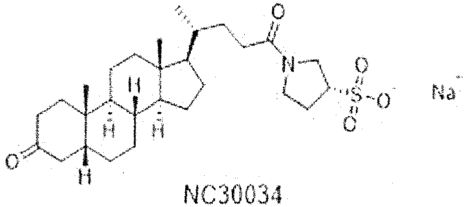

### NC30034

¹H NMR (400 MHz, DMSO-d₆) δ 3.64-3.30 (m, 3.5H), 3.25-3.15 (m, 1H), 3.12-3.03 (m, 0.5H), 2.81-2.69 (m, 1H), 2.43-2.30 (m, 1H), 2.26-2.14 (m, 1H), 2.14-1.86 (m, 6H), 1.86-1.49 (m, 7H), 1.49-1.00 (m, 14H), 0.96 (s, 3H), 0.92-0.83 (m, 3H), 0.66-0.62 (m, 3H); LCMS (ESI) m/Z calcd for C₂₈H₄₄NO₅S [M-Na]⁻506.3, found 506.3.

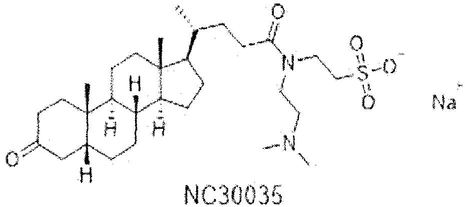

### NC30035

¹H NMR (400 MHz, DMSO-d₆) δ 3.55-3.39 (m, 2H), 3.32-3.22 (m, 2H), 2.82-2.70 (m, 1H), 2.70-2.61 (m, 1.2H), 2.60-2.54 (m, 0.8H), 2.43-2.18 (m, 5H), 2.16 (s, 2.4H), 2.12 (s, 3.6H), 2.00-1.90 (m, 3H), 1.87-1.50 (m, 7H), 1.47-0.99 (m, 14H), 0.96 (s, 3H), 0.91-0.87 (m, 3H), 0.65 (s, 3H); LCMS (ESI) *m*/*z* calcd for C₃₀H₅₁N₂O₅S [M-Na]⁻ 551.4, found 551.3.

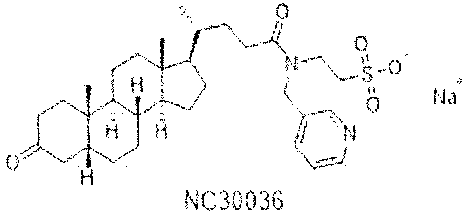

### NC30036

¹H NMR (500 MHz, CD₃OD) δ8.52-8.42 (m, 2H), 7.80-7.77 (m, 0.6H), 7.77-7.72 (m, 0.4H), 7.50-7.44 (m, 0.4H), 7.43-7.38 (m, 0.6H), 4.84-4.79 (m, 0.8H), 4.71-4.62 (m, 1.2H), 3.82-3.71 (m, 2H), 3.11-3.04 (m, 2H), 2.85-2.78 (m, 1H), 2.63-2.55 (m, 0.6H), 2.51-2.37 (m, 2H), 2.35-2.26 (m, 0.4H), 2.12-1.72 (m, 7H), 1.69-1.56 (m, 2H), 1.55-1.06 (m, 14.6H), 1.05 (s, 1.8H), 1.03 (s, 1.2H), 1.01 (d, *J* = 6.5 Hz, 1.8H), 0.99-0.93 (m, 0.4H), 0.88 (d, *J* = 6.5 Hz, 1.2H), 0.74 (s, 1.8H), 0.70 (s, 1.2H); LCMS (ESI) *MIZ* calcd for C₃₂H₄₇N₂O₅S [M-Na]⁻571.3, found 571.4.

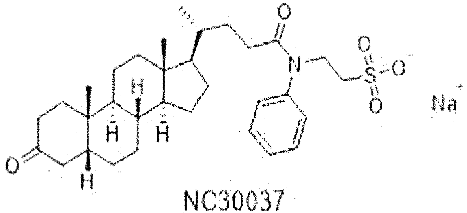

### NC30037

¹H NMR (500 MHz, CD₃OD) δ 7.53-7.47 (m, 2H), 7.46-7.39 (m, 1H), 7.35-7.29 (m, 2H), 4.15-4.05 (m, 2H), 3.09-3.02 (m, 2H), 2.82 (t, *J* = 14.3 Hz, 1H), 2.43 (td, *J* = 14.7, 5.6 Hz, 1H), 2.17-2.02 (m, 3H), 2.02-1.85 (m, 4H), 1.82-1.64 (m, 3H), 1.61-0.91 (m, 19H), 0.71 (d, J = 6.2 Hz, 3H), 0.65 (s, 3H); LCMS (ESI) *m*/*z* calcd for C₃₂H₄₆NO₅S [M-Na]⁻ 556.3, found 556.3.

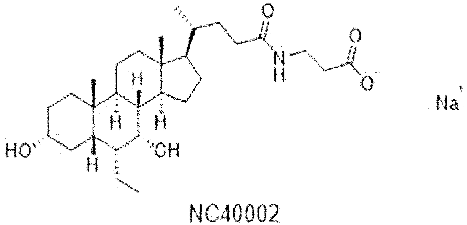

### NC40002

¹H NMR (500 MHz, CD₃OD) δ 3.65 (t, *J* = 2.6 Hz, 1H), 3.39 (t, *J* = 7.0 Hz, 2H), 3.34-3.26 (m, 1H), 2.34 (t, *J* = 7.0 Hz, 2H), 2.23 (ddd, *J* = 13.8, 10.4, 5.2 Hz, 1H), 2.07 (ddd, *J* = 13.8, 10.0, 6.4 Hz, 1H), 2.00 (dt, *J* = 12.4, 3.4 Hz, 1H), 1.97-1.68 (m, 7H), 1.64-1.24 (m, 13H), 1.23-1.06 (m, 3H), 1.05-0.94 (m, 4H), 0.94-0.87 (m, 6H), 0.69 (s, 3H); LCMS (ESI) m/z calcd for C₂₉H₄₈NO₅ [M-Na]⁻ 490.4, found 490.3.

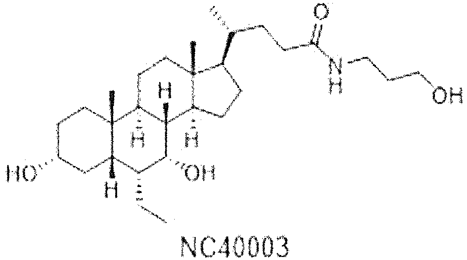

### NC40003

¹H NMR (500 MHz, CD₃OD) δ 3.65 (t, *J* = 2.8 Hz, 1H), 3.58 (t, *J* = 6.3 Hz, 2H), 3.35-3.27 (m, 1H), 3.27-3.22 (m, 2H), 2.24 (ddd, *J* = 13.7, 10.0, 5.2 Hz, 1H), 2.10 (ddd, *J* = 13.8, 9.7, 6.7 Hz, 1H), 2.00 (dt, *J* = 12.5, 3.4 Hz, 1H), 1.97-1.66 (m, 9H), 1.64-1.25 (m, 12H), 1.24-1.06 (m, 3H), 1.06-0.95 (m, 4H), 0.94-0.87 (m, 6H), 0.69 (s, 3H); LCMS (ESI) *m*/*z* calcd for C₂₉H₅₀NO₄ [M-H]⁻ 476.4, found 476.4.

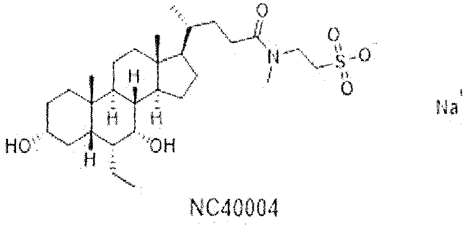

### NC40004

¹H NMR (500 MHz, CD₃OD) δ 3.84-3.70 (m, 2H), 3.66 (t, *J* = 2.6 Hz, 1H), 3.35-3.25 (m, 1H), 3.12 (s, 1.5H), 3.09-2.99 (m, 2H), 2.93 (s, 1.5H), 2.52 (ddd, *J* = 15.6, 10.9, 5.1 Hz, 0.5H), 2.46-2.33 (m, 1H), 2.26 (ddd, *J* = 15.3, 10.7, 5.7 Hz, 0.5H), 2.04-1.69 (m, 8H), 1.63-1.26 (m, 13H), 1.25-1.06 (m, 3H), 1.05-0.95 (m, 4H), 0.94-0.87 (m, 6H), 0.70 (s, 3H); LCMS (ESI) *m*/*z* calcd for C₂₉H₅₀NO₆S [M-Na]⁻ 540.3, found 540.3.

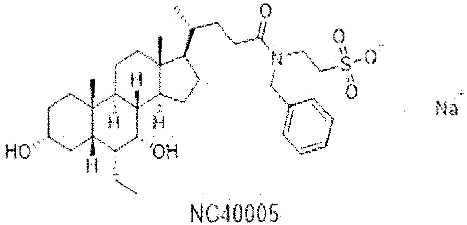

### NC40005

¹H NMR (400 MHz, DMSO-d₆) δ 7.41-7.14 (m, 5H), 4.59-4.48 (m, 2H), 4.28 (d, *J* = 4.8 Hz, 1H), 4.08-4.03 (m, 1H), 3.54-3.39 (m, 3H), 3.17-3.05 (m, 1H), 2.64-2.51 (m, 2H), 2.45-2.10 (m, 2H), 1.97-1.59 (m, 7H), 1.55-0.74 (m, 27H), 0.62 (s, 2H), 0.57 (s, 1H); LCMS (ESI) *m*/*z* calcd for C₃₅H₅₄NO₆S [M-Na]⁻616.4, found 616.3.

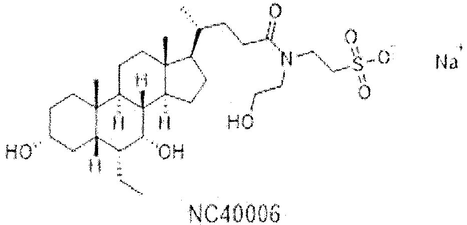

### NC40006

¹H NMR (500 MHz, CD₃OD) δ 3.89-3.78 (m, 1H), 3.78-3.63 (m, 4H), 3.58-3.51 (m, 1H), 3.51-3.43 (m, 1H), 3.35-3.26 (m, 1H), 3.14-3.03 (m, 2H), 2.57-2.44 (m, 1H), 2.42-2.29 (m, 1H), 2.05-1.69 (m, 8H), 1.64-1.25 (m, 13H), 1.25-1.05 (m, 3H), 1.05-0.94 (m, 4H), 0.94-0.86 (m, 6H), 0.70 (s, 3H); LCMS (ESI) m/z calcd for C₃₀H₅₂NO₇S [M-Na]⁻ 570.3, found 570.4.

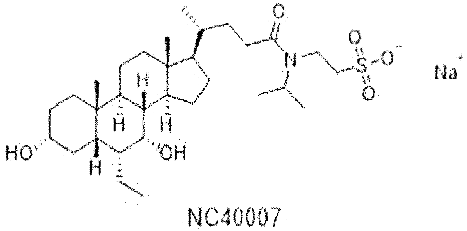

### NC40007

¹H NMR (400 MHz, DMSO-d₆) δ 4.55-4.43 (m, 0.5H), 4.29 (t, J = 5.7 Hz, 1H), 4.11-3.98 (m, 1.5H), 3.49 (s, 1H), 3.45-3.37 (m, 1H), 3.37-3.28 (m, 1H), 3.18-3.05 (m, 1H), 2.67-2.55 (m, 2H), 2.34-2.11 (m, 2H), 1.95-1.57 (m, 7H), 1.55-0.94 (m, 23H), 0.93-0.78 (m, 10H), 0.61 (s, 3H); LCMS (ESI) *m*/*z* calcd for C₃₁H₅₄NO₆S [M-Na]⁻ 568.4, found 568.4.

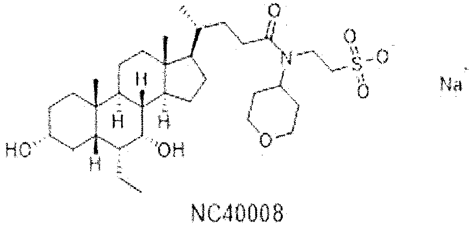

### NC40008

¹H NMR (500 MHz, CD₃OD) δ 4.43 (tt, *J* = 12.1, 3.9 Hz, 0.5H), 4.06-3.90 (m, 2.5H), 3.76-3.62 (m, 3H), 3.53-3.41 (m, 2H), 3.35-3.26 (m, 1H), 3.08-3.01 (m, 2H), 2.54-2.43 (m, 1H), 2.41-2.30 (m, 1H), 2.05-1.64 (m, 11H), 1.64-1.43 (m, 8H), 1.43-1.25 (m, 6H), 1.25-1.06 (m, 3H), 1.06-0.95 (m, 4H), 0.94-0.87 (m, 6H), 0.71(s, 1.5H), 0.69 (s, 1.5H); LCMS (ESI) *m*/*z* calcd for C₃₃H₅₆NO₇S [M-Na]⁻ 610.4, found 610.4.

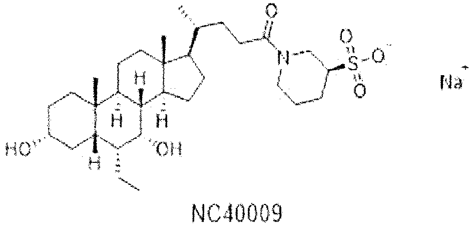

### NC40009

¹H NMR (500 MHz, CD₃OD) δ 5.05-4.99 (m, 0.5H), 4.43 (d, *J* = 13.0 Hz, 0.5H), 4.33-4.25 (m, 0.5H), 3.92 (d, *J* = 13.3 Hz, 0.5H), 3.65 (t, *J* = 2.7 Hz, 1H), 3.35-3.27 (m, 1H), 3.27-3.20 (m, 0.5H), 3.06-2.96 (m, 0.5H), 2.84-2.56 (m, 2H), 2.56-2.40 (m, 1H), 2.38-2.23 (m, 2H), 2.04-1.69 (m, 10H), 1.64-1.26 (m, 14H), 1.25-1.06 (m, 3H), 1.05-0.95 (m, 4H), 0.94-0.87 (m, 6H), 0.70 (s, 3H); LCMS (ESI) *m*/*z* calcd for C₃₁H₅₂NO₆S [M-Na]⁻ 566.4, found 566.4.

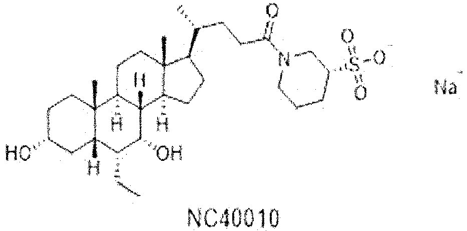

### NC40010

¹H NMR (500 MHz, CD₃OD) δ 5.02 (d, *J* = 9.8 Hz, 0.5H), 4.44 (d, *J* = 13.1 Hz, 0.5H), 4.34-4.26 (m, 0.5H), 3.92 (d, *J* = 13.2 Hz, 0.5H), 3.66 (t, *J* = 2.7 Hz, 1H), 3.34-3.26 (m, 1H), 3.23 (dd, *J* = 13.5, 11.2 Hz, 0.5H), 3.01 (td, *J* = 13.2, 2.8 Hz, 0.5H), 2.84-2.56 (m, 2H), 2.53-2.41 (m, 1H), 2.41-2.24 (m, 2H), 2.04-1.69 (m, 10H), 1.64-1.25 (m, 14H), 1.24-1.06 (m, 3H), 1.05-0.96 (m, 4H), 0.94-0.87 (m, 6H), 0.70 (s, 3H); LCMS (ESI) *m*/*z* calcd for C₃₁H₅₂NO₆S [M-Na]⁻ 566.4, found 566.4.

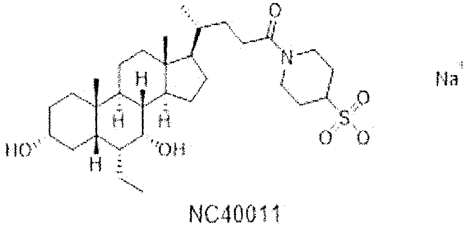

### NC40011

¹H NMR (400 MHz, DMSO-d₆) δ 4.37 (d, *J* = 13.1 Hz, 1H), 4.29 (d, *J* = 4.7 Hz, 1H), 4.05 (d, *J* = 4.1 Hz, 1H), 3.86 (d, *J* = 13.4 Hz, 1H), 3.49 (s, 1H), 3.18-3.05 (m, 1H), 2.98-2.87 (m, 1H), 2.48-2.38 (m, 2H), 2.36-2.10 (m, 2H), 1.98-1.57 (m, 9H), 1.55-1.03 (m, 18H), 1.03-0.75 (m, 11H), 0.61 (s, 3H); LCMS (ESI) *m*/*z* calcd for C₃₁H₅₂NO₆S [M-Na]⁻ 566.4, found 566.3.

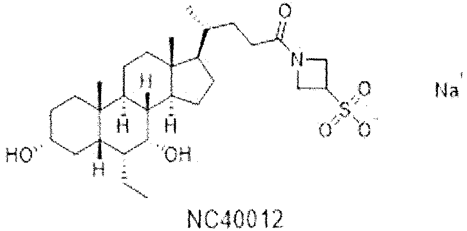

### NC40012

¹H NMR (500 MHz, CD₃OD) δ 4.42 (t, *J* = 8.8 Hz, 1H), 4.37-4.30 (m, 1H), 4.21-4.11 (m, 2H), 3.85-3.76 (m, 1H), 3.65 (t, *J* = 2.6 Hz, 1H), 3.35-3.25 (m, 1H), 2.25-2.13 (m, 1H), 2.11-1.97 (m, 2H), 1.97-1.69 (m, 7H), 1.64-1.24 (m, 13H), 1.24-1.06 (m, 3H), 1.05-0.94 (m, 4H), 0.94-0.87 (m, 6H), 0.70 (s, 3H); LCMS (ESI) *m*/*z* calcd for C₂₉H₄₈NO₆S [M-Na]⁻ 538.3, found 538.3.

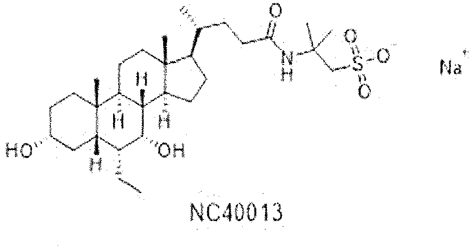

### NC40013

¹H NMR (500 MHz, CD₃OD) δ 3.65 (t, *J* = 2.7 Hz, 1H), 3.34-3.25 (m, 1H), 3.20-3.10 (m, 2H), 2.20 (ddd, *J* = 13.9, 10.3, 5.1 Hz, 1H), 2.09-1.68 (m, 9H), 1.64-1.23 (m, 19H), 1.23-1.05 (m, 3H), 1.05-0.94 (m, 4H), 0.93-0.86 (m, 6H), 0.69 (s, 3H); LCMS (ESI) *m*/*z* calcd for C₃₀H₅₂NO₆S [M-Na]⁻ 554.4, found 554.3.

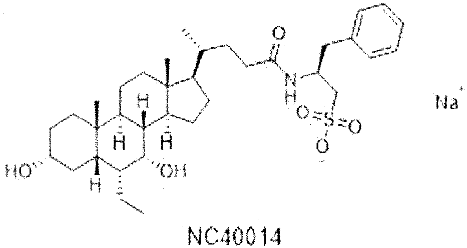

### NC40014

¹H NMR (400 MHz, DMSO-d₆) δ 7.71 (d, *J* = 7.6 Hz, 1H), 7.29-7.12 (m, 5H), 4.29 (d, *J* = 4.6 Hz, 1H), 4.19-4.07 (m, 1H), 4.05 (d, *J* = 5.0 Hz, 1H), 3.50 (s, 1H), 3.18-3.07 (m, 2H), 2.74 (dd, *J* = 13.4, 7.1 Hz, 1H), 2.53 (d, *J* = 6.2 Hz, 2H), 2.05-1.94 (m, 1H), 1.93-1.64 (m, 7H), 1.64-0.74 (m, 28H), 0.59 (s, 3H); LCMS (ESI) *m*/*z* calcd for C₃₅H₅₄NO₆S [M-Na]⁻ 616.4, found 616.4.

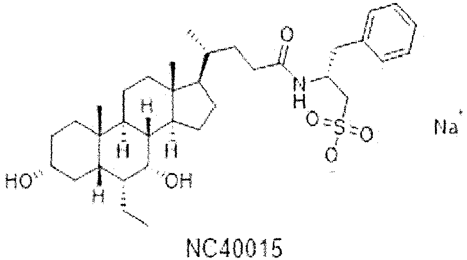

### NC40015

¹H NMR (500 MHz, CD₃OD) δ 7.30-7.22 (m, 4H), 7.21-7.14 (m, 1H), 4.52-4.43 (m, 1H), 3.65 (t, *J* = 2.8 Hz, 1H), 3.34-3.26 (m, 1H), 3. 16 (dd, *J* = 13.6, 6.1 Hz, 1H), 3.03-2.94 (m, 2H), 2.91 (dd, *J* = 13.7, 7.8 Hz, 1H), 2.18 (ddd, *J* = 13.8, 10.3, 5.1 Hz, 1H), 2.07-1.95 (m, 2H), 1.95-1.65 (m, 7H), 1.64-1.05 (m, 16H), 1.00 (td, *J* = 14.2, 3.5 Hz, 1H), 0.95-0.85 (m, 9H), 0.67 (s, 3H); LCMS (ESI) *m*/*z* calcd for C₃₅H₅₄NO₆S [M-Na]⁻ 616.4, found 616.4.

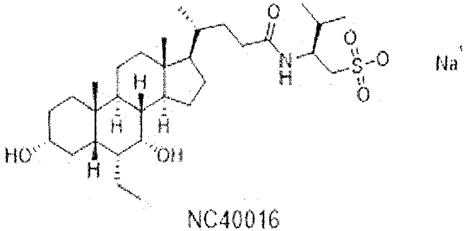

### NC40016

¹H NMR (500 MHz, CD₃OD) δ 4.24-4.16 (m, 1H), 3.65 (t, *J* = 2.8 Hz, 1H), 3.35-3.26 (m, 1H), 2.98-2.93 (m, 2H), 2.29 (ddd, *J* = 13.7, 10.6, 5.1 Hz, 1H), 2.15-2.03 (m, 2H), 2.03-1.69 (m, 8H), 1.63-1.25 (m, 13H), 1.24-1.05 (m, 3H), 1.05-0.96 (m, 4H), 0.95-0.87 (m, 12H), 0.70 (s, 3H); LCMS (ESI) *m*/*z* calcd for C₃₁H₅₄NO₆S [M-Na]⁻ 568.4, found 568.4.

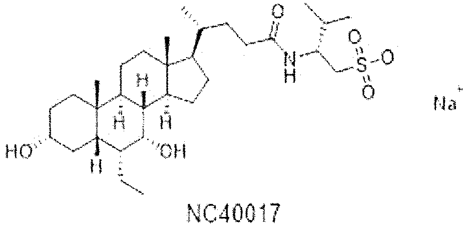

### NC40017

¹H NMR (500 MHz, CD₃OD) δ 4.25-4.18 (m, 1H), 3.65 (t, *J* = 2.7 Hz, 1H), 3.35-3.26 (m, 1H), 2.95 (d, J = 6.1 Hz, 2H), 2.26 (ddd, *J* = 13.7, 9.7, 5.0 Hz, 1H), 2.20-2.04 (m, 2H), 2.04-1.69 (m, 8H), 1.63-1.24 (m, 13H), 1.24-1.05 (m, 3H), 1.05-0.95 (m, 4H), 0.95-0.85 (m, 12H), 0.69 (s, 3H); LCMS (ESI) *m*/*z* calcd for C₃₁H₅₄NO₆S [M-Na]⁻ 568.4, found 568.4.

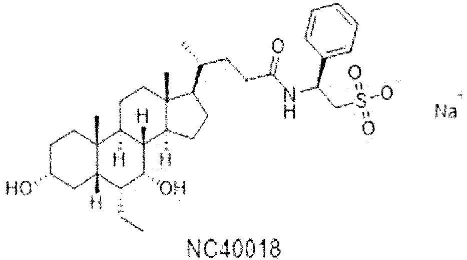

### NC40018

¹H NMR (400 MHz, DMSO-d₆) δ 8.15 (d, *J* = 6.3 Hz, 1H), 7.29-7.19 (m, 4H), 7.18-7.11 (m, 1H), 5.03-4.93 (m, 1H), 4.29 (d, *J* = 4.7 Hz, 1H), 4.05 (d, J = 5.0 Hz, 1H), 3.49 (s, 1H), 3.18-3.05 (m, 1H), 2.85 (dd, *J* = 13.9, 9.1 Hz, 1H), 2.67 (dd, *J* = 13.8, 4.1 Hz, 1H), 2.17-2.05 (m, 1H), 2.01-1.58 (m, 8H), 1.57-0.74 (m, 27H), 0.59 (s, 3H); LCMS (ESI) *m*/*z* calcd for C₃₄H₅₂NO₆S [M-Na]⁻ 602.4, found 602.4.

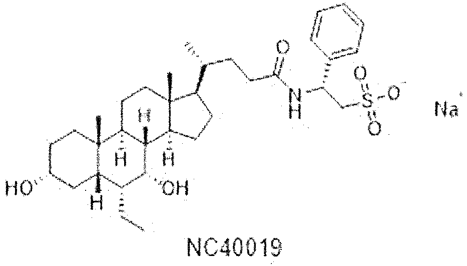

### NC40019

¹H NMR (500 MHz, CD₃OD) δ 7.37-7.26 (m, 4H), 7.25-7.18 (m, 1H), 5.36 (dd, *J* = 10.0, 3.7 Hz, 1H), 3.64 (t, *J* = 2.7 Hz, 1H), 3.34-3.22 (m, 2H), 3.09 (dd, *J* = 14.3, 3.7 Hz, 1H), 2.30 (ddd, *J* = 14.3, 9.6, 5.0 Hz, 1H), 2.18 (ddd, *J* = 13.9, 9.1, 7.2 Hz, 1H), 1.98 (dt, *J* = 12.4, 3.4 Hz, 1H), 1.95-1.67 (m, 7H), 1.64-1.11 (m, 15H), 1.10-0.99 (m, 2H), 0.97 (d, *J* = 6.4 Hz, 3H), 0.93-0.87 (m, 6H), 0.64 (s, 3H); LCMS (ESI) *m*/*z* calcd for C₃₄H₅₂NO₆S [M-Na]⁻ 602.4, found 602.4.

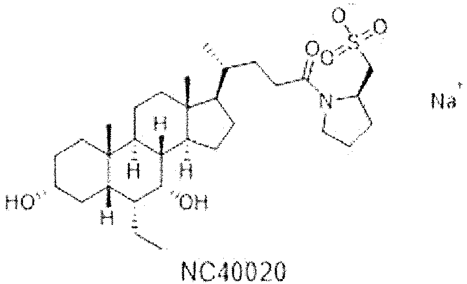

### NC40020

¹H NMR (500 MHz, CD₃OD) δ 4.52-4.45 (m, 0.5H), 4.44-4.37 (m, 0.5H), 3.66 (t, *J* = 2.7 Hz, 1H), 3.58-3.52 (m, 0.5H), 3.50-3.27 (m, 3H), 3.02-2.90 (m, 1H), 2.74 (dd, *J* = 13.3, 10.6 Hz, 0.5H), 2.56 (ddd, *J* = 15.3, 10.6, 5.2 Hz, 0.5H), 2.48-2.27 (m, 2H), 2.20 (ddd, *J* = 14.6, 10.4, 5.9 Hz, 0.5H), 2.11-1.70 (m, 11H), 1.64-1.25 (m, 13H), 1.24-1.06 (m, 3H), 1.05-0.95 (m, 4H), 0.94-0.87 (m, 6H), 0.70 (s, 3H); LCMS (ESI) *m*/*z* calcd for C₃₁H₅₂NO₆S [M-Na]⁻566.4, found 566.4.

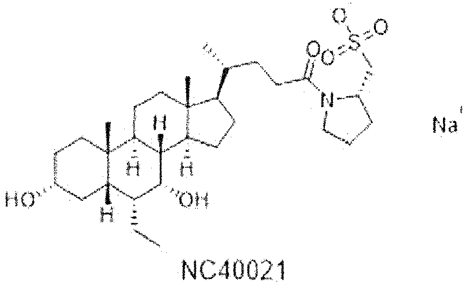

### NC40021

¹H NMR (500 MHz, CD₃OD) δ 4.52-4.44 (m, 0.5H), 4.44-4.37 (m, 0.5H), 3.66 (t, *J* = 2.7 Hz, 1H), 3.58-3.52 (m, 0.5H), 3.51-3.26 (m, 3H), 3.02-2.90 (m, 1H), 2.74 (dd, *J* = 13.3, 10.6 Hz, 0.5H), 2.49-2.39 (m, 2H), 2.34 (ddd, *J* = 14.6, 10.8, 5.2 Hz, 0.5H), 2.22 (ddd, *J* = 14.7, 10.5, 5.8 Hz, 0.5H), 2.11-1.69 (m, 11H), 1.64-1.25 (m, 13H), 1.25-1.05 (m, 3H), 1.05-0.95 (m, 4H), 0.94-0.87 (m, 6H), 0.701 (s, 1.5H), 0.697 (s, 1.5H); LCMS (ESI) *m*/*z* calcd for C₃₁H₅₂NO₆S [M-Na]⁻ 566.4, found 566.3.

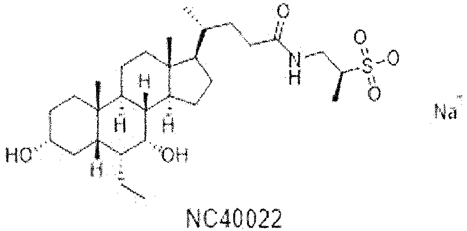

### NC40022

¹H NMR (500 MHz, CD₃OD) δ 3.65 (t, *J* = 2.7 Hz, 1H), 3.57 (dd, *J* = 13.7, 6.0 Hz, 1H), 3.44 (dd, *J* = 13.7, 6.8 Hz, 1H), 3.31-3.26 (m, 1H), 3.00-2.90 (m, 1H), 2.26 (ddd, *J* = 13.9, 10.1, 5.1 Hz, 1H), 2.12 (ddd, *J* = 13.9, 9.7, 6.7 Hz, 1H), 2.04-1.69 (m, 8H), 1.64-1.25 (m, 16H), 1.24-1.05 (m, 3H), 1.05-0.95 (m, 4H), 0.94-0.87 (m, 6H), 0.69 (s, 3H); LCMS (ESI) m/z calcd for C₂₉H₅₀NO₆S [M-Na]⁻ 540.3, found 540.3.

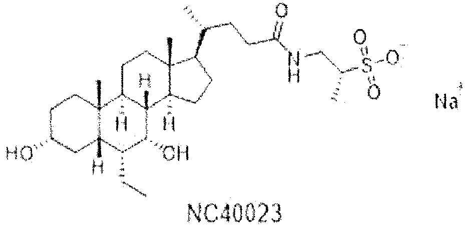

### NC40023

¹H NMR (400 MHz, DMSO-d₆) δ 7.63-7.56 (m, 1H), 4.30 (d, *J* = 4.6 Hz, 1H), 4.05 (d, *J* = 5.0 Hz, 1H), 3.49 (s, 1H), 3.30-3.22 (m, 1H), 3.19-3.05 (m, 2H), 2.49-2.41 (m, 1H), 2.13-2.01 (m, 1H), 2.00-1.58 (m, 8H), 1.57-0.74 (m, 30H), 0.59 (s, 3H). ; LCMS (ESI) m/Z calcd for C₂₉H₅₀NO₆S [M-Na]⁻ 540.3, found 540.3.

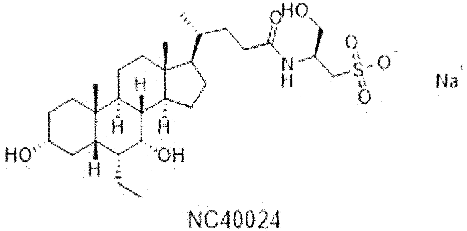

### NC40024

¹H NMR (500 MHz, CD₃OD) δ 4.34-4.25 (m, 1H), 3.76-3.67 (m, 2H), 3.65 (t, *J* = 2.6 Hz, 1H), 3.36-3.25 (m, 1H), 3.14-2.99 (m, 2H), 2.33-2.22 (m, 1H), 2.17-2.06 (m, 1H), 2.04-1.70 (m, 8H), 1.63-1.25 (m, 13H), 1.24-1.05 (m, 3H), 1.05-0.95 (m, 4H), 0.94-0.87 (m, 6H), 0.70 (s, 3H); LCMS (ESI) *m*/*z* calcd for C₂₉H₅₀NO₇S [M-Na]⁻ 556.3, found 556.3.

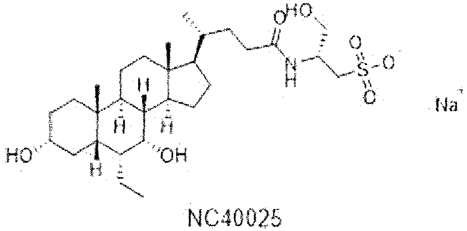

### NC40025

¹H NMR (500 MHz, CD₃OD) δ 4.34-4.26 (m, 1H), 3.76-3.67 (m, 2H), 3.65 (t, *J* = 2.6 Hz, 1H), 3.36-3.26 (m, 1H), 3.14-2.99 (m, 2H), 2.32-2.24 (m, 1H), 2.16-2.07 (m, 1H), 2.03-1.68 (m, 8H), 1.65-1.25 (m, 13H), 1.24-1.05 (m, 3H), 1.05-0.94 (m, 4H), 0.94-0.87 (m, 6H), 0.70 (s, 3H); LCMS (ESI) *m*/*z* calcd for C₂₉H₅₀NO₇S [M-Na]⁻ 556.3, found 556.3.

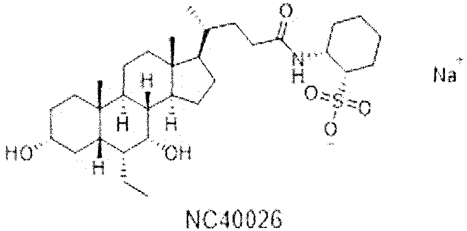

### NC40026

¹H NMR (500 MHz, CD₃OD) δ 4.34-4.28 (m, 1H), 3.65 (t, J = 2.6 Hz, 1H), 3.35-3.25 (m, 1H), 2.91 (dt, *J* = 10.2, 4.0 Hz, 1H), 2.32-2.11 (m, 3H), 2.07-1.69 (m, 11H), 1.64-1.25 (m, 17H), 1.24-1.05 (m, 3H), 1.05-0.94 (m, 4H), 0.94-0.87 (m, 6H), 0.69 (s, 3H); LCMS (ESI) *m*/*z* calcd for C₃₂H₅₄NO₆S [M-Na]⁻580.4, found 580.4.

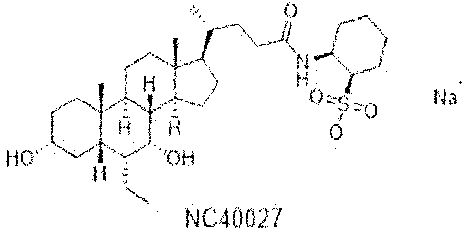

### NC40027

¹H NMR (500 MHz, CD₃OD) δ 4.33-4.26 (m, 1H), 3.65 (t, *J* = 2.7 Hz, 1H), 3.34-3.26 (m, 1H), 2.91 (dt, *J* = 10.1, 4.1 Hz, 1H), 2.31 (ddd, *J* = 14.2, 10.4, 5.2 Hz, 1H), 2.26-2.18 (m, 1H), 2.13 (ddd, *J* = 14.2, 10.1, 6.3 Hz, 1H), 2.07-1.69 (m, 11H), 1.65-1.25 (m, 17H), 1.24-1.05 (m, 3H), 1.05-0.95 (m, 4H), 0.94-0.87 (m, 6H), 0.70 (s, 3H); LCMS (ESI) *m*/*z* calcd for C₃₂H₅₄NO₆S [M-Na]⁻ 580.4, found 580.4.

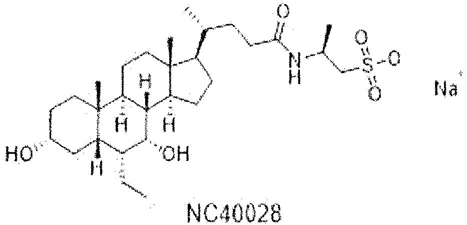

### NC40028

¹H NMR (500 MHz, CD₃OD) δ 4.37-4.27 (m, 1H), 3.65 (t, *J* = 2.7 Hz, 1H), 3.35-3.26 (m, 1H), 3.05 (dd, *J* = 13.9, 5.5 Hz, 1H), 2.88 (dd, *J* = 13.9, 7.0 Hz, 1H), 2.22 (ddd, *J* = 13.8, 10.3, 5.2 Hz, 1H), 2.07 (ddd, *J* = 13.8, 10.0, 6.4 Hz, 1H), 2.03-1.69 (m, 8H), 1.63-1.24 (m, 16H), 1.24-1.05 (m, 3H), 1.05-0.95 (m, 4H), 0.94-0.87 (m, 6H), 0.69 (s, 3H); LCMS (ESI) *m*/*z* calcd for C₂₉H₅₀NO₆S [M-Na]⁻ 540.3, found 540.3.

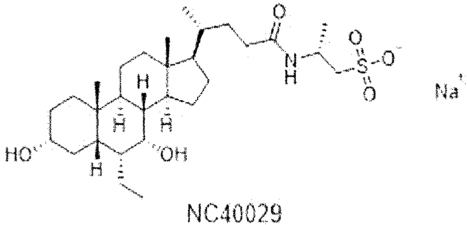

### NC40029

¹H NMR (500 MHz, CD₃OD) δ 4.37-4.27 (m, 1H), 3.65 (t, *J* = 2.7 Hz, 1H), 3.35-3.27 (m, 1H), 3.05 (dd, *J* = 13.9, 5.6 Hz, 1H), 2.88 (dd, *J* = 13.9, 6.9 Hz, 1H), 2.23 (ddd, *J* = 13.8, 10.1, 5.1 Hz, 1H), 2.07 (ddd, *J* = 13.8, 9.8, 6.6 Hz, 1H), 2.03-1.70 (m, 8H), 1.63-1.25 (m, 16H), 1.24-1.05 (m, 3H), 1.05-0.95 (m, 4H), 0.94-0.87 (m, 6H), 0.69 (s, 3H); LCMS (ESI) *m*/*z* calcd for C₂₉H₅₀NO₆S [M-Na]⁻ 540.3, found 540.3.

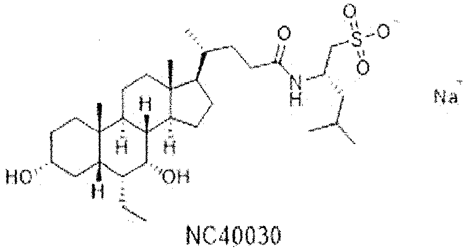

### NC40030

¹H NMR (500 MHz, CD₃OD) δ 4.39-4.30 (m, 1H), 3.65 (t, *J* = 2.8 Hz, 1H), 3.35-3.26 (m, 1H), 3.03 (dd, *J* = 14.0, 5.6 Hz, 1H), 2.90 (dd, *J* = 14.0, 6.3 Hz, 1H), 2.24 (ddd, *J* = 13.8, 10.1, 5.2 Hz, 1H), 2.09 (ddd, *J* = 13.8, 9.8, 6.7 Hz, 1H), 2.04-1.70 (m, 8H), 1.70-1.24 (m, 16H), 1.23-1.06 (m, 3H), 1.05-0.95 (m, 4H), 0.95-0.85 (m, 12H), 0.69 (s, 3H); LCMS (ESI) *m*/*z* calcd for C₃₂H₅₆NO₆S [M-Na]⁻ 582.4, found 582.4.

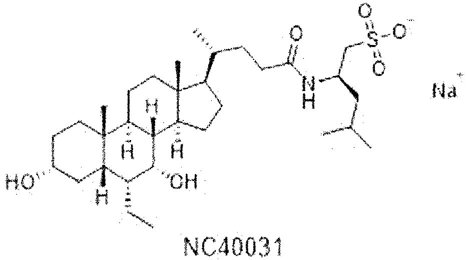

### NC40031

¹H NMR (500 MHz, CD₃OD) δ 4.42-4.33 (m, 1H), 3.65 (t, *J* = 2.7 Hz, 1H), 3.35-3.26 (m, 1H), 3.01 (dd, *J* = 14.0, 5.6 Hz, 1H), 2.90 (dd, *J* = 14.0, 6.3 Hz, 1H), 2.22 (ddd, *J* = 14.1, 9.3, 5.1 Hz, 1H), 2.11 (ddd, *J* = 13.7, 9.0, 7.5 Hz, 1H), 2.04-1.70 (m, 8H), 1.70-1.24 (m, 16H), 1.23-1.06 (m, 3H), 1.05-0.95 (m, 4H), 0.95-0.86 (m, 12H), 0.69 (s, 3H); LCMS (ESI) *m*/*z* calcd for C₃₂H₅₆NO₆S [M-Na]⁻ 582.4, found 582.4.

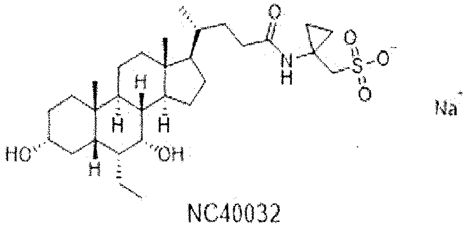

### NC40032

¹H NMR (400 MHz, DMSO-d₆) δ 7.79 (s, 1H), 4.28 (d, *J* = 4.7 Hz, 1H), 4.05 (d, *J* = 5.0 Hz, 1H), 3.49 (s, 1H), 3.17-3.06 (m, 1H), 2.71-2.57 (m, 2H), 2.04-1.55 (m, 9H), 1.55-0.74 (m, 29H), 0.62-0.50 (m, 5H); LCMS (ESI) *m*/*Z* calcd for C₃₀H₅₀NO₆S [M-Na]⁻ 552.3, found 552.3.

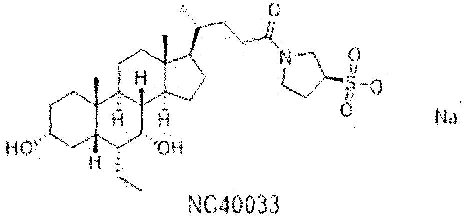

### NC40033

¹H NMR (500 MHz, CD₃OD) δ 3.87-3.40 (m, 6H), 3.35-3.24 (m, 1H), 2.47-2.17 (m, 4H), 2.06-1.69 (m, 8H), 1.64-1.25 (m, 13H), 1.24-1.06 (m, 3H), 1.05-0.94 (m, 4H), 0.94-0.87 (m, 6H), 0.71 (s, 1.5H), 0.70 (s, 1.5H); LCMS (ESI) *m*/*z* calcd for C₃₀H₅₀NO₆S [M-Na]⁻ 552.3, found 552.3.

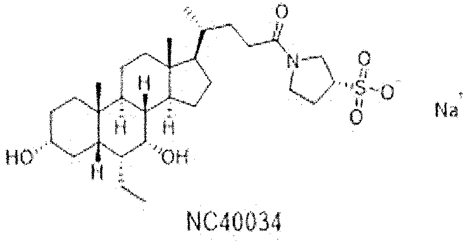

### NC40034

¹H NMR (500 MHz, CD₃OD) δ 3.87-3.40 (m, 6H), 3.36-3.26 (m, 1H), 2.47-2.17 (m, 4H), 2.04-1.69 (m, 8H), 1.64-1.25 (m, 13H), 1.25-1.06 (m, 3H), 1.05-0.94 (m, 4H), 0.94-0.87 (m, 6H), 0.71 (s, 1.5H), 0.70 (s, 1.5H); LCMS (ESI) *m*/*z* calcd for C₃₀H₅₀NO₆S [M-Na]⁻ 552.3, found 552.3.

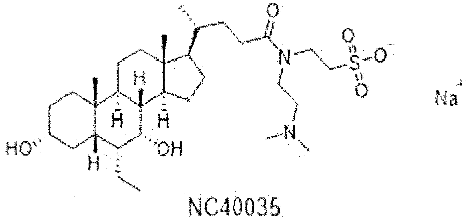

### NC40035

¹H NMR (500 MHz, CD₃OD) δ 3.81-3.75 (m, 1H), 3.73-3.68 (m, 1H), 3.65 (t, *J* = 2.7 Hz, 1H), 3.59-3.44 (m, 2H), 3.35-3.27 (m, 1H), 3.11-3.02 (m, 2H), 2.58-2.32 (m, 4H), 2.31 (s, 3H), 2.29 (s, 3H), 2.06-1.68 (m, 8H), 1.65-1.25 (m, 13H), 1.25-1.06 (m, 3H), 1.05-0.95 (m, 4H), 0.94-0.86 (m, 6H), 0.70 (s, 3H); LCMS (ESI) MIZ calcd for C₃₂H₅₇N₂O₆S [M-Na]⁻ 597.4, found 597.4.

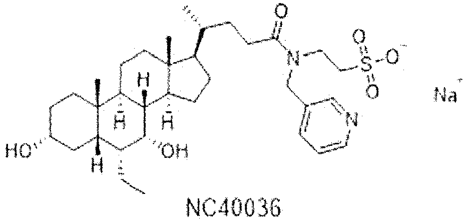

### NC40036

¹H NMR (500 MHz, CD₃OD) δ 8.52-8.41 (m, 2H), 7.81-7.76 (m, 0.6H), 7.76-7.71 (m, 0.4H), 7.51-7.45 (m, 0.4H), 7.43-7.37 (m, 0.6H), 4.84-4.81 (m, 1H), 4.72-7.62 (m, 1H), 3.85-3.70 (m, 2H), 3.68-3.62 (m, 1H), 3.36-3.26 (m, 1H), 3.12-3.03 (m, 2H), 2.64-2.54 (m, 0.6H), 2.51-2.37 (m, 1H), 2.35-2.25 (m, 0.4H), 2.04-1.67 (m, 8H), 1.64-1.05 (m, 16H), 1.05-0.95 (m, 3H), 0.94-0.85 (m, 7H), 0.69 (s, 1.8H), 0.66 (s, 1.2H); LCMS (ESI) *m*/*z* calcd for C₃₄H₅₃N₂O₆S [M-Na]⁻ 617.4, found 617.4.

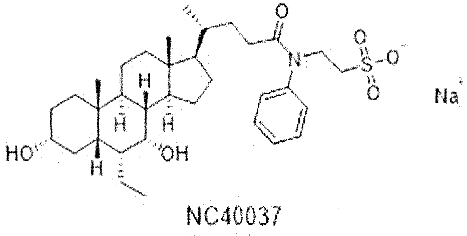

### NC40037

¹H NMR (500 MHz, CD₃OD) δ 7.53-7.46 (m, 2H), 7.46-7.38 (m, 1H), 7.35-7.29 (m, 2H), 4.13-4.06 (m, 2H), 3.63 (s, 1H), 3.34-3.25 (m, 1H), 3.09-3.02 (m, 2H), 2.17-2.08 (m, 1H), 2.02-1.93 (m, 1H), 1.93-1.64 (m, 8H), 1.62-1.15 (m, 13H), 1.14-0.85 (m, 10H), 0.70 (d, *J* = 5.9 Hz, 3H), 0.62 (s, 3H); LCMS (ESI) *m*/*z* calcd for C₃₄H₅₂NO₆S [M-Na]⁻ 602.4, found 602.4.

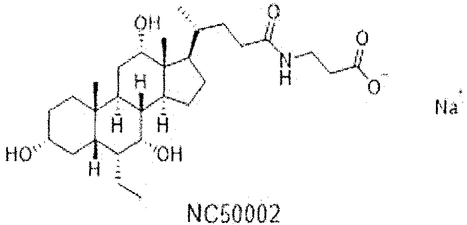

### NC50002

¹H NMR (400 MHz, DMSO-d₆) δ 7.83 (t, *J* = 5.5 Hz, 1H), 4.29 (s, 1H), 4.08 (s, 1H), 3.93 (s, 1H), 3.77 (s, 1H), 3.48 (s, 1H), 3.23-3.16 (m, 2H), 3.15-3.08 (m, 1H), 2.32 (t, *J* = 6.9 Hz, 2H), 2.14-1.55 (m, 10H), 1.55-1.05 (m, 13H), 1.01-0.70 (m, 11H), 0.58 (s, 3H). ; LCMS (ESI) *m*/*z* calcd for C₂₉H₄₈NO₆ [M-Na]⁻ 506.3, found 506.3.

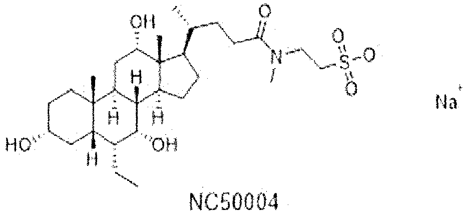

### NC50004

¹H NMR (500 MHz, CD₃OD) δ 3.98-3.93 (m, 1H), 3.86-3.70 (m, 2H), 3.66 (t, *J* = 2.7 Hz, 1H), 3.35-3.26 (m, 1H), 3.13 (s, 1.5H), 3.08-3.00 (m, 2H), 2.93 (s, 1.5H), 2.53 (ddd, *J* = 15.4, 10.8, 5.1 Hz, 0.5H), 2.48-2.33 (m, 1H), 2.33-2.15 (m, 1.5H), 2.02 (td, *J* = 12.1, 7.4 Hz, 1H), 1.97-1.68 (m, 7H), 1.64-1.25 (m, 12H), 1.18-1.08 (m, 1H), 1.08-0.93 (m, 4H), 0.93-0.87 (m, 6H), 0.72 (s, 3H); LCMS (ESI) *m*/*z* calcd for C₂₉H₅₀NO₇S [M-Na]⁻ 556.3, found 556.3.

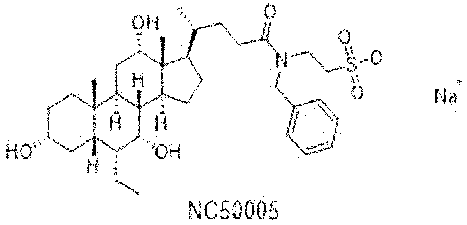

### NC50005

¹H NMR (400 MHz, DMSO-d₆) δ 7.42-7.14 (m, 5H), 4.59 (s, 0.6H), 4.56-4.43 (m, 1.4H), 4.29 (d, *J* = 4.4 Hz, 1H), 4.13 (d, *J* = 3.4 Hz, 0.7H), 4.06 (d, *J* = 3.1 Hz, 0.3H), 3.97-3.90 (m, 1H), 3.81 (s, 0.7H), 3.74 (s, 0.3H), 3.54-3.39 (m, 3H), 3.17-3.06 (m, 1H), 2.66-2.54 (m, 2H), 2.46-1.91 (m, 4H), 1.89-1.58 (m, 6H), 1.54-1.04 (m, 13H), 1.02-0.74 (m, 11H), 0.60 (s, 2.1H), 0.56 (s, 0.9H); LCMS (ESI) *m*/*z* calcd for C₃₅H₅₄NO₇S [M-Na]⁻ 632.4, found 632.3.

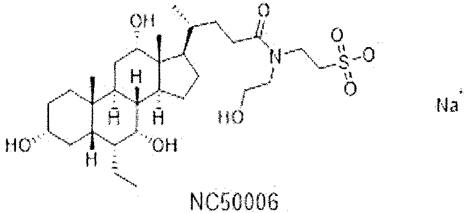

### NC50006

¹H NMR (500 MHz, CD₃OD) δ 3.98-3.94 (m, 1H), 3.90-3.79 (m, 1H), 3.77-3.64 (m, 4H), 3.59-3.53 (m, 1H), 3.52-3.43 (m, 1H), 3.35-3.26 (m, 1H), 3.13-3.04 (m, 2H), 2.58-2.45 (m, 1H), 2.43-2.30 (m, 1H), 2.24-2.15 (m, 1H), 2.05-1.68 (m, 8H), 1.65-1.26 (m, 12H), 1.20-0.94 (m, 5H), 0.94-0.86 (m, 6H), 0.76-0.67 (m, 3H); LCMS (ESI) *m*/*z* calcd for C₃₀H₅₂NO₈S [M-Na]⁻ 586.3, found 586.4.

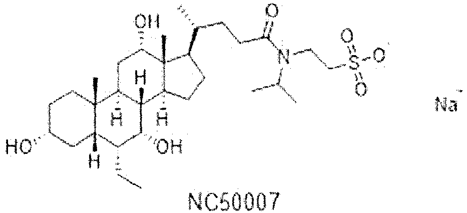

### NC50007

¹H NMR (500 MHz, CD₃OD) δ 4.66-4.56 (m, 0.4H) and 4.25-4.13 (m, 0.6H), 3.98-3.93 (m, 1H), 3.72-3.58 (m, 3H), 3.36-3.26 (m, 1H), 3.09-3.02 (m, 2H), 2.51-2.41 (m, 1H), 2.38-2.27 (m, 1H), 2.25-2.15 (m, 1H), 2.08-1.97 (m, 1H), 1.96-1.68 (m, 7H), 1.65-1.23 (m, 16H), 1.20-1.08 (m, 3H), 1.08-0.95 (m, 4H), 0.94-0.86 (m, 6H), 0.723 (s, 1.8H), 0.720 (s, 1.2H); LCMS (ESI) *m*/*z* calcd for C₃₁H₅₄NO₇S [M-Na]⁻ 584.4, found 584.4.

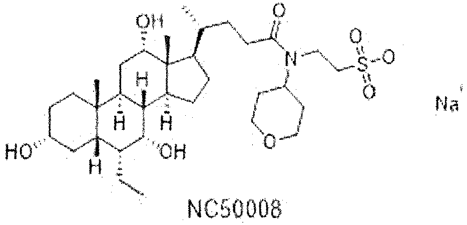

### NC50008

¹H NMR (500 MHz, CD₃OD) δ 4.44 (tt, *J* = 12.1, 3.9 Hz, 0.5H), 4.06-3.91 (m, 3.5H), 3.78-3.69 (m, 1H), 3.69-3.62 (m, 2H), 3.53-3.41 (m, 2H), 3.37-3.24 (m, 1H), 3.09-3.00 (m, 2H), 2.55-2.44 (m, 1H), 2.41-2.31 (m, 1H), 2.25-2.15 (m, 1H), 2.08-1.64 (m, 11H), 1.64-1.25 (m, 13H), 1.17-0.95 (m, 5H), 0.94-0.87 (m, 6H), 0.72 (s, 1.5H), 0.71 (s, 1.5H); LCMS (ESI) *m*/*z* calcd for C₃₃H₅₆NO₈S [M-Na]⁻ 626.4, found 626.4.

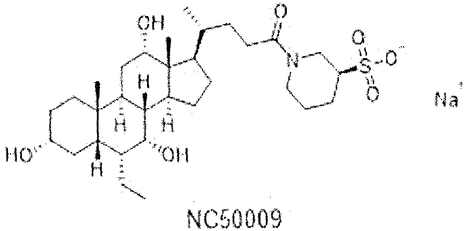

### NC50009

¹H NMR (400 MHz, DMSO-d₆) δ 4.72 (d, *J* = 12.5 Hz, 0.5H), 4.34-4.23 (m, 1.5H), 4.15-4.07 (m, 1H), 4.03 (d, *J* = 13.4 Hz, 0.5H), 3.93 (t, *J* = 4.9 Hz, 1H), 3.83-3.72 (m, 1.5H), 3.48 (s, 1H), 3.18-3.07 (m, 1H), 3.00-2.89 (m, 0.5H), 2.87-2.75 (m, 0.5H), 2.42-1.92 (m, 7H), 1.90-1.08 (m, 22H), 1.02-0.73 (m, 11H), 0.59 (s, 3H); LCMS (ESI) *m*/*z* calcd for C₃₁H₅₂NO₇S [M-Na]⁻ 582.3, found 582.3.

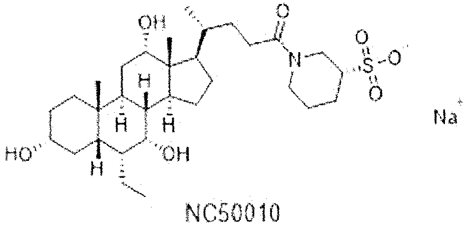

### NC50010

¹H NMR (500 MHz, CD₃OD) δ 5.02 (d, *J* = 9.7 Hz, 0.5H), 4.45 (d, *J* = 12.9 Hz, 0.5H), 4.34-4.28 (m, 0.5H), 3.98-3.90 (m, 1.5H), 3.66 (t, *J* = 2.7 Hz, 1H), 3.35-3.27 (m, 1H), 3.23 (dd, *J* = 13.5, 11.2 Hz, 0.5H), 3.01 (td, *J* = 13.2, 2.8 Hz, 0.5H), 2.79 (tt, *J* = 11.3, 3.8 Hz, 0.5H), 2.75-2.65 (m, 1H), 2.59 (td, *J* = 12.6, 2.7 Hz, 0.5H), 2.54-2.26 (m, 3H), 2.20 (td, *J* = 12.1, 5.2 Hz, 1H), 2.02 (td, *J* = 11.9, 7.2 Hz, 1H), 1.97-1.67 (m, 9H), 1.64-1.26 (m, 13H), 1.18-1.09 (m, 1H), 1.09-0.95 (m, 4H), 0.95-0.87 (m, 6H), 0.72 (s, 3H); LCMS (ESI) *m*/*z* calcd for C₃₁H₅₂NO₇S [M-Na]⁻ 582.3, found 582.4.

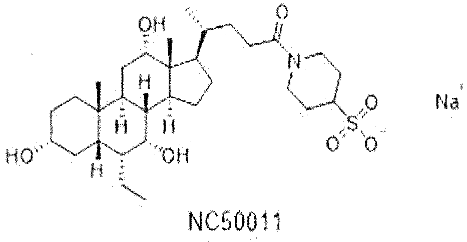

### NC50011

¹H NMR (400 MHz, DMSO-d₆) δ 4.37 (d, *J* = 13.0 Hz, 1H), 4.29 (d, *J* = 4.3 Hz, 1H), 4.10 (d, *J* = 3.4 Hz, 1H), 3.96-3.90 (m, 1H), 3.85 (d, *J* = 13.5 Hz, 1H), 3.79 (s, 1H), 3.48 (s, 1H), 3.18-3.06 (m, 1H), 2.99-2.87 (m, 1H), 2.48-2.37 (m, 2H), 2.36-2.04 (m, 3H), 2.04-1.55 (m, 9H), 1.54-1.07 (m, 15H), 1.04-0.75 (m, 11H), 0.59 (s, 3H); LCMS (ESI) *m*/*z* calcd for C₃₁H₅₂NO₇S [M-Na]⁻ 582.3, found 582.3.

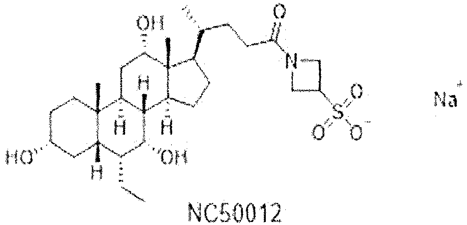

### NC50012

¹H NMR (400 MHz, DMSO-d₆) δ 4.30 (d, *J* = 4.3 Hz, 1H), 4.21-4.09 (m, 2H), 4.05-3.97 (m, 1H), 3.94 (d, J = 4.9 Hz, 1H), 3.85 (t, *J* = 9.0 Hz, 1H), 3.81-3.71 (m, 2H), 3.48 (s, 1H), 3.45-3.38 (m, 1H), 3.17-3.06 (m, 1H), 2.15-1.06 (m, 23H), 1.00-0.74 (m, 11H), 0.58 (s, 3H); LCMS (ESI) *m*/*z* calcd for C₂₉H₄₈NO₇S [M-Na]⁻ 554.3, found 554.3.

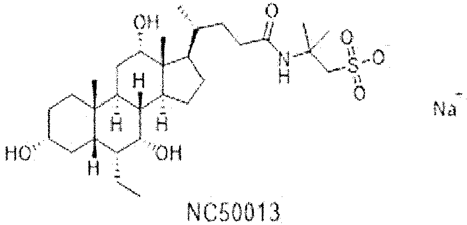

### NC50013

¹H NMR (500 MHz, CD₃OD) δ 3.97-3.93 (m, 1H), 3.66 (t, *J* = 2.7 Hz, 1H), 3.35-3.27 (m, 1H), 3.20-3.12 (m, 2H), 2.26-2.15 (m, 2H), 2.10-1.96 (m, 2H), 1.96-1.70 (m, 7H), 1.64-1.24 (m, 18H), 1.12 (qd, *J* = 12.0, 5.9 Hz, 1H), 1.05-0.95 (m, 4H), 0.91 (d, *J* = 6.7 Hz, 6H), 0.71 (s, 3H); LCMS (ESI) *m*/*z* calcd for C₃₀H₅₂NO₇S [M-Na]⁻ 570.3, found 570.3.

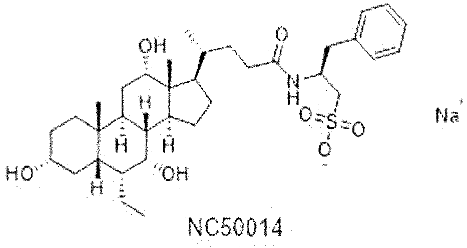

### NC50014

¹H NMR (500 MHz, CD₃OD) δ 7.29-7.22 (m, 4H), 7.21-7.14 (m, 1H), 4.53-4.44 (m, 1H), 3.96-3.91 (m, 1H), 3.66 (t, *J* = 2.8 Hz, 1H), 3.36-3.26 (m, 1H), 3.16 (dd, *J* = 13.7, 6.0 Hz, 1H), 3.04-2.95 (m, 2H), 2.88 (dd, *J* = 13.7, 8.1 Hz, 1H), 2.24-2.13 (m, 2H), 2.07-1.96 (m, 2H), 1.96-1.63 (m, 7H), 1.63-1.18 (m, 12H), 1.17-1.05 (m, 1H), 1.05-0.95 (m, 4H), 0.95-0.86 (m, 6H), 0.70 (s, 3H); LCMS (ESI) *m*/*z* calcd for C₃₅H₅₄NO₇S [M-Na]⁻ 632.4, found 632.4.

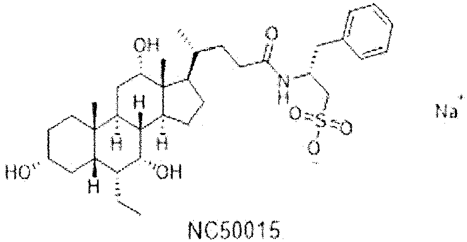

### NC50015

¹H NMR (400 MHz, DMSO-d₆) δ 7.70 (d, *J* = 7.5 Hz, 1H), 7.29-7.11 (m, 5H), 4.29 (d, *J* = 4.3 Hz, 1H), 4.17-4.06 (m, 2H), 3.93 (d, *J* = 4.9 Hz, 1H), 3.78 (s, 1H), 3.49 (s, 1H), 3.17-3.07 (m, 2H), 2.75 (dd, *J* = 13.3, 6.9 Hz, 1H), 2.53-2.51 (m, 2H), 2.15-1.05 (m, 23H), 1.00-0.75 (m, 11H), 0.57 (s, 3H); LCMS (ESI) *m*/*z* calcd for C₃₅H₅₄NO₇S [M-Na]⁻ 632.4, found 632.4.

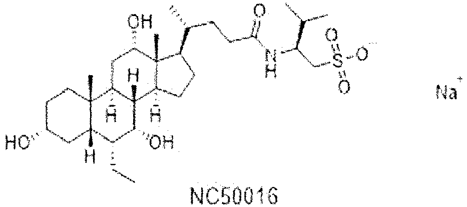

### NC50016

¹H NMR (500 MHz, CD₃OD) δ 4.24-4.17 (m, 1H), 3.98-3.93 (m, 1H), 3.66 (t, *J* = 2.7 Hz, 1H), 3.34-3.27 (m, 1H), 2.98-2.92 (m, 2H), 2.30 (ddd, *J* = 13.7, 10.5, 5.1 Hz, 1H), 2.24-1.97 (m, 4H), 1.97-1.70 (m, 7H), 1.64-1.26 (m, 12H), 1.12 (qd, *J* = 12.0, 5.8 Hz, 1H), 1.06-0.95 (m, 4H), 0.95-0.87 (m, 12H), 0.72 (s, 3H); LCMS (ESI) *m*/*z* calcd for C₃₁H₅₄NO₇S [M-Na]⁻ 584.4, found 584.4.

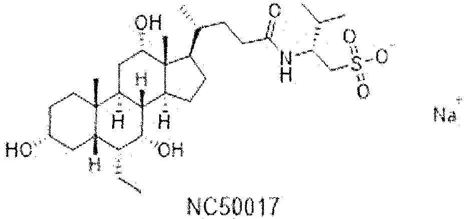

### NC50017

¹H NMR (500 MHz, CD₃OD) δ 4.27-4.20 (m, 1H), 3.98-3.93 (m, 1H), 3.66 (t, *J* = 2.8 Hz, 1H), 3.35-3.27 (m, 1H), 2.97-2.91 (m, 2H), 2.27 (ddd, *J* = 14.2, 9.6, 4.9 Hz, 1H), 2.23-2.11 (m, 2H), 2.08-1.96 (m, 2H), 1.96-1.69 (m, 7H), 1.65-1.24 (m, 12H), 1.17-1.06 (m, 1H), 1.06-0.96 (m, 4H), 0.95-0.85 (m, 12H), 0.70 (s, 3H); LCMS (ESI) *m*/*z* calcd for C₃₁H₅₄NO₇S [M-Na]⁻ 584.4, found 584.3.

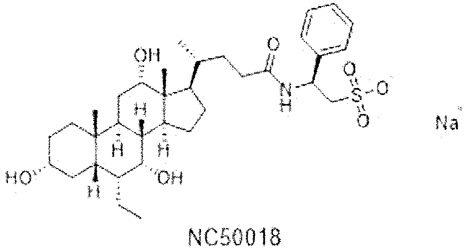

### NC50018

¹H NMR (500 MHz, CD₃OD) δ 7.37-7.27 (m, 4H), 7.24-7.18 (m, 1H), 5.34 (dd, *J* = 9.9, 3.8 Hz, 1H), 3.98-3.92 (m, 1H), 3.66 (d, *J* = 2.8 Hz, 1H), 3.34-3.29 (m, 1H), 3.29-3.23 (m, 1H), 3.10 (dd, *J* = 14.3, 3.8 Hz, 1H), 2.32 (ddd, *J* = 13.8, 10.1, 5.0 Hz, 1H), 2.23-2.12 (m, 2H), 2.05-1.95 (m, 1H), 1.95-1.68 (m, 7H), 1.63-1.20 (m, 12H), 1.15-1.05 (m, 1H), 1.05-0.96 (m, 4H), 0.94-0.85 (m, 6H), 0.68 (s, 3H). ; LCMS (ESI) *m*/*z* calcd for C₃₄H₅₂NO₇S [M-Na]⁻ 618.3, found 618.3.

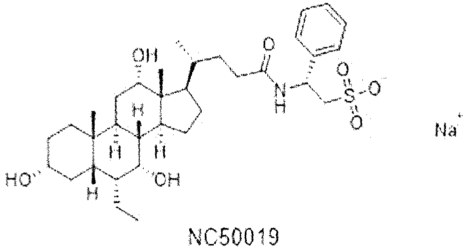

### NC50019

¹H NMR (400 MHz, DMSO-d₆) δ 8.16 (d, *J* = 6.3 Hz, 1H), 7.28-7.19 (m, 4H), 7.18-7.10 (m, 1H), 5.03-4.94 (m, 1H), 4.30 (d, *J* = 4.3 Hz, 1H), 4.11 (d, *J* = 3.5 Hz, 1H), 3.93 (d, *J* = 4.9 Hz, 1H), 3.79 (s, 1H), 3.48 (s, 1H), 3.17-3.06 (m, 1H), 2.85 (dd, *J* = 13.9, 9.2 Hz, 1H), 2.67 (dd, *J* = 13.8, 4.0 Hz, 1H), 2.15-1.91 (m, 4H), 1.90-1.56 (m, 6H), 1.54-1.03 (m, 13H), 0.99-0.75 (m, 11H), 0.56 (s, 3H); LCMS (ESI) *m*/*z* calcd for C₃₄H₅₂NO₇S [M-Na]⁻ 618.3, found 618.3.

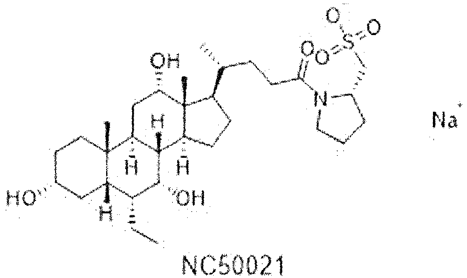

### NC50021

¹H NMR (500 MHz, CD₃OD) δ 4.52-4.45 (m, 0.5H), 4.45-4.38 (m, 0.5H), 3.99-3.93 (m, 1H), 3.66 (t, *J* = 2.8 Hz, 1H), 3.59-3.53 (m, 0.5H), 3.51-3.26 (m, 3H), 3.02-2.92 (m, 1H), 2.74 (dd, *J* = 13.3, 10.6 Hz, 0.5H), 2.50-2.39 (m, 2H), 2.35 (ddd, *J* = 15.3, 10.6, 5.1 Hz, 0.5H), 2.28-2.15 (m, 1.5H), 2.11-1.70 (m, 11H), 1.64-1.27 (m, 12H), 1.18-0.95 (m, 5H), 0.95-0.86 (m, 6H), 0.72 (s, 3H); LCMS (ESI) *m*/*z* calcd for C₃₁H₅₂NO₇S [M-Na]⁻ 582.3, found 582.3.

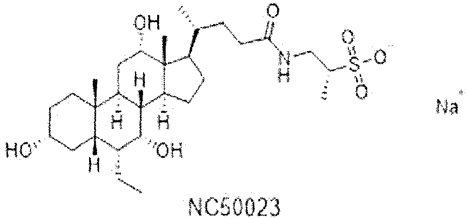

### NC50023

¹H NMR (500 MHz, CD₃OD) δ 3.97-3.93 (m, 1H), 3.66 (t, J = 2.7 Hz, 1H), 3.58 (dd, *J* = 13.7, 5.8 Hz, 1H), 3.42 (dd, *J* = 13.7, 7.2 Hz, 1H), 3.36-3.27 (m, 1H), 2.99-2.90 (m, 1H), 2.28 (ddd, J = 13.8, 10.2, 5.0 Hz, 1H), 2.23-2.08 (m, 2H), 2.01 (td, J = 12.1, 7.4 Hz, 1H), 1.96-1.69 (m, 7H), 1.64-1.47 (m, 6H), 1.47-1.24 (m, 9H), 1.12 (qd, J = 12.1, 5.8 Hz, 1H), 1.05-0.94 (m, 4H), 0.94-0.86 (m, 6H), 0.71 (s, 3H); LCMS (ESI) *m*/*z* calcd for C₂₉H₅₀NO₇S [M-Na]⁻ 556.3, found 556.2.

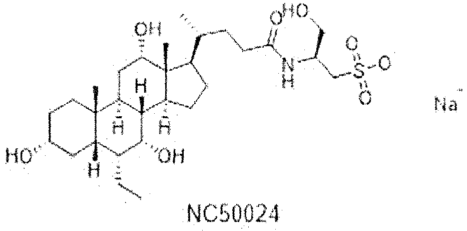

### NC50024

¹H NMR (500 MHz, CD₃OD) δ 4.34-4.26 (m, 1H), 3.95 (t, J = 3.0 Hz, 1H), 3.76-3.64 (m, 3H), 3.35-3.27 (m, 1H), 3.14-3.07 (m, 1H), 3.06-2.99 (m, 1H), 2.34-2.24 (m, 1H), 2.24-2.08 (m, 2H), 2.06-1.96 (m, 1H), 1.96-1.70 (m, 7H), 1.64-1.47 (m, 6H), 1.47-1.27 (m, 6H), 1.18-1.07 (m, 1H), 1.06-0.95 (m, 4H), 0.94-0.86 (m, 6H), 0.72 (d, J = 1.1 Hz, 3H); LCMS (ESI) *m*/*z* calcd for C₂₉H₅₀NO₈S [M-Na]⁻ 572.3, found 572.2.

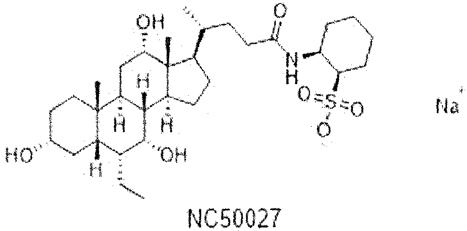

### NC50027

¹H NMR (500 MHz, CD₃OD) δ 4.33-4.27 (m, 1H), 3.99-3.93 (m, 1H), 3.69-3.64 (m, 1H), 3.34-3.27 (m, 1H), 2.91 (dt, *J* = 10.1, 4.1 Hz, 1H), 2.33 (ddd, J = 14.2, 10.2, 5.1 Hz, 1H), 2.27-2.09 (m, 3H), 2.06-1.70 (m, 11H), 1.64-1.27 (m, 16H), 1.18-1.06 (m, 1H), 1.06-0.94 (m, 4H), 0.94-0.86 (m, 6H), 0.72 (s, 3H); LCMS (ESI) *m*/*z* calcd for C₃₂H₅₄NO₇S [M-Na]⁻ 596.4, found 596.3.

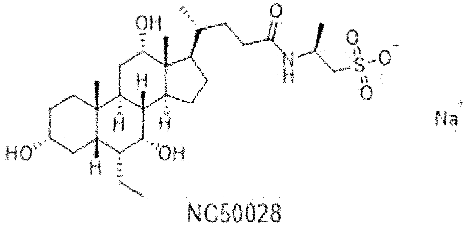

### NC50028

¹H NMR (400 MHz, DMSO-d₆) δ 7.68 (d, J = 7.5 Hz, 1H), 4.30 (d, J = 4.2 Hz, 1H), 4.10 (d, *J* = 3.4 Hz, 1H), 4.04-3.91 (m, 2H), 3.78 (s, 1H), 3.48 (s, 1H), 3.18-3.06 (m, 1H), 2.60 (dd, J = 13.4, 4.6 Hz, 1H), 2.45 (dd, J = 13.4, 8.0 Hz, 1H), 2.14-1.06 (m, 26H), 1.02-0.74 (m, 11H), 0.58 (s, 3H); LCMS (ESI) *m*/*z* calcd for C₂₉H₅₀NO₇S [M-Na]⁻ 556.3, found 556.2.

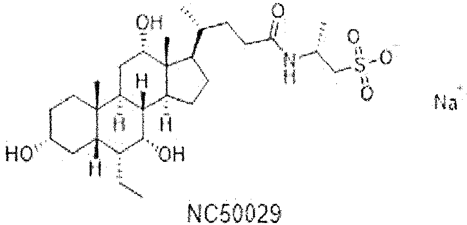

### NC50029

¹H NMR (500 MHz, CD₃OD) δ 4.37-4.27 (m, 1H), 3.97-3.93 (m, 1H), 3.66 (t, *J* = 2.8 Hz, 1H), 3.34-3.27 (m, 1H), 3.05 (dd, *J* = 13.9, 5.5 Hz, 1H), 2.88 (dd, *J* = 13.9, 7.0 Hz, 1H), 2.29-2.15 (m, 2H), 2.13-1.97 (m, 2H), 1.97-1.69 (m, 7H), 1.64-1.47 (m, 6H), 1.47-1.26 (m, 9H), 1.12 (qd, *J* = 11.9, 5.7 Hz, 1H), 1.05-0.94 (m, 4H), 0.93-0.87 (m, 6H), 0.71 (s, 3H); LCMS (ESI) *m*/*z* calcd for C₂₉H₅₀NO₇S [M-Na]⁻ 556.3, found 556.2.

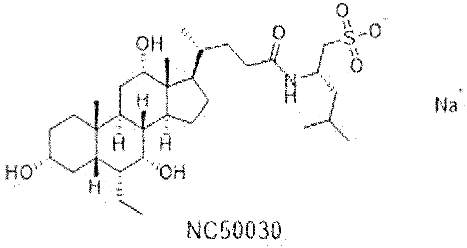

### NC50030

¹H NMR (500 MHz, CD₃OD) δ 4.39-4.30 (m, 1H), 3.99-3.92 (m, 1H), 3. 66 (t, *J* = 2.6 Hz, 1H), 3.35-3.26 (m, 1H), 3.03 (dd, *J* = 14.0, 5.5 Hz, 1H), 2.90 (dd, *J* = 14.0, 6.4 Hz, 1H), 2.30-2.15 (m, 2H), 2.15-2.06 (m, 1H), 2.06-1.97 (m, 1H), 1.97-1.25 (m, 22H), 1.18-1.06 (m, 1H), 1.05-0.85 (m, 16H), 0.72 (s, 3H); LCMS (ESI) *m*/*z* calcd for C₃₂H₅₆NO₇S [M-Na]⁻ 598.4, found 598.4.

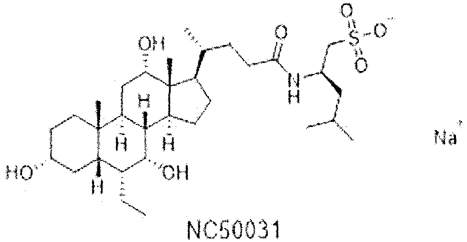

### NC50031

¹H NMR (400 MHz, DMSO-d₆) δ 7.55 (d, *J* = 8.6 Hz, 1H), 4.30 (d, *J* = 4.3 Hz, 1H), 4.13-4.02 (m, 2H), 3.94 (d, *J* = 4.9 Hz, 1H), 3.78 (s, 1H), 3.48 (s, 1H), 3.17-3.06 (m, 1H), 2.59 (dd, *J* = 13.5, 4.5 Hz, 1H), 2.49-2.41 (m, 1H), 2.14-1.86 (m, 4H), 1.87-1.05 (m, 22H), 1.03-0.72 (m, 17H), 0.57 (s, 3H); LCMS (ESI) *m*/*z* calcd for C₃₂H₅₆NO₇S [M-Na]⁻ 598.4, found 598.4.

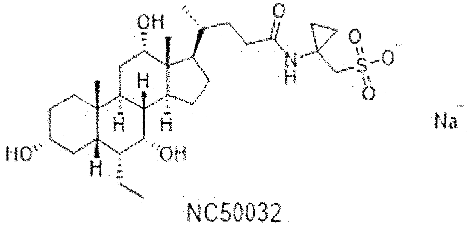

### NC50032

¹H NMR (500 MHz, CD₃OD) δ 3.96-3.92 (m, 1H), 3.66 (t, *J* = 2.7 Hz, 1H), 3.34-3.25 (m, 1H), 3.03 (s, 2H), 2.27-2.14 (m, 2H), 2.11-1.96 (m, 2H), 1.96-1.70 (m, 7H), 1.64-1.24 (m, 12H), 1.11 (qd, *J* = 12.0, 5.8 Hz, 1H), 1.05-0.86 (m, 12H), 0.81-0.76 (m, 2H), 0.71 (s, 3H); LCMS (ESI) MIZ calcd for C₃₀H₅₀NO₇S [M-Na]⁻ 568.3, found 568.3.

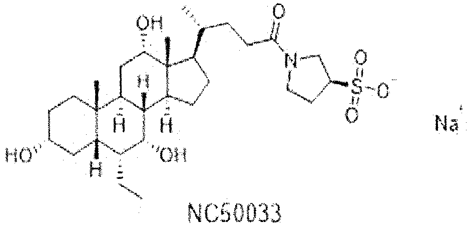

### NC50033

¹H NMR (500 MHz, CD₃OD) δ 4.00-3.91 (m, 1H), 3.89-3.40 (m, 6H), 3.36-3.25 (m, 1H), 2.47-2.15 (m, 5H), 2.07-1.97 (m, 1H), 1.97-1.70 (m, 7H), 1.64-1.27 (m, 12H), 1.13 (qd, *J* = 11.9, 5.3 Hz, 1H), 1.07-0.94 (m, 4H), 0.94-0.86 (m, 6H), 0.73 (s, 1.5H), 0.72 (s, 1.5H); LCMS (ESI) *m*/*z* calcd for C₃₀H₅₀NO₇S [M-Na]⁻ 568.3, found 568.3.

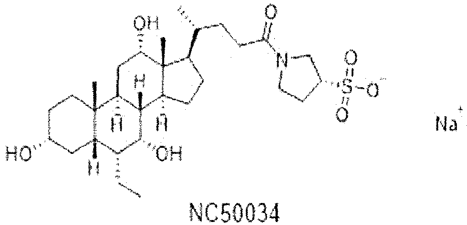

### NC50034

¹H NMR (500 MHz, CD₃OD) δ 3.95 (d, *J* = 2.3 Hz, 1H), 3.88-3.40 (m, 6H), 3.34-3.26 (m, 1H), 2.47-2.15 (m, 5H), 2.08-1.97 (m, 1H), 1.97-1.70 (m, 7H), 1.64-1.26 (m, 12H), 1.13 (qd, *J* = 11.7, 5.2 Hz, 1H), 1.07-0.93 (m, 4H), 0.93-0.86 (m, 6H), 0.73 (s, 1.5H), 0.72 (s, 1.5H); LCMS (ESI) *m*/*z* calcd for C₃₀H₅₀NO₇S [M-Na]⁻ 568.3, found 568.3.

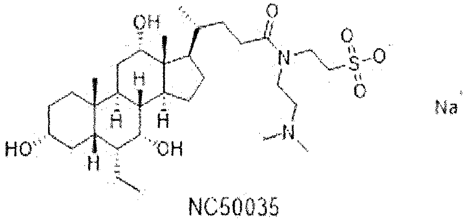

### NC50035

¹H NMR (500 MHz, CD₃OD) δ 3.98-3.93 (m, 1H), 3.81-3.75 (m, 1H), 3.73-3.68 (m, 1H), 3.66 (t, *J* = 2.8 Hz, 1H), 3.59-3.45 (m, 2H), 3.35-3.27 (m, 1H), 3.11-3.02 (m, 2H), 2.58-2.32 (m, 4H), 2.31 (s, 3H), 2.28 (s, 3H), 2.20 (td, *J* = 12.3, 5.2 Hz, 1H), 2.02 (td, *J* = 12.1, 7.4 Hz, 1H), 1.97-1.70 (m, 7H), 1.64-1.27 (m, 12H), 1.18-0.96 (m, 5H), 0.94-0.87 (m, 6H), 0.72 (s, 3H); LCMS (ESI) *m*/*z* calcd for C₃₂H₅₇N₂O₇S [M-Na]⁻ 613.4, found 613.4.

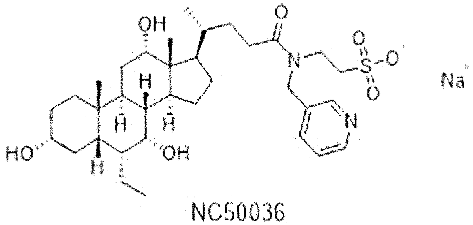

### NC50036

¹H NMR (400 MHz, DMSO-d₆) δ8.51-8.40 (m, 2H), 7.62-7.55 (m, 1H), 7.43-7.38 (m, 0.4H), 7.36-7.31 (m, 0.6H), 4.66 (s, 0.4H), 4.59-4.46 (m, 1.6H), 4.29 (d, *J* = 4.3 Hz, 1H), 4.15-4.05 (m, 1H), 3.97-3.91 (m, 1H), 3.83-3.72 (m, 1H), 3.56-3.38 (m, 3H), 3.18-3.06 (m, 1H), 2.68-2.54 (m, 2H), 2.46-2.37 (m, 1H), 2.37-2.24 (m, 1H), 2.14-1.91 (m, 2H), 1.90-1.57 (m, 6H), 1.56-1.08 (m, 13H), 1.03-0.74 (m, 11H), 0.59 (s, 1.8H), 0.56 (s, 1.2H); LCMS (ESI) *m*/*z* calcd for C₃₄H₅₃N₂O₇S [M-Na]⁻ 633.4, found 633.4.

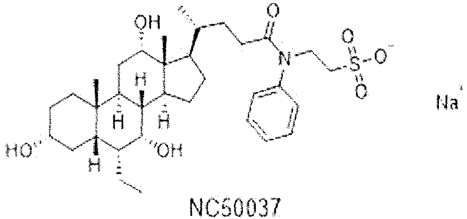

### NC50037

¹H NMR (500 MHz, CD₃OD) δ 7.52-7.46 (m, 2H), 7.45-7.38 (m, 1H), 7.35-7.29 (m, 2H), 4.13-4.06 (m, 2H), 3.85 (s, 1H), 3.64 (s, 1H), 3.34-3.25 (m, 1H), 3.09-3.03 (m, 2H), 2.20-2.10 (m, 2H), 2.04-1.65 (m, 9H), 1.60-1.34 (m, 8H), 1.33-1.14 (m, 4H), 1.13-0.84 (m, 8H), 0.77 (d, *J* = 6.1 Hz, 3H), 0.64 (s, 3H); LCMS (ESI) *m*/*z* calcd for C₃₄H₅₂NO₇S [M-Na]⁻ 618.3, found 618.4.

### <Experimental Example 1> Evaluation 1 of the inflammation-inhibiting efficacy of taurine-bile acid derivatives

To evaluate anti-inflammatory efficacy, an inflammatory response was induced in J774A.1 (Mouse BALB/c monocyte macrophage cell line) cells derived from mouse macrophages by LPS (lipopolysaccharide) and LPS+ATP (adenosine triphosphate), and the efficacy of taurine-bile acid derivatives to inhibit the production of inflammatory cytokines (IL-1β, TNF-α) was measured. After treating J774A.1 cells with 1 µM of a taurine-bile acid derivative, the degree to which the secretion of TNF-α among cytokines was suppressed was measured in the experimental group in which an inflammatory response was induced by LPS, and the extent to which IL-1β secretion among cytokines was suppressed was measured in the experimental group in which an inflammatory response was induced with LPS+ATP. The ability of taurine-bile acid derivatives to inhibit cytokine production was graded compared to taurodeoxycholic acid, a positive control. The grading criteria are A: over 100%, B: over 50% and under 100%, C: between 20% and under 50%, and D: under 20%. The measurement results are as shown in Table 1 below.

**Table 1**

| | Grade of inhibition of inflammatory cytokine production | |
|---|---|---|
| compound code | TNF-α production inhibition grade | IL-1β production inhibition grade |
| NC10001 | D | A |
| NC10002 | A | A |
| NC10003 | A | A |
| NC10004 | C | B |
| NC10005 | D | C |
| NC10006 | D | B |
| NC10007 | B | B |
| NC10008 | A | A |
| NC10009 | A | A |
| NC10010 | A | A |
| NC10011 | A | A |
| NC10012 | A | A |
| NC10013 | D | B |
| NC10014 | D | D |
| NC10015 | D | D |
| NC10016 | A | A |
| NC10017 | A | A |
| NC10018 | B | C |
| NC10019 | D | C |
| NC10021 | - | - |
| NC10022 | C | B |
| NC10024 | D | A |
| NC10025 | - | - |
| NC10026 | D | A |
| NC10027 | D | A |
| NC10028 | D | A |
| NC10029 | D | A |
| NC10031 | D | D |
| NC10032 | D | B |
| NC10033 | D | B |
| NC10034 | - | - |
| NC10035 | D | B |
| NC10036 | D | A |
| NC10037 | D | B |
| NC10038 | D | B |
| NC10039 | C | B |
| NC10040 | D | D |
| NC10041 | D | D |
| NC10042 | D | D |
| NC10043 | D | A |
| NC10045 | D | B |
| NC10046 | D | B |
| NC10047 | D | C |
| NC10048 | D | B |
| NC10049 | D | A |
| NC10050 | D | A |
| NC10051 | D | B |
| NC10052 | B | B |
| NC10053 | B | B |
| NC10054 | D | B |

| -continued | Grade of inhibition of inflammatory cytokine production | |
|---|---|---|
| compound code | TNF-α production inhibition grade | IL-1β production inhibition grade |
| NC10055 | D | B |
| NC10056 | C | B |
| NC10058 | D | A |
| NC10059 | D | A |
| NC10060 | D | B |
| NC10061 | D | A |
| NC10062 | D | A |
| NC10063 | D | A |
| NC10064 | D | D |
| NC10065 | D | A |
| NC10066 | D | A |
| NC10067 | D | D |
| NC10068 | D | A |
| NC10069 | D | A |
| NC10070 | B | A |
| NC10071 | D | A |
| NC10072 | D | C |
| NC10073 | D | B |
| NC10074 | D | A |
| NC10075 | D | B |
| NC10076 | D | A |
| NC10077 | D | A |
| NC10078 | D | A |
| NC10079 | C | A |
| NC10080 | D | A |
| NC10081 | A | A |
| NC10082 | A | A |
| NC10083 | A | A |
| NC10084 | D | D |
| NC10085 | D | D |
| NC10086 | D | D |
| NC10087 | A | A |
| NC10088 | C | D |
| NC10089 | D | C |
| NC10090 | D | D |
| NC10091 | A | A |
| NC10092 | - | - |
| NC10093 | - | - |
| NC20002 | B | B |
| NC20003 | B | C |
| NC20004 | D | A |
| NC20005 | D | A |
| NC20006 | D | A |
| NC20007 | D | A |
| NC20008 | D | A |
| NC20009 | D | D |
| NC20010 | D | B |
| NC20011 | D | B |
| NC20012 | D | A |

| -continued | Grade of inhibition of inflammatory cytokine production | |
|---|---|---|
| compound code | TNF-α production inhibition grade | IL-1β production inhibition grade |
| NC20013 | D | A |
| NC20014 | D | A |
| NC20015 | D | A |
| NC20016 | D | A |
| NC20017 | D | B |
| NC20018 | D | D |
| NC20019 | D | D |
| NC20020 | B | C |
| NC20021 | D | D |
| NC20022 | D | D |
| NC20023 | D | D |
| NC20024 | D | C |
| NC20025 | D | D |
| NC20026 | D | B |
| NC20027 | D | A |
| NC20028 | D | D |
| NC20029 | D | D |
| NC20030 | D | D |
| NC20031 | D | B |
| NC20032 | D | B |
| NC20033 | D | C |
| NC20034 | D | D |
| NC20035 | D | D |
| NC20036 | D | D |
| NC20037 | D | B |
| NC30002 | D | B |
| NC30004 | C | C |
| NC30005 | - | - |
| NC30006 | D | D |
| NC30007 | - | - |
| NC30008 | D | B |
| NC30009 | B | C |
| NC30010 | A | B |
| NC30011 | D | D |
| NC30012 | D | C |
| NC30013 | B | B |
| NC30014 | D | C |
| NC30015 | D | D |
| NC30016 | C | C |
| NC30017 | D | D |
| NC30018 | D | D |
| NC30019 | D | D |
| NC30020 | D | C |
| NC30021 | - | - |
| NC30022 | C | C |
| NC30023 | A | B |
| NC30024 | - | - |
| NC30025 | A | D |
| NC30026 | - | - |
| NC30027 | A | A |

| -continued | Grade of inhibition of inflammatory cytokine production | |
|---|---|---|
| compound code | TNF-α production inhibition grade | IL-1β production inhibition grade |
| NC30028 | D | D |
| NC30029 | D | C |
| NC30030 | D | C |
| NC30031 | D | C |
| NC30032 | - | - |
| NC30033 | D | C |
| NC30034 | D | D |
| NC30035 | A | A |
| NC30036 | - | - |
| NC30037 | C | A |
| NC40002 | D | C |
| NC40003 | - | - |
| NC40004 | D | B |
| NC40005 | - | - |
| NC40006 | C | B |
| NC40007 | D | B |
| NC40008 | A | A |
| NC40009 | D | D |
| NC40010 | B | D |
| NC40011 | D | C |
| NC40012 | D | B |
| NC40013 | D | A |
| NC40014 | D | A |
| NC40015 | D | A |
| NC40016 | - | - |
| NC40017 | D | C |
| NC40018 | D | A |
| NC40019 | D | A |
| NC40020 | D | A |
| NC40021 | D | B |
| NC40022 | C | C |
| NC40023 | D | D |
| NC40024 | D | D |
| NC40025 | C | C |
| NC40026 | - | - |
| NC40027 | D | B |
| NC40028 | D | B |
| NC40029 | D | D |
| NC40030 | D | D |
| NC40031 | D | D |
| NC40032 | D | D |
| NC40033 | A | A |
| NC40034 | C | A |
| NC40035 | D | A |
| NC40036 | D | B |
| NC40037 | D | C |
| NC50002 | - | - |
| NC50004 | D | D |
| NC50005 | - | - |
| NC50006 | D | D |
| NC50007 | D | A |

| -continued | Grade of inhibition of inflammatory cytokine production | |
|---|---|---|
| compound code | TNF-α production inhibition grade | IL-1β production inhibition grade |
| NC50008 | D | A |
| NC50009 | - | - |
| NC50010 | D | A |
| NC50011 | - | - |
| NC50012 | D | A |
| NC50013 | D | A |
| NC50014 | - | - |
| NC50015 | D | A |
| NC50016 | D | A |
| NC50017 | - | - |
| NC50018 | D | B |
| NC50019 | D | B |
| NC50021 | D | C |
| NC50023 | - | - |
| NC50024 | D | C |
| NC50027 | D | C |
| NC50028 | D | B |
| NC50029 | D | B |
| NC50030 | D | B |
| NC50031 | D | B |
| NC50032 | D | B |

### <Experimental Example 2> Evaluation 2 of the inflammation-inhibiting efficacy of taurine-bile acid derivatives

To evaluate anti-inflammatory efficacy, U937 cells (human monocytic cell line) were differentiated into macrophages using PMA (phorbol 12-myristate 13-acetate) and then induced an inflammatory response with Pam2CSK4 (Pam2CysSerLys4) or BzATP(2'(3')-O-(4-Benzoylbenzoyl)adenosine 5'-triphosphate), the efficacy of taurine-bile acid derivatives in suppressing inflammatory cytokine (IL-1β, TNF-α) production was measured.

Using PMA, U937 cells were differentiated into macrophages, and the extent of TNF-α secretion inhibition was measured in an experimental group where taurine-bile acid derivatives was treated, followed by induction of inflammatory response with Pam2CSK4, and in the experimental group in which an inflammatory response was induced with BzATP, the extent to which IL-1β secretion among cytokines was suppressed was measured.

The ability of taurine-bile acid derivatives to inhibit cytokine production was expressed in terms of IC₅₀ (µM) values. The standards for TNF α inhibition ability are A: less than 0.5, B: 0.5 to less than 1.0, C: 1.0 to less than 2.0, D: 2.0 or more, and the IL-1β inhibition ability grade standards are A: less than 0.05, B: 0.05 to less than 0.10, C: 0.10 or more and less than 0.20, D: 0.20 or more. The measurement results are as shown in Table 2 below.

**Table 2**

| | Grade of inhibition of inflammatory cytokine production | |
|---|---|---|
| compound code | TNF-α production inhibition grade | IL-1β production inhibition grade |
| NC10001 | D | A |
| NC10002 | A | A |
| NC10003 | A | A |
| NC10004 | D | D |
| NC10005 | C | C |
| NC10006 | B | D |
| NC10007 | D | A |
| NC10008 | A | A |
| NC10009 | A | A |
| NC10010 | A | A |
| NC10011 | A | A |
| NC10012 | A | A |
| NC10013 | B | B |
| NC10014 | C | A |
| NC10015 | D | A |
| NC10016 | A | A |
| NC10017 | B | A |
| NC10018 | D | B |
| NC10019 | B | D |
| NC10021 | C | B |
| NC10022 | B | C |
| NC10024 | C | B |
| NC10025 | C | B |
| NC10026 | C | A |
| NC10027 | B | A |
| NC10028 | D | B |
| NC10029 | D | B |
| NC10031 | D | B |
| NC10032 | D | B |
| NC10033 | B | C |
| NC10034 | B | B |
| NC10035 | B | D |
| NC10036 | D | C |
| NC10037 | A | D |
| NC10038 | D | B |
| NC10039 | B | B |
| NC10040 | C | D |
| NC10041 | B | D |
| NC10042 | B | D |
| NC10043 | A | C |
| NC10045 | A | B |
| NC10046 | A | C |
| NC10047 | B | C |
| NC10048 | A | B |
| NC10049 | A | A |
| NC10050 | A | B |
| NC10051 | C | B |
| NC10052 | C | B |
| NC10053 | C | B |
| NC10054 | C | B |
| NC10055 | C | A |

| - continued | Grade of inhibition of inflammatory cytokine production | |
|---|---|---|
| compound code | TNF-α production inhibition grade | IL-1β production inhibition grade |
| NC10056 | D | B |
| NC10058 | D | A |
| NC10059 | C | A |
| NC10060 | D | D |
| NC10061 | B | C |
| NC10062 | B | A |
| NC10063 | B | A |
| NC10064 | A | A |
| NC10065 | C | C |
| NC10066 | C | A |
| NC10067 | C | D |
| NC10068 | A | A |
| NC10069 | A | A |
| NC10070 | A | A |
| NC10071 | A | A |
| NC10072 | A | A |
| NC10073 | A | B |
| NC10074 | A | B |
| NC10075 | A | C |
| NC10076 | D | B |
| NC10077 | A | A |
| NC10078 | A | A |
| NC10079 | B | A |
| NC10080 | B | C |
| NC10081 | B | A |
| NC10082 | A | A |
| NC10083 | B | A |
| NC10084 | A | D |
| NC10085 | D | D |
| NC10086 | A | D |
| NC10087 | B | A |
| NC10088 | A | D |
| NC10089 | A | D |
| NC10090 | B | D |
| NC10091 | B | A |
| NC10092 | D | B |
| NC10093 | D | C |
| NC20002 | D | C |
| NC20003 | - | B |
| NC20004 | B | A |
| NC20005 | B | B |
| NC20006 | C | A |
| NC20007 | C | A |
| NC20008 | B | B |
| NC20009 | D | A |
| NC20010 | C | A |
| NC20011 | D | B |
| NC20012 | B | A |
| NC20013 | C | A |
| NC20014 | B | A |
| NC20015 | A | A |

| - continued | Grade of inhibition of inflammatory cytokine production | |
|---|---|---|
| compound code | TNF-α production inhibition grade | IL-1β production inhibition grade |
| NC20016 | C | A |
| NC20017 | A | D |
| NC20018 | C | D |
| NC20019 | C | B |
| NC20020 | D | A |
| NC20021 | C | B |
| NC20022 | C | A |
| NC20023 | D | B |
| NC20024 | A | D |
| NC20025 | D | D |
| NC20026 | C | C |
| NC20027 | D | A |
| NC20028 | C | D |
| NC20029 | A | B |
| NC20030 | A | D |
| NC20031 | A | B |
| NC20032 | D | A |
| NC20033 | C | B |
| NC20034 | C | B |
| NC20035 | C | B |
| NC20036 | B | B |
| NC20037 | A | A |
| NC30002 | C | C |
| NC30004 | C | D |
| NC30005 | B | D |
| NC30006 | B | D |
| NC30007 | A | D |
| NC30008 | B | D |
| NC30009 | B | D |
| NC30010 | B | A |
| NC30011 | B | D |
| NC30012 | D | D |
| NC30013 | B | D |
| NC30014 | B | D |
| NC30015 | A | D |
| NC30016 | A | D |
| NC30017 | D | D |
| NC30018 | D | D |
| NC30019 | C | D |
| NC30020 | D | D |
| NC30021 | B | B |
| NC30022 | D | D |
| NC30023 | D | D |
| NC30024 | C | D |
| NC30025 | C | D |
| NC30026 | A | D |
| NC30027 | B | A |
| NC30028 | B | D |
| NC30029 | B | D |
| NC30030 | B | D |

| - continued | Grade of inhibition of inflammatory cytokine production | |
|---|---|---|
| compound code | TNF-α production inhibition grade | IL-1β production inhibition grade |
| NC30031 | B | D |
| NC30032 | B | D |
| NC30033 | B | B |
| NC30034 | B | D |
| NC30035 | B | A |
| NC30036 | C | D |
| NC30037 | D | A |
| NC40002 | D | D |
| NC40003 | D | D |
| NC40004 | D | D |
| NC40005 | D | D |
| NC40006 | C | D |
| NC40007 | C | D |
| NC40008 | A | A |
| NC40009 | C | C |
| NC40010 | B | D |
| NC40011 | B | D |
| NC40012 | B | D |
| NC40013 | C | A |
| NC40014 | B | A |
| NC40015 | B | B |
| NC40016 | B | D |
| NC40017 | B | D |
| NC40018 | A | A |
| NC40019 | C | C |
| NC40020 | C | C |
| NC40021 | B | D |
| NC40022 | B | D |
| NC40023 | B | D |
| NC40024 | B | D |
| NC40025 | B | B |
| NC40026 | B | B |
| NC40027 | D | D |
| NC40028 | C | C |
| NC40029 | C | D |
| NC40030 | C | D |
| NC40031 | C | D |
| NC40032 | C | D |
| NC40033 | A | A |
| NC40034 | A | A |
| NC40035 | A | B |
| NC40036 | C | B |
| NC40037 | B | B |
| NC50002 | D | D |
| NC50004 | D | C |
| NC50005 | C | D |
| NC50006 | D | A |
| NC50007 | C | B |
| NC50008 | B | A |
| NC50009 | C | C |

| - continued | Grade of inhibition of inflammatory cytokine production | |
|---|---|---|
| compound code | TNF-aproduction inhibition grade | IL-1β production inhibition grade |
| NC50010 | D | C |
| NC50011 | D | D |
| NC50012 | C | A |
| NC50013 | C | A |
| NC50014 | B | A |
| NC50015 | C | B |
| NC50016 | B | B |
| NC50017 | C | B |
| NC50018 | D | B |
| NC50019 | C | D |
| NC50021 | B | D |
| NC50023 | D | D |
| NC50024 | D | D |
| NC50027 | C | D |
| NC50028 | C | D |
| NC50029 | B | D |
| NC50030 | D | C |
| NC50031 | D | B |
| NC50032 | D | B |

### Industrial Applicability

A new compound containing the taurine derivative of the present invention can be prepared, and it can be developed into a useful therapeutic agent, so it can be used in the pharmaceutical industry.

## Claims

1. A compound represented by formula 1 or a pharmaceutically acceptable salt thereof: 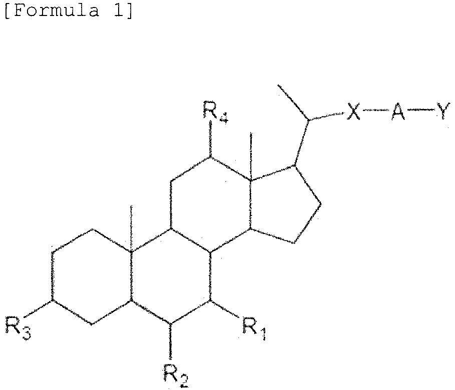 wherein,
R₁, R₂, R₃ and R₄ are each independently H, halo, C₁₋₅ alkyl, OH, or =O,
X is substituted or unsubstituted C₁₋₁₀ alkylene,
wherein when X is substituted, one or more hydrogens are substituted with linear or branched C₁₋₁₀ alkyl, halo, OH, SH, NH₂, CONH₂, or COOH,
A is C₁₋₁₀ alkylene, C₁₋₁₀ heteroalkylene, C₁₋₁₀ alkenylene, C₁₋₅ alkynylene, C₃₋₈ cycloalkylene, C₃₋₈ heterocycloalkylene, C₆₋₂₀ arylene, C₆₋₂₀ heteroarylene, -NR₅R₆C(O)-, -OR₆, -SR₆, -NR₅R₆, -C(O)R₆, -C(O)OR₆, -C(O)NR₅R₆, -CH(R₅)(R₆), -CH(R₅)(OR₆), -CH(OR₅)(OR₆), -CH(R₅)(SR₆), or -CH(SR₅)(SR₆);
R₅ is H, substituted or unsubstituted C₁₋₁₀ alkyl, substituted or unsubstituted C₁₋₁₀ heteroalkyl, substituted or unsubstituted C₃₋₂₀ cycloalkyl, substituted or unsubstituted C₃₋₂₀ heterocycloalkyl, substituted or unsubstituted C₆₋₂₀ aryl, substituted or unsubstituted C₆₋₂₀ heteroaryl, substituted or unsubstituted C₁₋₁₀ alkyl-C₆₋₂₀ cycloalkyl, substituted or unsubstituted C₁₋₁₀ alkyl-C₆₋₂₀ heterocycloalkyl, substituted or unsubstituted C₁₋₁₀alkyl-C₆₋₂₀ aryl or substituted or unsubstituted C₁₋₁₀alkyl-C₆₋₂₀ heteroaryl,
wherein if R₅ is substituted, one or more hydrogen(s) is(are) substituted with linear or branched C₁₋₅ alkyl, =O, ORₐ, SRₐ, N(Rₐ)₂, or CON(Rₐ)₂, and Rₐ is H or C₁₋₅ alkyl,
R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, substituted or unsubstituted C₆₋₂₀ arylene, or substituted or unsubstituted C₆₋₂₀ heteroarylene,
wherein if R₆ is substituted, one or more hydrogen(s) is(are) substituted with =O, linear or branched C₁₋₁₀ alkyl, C₀₋₁₀alkyl-C₆₋₂₀aryl, C₀₋₁₀alkyl-OH, C₀₋₁₀alkyl-NH₂, C₀₋₁₀alkyl-SH, or C₀₋₁₀alkyl-CONH₂; or R₅ and R₆ together form C₃₋₂₀ cycloalkylene, C₃₋₂₀ heterocycloalkylene, C₆₋₂₀ arylene or C₆₋₂₀ heteroarylene;
Y combines directly with A or with R₆, and is H, -(CH₂)ₙCH₃, -(CH₂)ₙCOOH, -(CH₂)ₙOH -(CH₂)ₙSO₃H, -(CH₂)ₙOSO₃H, -(CH₂)ₙPO₃H₂, -(CH₂)ₙOPO₃H₂, -(CH₂)ₙSO₃NH₂, -(CH₂)ₙCONH₂, or -(CH₂)ₙC(O)SO₃H,
wherein n is an integer from 0 to 10.

2. The compound as in claim 1, wherein R₁, R₂, R₃ and R₄ are each independently H, OH, =O or C₁₋₅ alkyl; or a pharmaceutically acceptable salt thereof.

3. The compound as in claim 1, wherein R₁, R₂, R₃ and R₄ are each independently H, or OH, and wherein contains at least one or more OH; or a pharmaceutically acceptable salt thereof.

4. The compound as in claim 1, wherein R₁, R₂, R₃ and R₄ are each independently H, OH, =O or C₁₋₅ alkyl, and contains at least one or more =O or C₁₋₅ alkyl; or a pharmaceutically acceptable salt thereof.

5. The compound as in claim 1, wherein R₁, R₂, R₃ and R₄ are each independently H, or OH; or a pharmaceutically acceptable salt thereof.

6. The compound as in claim 1, wherein R₃ is OH; or a pharmaceutically acceptable salt thereof.

7. The compound as in claim 1, wherein R₁ and R₃ are OH; or a pharmaceutically acceptable salt thereof.

8. The compound as in claim 1, wherein R₃ is OH; or a pharmaceutically acceptable salt thereof.

9. The compound as in claim 1, wherein R₁ and R₃ are OH, and R₂ is C₂ alkyl; or a pharmaceutically acceptable salt thereof.

10. The compound as in claim 1, wherein R₁, R₃ and R₄ are OH, R₂ is C₂ alkyl; or a pharmaceutically acceptable salt thereof.

11. The compound as in claim 1, wherein X is substituted or unsubstituted C₁₋₄ alkylene, and if substituted, one or more hydrogen(s) is(are) substituted with linear or branched C₁₋₄ alkyl; or a pharmaceutically acceptable salt thereof.

12. The compound as in claim 1, wherein X is unsubstituted C₁₋₄ alkylene; or a pharmaceutically acceptable salt thereof.

13. The compound as in claim 1, wherein A is -NR₅R₆C(O)-, -OR₆, -SR₆, -NR₅R₆, -C(O)R₆, -C(O)OR₆, -C(O)NR₅R₆, -CH(R₅)(R₆), -CH(R₅)(OR₆), -CH(OR₅)(OR₆), - CH(R₅)(SR₆), or -CH(SR₅)(SR₆), and wherein R₅, R₆ are **characterized in that** they are the same as the definition in claim 1; or a pharmaceutically acceptable salt thereof.

14. The compound as in claim 1, wherein A is -NR₅R₆C(O)-, -C(O)NR₅R₆, or - CH(R₅)(R₆) and wherein R₅, R₆ are **characterized in that** they are the same as the definition in claim 1; or a pharmaceutically acceptable salt thereof.

15. The compound as in claim 14, wherein R₅ is H, substituted or unsubstituted C₁₋₁₀ alkyl, substituted or unsubstituted C₁₋₁₀ heteroalkyl, substituted or unsubstituted C₃₋₂₀ cycloalkyl, substituted or unsubstituted C₃₋₂₀ heterocycloalkyl, substituted or unsubstituted C₆₋₂₀ aryl, substituted or unsubstituted C₁₋₁₀alkyl-C₆₋₂₀ aryl or substituted or unsubstituted C₁₋₁₀alkyl-C₆₋₂₀ heteroaryl, and wherein if R₅ is substituted, one or more hydrogen(s) is (are) substituted with linear or branched C₁₋₅ alkyl, =O, ORₐ, SRₐ, N(Rₐ)₂, or CON(Rₐ)₂ and Rₐ is H or C₁₋₅ alkyl; or a pharmaceutically acceptable salt thereof.

16. The compound as in claim 14, wherein R₅ is H, or substituted or unsubstituted C₁₋₁₀ alkyl, and wherein if R₅ is substituted, one or more hydrogen(s) is (are) substituted with =O, OH, SH, NH₂, or CONH₂; or a pharmaceutically acceptable salt thereof.

17. The compound as in claim 14, wherein R₅ is substituted or unsubstituted C₃₋₂₀ cycloalkyl, substituted or unsubstituted C₃₋₂₀ heterocycloalkyl or substituted or unsubstituted C₆₋₂₀ aryl, and wherein if R₅ is substituted, one or more hydrogen(s) is(are) substituted with =O, OH, SH, NH₂, or CONH₂; or a pharmaceutically acceptable salt thereof.

18. The compound as in claim 14, wherein R₅ is unsubstituted C₃₋₂₀ C₃₋₁₀ cycloalkyl, unsubstituted C₃₋₁₀ heterocycloalkyl or unsubstituted C₆₋₁₀ aryl; or a pharmaceutically acceptable salt thereof.

19. The compound as in claim 14, wherein R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, or substituted or unsubstituted C₆₋₂₀ arylene, and wherein if R₆ is substituted, one or more hydrogen (s) is (are) substituted with =O, linear or branched C₁₋₁₀ alkyl, C₀₋₁₀alkyl-C₆₋₂₀aryl or C₀₋₁₀alkyl-OH; or a pharmaceutically acceptable salt thereof.

20. The compound as in claim 14, wherein R₆ is a bond, substituted or unsubstituted C₁₋₁₀ alkylene, substituted or unsubstituted C₁₋₁₀ heteroalkylene, substituted or unsubstituted C₃₋₂₀ cycloalkylene, substituted or unsubstituted C₃₋₂₀ heterocycloalkylene, or substituted or unsubstituted C₆₋₂₀ arylene, and wherein if R₆ is substituted, one or more hydrogen(s) is(are) substituted with linear or branched C₁₋₅ alkyl, C₀₋₅ alkyl-OH, C₆₋₂₀aryl or CH₂-C₆₋₂₀aryl; or a pharmaceutically acceptable salt thereof.

21. The compound as in claim 14, wherein R₆ is a bond; or a pharmaceutically acceptable salt thereof.

22. The compound as in claim 14, wherein R₆ is substituted or unsubstituted alkylene; and wherein if R₆ is substituted, one or more hydrogen(s) is(are) substituted with linear or branched C₁₋₅ alkyl, C₀₋₃ alkyl-OH, C₆ aryl or CH₂-C₆ aryl; or a pharmaceutically acceptable salt thereof.

23. The compound as in claim 1, wherein R₅ and R₆ together form C₃₋₁₀ cycloalkylene, C₃₋₁₀ heterocycloalkylene, C₆₋₁₀ arylene or C₆₋₁₀ heteroarylene; or a pharmaceutically acceptable salt thereof.

24. The compound as in claim 1, wherein R₅ and R₆ together form 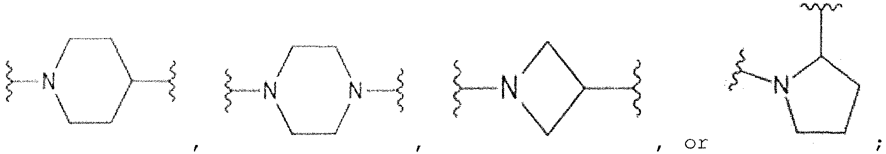 or a pharmaceutically acceptable salt thereof.

25. The compound as in claim 1, wherein Y is H, -(CH₂)ₙCH₃, -(CH₂)ₙCOOH, - (CH₂)ₙOH, -(CH₂)nSO₃H or -(CH₂)ₙOSO₃H, and n is an integer from 0 to 10; or a pharmaceutically acceptable salt thereof.

26. The compound as in claim 1, wherein Y is -(CH₂)ₙSO₃H, and n is an integer from 0 to 5; or a pharmaceutically acceptable salt thereof.

27. The compound as claim 1, the compound represented by Formula 1 or a pharmaceutically acceptable salt thereof is any one selected from the group consisting of the following compounds:

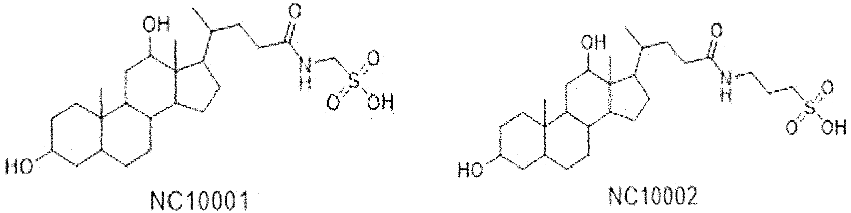

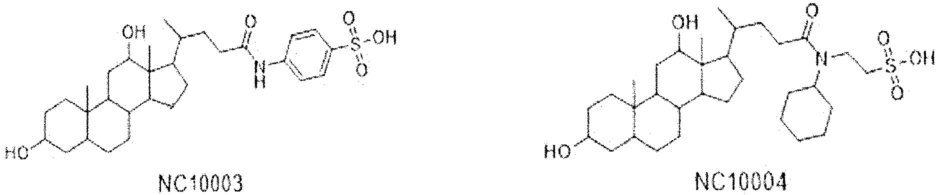

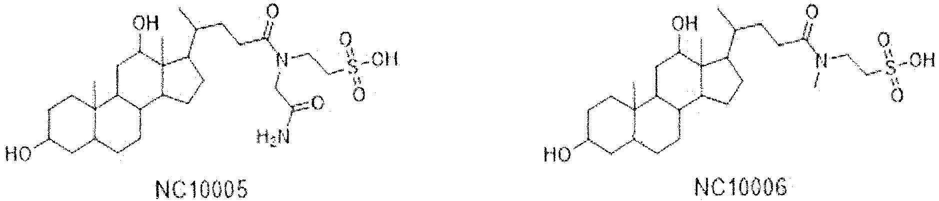

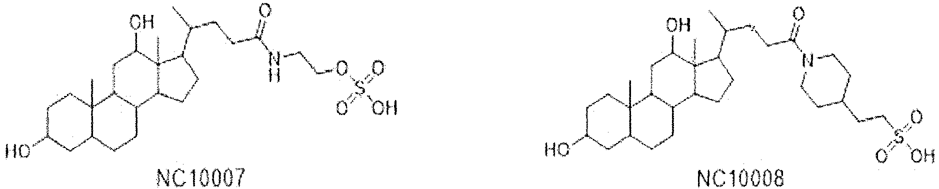

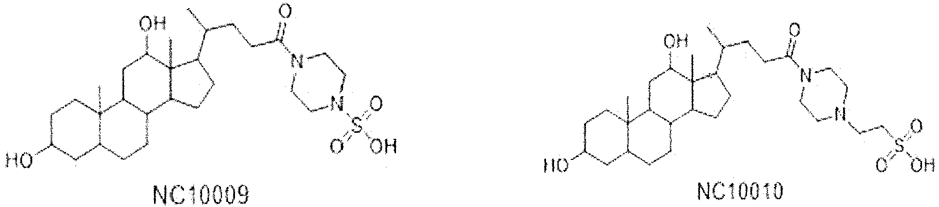

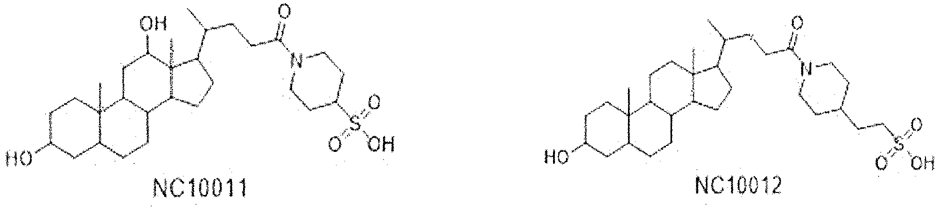

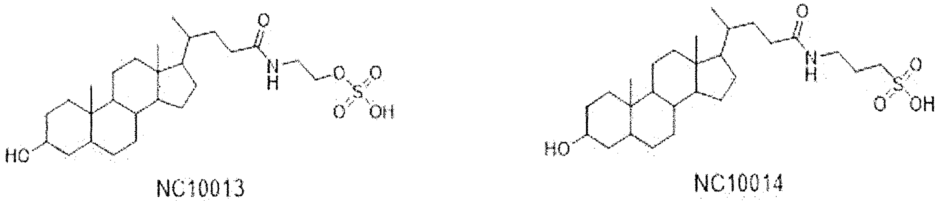

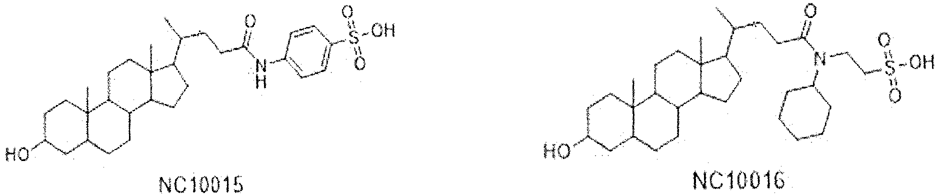

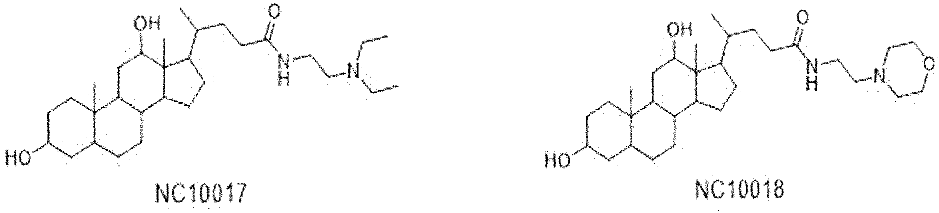

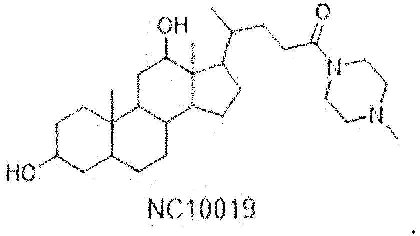

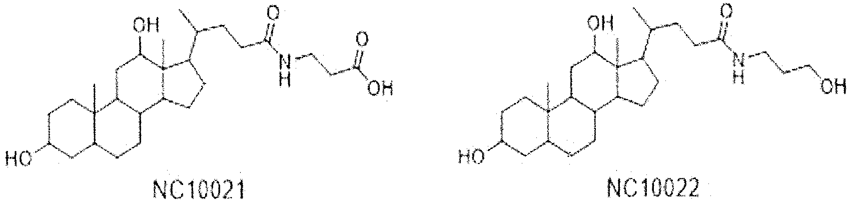

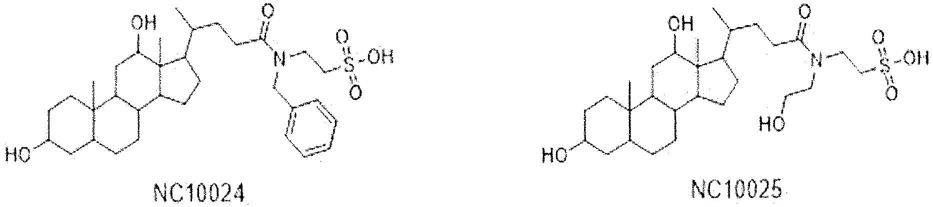

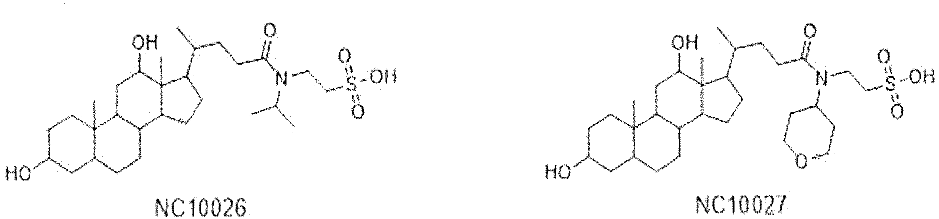

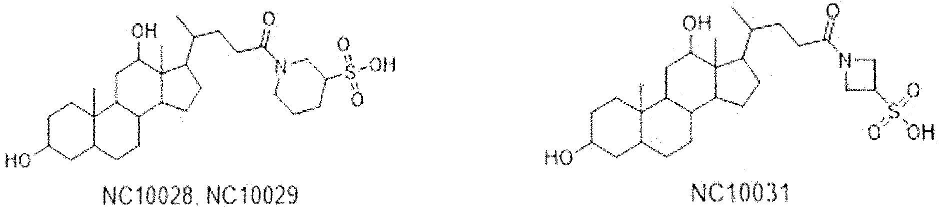

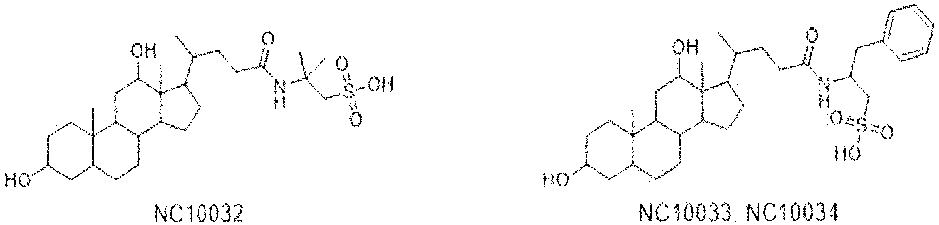

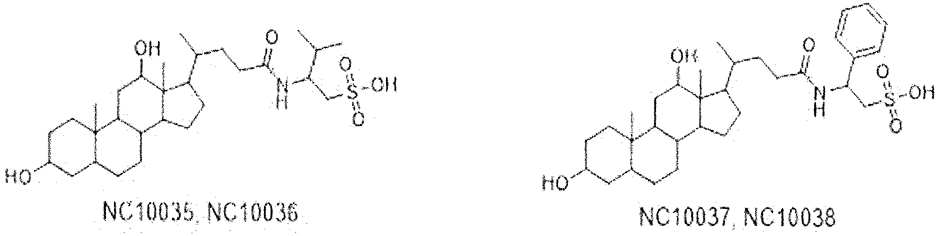

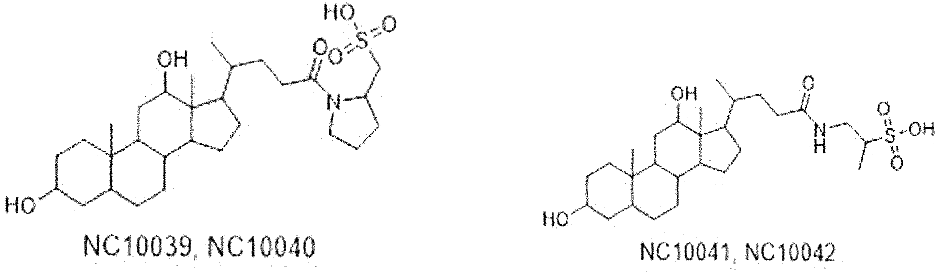

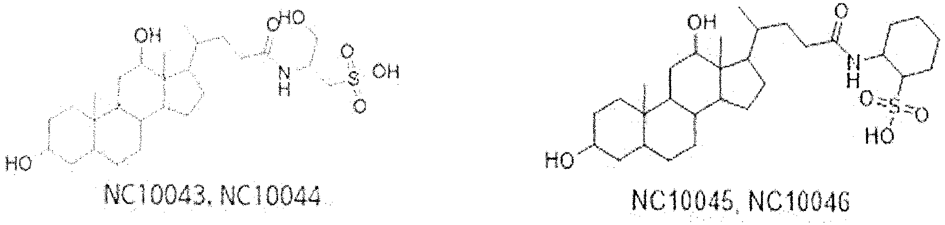

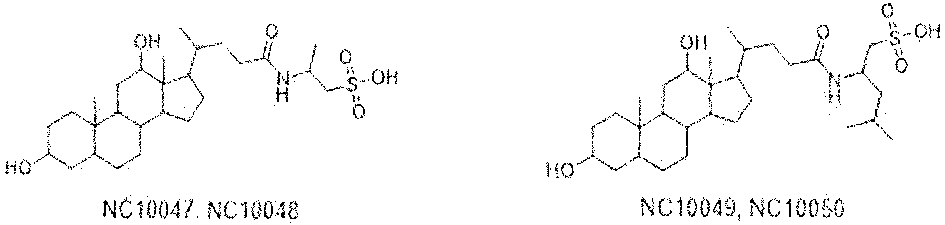

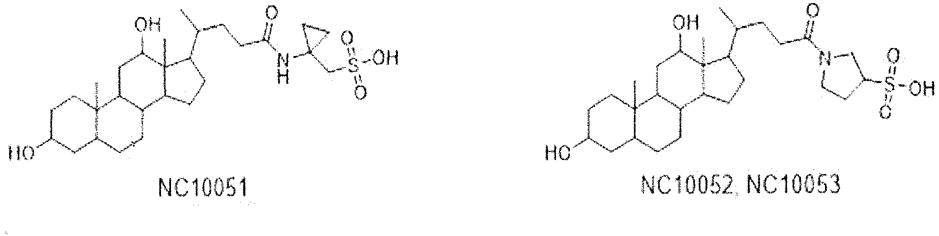

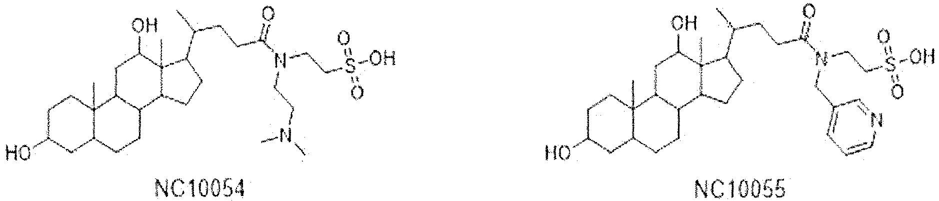

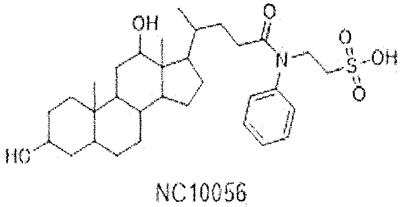

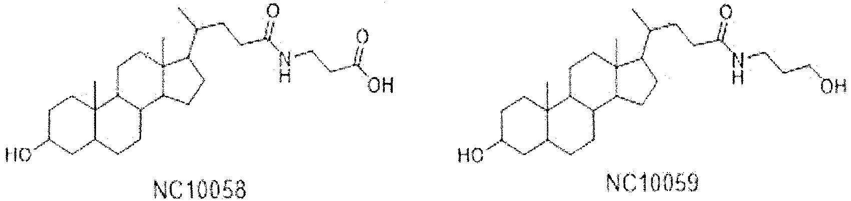

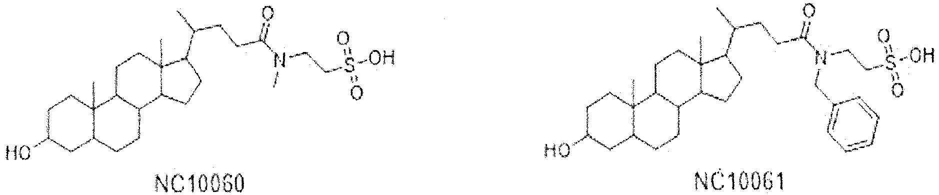

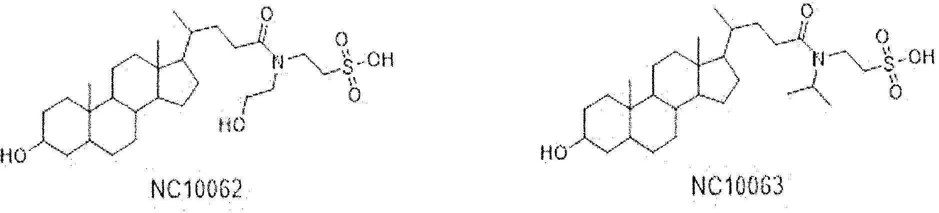

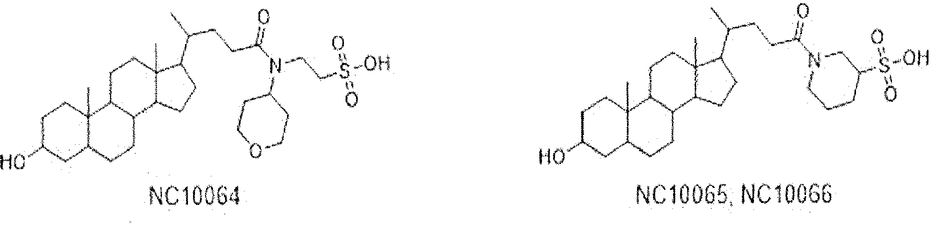

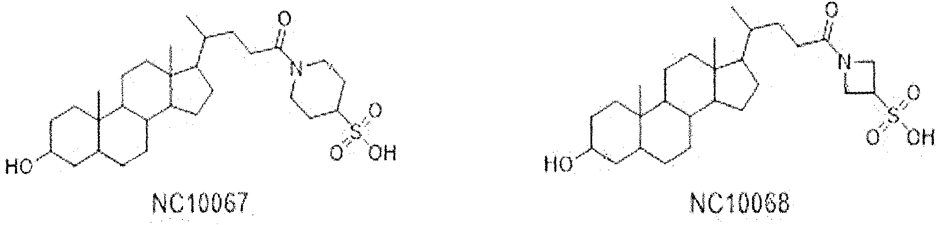

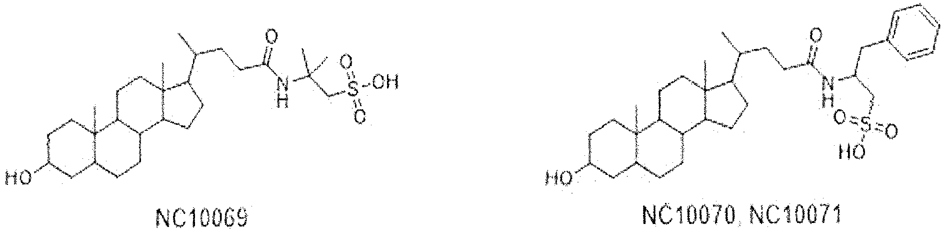

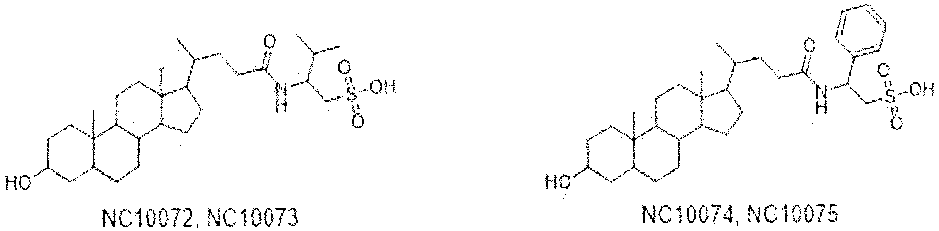

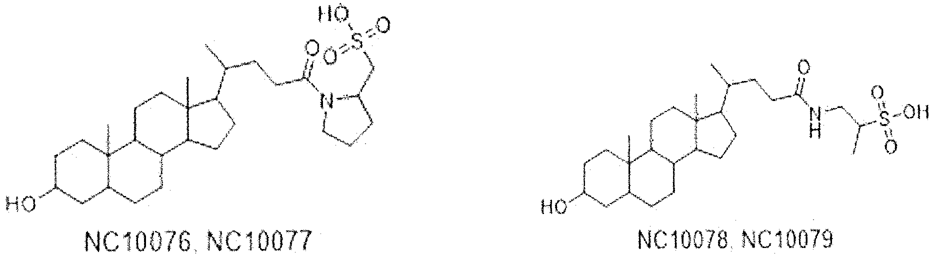

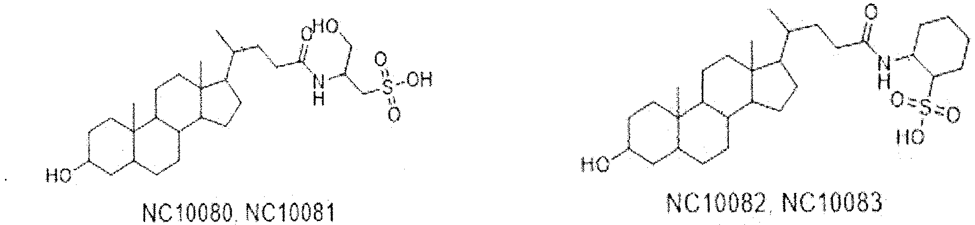

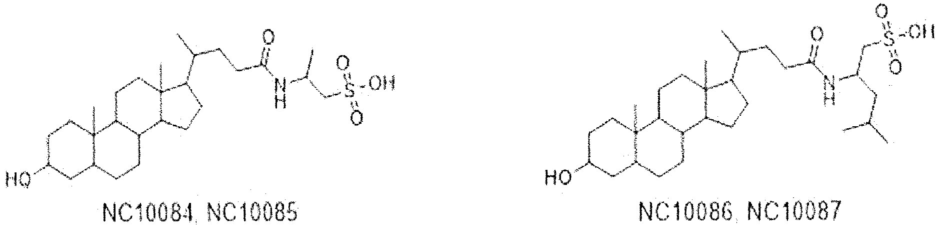

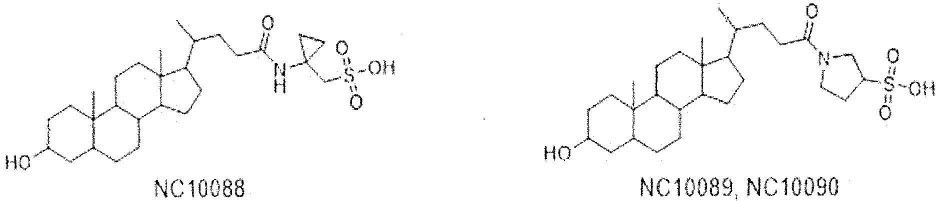

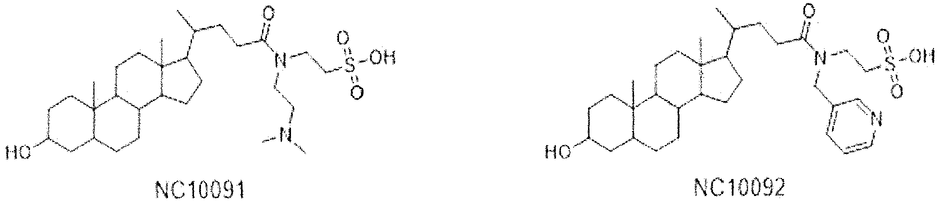

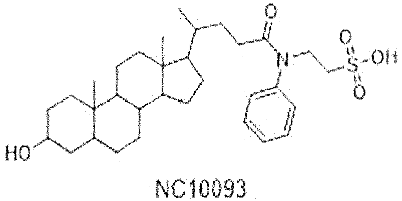

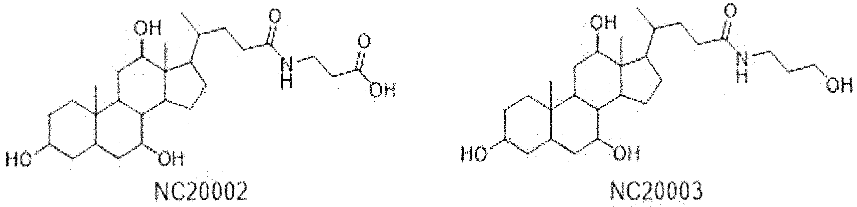

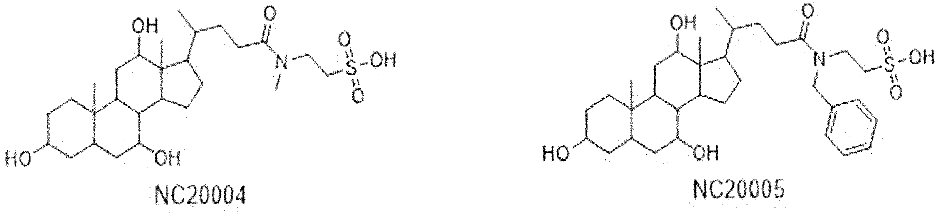

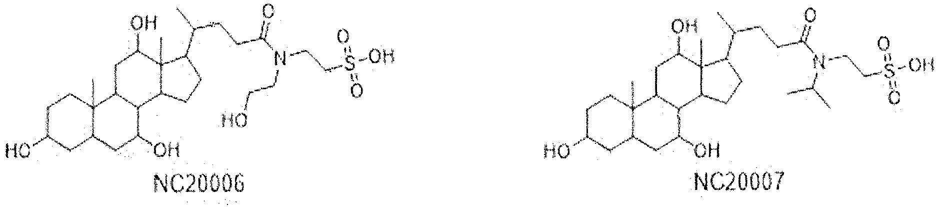

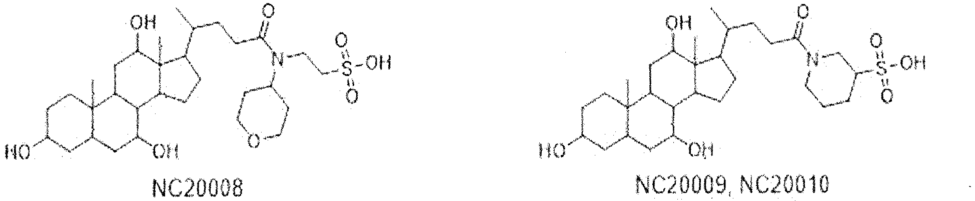

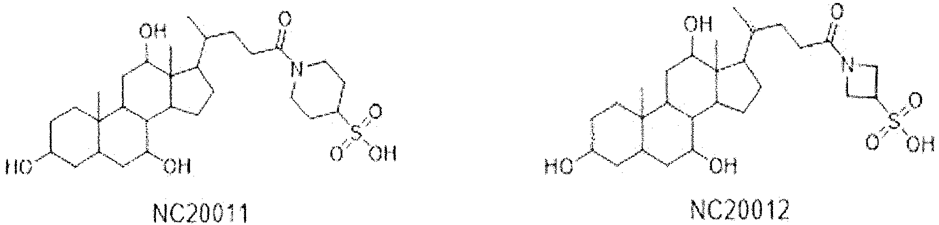

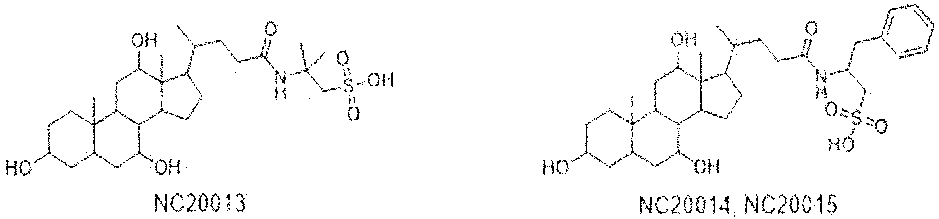

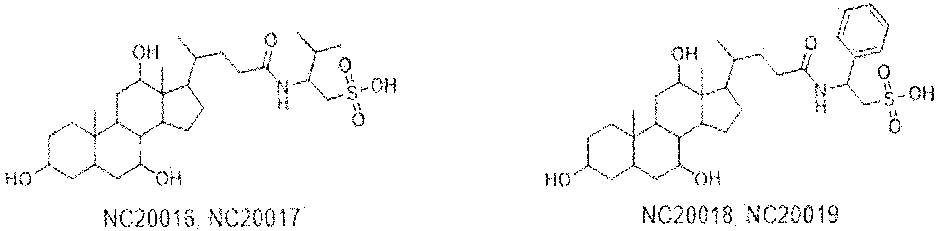

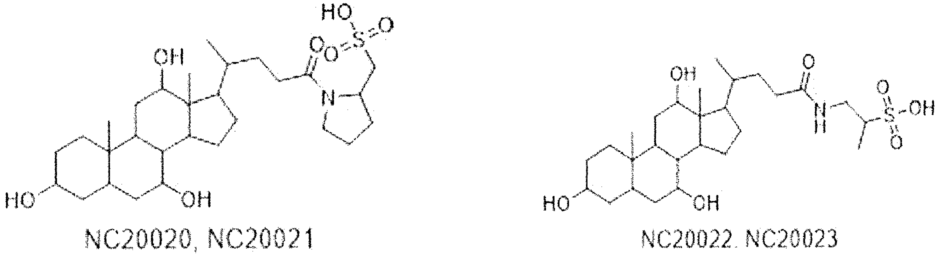

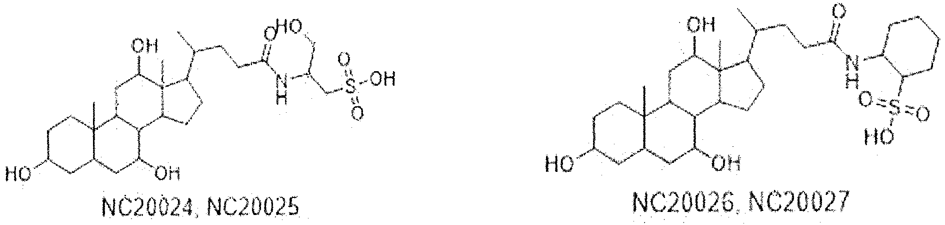

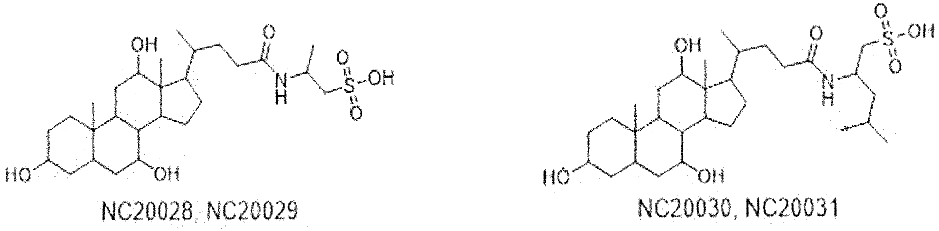

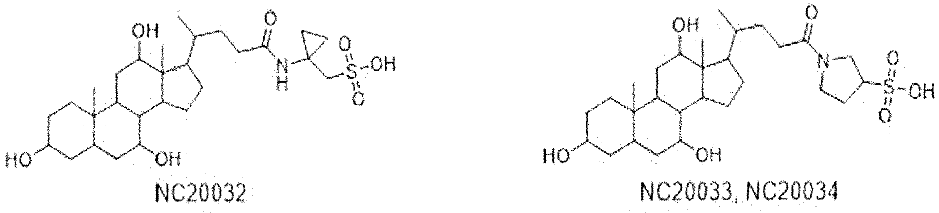

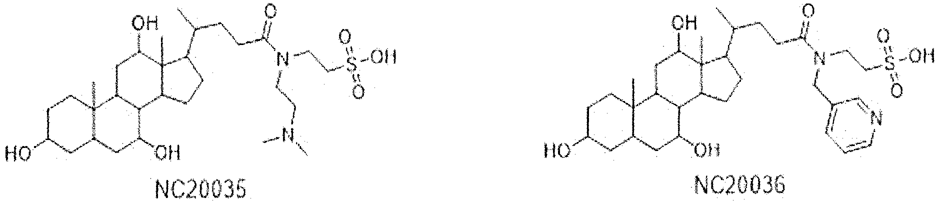

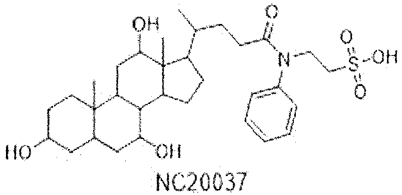

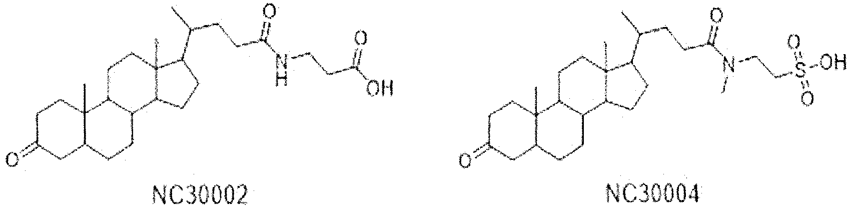

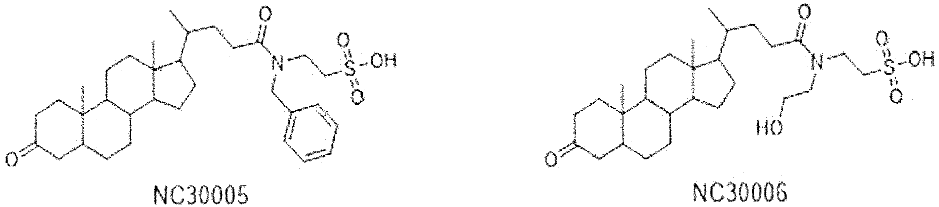

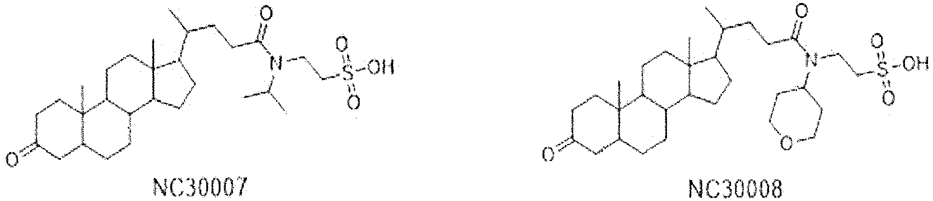

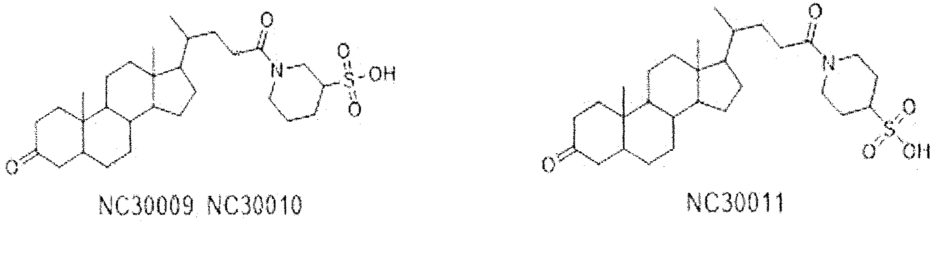

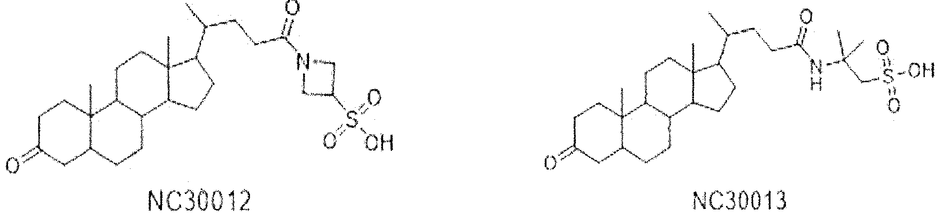

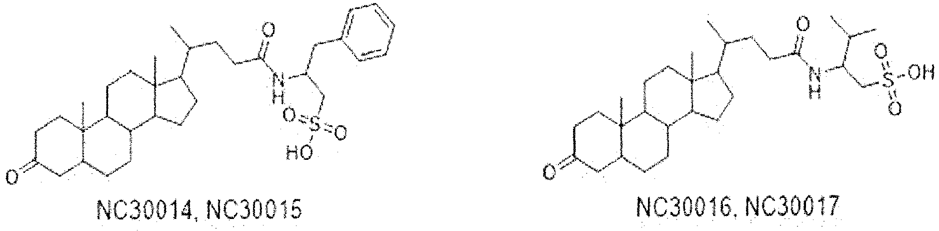

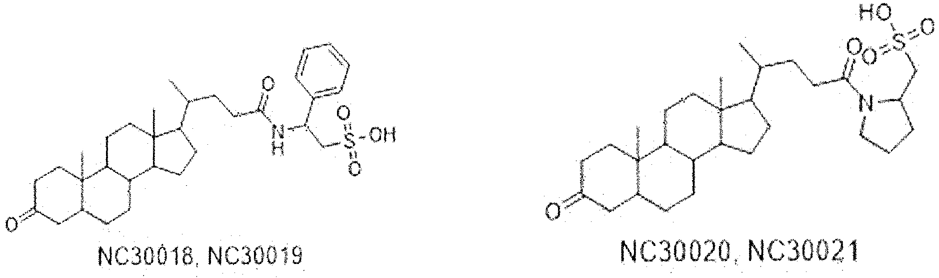

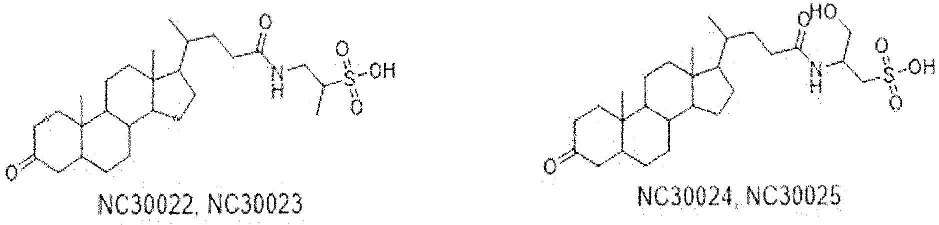

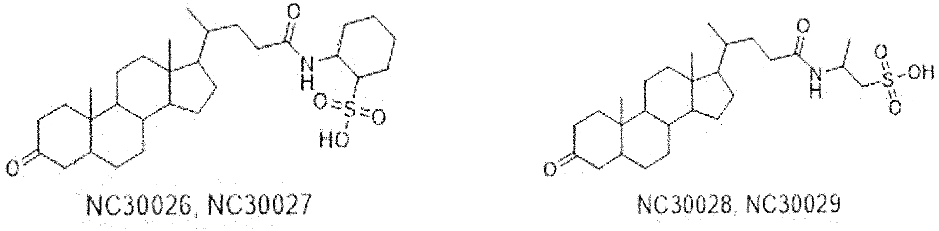

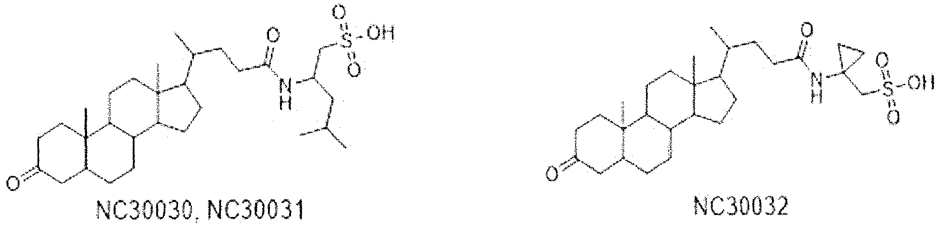

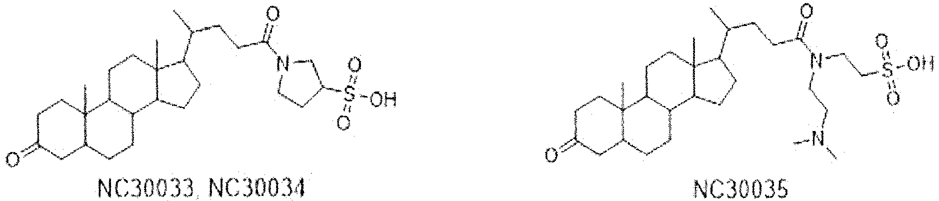

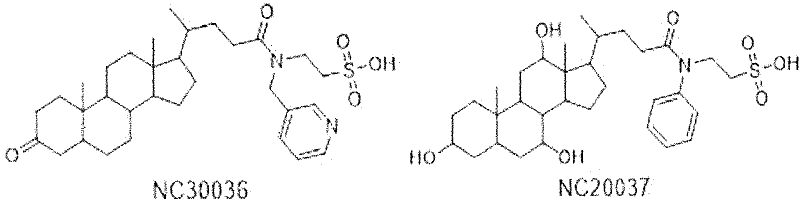

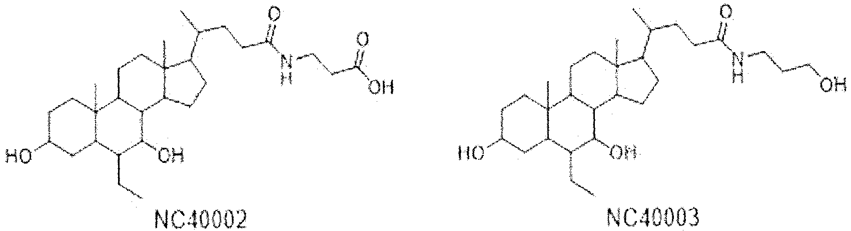

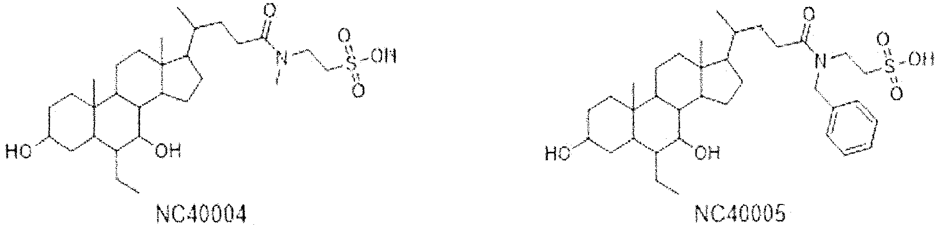

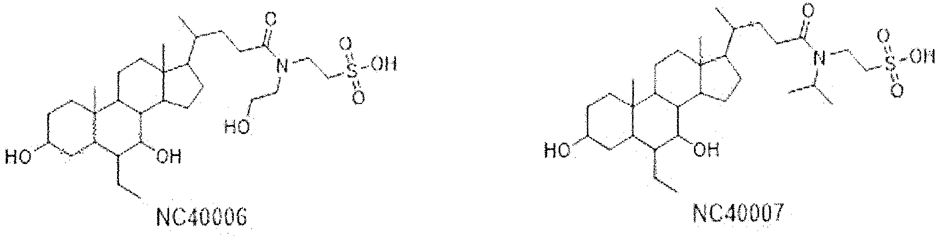

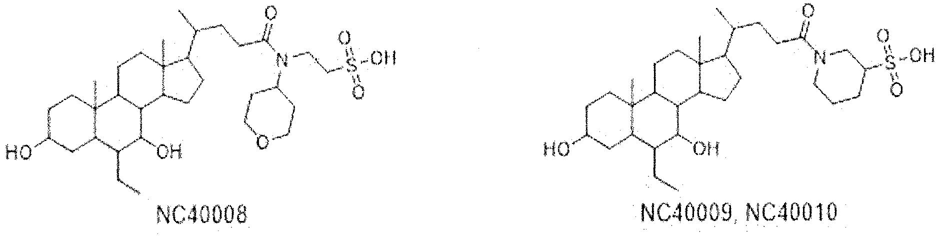

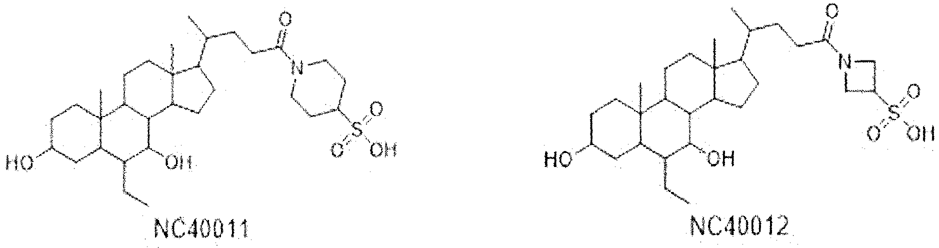

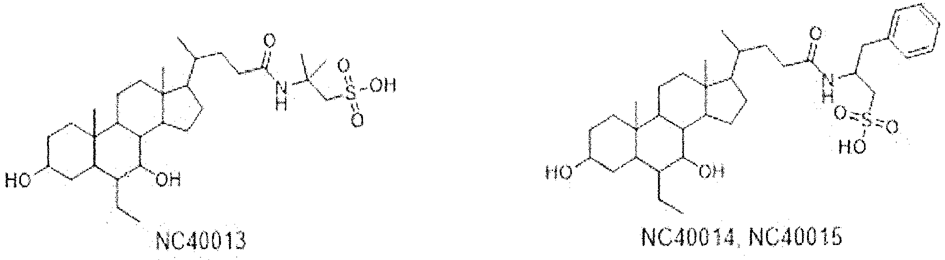

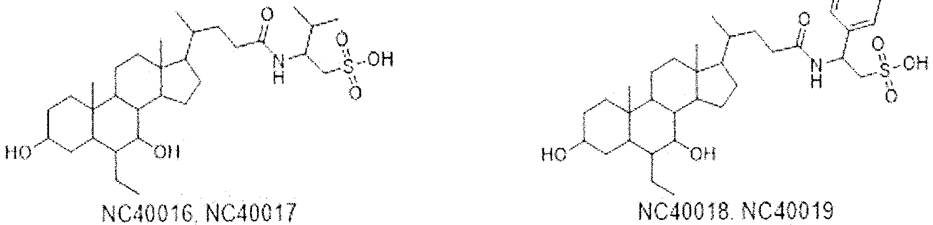

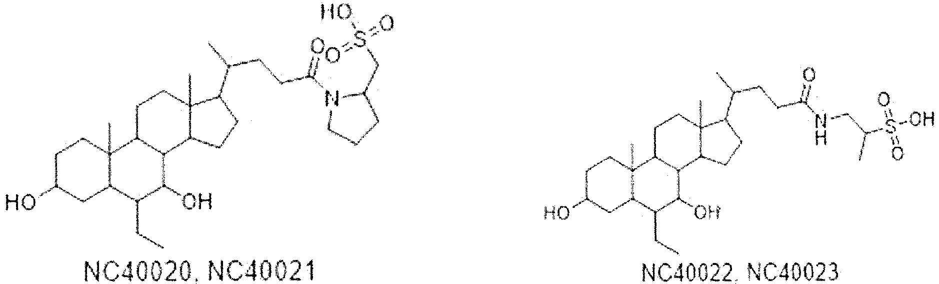

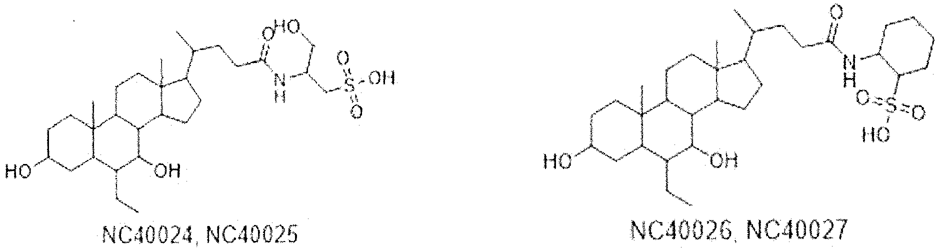

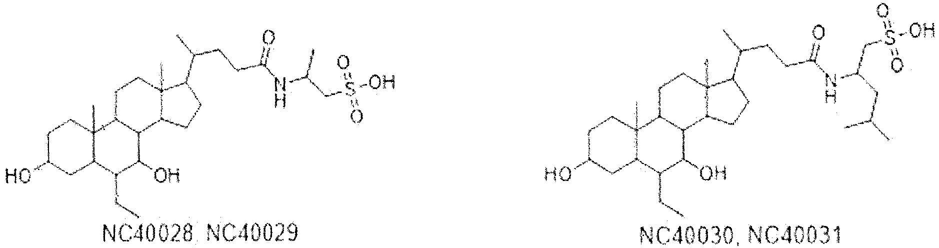

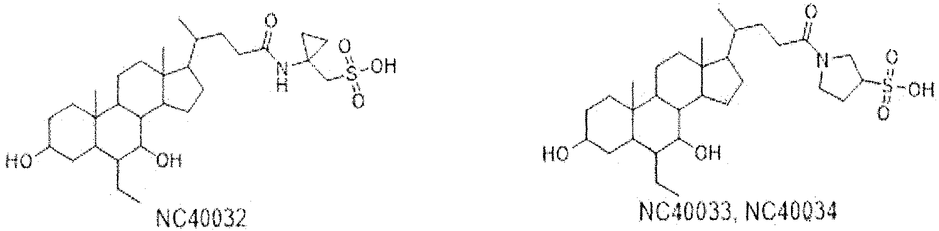

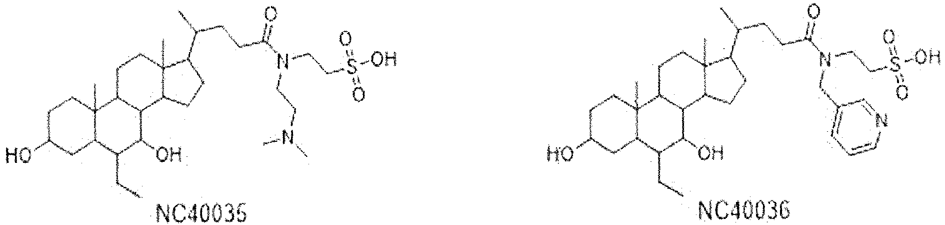

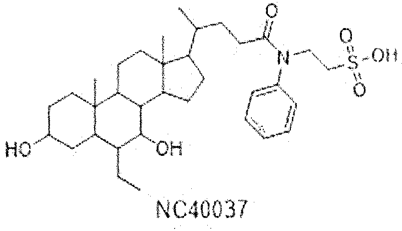

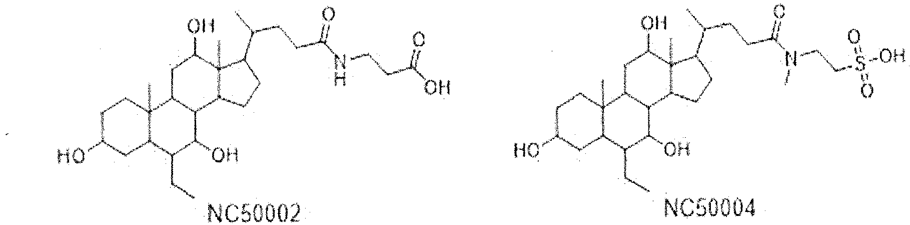

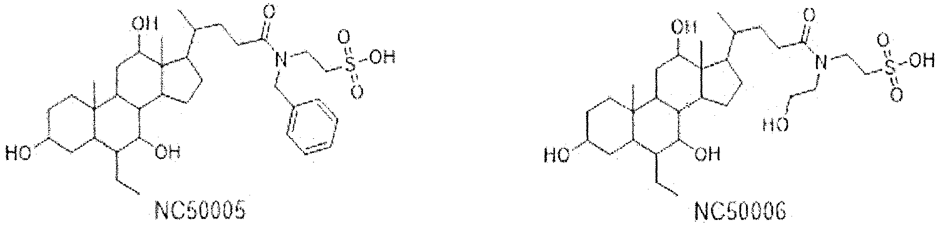

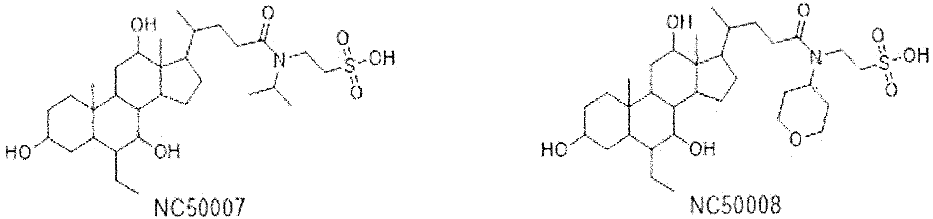

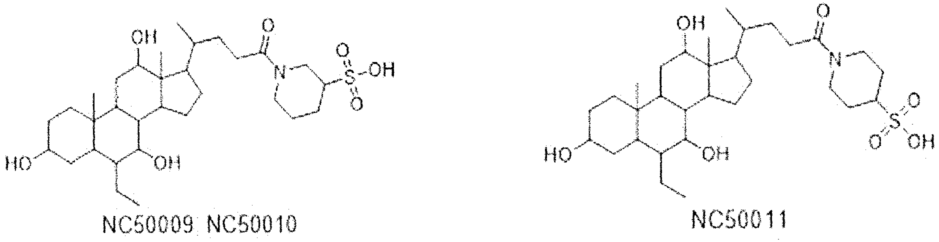

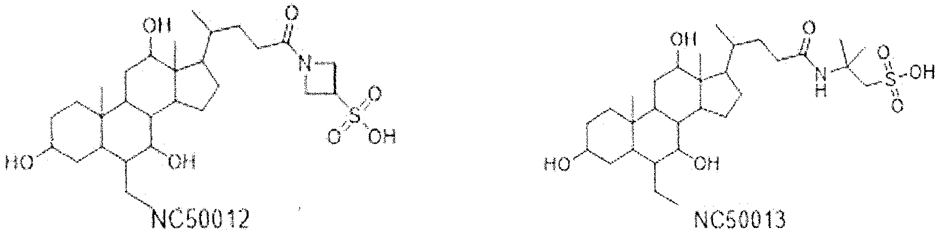

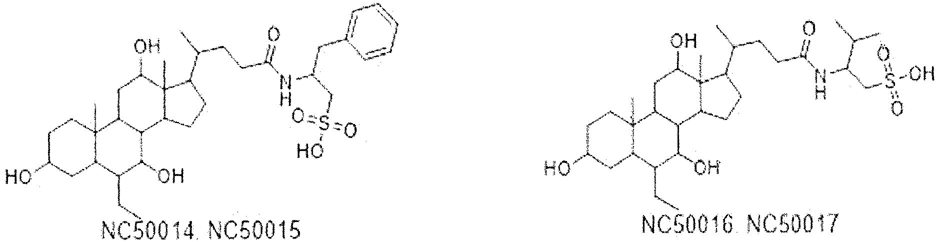

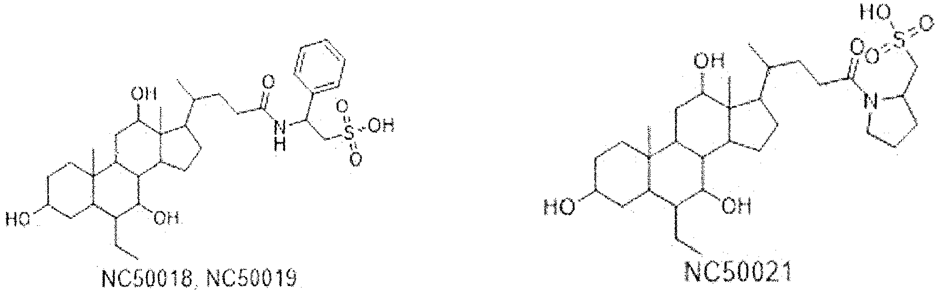

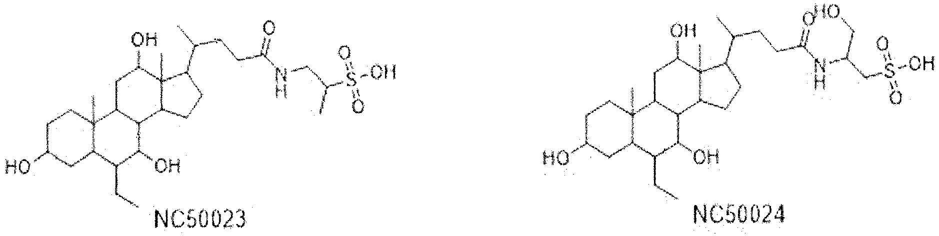

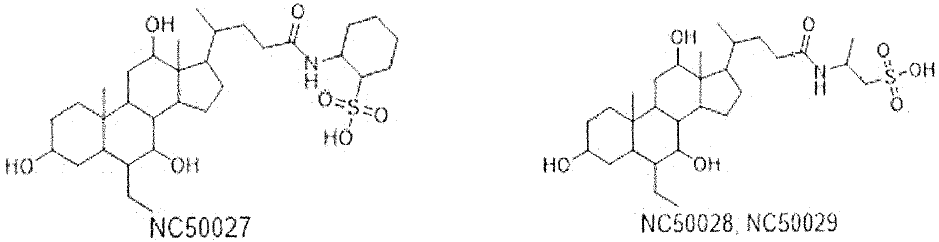

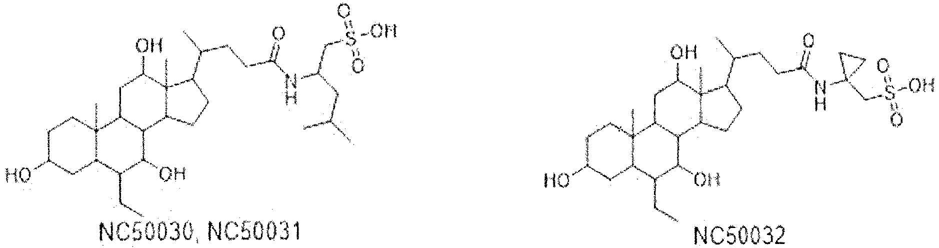

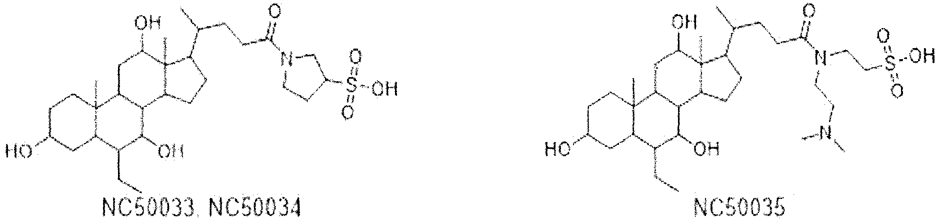

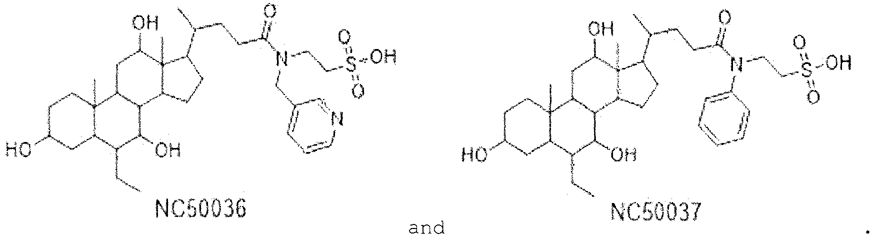

28. The compound as claim 1, the compound represented by Formula 1 or a pharmaceutically acceptable salt thereof is any one selected from the group consisting of the following compounds: 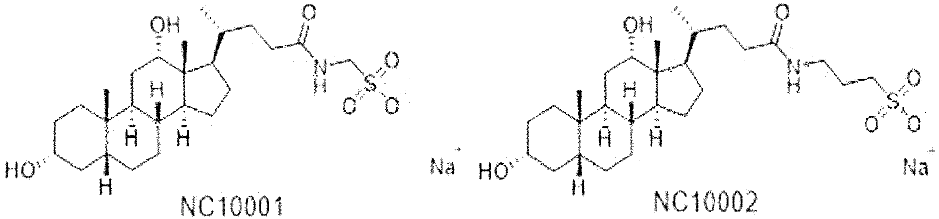 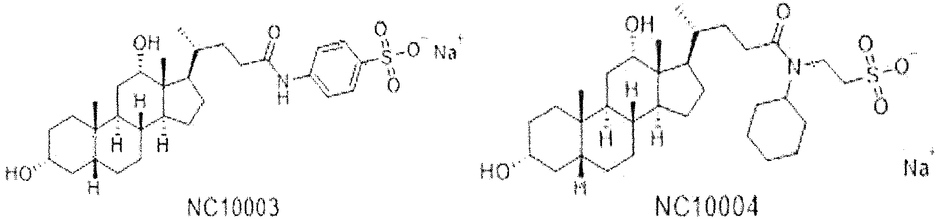 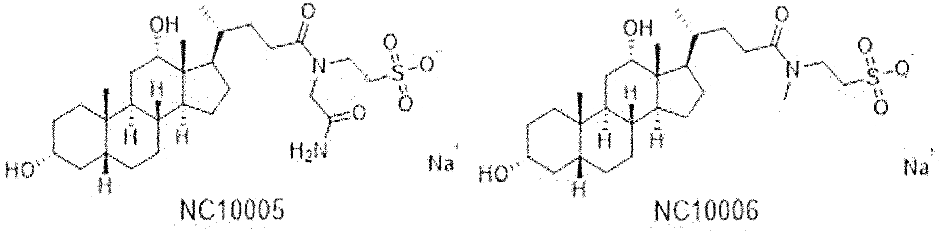 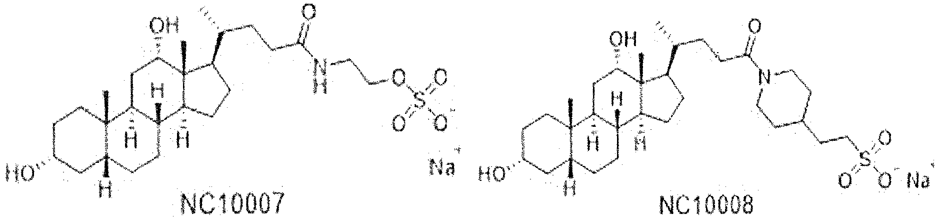 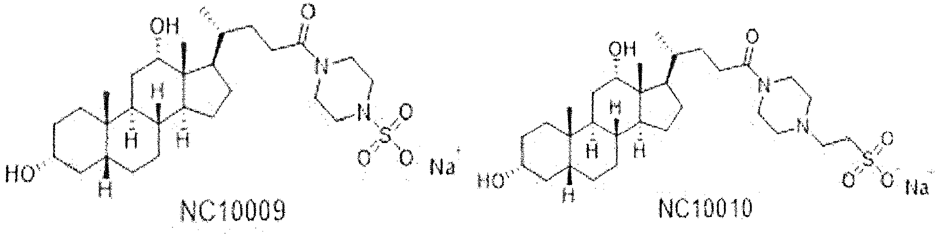 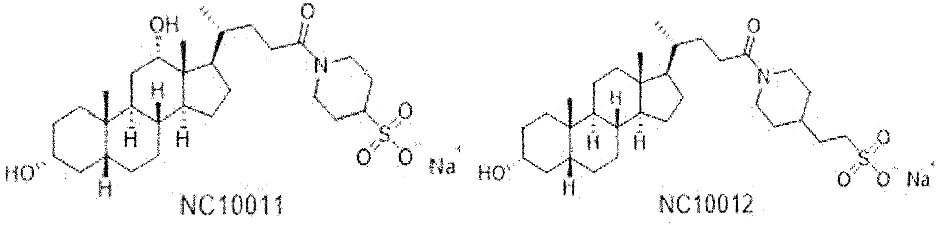 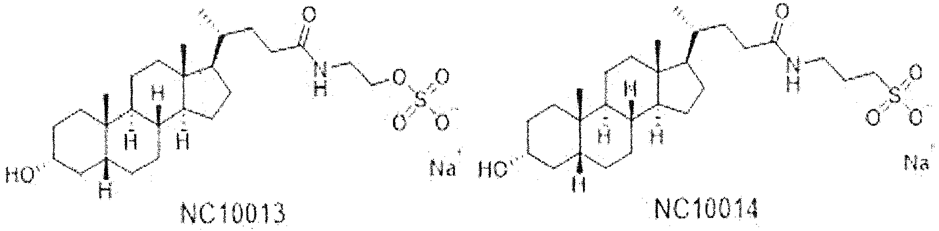 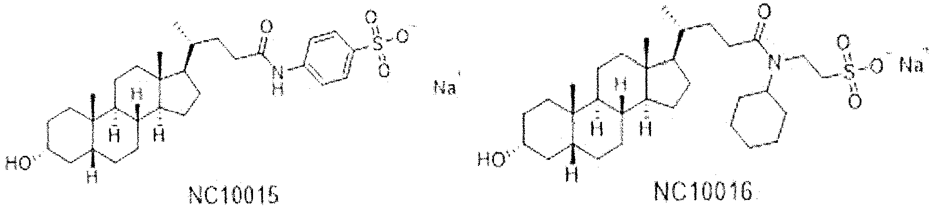 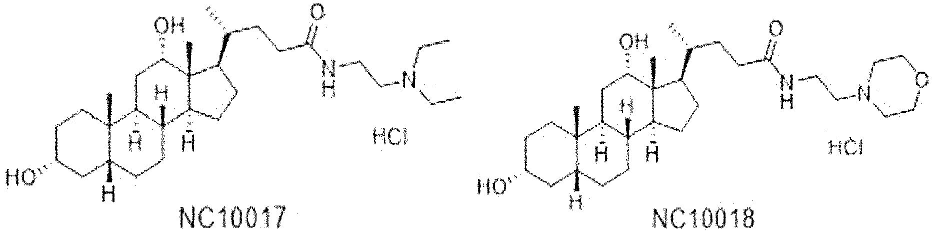 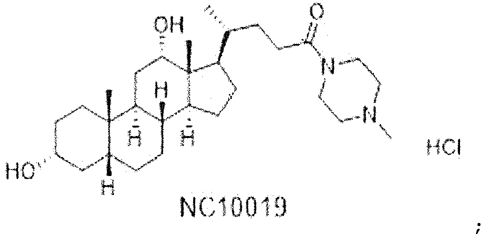 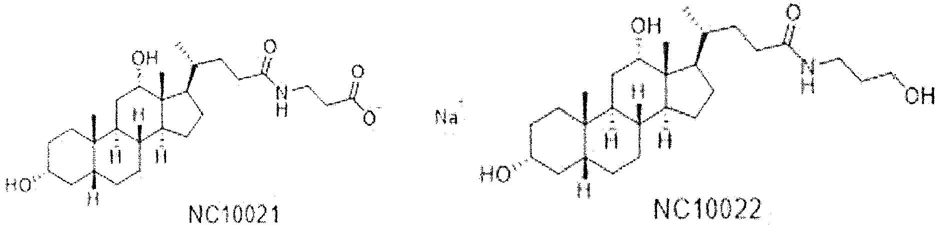 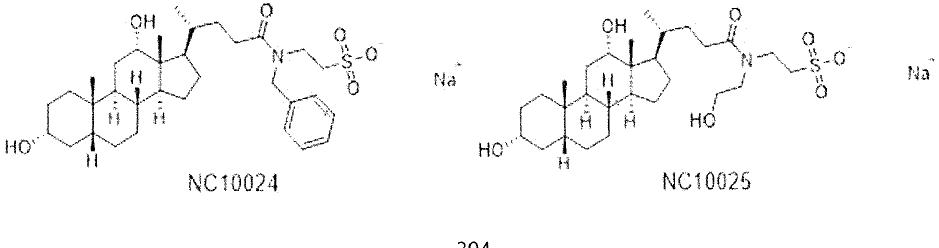 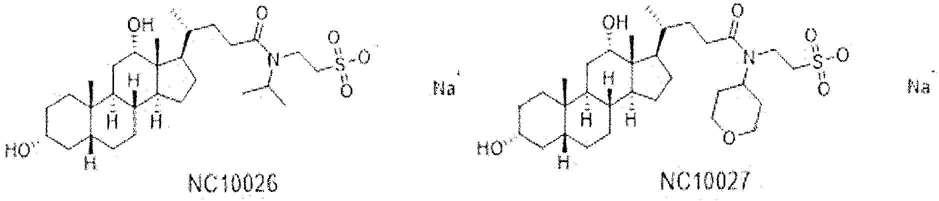 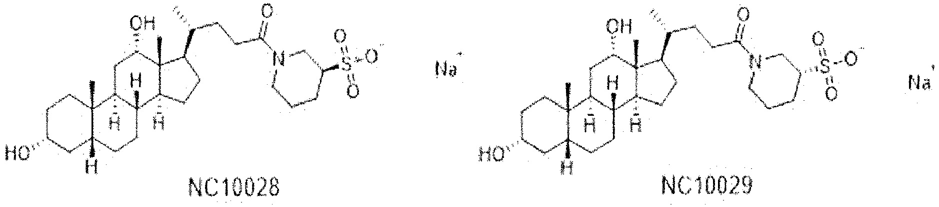 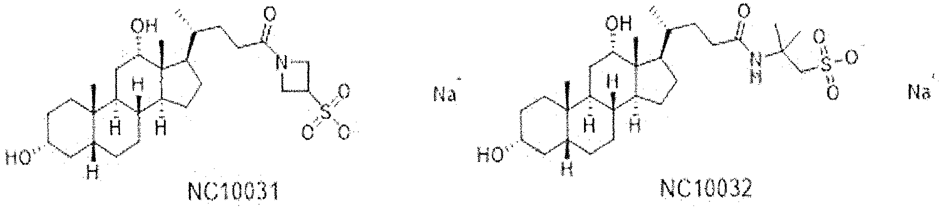 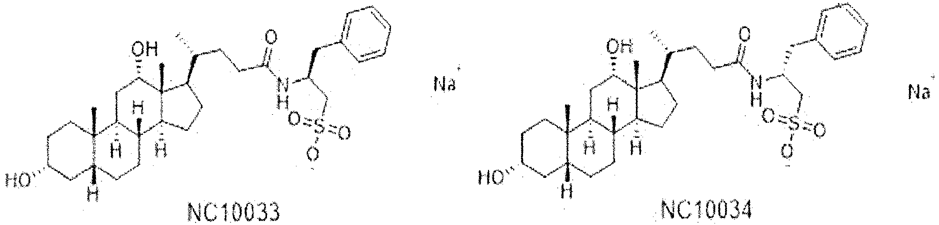 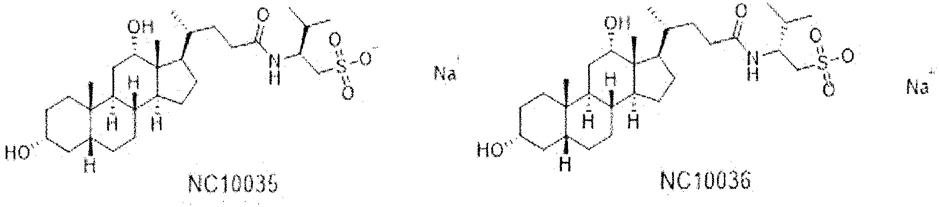 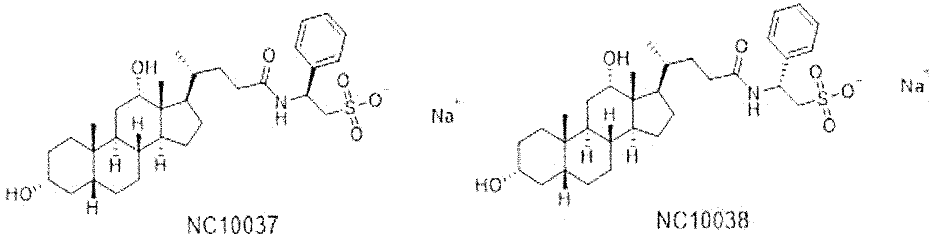 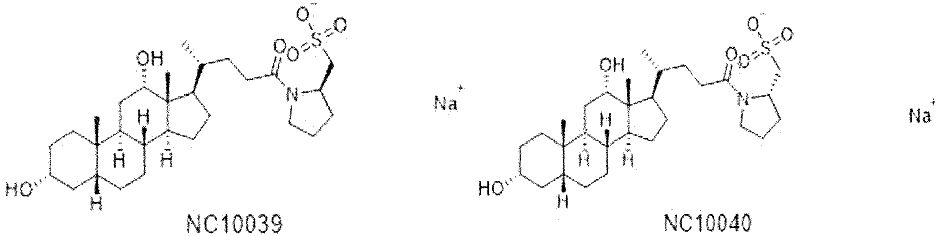 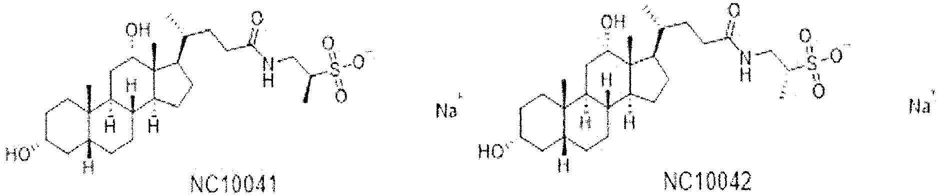 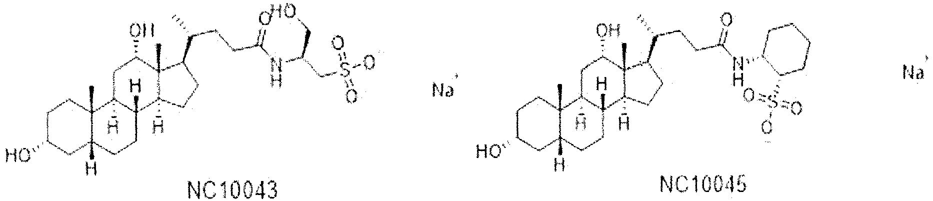 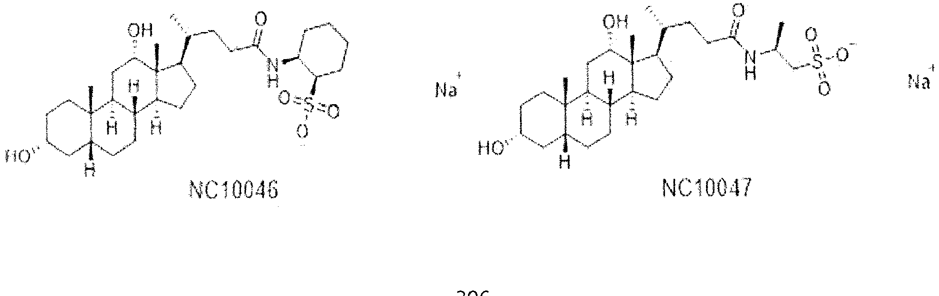 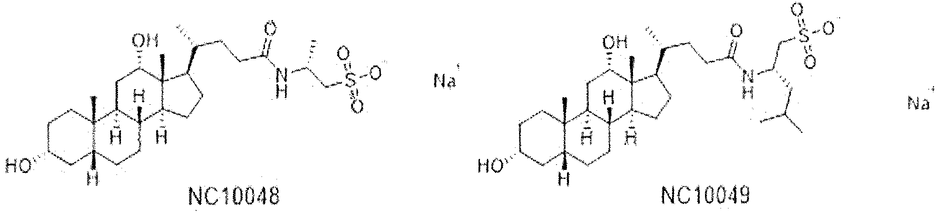 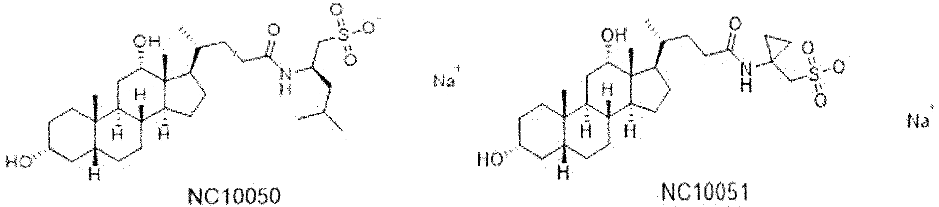 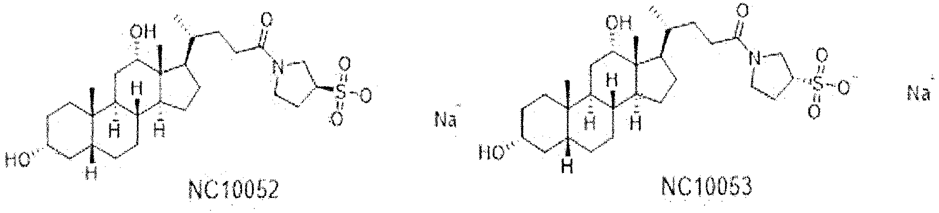 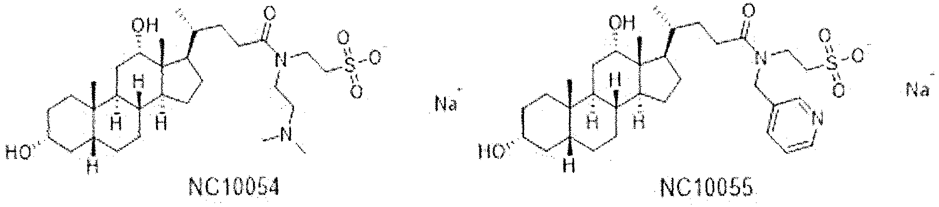 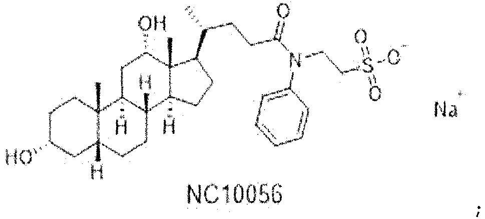 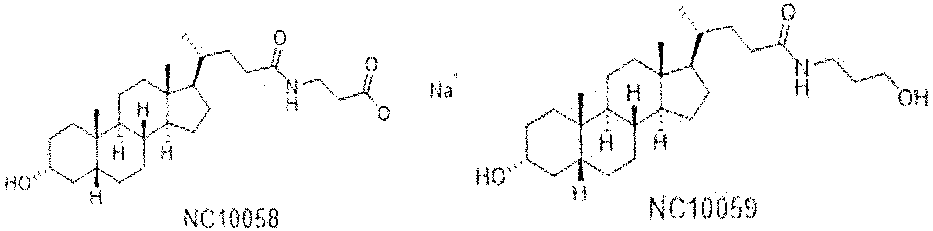 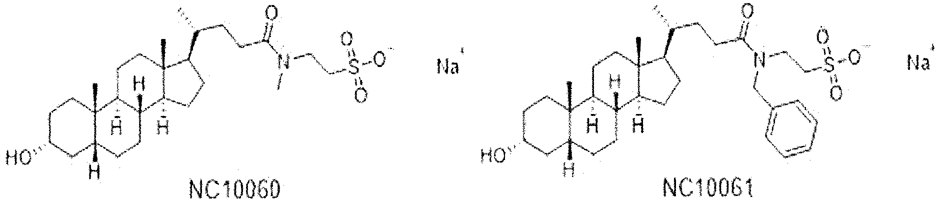 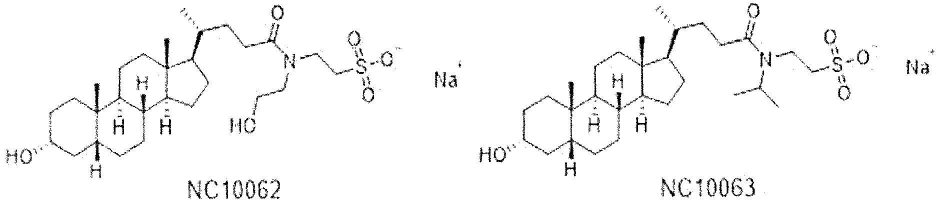 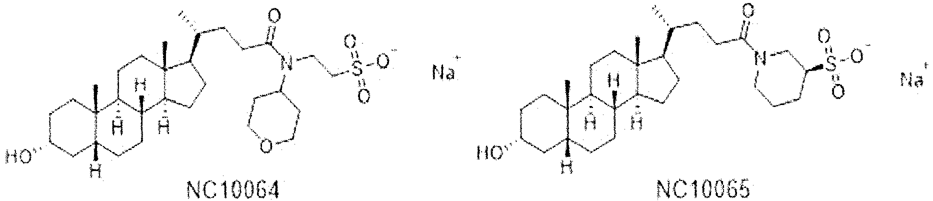 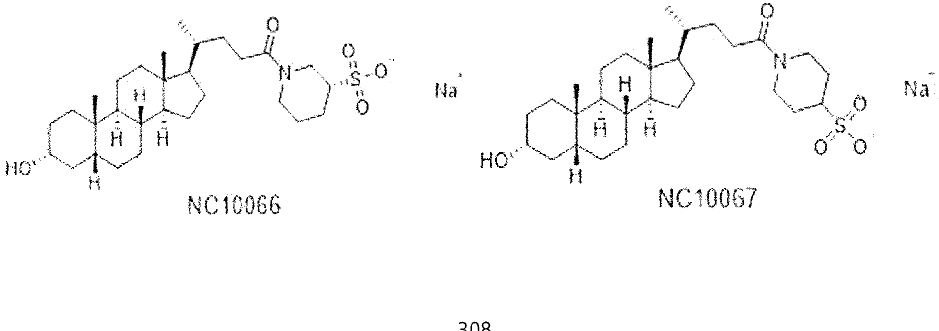 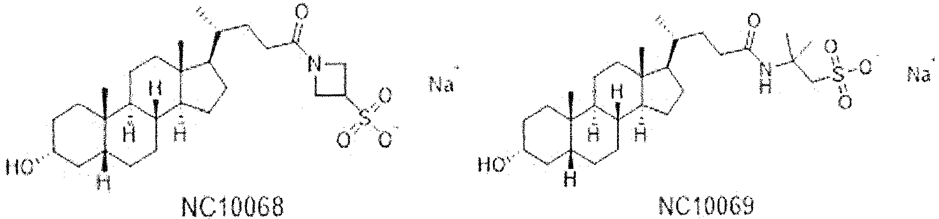 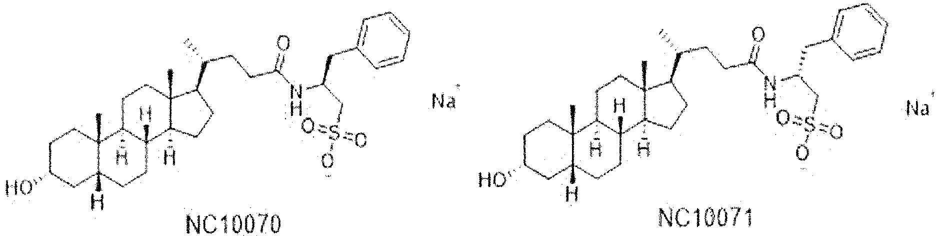 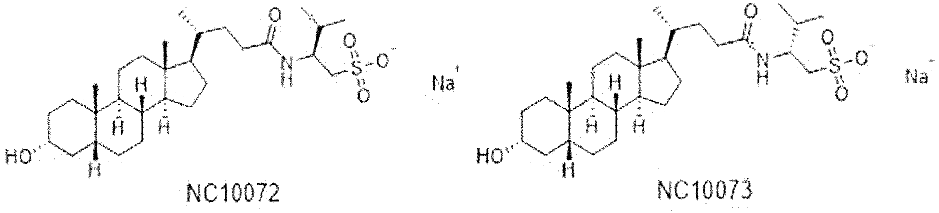 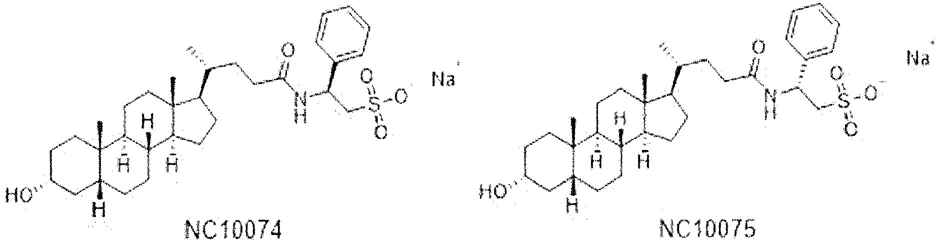 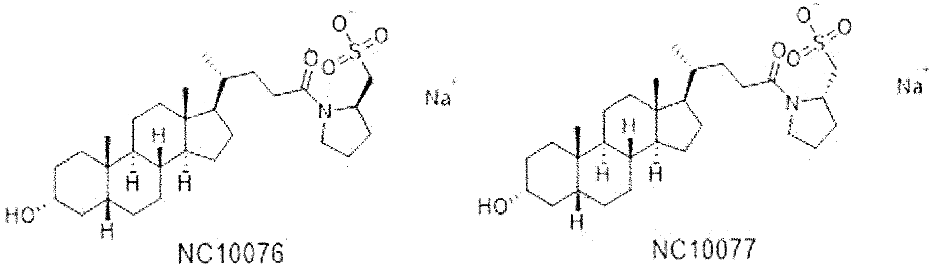 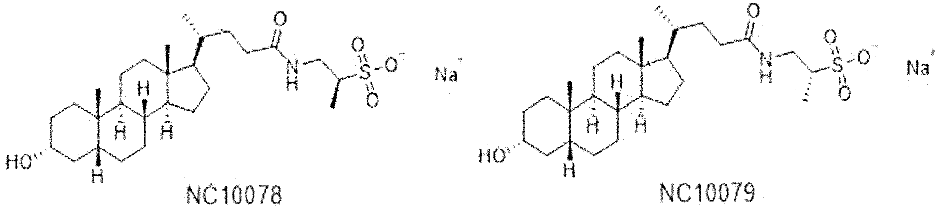 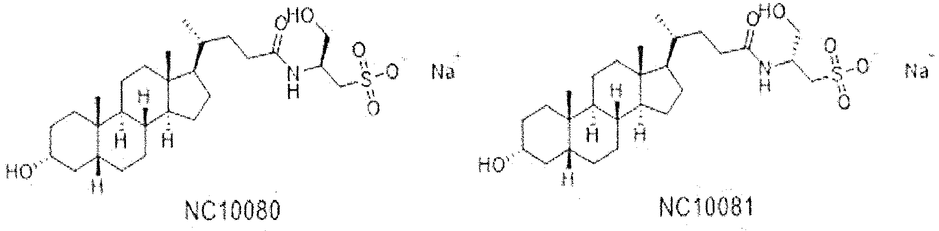 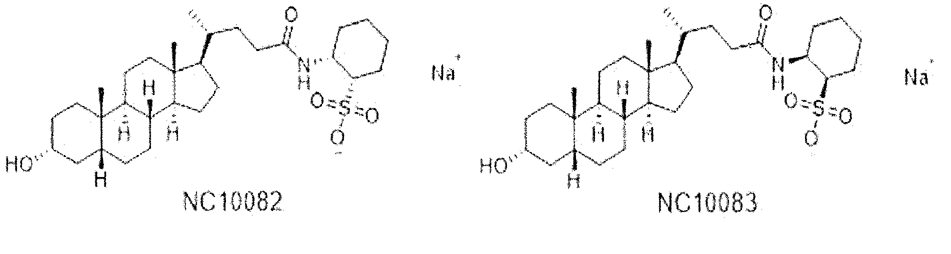 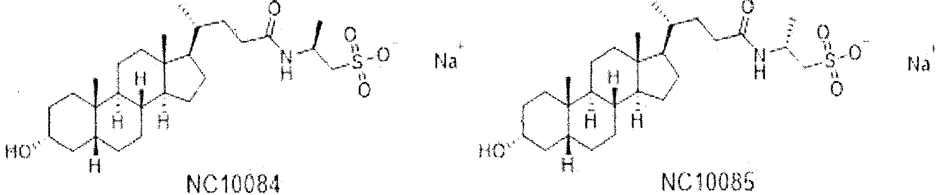 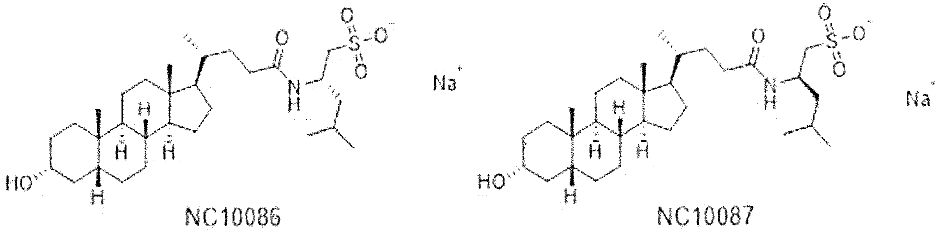 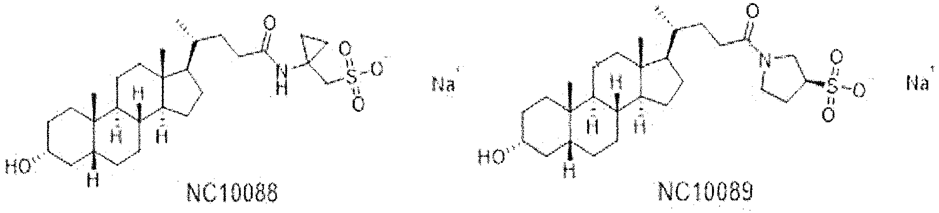 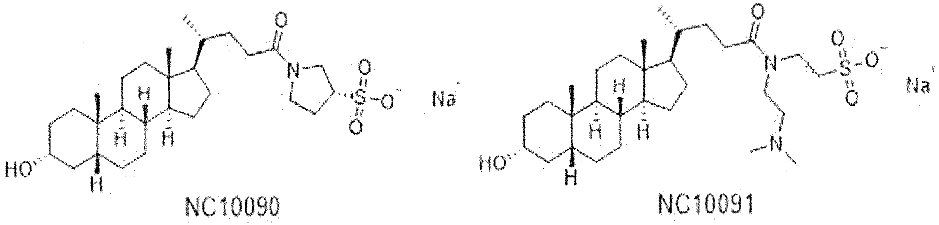 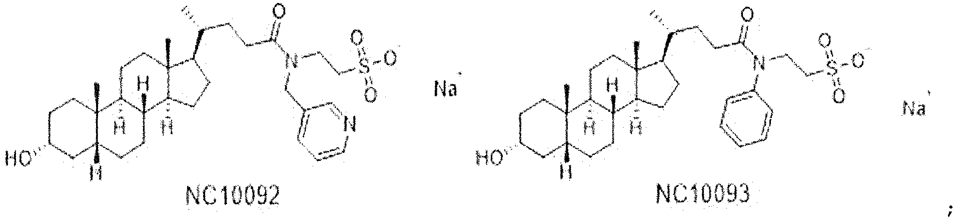 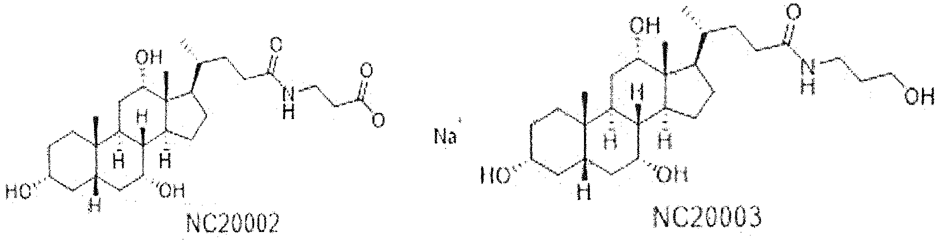 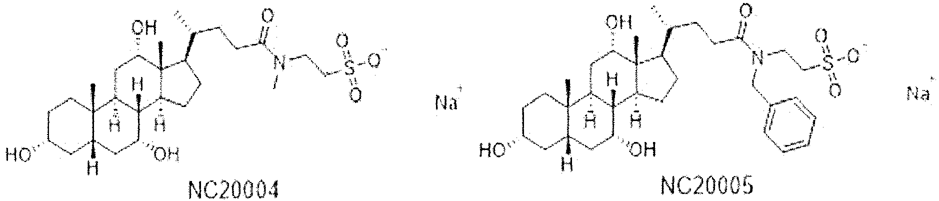 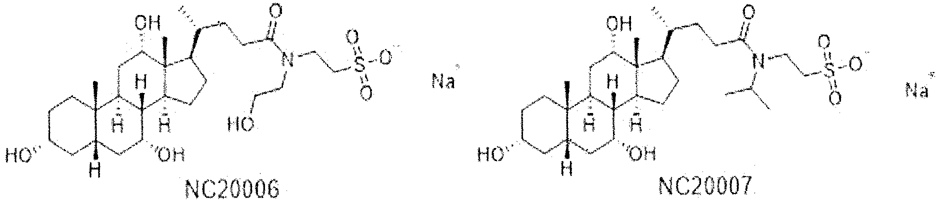 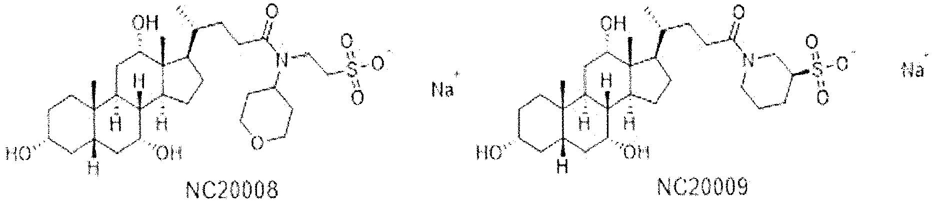 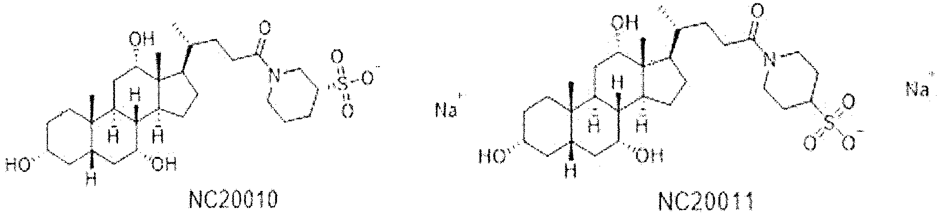 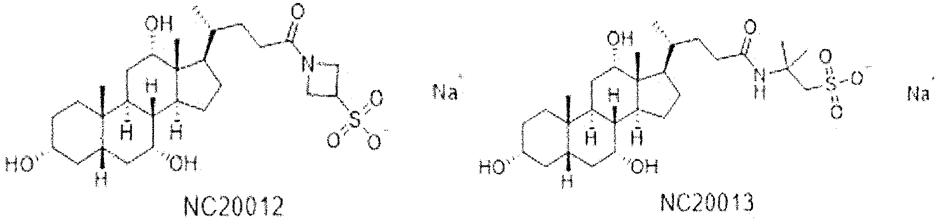 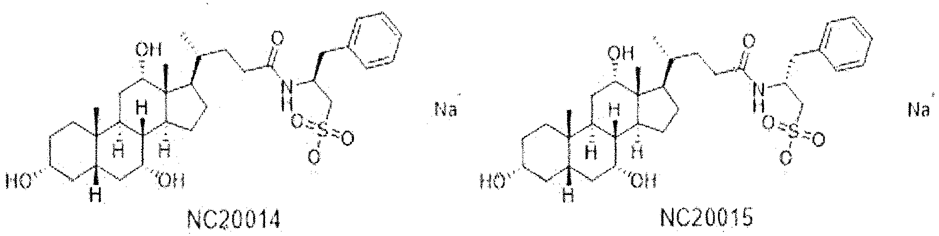 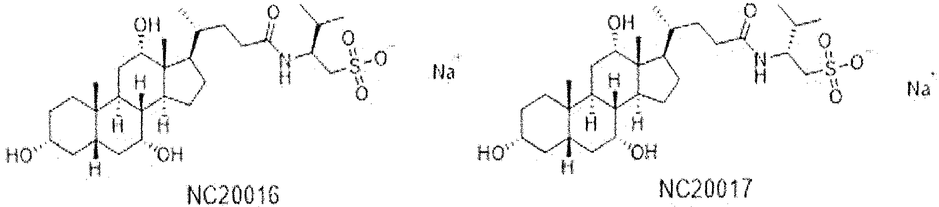 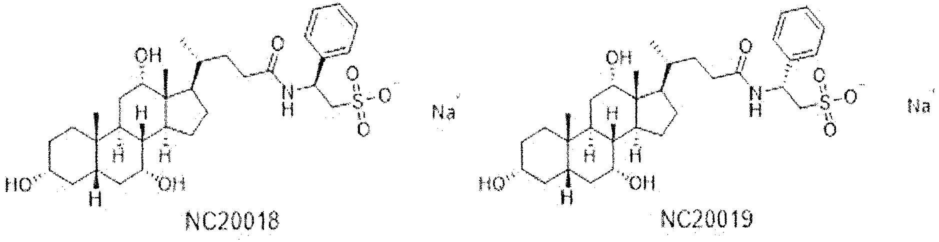 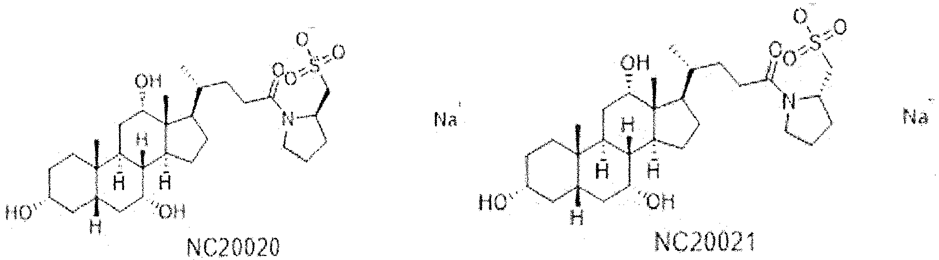 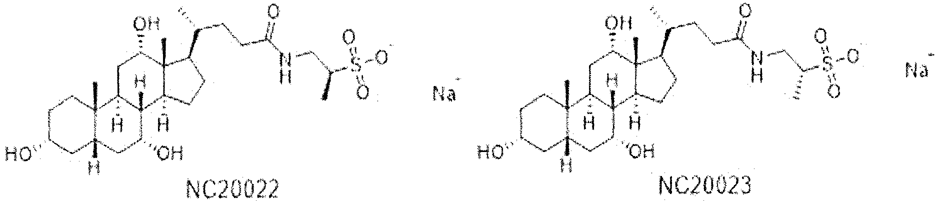 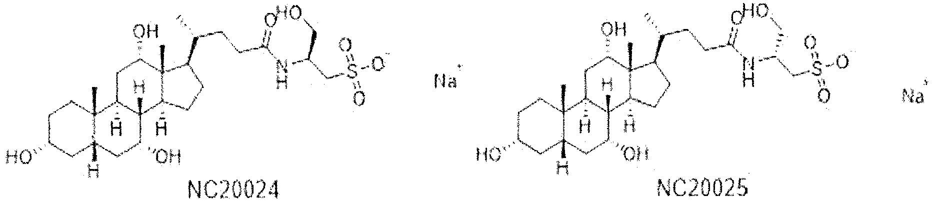 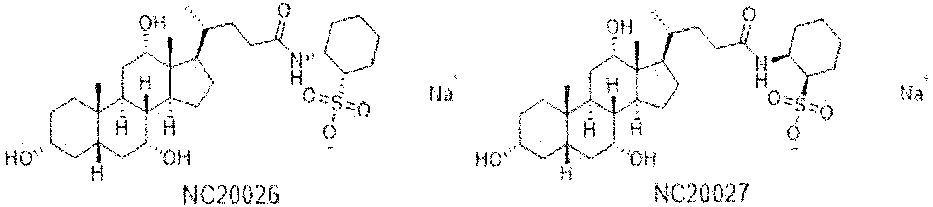 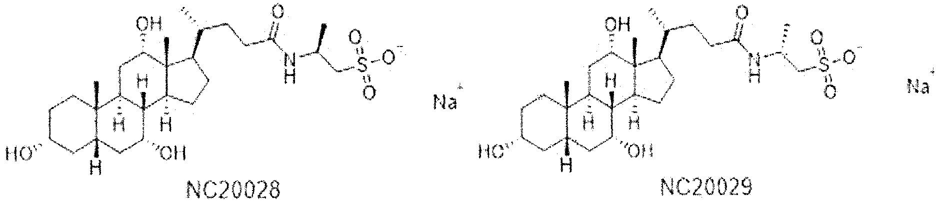 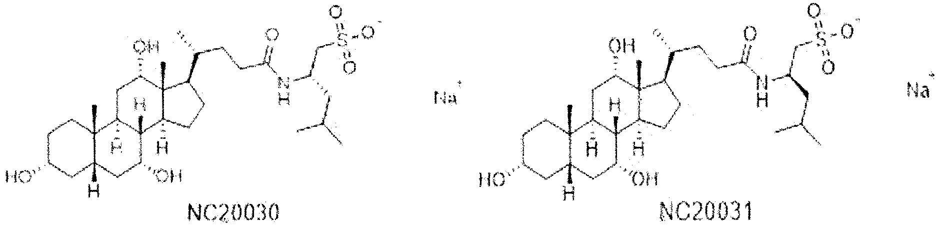 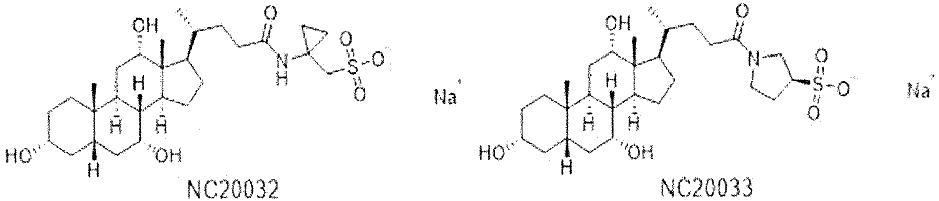 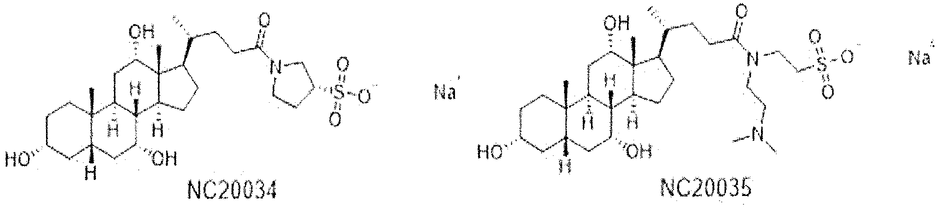 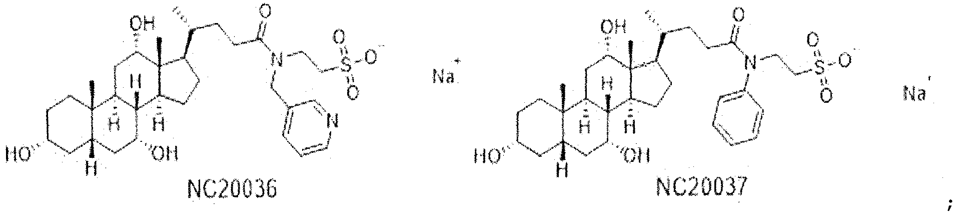 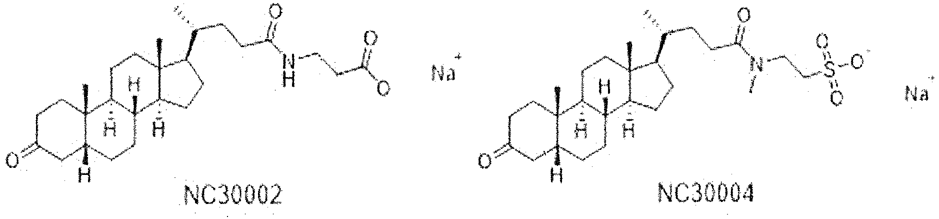 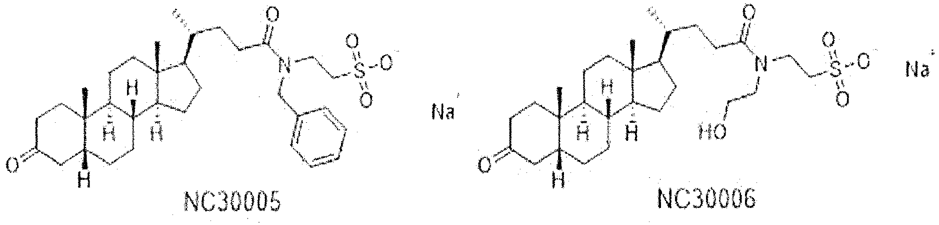 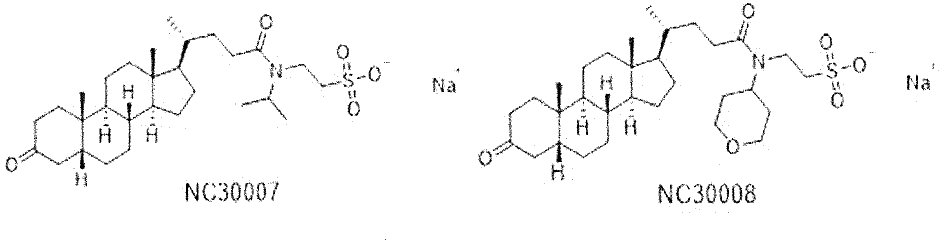 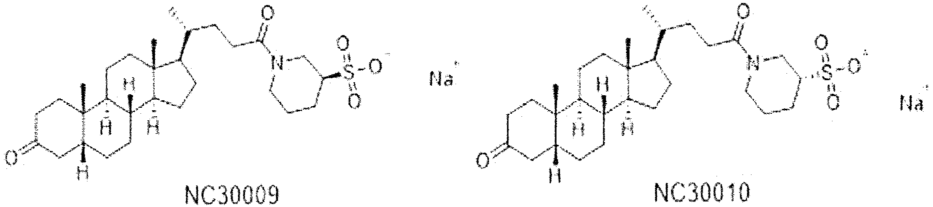 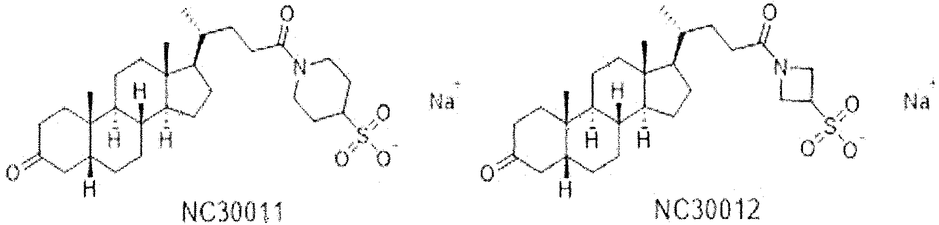 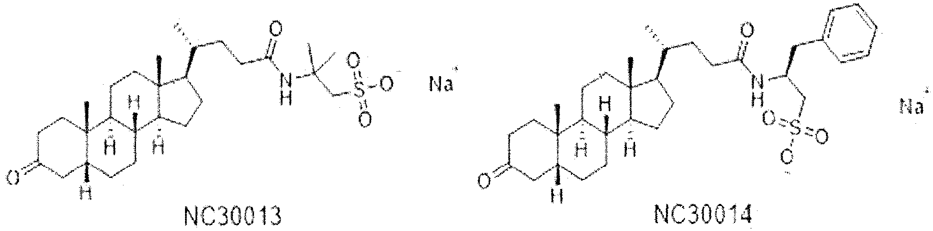 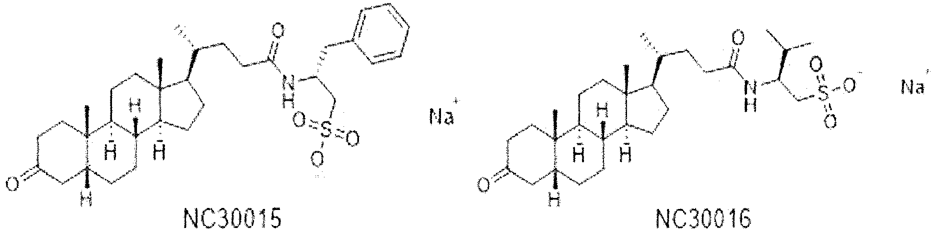 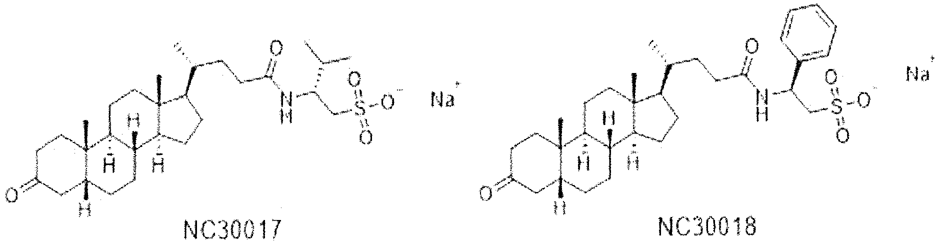 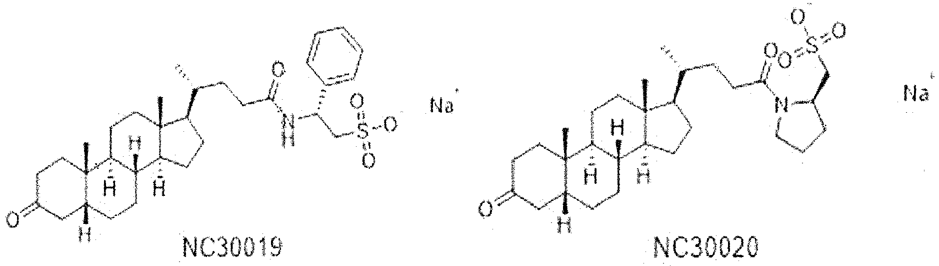 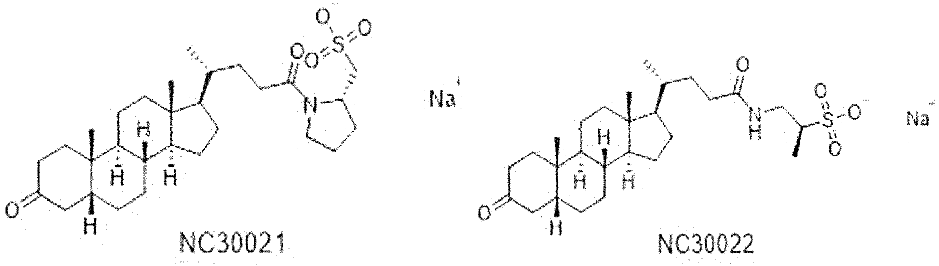 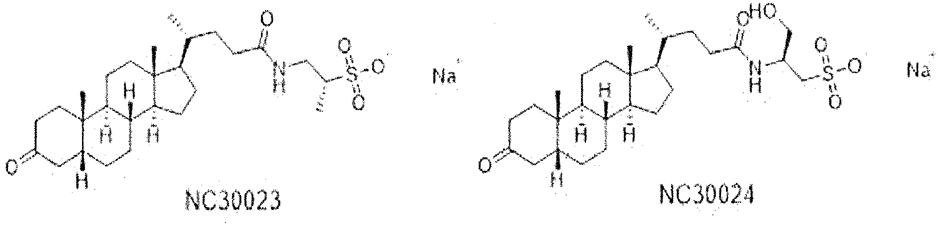 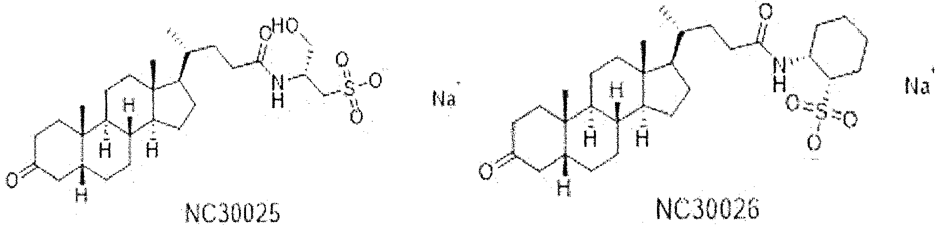 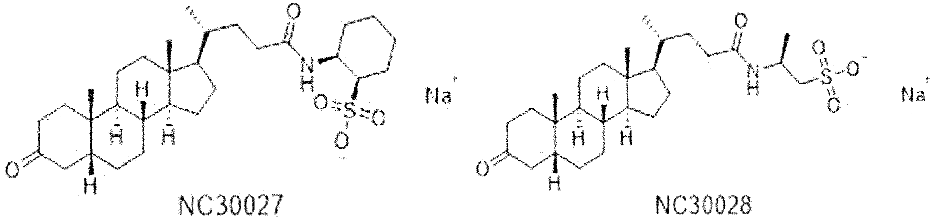 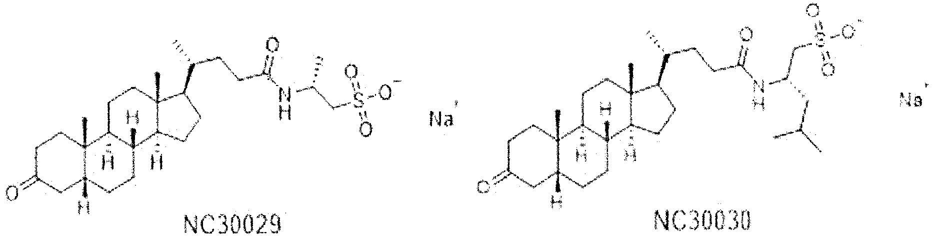 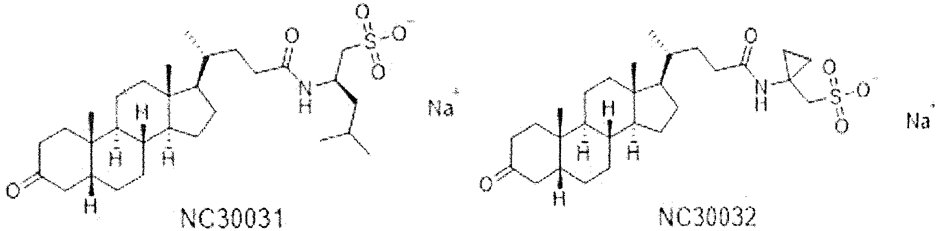 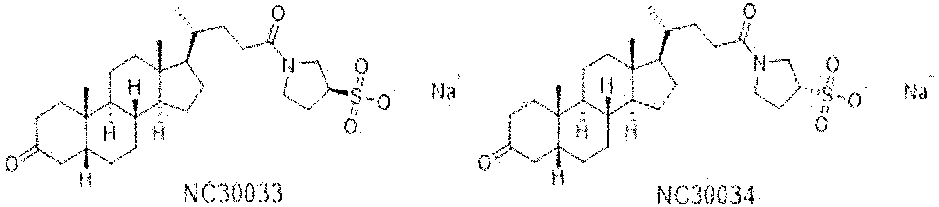 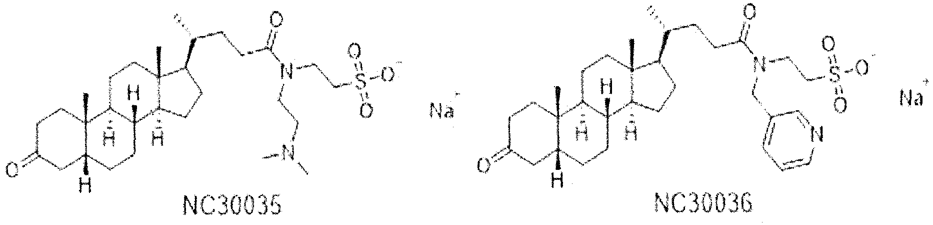 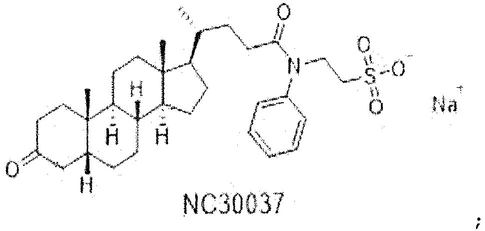 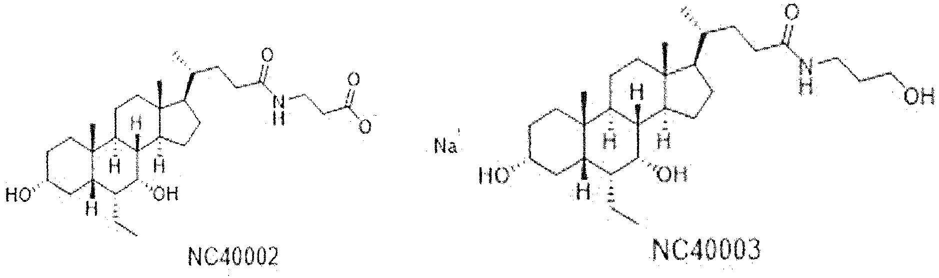 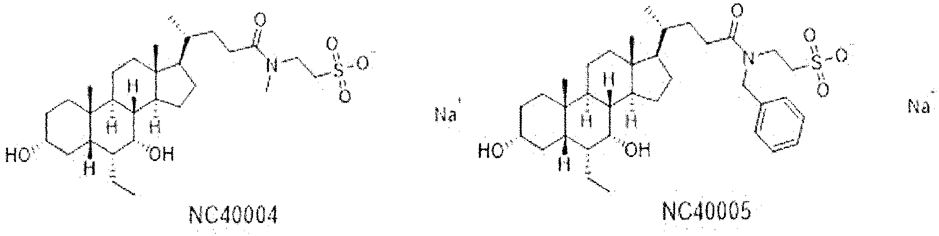 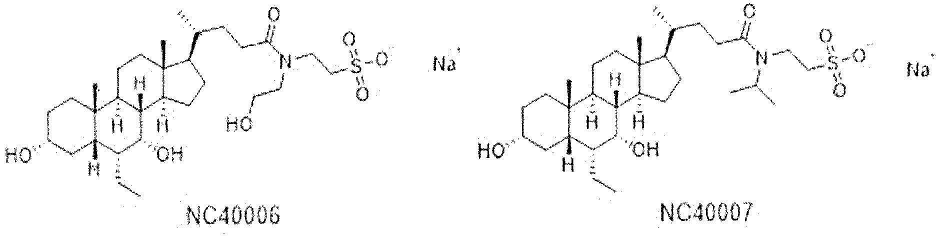 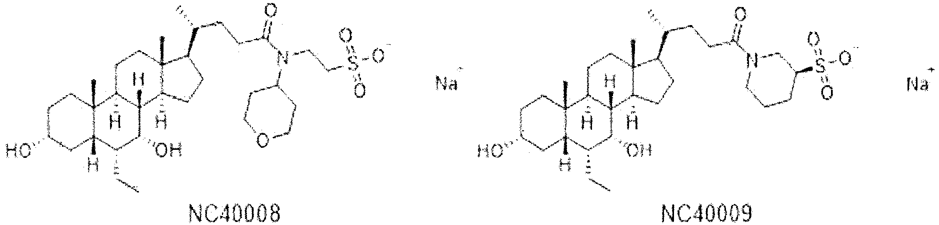 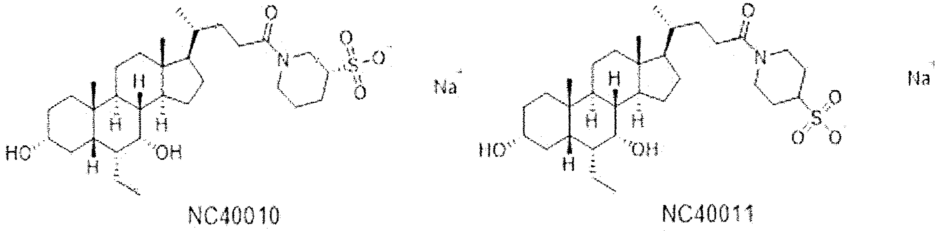 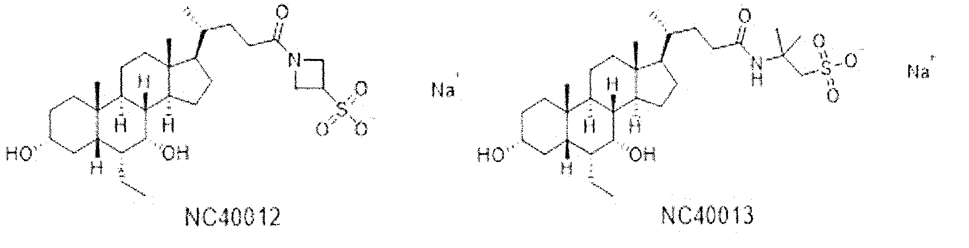 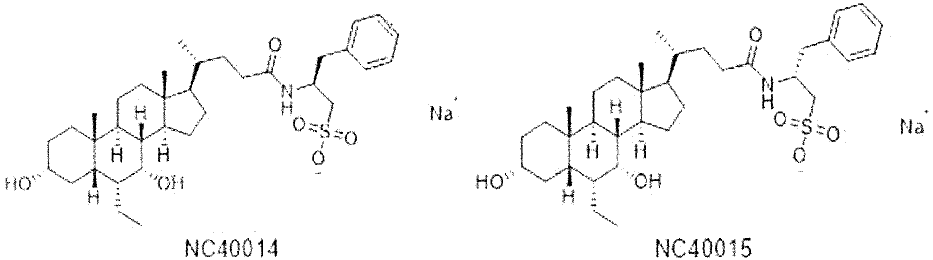 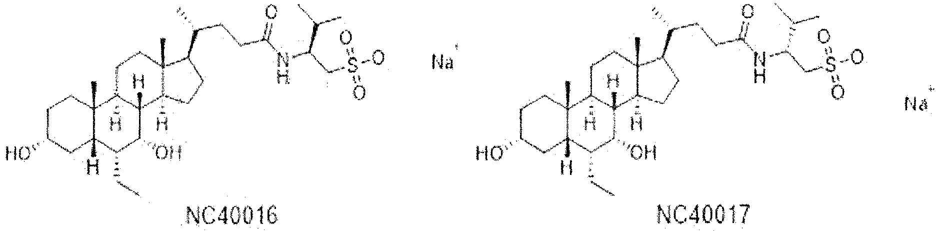 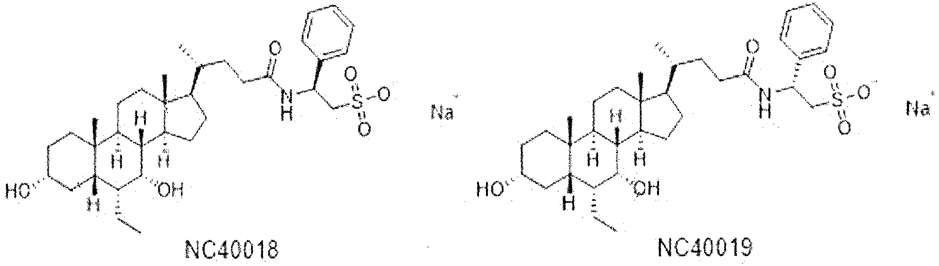 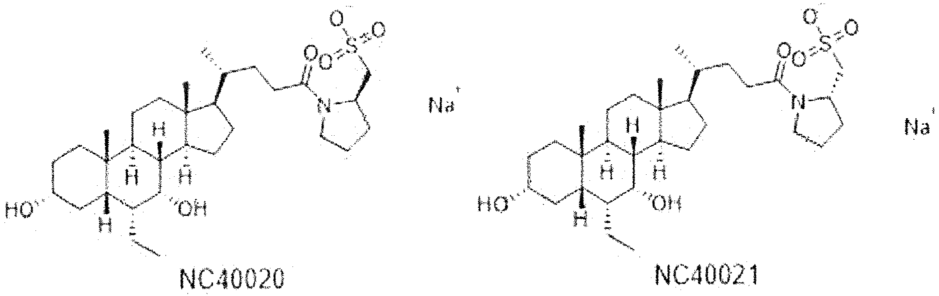 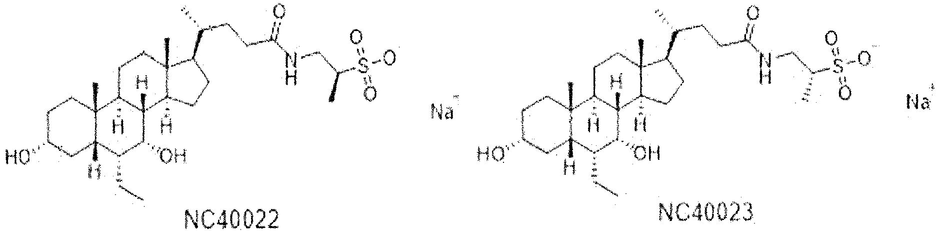 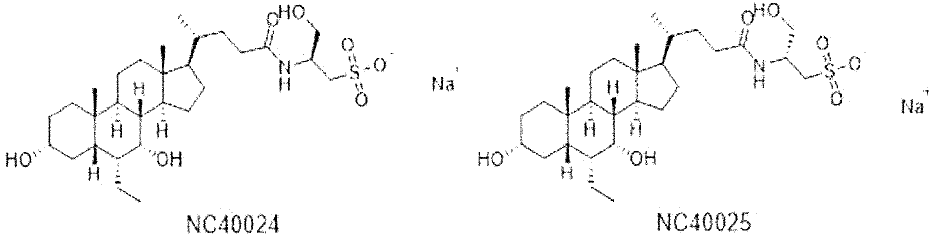 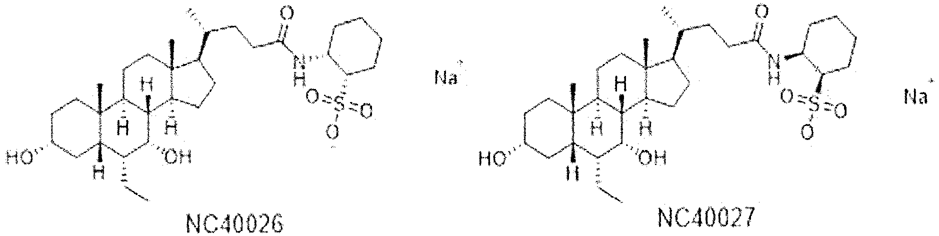 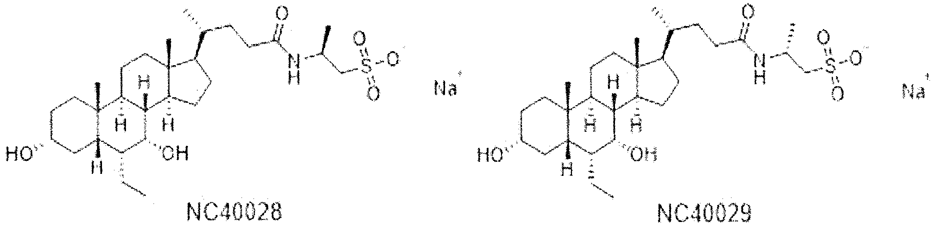 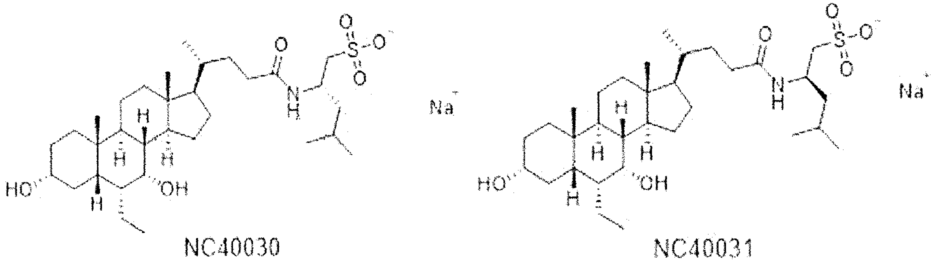 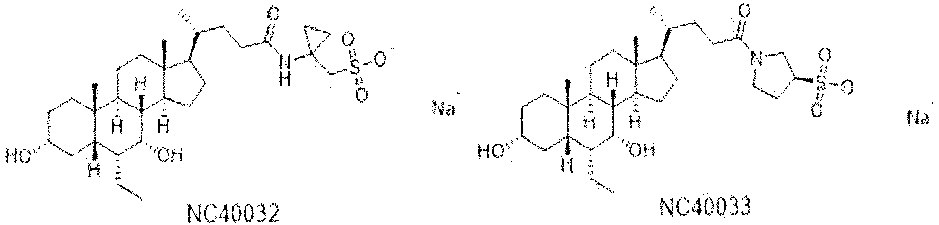 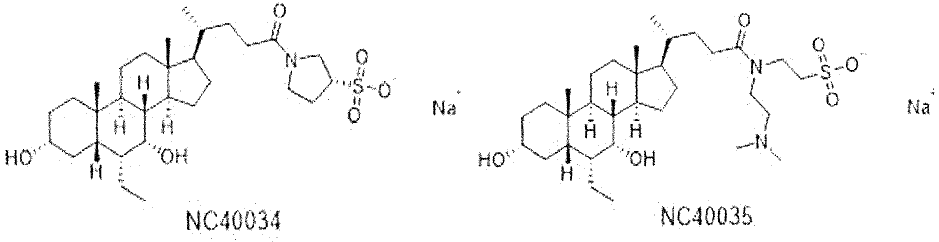 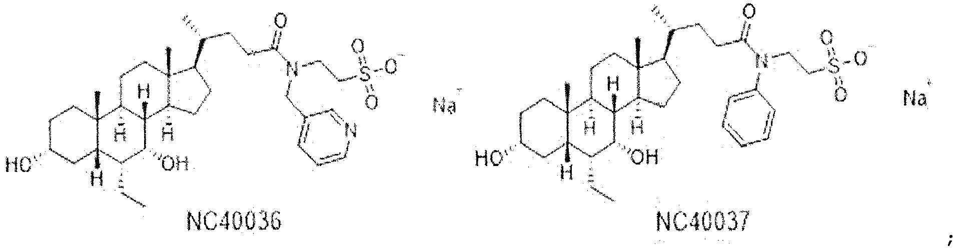 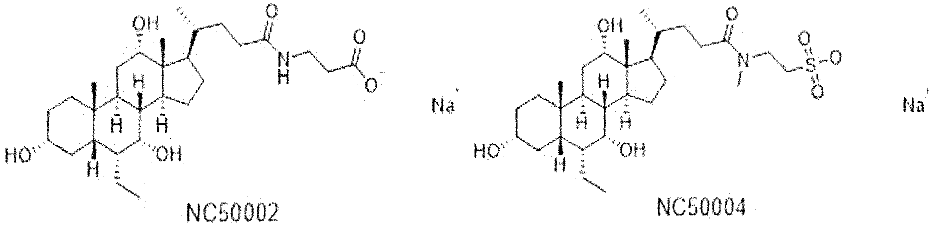 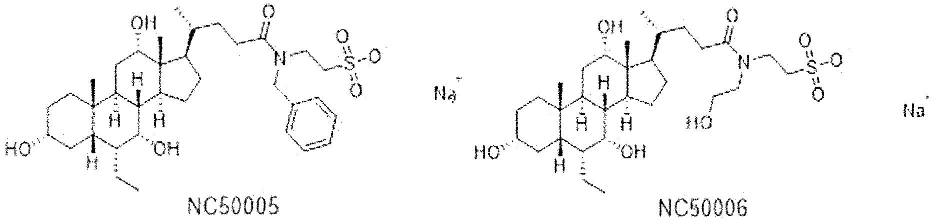 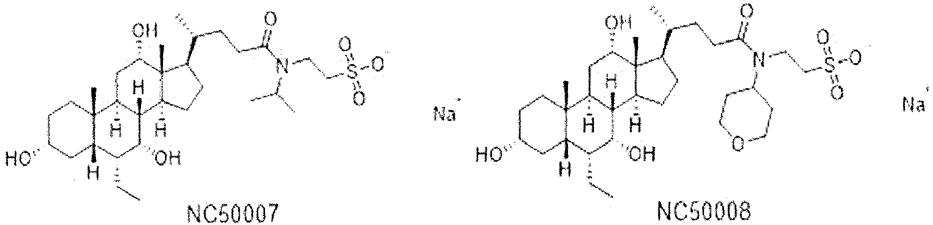 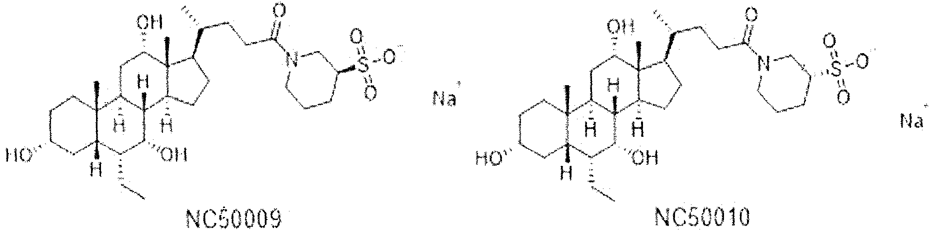 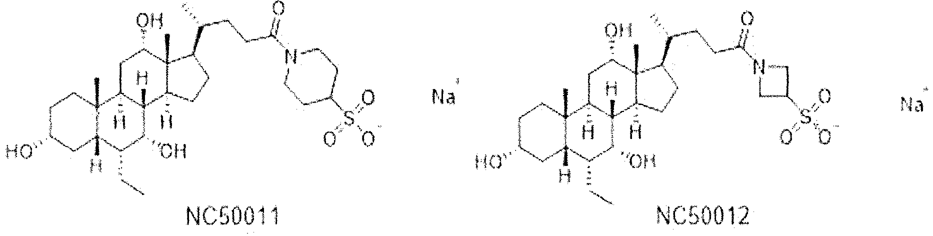 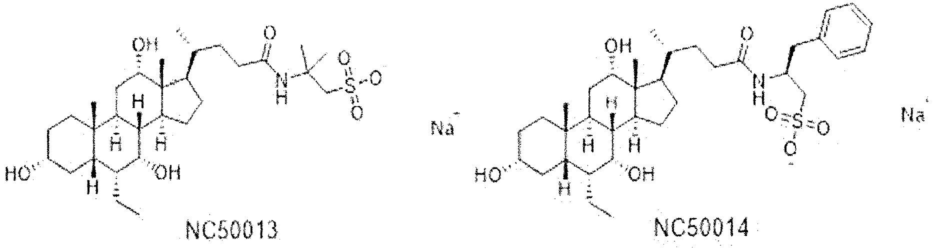 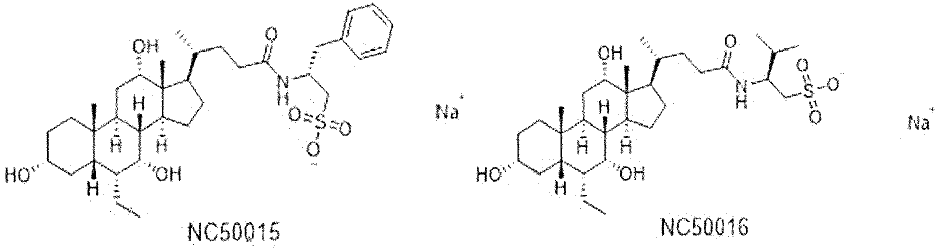 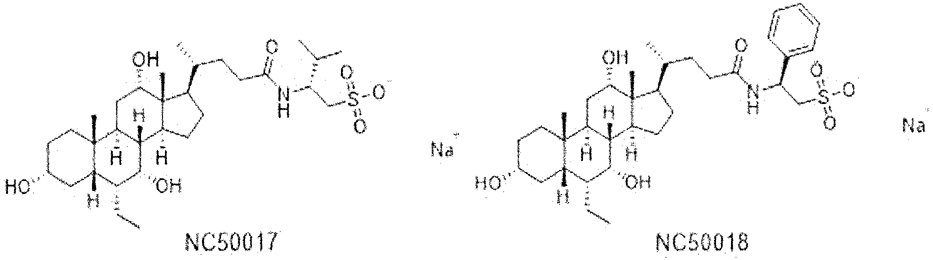 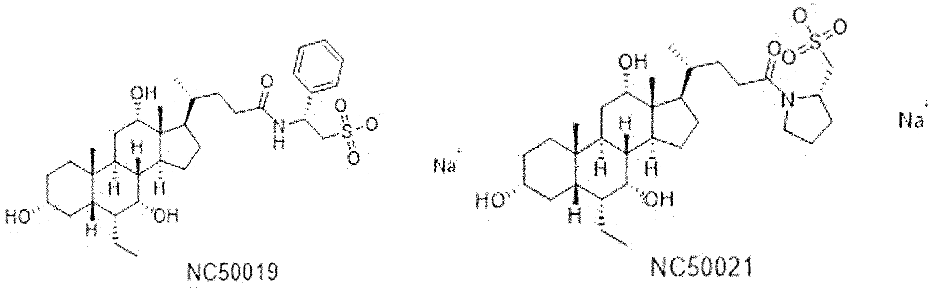 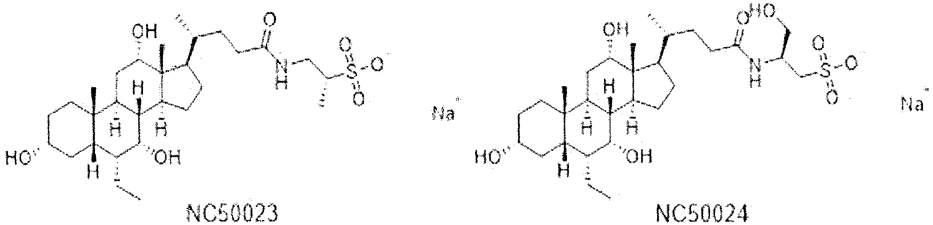 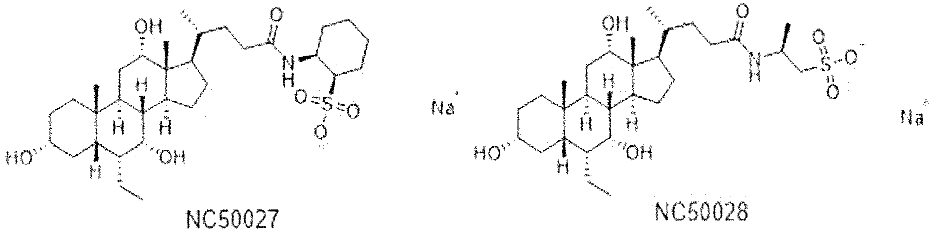 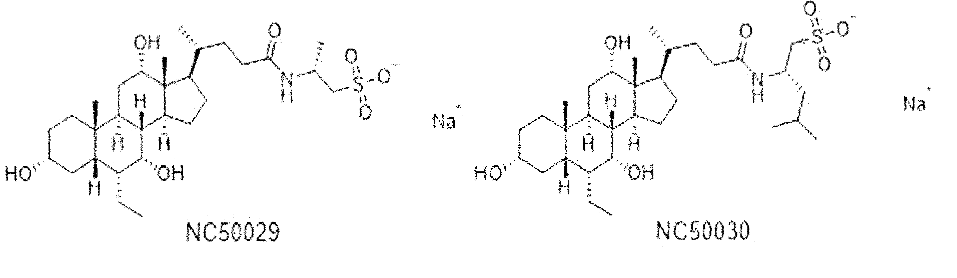 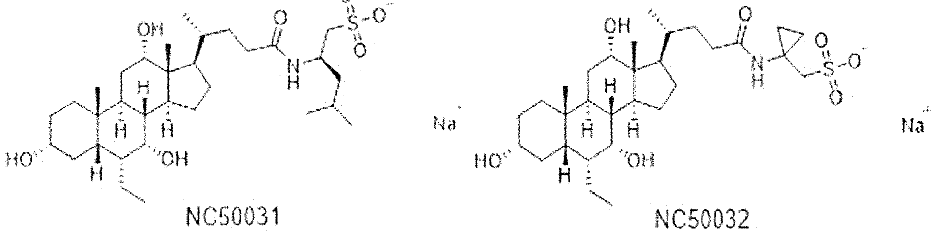 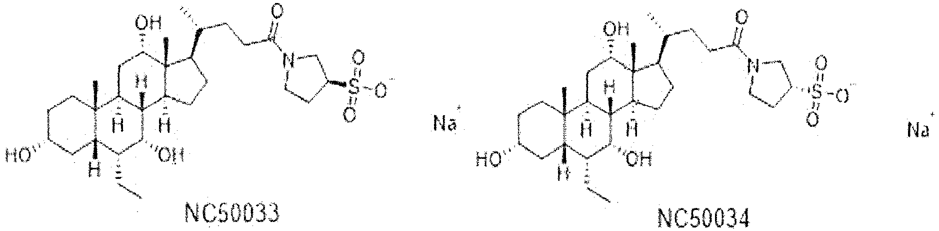 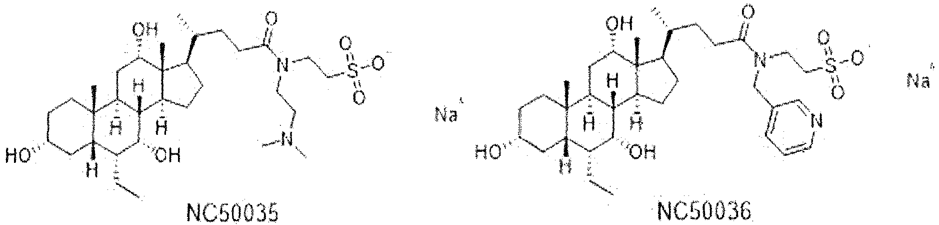 and 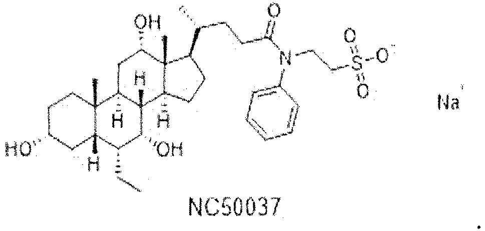 (wherein, Na⁺ can be replaced by other pharmaceutically acceptable salts)

29. A pharmaceutical composition comprising the compound of claim 1 or a pharmaceutically accpetable salt thereof.

30. The pharmaceutical composition as claim 29, wherien the pharmaceutical composition is for the treatment of inflammatory diseases.
